# EUROPEAN PATENT APPLICATION

(11) **EP 1 923 072 A1**
(43) Date of publication of application: **21.05.2008**
(21) Application number: 06782749.3
(22) Date of filing: 09.08.2006
(51) Int. Cl.: A61K 39/395, A61K 31/7088, A61K 45/00, A61K 48/00, A61P 35/00

(54) **PROPHYLACTIC/THERAPEUTIC AGENT FOR CANCER**

(30) Priority: 09.08.2005 JP 2005230797
(71) Applicant: Takeda Pharmaceutical Company Limited, Osaka-shi, Osaka 541-0045 (JP)
(72) Inventor: KONDO, Shinichi, Osaka-shi, Osaka 5328686 (JP); SUNAHARA, Eiji, Osaka-shi, Osaka 5328686 (JP); ARAI, Toshimitsu, Osaka-shi, Osaka 5328686 (JP); UTSUMI, Hiroko, Osaka-shi, Osaka 5328686 (JP)
(74) Representative: Kalhammer, Georg
(86) International application number: PCT/JP2006/316070
(87) International publication number: WO 2007/018316

(57) **Abstract**

Identification of a novel target molecule for preventing/treating cancer and a preventive/remedy agent for cancer which targets the molecule, specifically, an agent for promoting the apoptosis in cancer cells and/or inhibiting the growth of cancer cells and a preventive/remedy agent for cancer, which comprise an antibody against a protein including the same or substantially the same amino acid sequence as the amino acid sequence represented by SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16 or SEQ ID NO: 18; an antisense polynucleotide of the protein (preferably, siRNA against mRNA of the protein) ; and a substance controlling the expression and/or activity of the protein, are provided by the present invention. A method of screening for a substance that promotes apoptosis in cancer cells and/or inhibits the growth of cancer cells and a preventive/remedy substance for cancer, in which the method comprises using a protein (preferably a cell producing the protein), an antibody against the protein, or a polynucleotide encoding the protein, is also provided by the present invention.

SEQUENCE LISTING

<110> Takeda Pharmaceutical Company Limited

<120> Prophylactic/Therapeutic Agent for Cancer

<130> 09961

<150> JP 2005-230797
<151> 2005-08-09

<160> 51

<170> PatentIn version 3.2

<210> 1
<211> 399
<212> DNA
<213> Homo sapiens


<220>
<221> CDS
<222> (1)..(399)

<400> 1
atg aaa ccc cag ttt gtt ggg atc ttg ctc agc tcc ctg cta ggg gct 48
Met Lys Pro Gln Phe Val Gly Ile Leu Leu Ser Ser Leu Leu Gly Ala
1               5                   10                  15

gcc ttg gga aac cga atg cgg tgc tac aac tgt ggt gga agc ccc agc 96
Ala Leu Gly Asn Arg Met Arg Cys Tyr Asn Cys Gly Gly Ser Pro Ser
            20                  25                  30

agt tct tgc aaa gag gcc gtg acc acc tgt ggc gag ggc aga ccc cag 144
Ser Ser Cys Lys Glu Ala Val Thr Thr Cys Gly Glu Gly Arg Pro Gln
        35                  40                  45

cca ggc ctg gaa cag atc aag cta cct gga aac ccc cca gtg acc ttg 192
Pro Gly Leu Glu Gln Ile Lys Leu Pro Gly Asn Pro Pro Val Thr Leu
    50                  55                  60

att cac caa cat cca gcc tgc gtc gca gcc cat cat tgc aat caa gtg 240
Ile His Gln His Pro Ala Cys Val Ala Ala His His Cys Asn Gln Val
65                  70                  75                  80

gag aca gag tcg gtg gga gac gtg act tat cca gcc cac agg gac tgc 288
Glu Thr Glu Ser Val Gly Asp Val Thr Tyr Pro Ala His Arg Asp Cys
                85                  90                  95

tac ctg gga gac ctg tgc aac agc gcc gtg gca agc cat gtg gcc cct 336
Tyr Leu Gly Asp Leu Cys Asn Ser Ala Val Ala Ser His Val Ala Pro
            100                 105                 110

gca ggc att ttg gct gca gca gct acc gcc ctg acc tgt ctc ttg cca 384
Ala Gly Ile Leu Ala Ala Ala Ala Thr Ala Leu Thr Cys Leu Leu Pro
        115                 120                 125

gga ctg tgg agc gga 399
Gly Leu Trp Ser Gly
    130


<210> 2
<211> 133
<212> PRT
<213> Homo sapiens

<400> 2

Met Lys Pro Gln Phe Val Gly Ile Leu Leu Ser Ser Leu Leu Gly Ala
1               5                   10                  15


Ala Leu Gly Asn Arg Met Arg Cys Tyr Asn Cys Gly Gly Ser Pro Ser
            20                  25                  30

Ser Ser Cys Lys Glu Ala Val Thr Thr Cys Gly Glu Gly Arg Pro Gln
        35                  40                  45

Pro Gly Leu Glu Gln Ile Lys Leu Pro Gly Asn Pro Pro Val Thr Leu
    50                  55                  60

Ile His Gln His Pro Ala Cys Val Ala Ala His His Cys Asn Gln Val
65                  70                  75                  80

Glu Thr Glu Ser Val Gly Asp Val Thr Tyr Pro Ala His Arg Asp Cys
                85                  90                  95

Tyr Leu Gly Asp Leu Cys Asn Ser Ala Val Ala Ser His Val Ala Pro
            100                 105                 110

Ala Gly Ile Leu Ala Ala Ala Ala Thr Ala Leu Thr Cys Leu Leu Pro
        115                 120                 125

Gly Leu Trp Ser Gly
    130

<210>  3
<211>  3648
<212>  DNA
<213>  Homo sapiens

<220>
<221>  CDS
<222>  (1)..(3648)

<400>  3
atg ggg cac ctg ccc acg ggg ata cac ggc gcc cgc cgc ctc ctg cct      48
Met Gly His Leu Pro Thr Gly Ile His Gly Ala Arg Arg Leu Leu Pro
1               5                   10                  15

ctg ctc tgg ctc ttt gtg ctg ttc aag aat gct aca gct ttc cat gta      96
Leu Leu Trp Leu Phe Val Leu Phe Lys Asn Ala Thr Ala Phe His Val
            20                  25                  30

act gtc caa gat gat aat aac atc gtt gtc tca tta gaa gct tca gac      144
Thr Val Gln Asp Asp Asn Asn Ile Val Val Ser Leu Glu Ala Ser Asp
        35                  40                  45

gtc atc agt cca gca tct gtg tat gtt gtg aag ata act ggt gaa tcc      192
Val Ile Ser Pro Ala Ser Val Tyr Val Val Lys Ile Thr Gly Glu Ser
    50                  55                  60

aaa aat tat ttc ttc gaa ttt gag gaa ttc aac agc act ttg cct cct      240
Lys Asn Tyr Phe Phe Glu Phe Glu Glu Phe Asn Ser Thr Leu Pro Pro
65                  70                  75                  80

cct gtt att ttc aag gcc agt tat cat ggc ctt tat tat ata atc act      288
Pro Val Ile Phe Lys Ala Ser Tyr His Gly Leu Tyr Tyr Ile Ile Thr
                85                  90                  95

ctg gta gtg gta aat gga aat gtg gtg acc aag cca tcc aga tca atc      336
Leu Val Val Val Asn Gly Asn Val Val Thr Lys Pro Ser Arg Ser Ile
            100                 105                 110

act gtg tta aca aaa cct cta cct gta acc agt gtt tcc ata tat gac      384
Thr Val Leu Thr Lys Pro Leu Pro Val Thr Ser Val Ser Ile Tyr Asp
        115                 120                 125

tat aaa cct tct cct gaa aca gga gtc ctg ttt gaa ata cat tat cca      432
Tyr Lys Pro Ser Pro Glu Thr Gly Val Leu Phe Glu Ile His Tyr Pro
    130                 135                 140

gaa aaa tat aac gtt ttc aca aga gtg aac att agc tac tgg gaa ggt 480
Glu Lys Tyr Asn Val Phe Thr Arg Val Asn Ile Ser Tyr Trp Glu Gly
145                 150                 155                 160

aaa gac ttc cgg aca atg cta tat aaa gat ttc ttt aag gga aaa aca 528
Lys Asp Phe Arg Thr Met Leu Tyr Lys Asp Phe Phe Lys Gly Lys Thr
                165                 170                 175

gta ttt aat cac tgg ctg cca gga atg tgt tat agt aat atc acc ttt 576
Val Phe Asn His Trp Leu Pro Gly Met Cys Tyr Ser Asn Ile Thr Phe
            180                 185                 190

cag ctg gta tct gag gca act ttt aat aaa agt acc ctt gtt gag tac 624
Gln Leu Val Ser Glu Ala Thr Phe Asn Lys Ser Thr Leu Val Glu Tyr
        195                 200                 205

agt ggt gtc agt cac gaa ccc aaa cag cac aga act gcc cct tat cca 672
Ser Gly Val Ser His Glu Pro Lys Gln His Arg Thr Ala Pro Tyr Pro
    210                 215                 220

cct caa aat att tcc gtt cgt atc gta aac ttg aac aaa aac aac tgg 720
Pro Gln Asn Ile Ser Val Arg Ile Val Asn Leu Asn Lys Asn Asn Trp
225                 230                 235                 240

gaa gaa cag agt ggc aat ttc cca gaa gaa tcc ttc atg aga tca caa 768
Glu Glu Gln Ser Gly Asn Phe Pro Glu Glu Ser Phe Met Arg Ser Gln
                245                 250                 255

gat aca ata gga aaa gaa aaa ctc ttc cat ttt aca gaa gaa acc cct 816
Asp Thr Ile Gly Lys Glu Lys Leu Phe His Phe Thr Glu Glu Thr Pro
            260                 265                 270

gaa att ccc tcg ggc aac att tct tcc ggt tgg cct gat ttt aat agc 864
Glu Ile Pro Ser Gly Asn Ile Ser Ser Gly Trp Pro Asp Phe Asn Ser
        275                 280                 285

agt gac tat gaa act acg tct cag cca tat tgg tgg gac agt gca tct 912
Ser Asp Tyr Glu Thr Thr Ser Gln Pro Tyr Trp Trp Asp Ser Ala Ser
    290                 295                 300

gca gct cct gaa agt gaa gat gaa ttt gtc agc gta ctt ccc atg gaa 960
Ala Ala Pro Glu Ser Glu Asp Glu Phe Val Ser Val Leu Pro Met Glu
305                 310                 315                 320

tac gaa aat aac agt aca ctc agt gag aca gag aag tca aca tca ggc 1008
Tyr Glu Asn Asn Ser Thr Leu Ser Glu Thr Glu Lys Ser Thr Ser Gly
                325                 330                 335

tct ttc tcc ttt ttc cct gtg caa atg ata ttg acc tgg tta cca ccc 1056
Ser Phe Ser Phe Phe Pro Val Gln Met Ile Leu Thr Trp Leu Pro Pro
            340                 345                 350

aaa cca ccc act gct ttt gat ggg ttc cat atc cat att gaa cga gaa 1104
Lys Pro Pro Thr Ala Phe Asp Gly Phe His Ile His Ile Glu Arg Glu
        355                 360                 365

gag aac ttt act gaa tat ttg atg gtg gat gaa gaa gca cat gaa ttt 1152
Glu Asn Phe Thr Glu Tyr Leu Met Val Asp Glu Glu Ala His Glu Phe
    370                 375                 380

gtt gca gaa ctg aag gaa cct ggg aaa tat aag tta tct gtg aca acc 1200
Val Ala Glu Leu Lys Glu Pro Gly Lys Tyr Lys Leu Ser Val Thr Thr
385                 390                 395                 400

ttt agt tcc tca gga tct tgt gaa act cga aaa agt cag tca gca aaa 1248
Phe Ser Ser Ser Gly Ser Cys Glu Thr Arg Lys Ser Gln Ser Ala Lys
                405                 410                 415

tca ctc agc ttt tat atc agt cct tca gga gag tgg att gaa gaa ctg 1296
Ser Leu Ser Phe Tyr Ile Ser Pro Ser Gly Glu Trp Ile Glu Glu Leu
            420                 425                 430

acc gag aag ccg cag cac gtg agt gtc cac gtt tta agc tca acc act 1344
Thr Glu Lys Pro Gln His Val Ser Val His Val Leu Ser Ser Thr Thr
        435                 440                 445

gcc ttg atg tcc tgg aca tct tcc caa gag aac tac aac agc acc att     1392
Ala Leu Met Ser Trp Thr Ser Ser Gln Glu Asn Tyr Asn Ser Thr Ile
    450                 455                 460

gtg tct gtg gtg tcg ctg acc tgc cag aaa caa aag gag agc cag agg     1440
Val Ser Val Val Ser Leu Thr Cys Gln Lys Gln Lys Glu Ser Gln Arg
465                 470                 475                 480

ctt gaa aag cag tac tgc act cag gtg aac tca agc aaa cct att att     1488
Leu Glu Lys Gln Tyr Cys Thr Gln Val Asn Ser Ser Lys Pro Ile Ile
                485                 490                 495

gaa aat ctg gtt cct ggt gcc cag tac cag gtt gta ata tac cta agg     1536
Glu Asn Leu Val Pro Gly Ala Gln Tyr Gln Val Val Ile Tyr Leu Arg
            500                 505                 510

aaa ggc cct ttg att gga cca cct tca gat cct gtg aca ttt gct att     1584
Lys Gly Pro Leu Ile Gly Pro Pro Ser Asp Pro Val Thr Phe Ala Ile
        515                 520                 525

gtt ccc aca gga ata aag gat tta atg ctc tat cct ttg ggt cct acg     1632
Val Pro Thr Gly Ile Lys Asp Leu Met Leu Tyr Pro Leu Gly Pro Thr
    530                 535                 540

gcc gtg gtt ctg agc tgg acc aga cct tat tta ggc gtg ttc aga aaa     1680
Ala Val Val Leu Ser Trp Thr Arg Pro Tyr Leu Gly Val Phe Arg Lys
545                 550                 555                 560

tac gtg gtt gaa atg ttt tat ttc aac cct gct aca atg aca tca gag     1728
Tyr Val Val Glu Met Phe Tyr Phe Asn Pro Ala Thr Met Thr Ser Glu
                565                 570                 575

tgg acc acc tac tat gaa ata gca gca act gtt tcc tta act gca tcc     1776
Trp Thr Thr Tyr Tyr Glu Ile Ala Ala Thr Val Ser Leu Thr Ala Ser
            580                 585                 590

gtg aga ata gct aat ctg ctg cca gca tgg tac tac aac ttc cgg gtt     1824
Val Arg Ile Ala Asn Leu Leu Pro Ala Trp Tyr Tyr Asn Phe Arg Val
        595                 600                 605

acc atg gtg acg tgg gga gat cca gaa ttg agc tgc tgt gac agc tct     1872
Thr Met Val Thr Trp Gly Asp Pro Glu Leu Ser Cys Cys Asp Ser Ser
    610                 615                 620

acc atc agc ttc ata aca gcc cca gtg gct ccg gaa atc act tct gtg     1920
Thr Ile Ser Phe Ile Thr Ala Pro Val Ala Pro Glu Ile Thr Ser Val
625                 630                 635                 640

gaa tat ttc aac agt ctg tta tat atc agt tgg aca tat ggg gat gat     1968
Glu Tyr Phe Asn Ser Leu Leu Tyr Ile Ser Trp Thr Tyr Gly Asp Asp
                645                 650                 655

aca acg gac ttg tcc cat tct aga atg ctt cac tgg atg gtg gtt gca     2016
Thr Thr Asp Leu Ser His Ser Arg Met Leu His Trp Met Val Val Ala
            660                 665                 670

gaa gga aaa aag aaa att aaa aag agt gta aca cgc aat gtc atg act     2064
Glu Gly Lys Lys Lys Ile Lys Lys Ser Val Thr Arg Asn Val Met Thr
        675                 680                 685

gca att ctc agc ttg cct cca ggc gac atc tat aac ctc tca gta act     2112
Ala Ile Leu Ser Leu Pro Pro Gly Asp Ile Tyr Asn Leu Ser Val Thr
    690                 695                 700

gct tgt act gaa aga gga agt aat acc tcc atg ctc cgc ctt gtc aag     2160
Ala Cys Thr Glu Arg Gly Ser Asn Thr Ser Met Leu Arg Leu Val Lys
705                 710                 715                 720

cta gaa cca gct cca ccc aaa tca ctc ttc gca gtg aac aaa acc cag     2208
Leu Glu Pro Ala Pro Pro Lys Ser Leu Phe Ala Val Asn Lys Thr Gln
                725                 730                 735

act tca gtg act ttg ctg tgg gtg gaa gag gga gta gct gat ttc ttt     2256
Thr Ser Val Thr Leu Leu Trp Val Glu Glu Gly Val Ala Asp Phe Phe
            740                 745                 750

gaa gtt ttc tgt caa caa gtt ggc tcc agt cag aaa acc aaa ctt cag     2304
Glu Val Phe Cys Gln Gln Val Gly Ser Ser Gln Lys Thr Lys Leu Gln
        755                 760                 765

gaa cca gtt gct gtt tct tcc cat gtc gtg acc atc tcc agc ctt ctt     2352
Glu Pro Val Ala Val Ser Ser His Val Val Thr Ile Ser Ser Leu Leu
    770                 775                 780

cct gcc act gcc tac aat tgt agt gtc acc agc ttt agc cat gac agc     2400
Pro Ala Thr Ala Tyr Asn Cys Ser Val Thr Ser Phe Ser His Asp Ser
785                 790                 795                 800

ccc agt gtc cct acg ttc ata gcc gtc tca aca atg gtt aca gag atg     2448
Pro Ser Val Pro Thr Phe Ile Ala Val Ser Thr Met Val Thr Glu Met
                805                 810                 815

aat ccc aat gtg gta gtg atc tcc gtg ctg gcc atc ctt agc aca ctt     2496
Asn Pro Asn Val Val Val Ile Ser Val Leu Ala Ile Leu Ser Thr Leu
            820                 825                 830

tta att gga ctg ttg ctt gtt acc ctc att att ctt agg aaa aag cat     2544
Leu Ile Gly Leu Leu Leu Val Thr Leu Ile Ile Leu Arg Lys Lys His
        835                 840                 845

ctg cag atg gct agg gag tgt gga gct ggt aca ttt gtc aat ttt gca     2592
Leu Gln Met Ala Arg Glu Cys Gly Ala Gly Thr Phe Val Asn Phe Ala
    850                 855                 860

tcc tta gag agg gat gga aag ctt cca tac aac tgg cgt agg agt ata     2640
Ser Leu Glu Arg Asp Gly Lys Leu Pro Tyr Asn Trp Arg Arg Ser Ile
865                 870                 875                 880

ttt gct ttc tta acc ctg cta ccc tca tgt ctt tgg act gat tat ctt     2688
Phe Ala Phe Leu Thr Leu Leu Pro Ser Cys Leu Trp Thr Asp Tyr Leu
                885                 890                 895

ttg gca ttt tat att aat cct tgg agt aaa aat ggt tta aag aag agg     2736
Leu Ala Phe Tyr Ile Asn Pro Trp Ser Lys Asn Gly Leu Lys Lys Arg
            900                 905                 910

aaa ctg aca aac ccg gtt caa ctg gat gac ttt gat gcc tat att aag     2784
Lys Leu Thr Asn Pro Val Gln Leu Asp Asp Phe Asp Ala Tyr Ile Lys
        915                 920                 925

gat atg gcc aaa gac tct gac tat aaa ttt tct ctt cag ttt gag gag     2832
Asp Met Ala Lys Asp Ser Asp Tyr Lys Phe Ser Leu Gln Phe Glu Glu
    930                 935                 940

ttg aaa ttg att gga ctg gat atc cca cac ttt gct gca gat ctt cca     2880
Leu Lys Leu Ile Gly Leu Asp Ile Pro His Phe Ala Ala Asp Leu Pro
945                 950                 955                 960

ctg aat cga tgt aaa aac cgt tac aca aac atc cta cca tat gac ttc     2928
Leu Asn Arg Cys Lys Asn Arg Tyr Thr Asn Ile Leu Pro Tyr Asp Phe
                965                 970                 975

agc cgt gtg aga tta gtc tcc atg aat gaa gag gaa ggt gca gac tac     2976
Ser Arg Val Arg Leu Val Ser Met Asn Glu Glu Glu Gly Ala Asp Tyr
            980                 985                 990

atc aat gcc aac tat att cct gga  tac aac tca ccc cag  gag tat att     3024
Ile Asn Ala Asn Tyr Ile Pro Gly  Tyr Asn Ser Pro Gln  Glu Tyr Ile
        995                 1000                 1005

gcc acc  cag ggg cca ctg cct  gaa acc aga aat gac  ttc tgg aag     3069
Ala Thr  Gln Gly Pro Leu Pro  Glu Thr Arg Asn Asp  Phe Trp Lys
    1010                 1015                 1020

atg gtc  ctg caa caa aag tct  cag att att gtc atg  ctc act cag     3114
Met Val  Leu Gln Gln Lys Ser  Gln Ile Ile Val Met  Leu Thr Gln
    1025                 1030                 1035

tgt aat  gag aaa agg agg gtg  aaa tgt gac cat tac  tgg cca ttc     3159
Cys Asn  Glu Lys Arg Arg Val  Lys Cys Asp His Tyr  Trp Pro Phe
    1040                 1045                 1050

acg gaa gaa cct ata gcc tat gga gac atc act gtg gag atg att 3204
Thr Glu Glu Pro Ile Ala Tyr Gly Asp Ile Thr Val Glu Met Ile
    1055                1060                1065

tca gag gaa gag cag gac gac tgg gcc tgt aga cac ttc cgg atc 3249
Ser Glu Glu Glu Gln Asp Asp Trp Ala Cys Arg His Phe Arg Ile
    1070                1075                1080

aac tat gct gac gag atg cag gat gtg atg cat ttt aac tac act 3294
Asn Tyr Ala Asp Glu Met Gln Asp Val Met His Phe Asn Tyr Thr
    1085                1090                1095

gca tgg cct gat cat ggt gtg ccc aca gca aat gct gca gaa agt 3339
Ala Trp Pro Asp His Gly Val Pro Thr Ala Asn Ala Ala Glu Ser
    1100                1105                1110

atc ctg cag ttt gta cac atg gtc cga cag caa gct acc aag agc 3384
Ile Leu Gln Phe Val His Met Val Arg Gln Gln Ala Thr Lys Ser
    1115                1120                1125

aaa ggt ccc atg atc att cac tgc agt gct ggc gtg gga cgg aca 3429
Lys Gly Pro Met Ile Ile His Cys Ser Ala Gly Val Gly Arg Thr
    1130                1135                1140

gga aca ttc att gcc ctg gac agg ctc ttg cag cac att cgg gat 3474
Gly Thr Phe Ile Ala Leu Asp Arg Leu Leu Gln His Ile Arg Asp
    1145                1150                1155

cat gag ttt gtt gac atc tta ggg ctg gtg tca gaa atg agg tca 3519
His Glu Phe Val Asp Ile Leu Gly Leu Val Ser Glu Met Arg Ser
    1160                1165                1170

tac cgg atg tct atg gta cag aca gag gag cag tac att ttt atc 3564
Tyr Arg Met Ser Met Val Gln Thr Glu Glu Gln Tyr Ile Phe Ile
    1175                1180                1185

cat cag tgt gtg caa ctg atg tgg atg aag aag aag cag cag ttc 3609
His Gln Cys Val Gln Leu Met Trp Met Lys Lys Lys Gln Gln Phe
    1190                1195                1200

tgc atc agt gat gtc ata tac gag aat gtt agc aag tcc 3648
Cys Ile Ser Asp Val Ile Tyr Glu Asn Val Ser Lys Ser
    1205                1210                1215

<210> 4
<211> 1216
<212> PRT
<213> Homo sapiens

<400> 4

Met Gly His Leu Pro Thr Gly Ile His Gly Ala Arg Arg Leu Leu Pro
1               5                   10                  15

Leu Leu Trp Leu Phe Val Leu Phe Lys Asn Ala Thr Ala Phe His Val
            20                  25                  30

Thr Val Gln Asp Asp Asn Asn Ile Val Val Ser Leu Glu Ala Ser Asp
        35                  40                  45

Val Ile Ser Pro Ala Ser Val Tyr Val Val Lys Ile Thr Gly Glu Ser
    50                  55                  60

Lys Asn Tyr Phe Phe Glu Phe Glu Glu Phe Asn Ser Thr Leu Pro Pro
65                  70                  75                  80

Pro Val Ile Phe Lys Ala Ser Tyr His Gly Leu Tyr Tyr Ile Ile Thr
                85                  90                  95

Leu Val Val Val Asn Gly Asn Val Val Thr Lys Pro Ser Arg Ser Ile
            100                 105                 110

Thr Val Leu Thr Lys Pro Leu Pro Val Thr Ser Val Ser Ile Tyr Asp
        115                 120                 125

Tyr Lys Pro Ser Pro Glu Thr Gly Val Leu Phe Glu Ile His Tyr Pro
    130                 135                 140

Glu Lys Tyr Asn Val Phe Thr Arg Val Asn Ile Ser Tyr Trp Glu Gly
145                 150                 155                 160

Lys Asp Phe Arg Thr Met Leu Tyr Lys Asp Phe Phe Lys Gly Lys Thr
                165                 170                 175

Val Phe Asn His Trp Leu Pro Gly Met Cys Tyr Ser Asn Ile Thr Phe
            180                 185                 190

Gln Leu Val Ser Glu Ala Thr Phe Asn Lys Ser Thr Leu Val Glu Tyr
        195                 200                 205

Ser Gly Val Ser His Glu Pro Lys Gln His Arg Thr Ala Pro Tyr Pro
    210                 215                 220

Pro Gln Asn Ile Ser Val Arg Ile Val Asn Leu Asn Lys Asn Asn Trp
225                 230                 235                 240

Glu Glu Gln Ser Gly Asn Phe Pro Glu Glu Ser Phe Met Arg Ser Gln
                245                 250                 255

Asp Thr Ile Gly Lys Glu Lys Leu Phe His Phe Thr Glu Glu Thr Pro
            260                 265                 270

Glu Ile Pro Ser Gly Asn Ile Ser Ser Gly Trp Pro Asp Phe Asn Ser
        275                 280                 285

Ser Asp Tyr Glu Thr Thr Ser Gln Pro Tyr Trp Trp Asp Ser Ala Ser
    290                 295                 300

Ala Ala Pro Glu Ser Glu Asp Glu Phe Val Ser Val Leu Pro Met Glu
305                 310                 315                 320

Tyr Glu Asn Asn Ser Thr Leu Ser Glu Thr Glu Lys Ser Thr Ser Gly
                325                 330                 335

Ser Phe Ser Phe Phe Pro Val Gln Met Ile Leu Thr Trp Leu Pro Pro
            340                 345                 350

Lys Pro Pro Thr Ala Phe Asp Gly Phe His Ile His Ile Glu Arg Glu
        355                 360                 365

Glu Asn Phe Thr Glu Tyr Leu Met Val Asp Glu Glu Ala His Glu Phe
    370                 375                 380

Val Ala Glu Leu Lys Glu Pro Gly Lys Tyr Lys Leu Ser Val Thr Thr
385                 390                 395                 400

Phe Ser Ser Ser Gly Ser Cys Glu Thr Arg Lys Ser Gln Ser Ala Lys
                405                 410                 415

Ser Leu Ser Phe Tyr Ile Ser Pro Ser Gly Glu Trp Ile Glu Glu Leu
            420                 425                 430

Thr Glu Lys Pro Gln His Val Ser Val His Val Leu Ser Ser Thr Thr
        435                 440                 445

Ala Leu Met Ser Trp Thr Ser Ser Gln Glu Asn Tyr Asn Ser Thr Ile
    450                 455                 460

Val Ser Val Val Ser Leu Thr Cys Gln Lys Gln Lys Glu Ser Gln Arg
465                 470                 475                 480

Leu Glu Lys Gln Tyr Cys Thr Gln Val Asn Ser Ser Lys Pro Ile Ile
                485                 490                 495

Glu Asn Leu Val Pro Gly Ala Gln Tyr Gln Val Val Ile Tyr Leu Arg
            500                 505                 510

Lys Gly Pro Leu Ile Gly Pro Pro Ser Asp Pro Val Thr Phe Ala Ile
        515                 520                 525

Val Pro Thr Gly Ile Lys Asp Leu Met Leu Tyr Pro Leu Gly Pro Thr
    530                 535                 540

Ala Val Val Leu Ser Trp Thr Arg Pro Tyr Leu Gly Val Phe Arg Lys
545                 550                 555                 560

Tyr Val Val Glu Met Phe Tyr Phe Asn Pro Ala Thr Met Thr Ser Glu
                565                 570                 575

Trp Thr Thr Tyr Tyr Glu Ile Ala Ala Thr Val Ser Leu Thr Ala Ser
            580                 585                 590

Val Arg Ile Ala Asn Leu Leu Pro Ala Trp Tyr Tyr Asn Phe Arg Val
        595                 600                 605

Thr Met Val Thr Trp Gly Asp Pro Glu Leu Ser Cys Cys Asp Ser Ser
    610                 615                 620

Thr Ile Ser Phe Ile Thr Ala Pro Val Ala Pro Glu Ile Thr Ser Val
625                 630                 635                 640

Glu Tyr Phe Asn Ser Leu Leu Tyr Ile Ser Trp Thr Tyr Gly Asp Asp
                645                 650                 655

Thr Thr Asp Leu Ser His Ser Arg Met Leu His Trp Met Val Val Ala
            660                 665                 670

Glu Gly Lys Lys Lys Ile Lys Lys Ser Val Thr Arg Asn Val Met Thr
        675                 680                 685

Ala Ile Leu Ser Leu Pro Pro Gly Asp Ile Tyr Asn Leu Ser Val Thr
    690                 695                 700

Ala Cys Thr Glu Arg Gly Ser Asn Thr Ser Met Leu Arg Leu Val Lys
705                 710                 715                 720

Leu Glu Pro Ala Pro Pro Lys Ser Leu Phe Ala Val Asn Lys Thr Gln
                725                 730                 735

Thr Ser Val Thr Leu Leu Trp Val Glu Glu Gly Val Ala Asp Phe Phe
            740                 745                 750

Glu Val Phe Cys Gln Gln Val Gly Ser Ser Gln Lys Thr Lys Leu Gln
        755                 760                 765

Glu Pro Val Ala Val Ser Ser His Val Val Thr Ile Ser Ser Leu Leu
    770                 775                 780

Pro Ala Thr Ala Tyr Asn Cys Ser Val Thr Ser Phe Ser His Asp Ser
785                 790                 795                 800

Pro Ser Val Pro Thr Phe Ile Ala Val Ser Thr Met Val Thr Glu Met
                805                 810                 815

Asn Pro Asn Val Val Val Ile Ser Val Leu Ala Ile Leu Ser Thr Leu
            820                 825                 830

Leu Ile Gly Leu Leu Leu Val Thr Leu Ile Ile Leu Arg Lys Lys His
        835                 840                 845

Leu Gln Met Ala Arg Glu Cys Gly Ala Gly Thr Phe Val Asn Phe Ala
    850                 855                 860

Ser Leu Glu Arg Asp Gly Lys Leu Pro Tyr Asn Trp Arg Arg Ser Ile
865                 870                 875                 880

Phe Ala Phe Leu Thr Leu Leu Pro Ser Cys Leu Trp Thr Asp Tyr Leu
                885                 890                 895

Leu Ala Phe Tyr Ile Asn Pro Trp Ser Lys Asn Gly Leu Lys Lys Arg
            900                 905                 910

Lys Leu Thr Asn Pro Val Gln Leu Asp Asp Phe Asp Ala Tyr Ile Lys
        915                 920                 925

Asp Met Ala Lys Asp Ser Asp Tyr Lys Phe Ser Leu Gln Phe Glu Glu
    930                 935                 940

Leu Lys Leu Ile Gly Leu Asp Ile Pro His Phe Ala Ala Asp Leu Pro
945                 950                 955                 960

Leu Asn Arg Cys Lys Asn Arg Tyr Thr Asn Ile Leu Pro Tyr Asp Phe
                965                 970                 975

Ser Arg Val Arg Leu Val Ser Met Asn Glu Glu Glu Gly Ala Asp Tyr
            980                 985                 990

Ile Asn Ala Asn Tyr Ile Pro Gly Tyr Asn Ser Pro Gln Glu Tyr Ile
        995                 1000                1005

Ala Thr Gln Gly Pro Leu Pro Glu Thr Arg Asn Asp Phe Trp Lys
    1010                1015                1020

Met Val Leu Gln Gln Lys Ser Gln Ile Ile Val Met Leu Thr Gln
    1025                1030                1035

Cys Asn Glu Lys Arg Arg Val Lys Cys Asp His Tyr Trp Pro Phe
    1040                1045                1050

Thr Glu Glu Pro Ile Ala Tyr Gly Asp Ile Thr Val Glu Met Ile
    1055                1060                1065

Ser Glu Glu Glu Gln Asp Asp Trp Ala Cys Arg His Phe Arg Ile
    1070                1075                1080

Asn Tyr Ala Asp Glu Met Gln Asp Val Met His Phe Asn Tyr Thr
    1085                1090                1095

Ala Trp Pro Asp His Gly Val Pro Thr Ala Asn Ala Ala Glu Ser
    1100                1105                1110

Ile Leu Gln Phe Val His Met Val Arg Gln Gln Ala Thr Lys Ser
    1115                1120                1125

Lys Gly Pro Met Ile Ile His Cys Ser Ala Gly Val Gly Arg Thr
    1130                1135                1140

Gly Thr Phe Ile Ala Leu Asp Arg Leu Leu Gln His Ile Arg Asp
    1145                1150                1155

His Glu Phe Val Asp Ile Leu Gly Leu Val Ser Glu Met Arg Ser
    1160                1165                1170

Tyr Arg Met Ser Met Val Gln Thr Glu Glu Gln Tyr Ile Phe Ile
    1175                1180                1185

His Gln Cys Val Gln Leu Met Trp Met Lys Lys Lys Gln Gln Phe
    1190                1195                1200

Cys Ile Ser Asp Val Ile Tyr Glu Asn Val Ser Lys Ser
    1205                1210                1215

<210> 5
<211> 996
<212> DNA
<213> Homo sapiens

<220>
<221> CDS
<222> (1)..(996)

<400> 5
atg ccc ggc aag cac cag cat ttc cag gaa cct gag gtc ggc tgc tgc 48
Met Pro Gly Lys His Gln His Phe Gln Glu Pro Glu Val Gly Cys Cys
1               5                   10                  15

ggg aaa tac ttc ctg ttt ggc ttc aac att gtc ttc tgg gtg ctg gga 96
Gly Lys Tyr Phe Leu Phe Gly Phe Asn Ile Val Phe Trp Val Leu Gly
            20                  25                  30

gcc ctg ttc ctg gct atc ggc ctc tgg gcc tgg ggt gag aag ggc gtt 144
Ala Leu Phe Leu Ala Ile Gly Leu Trp Ala Trp Gly Glu Lys Gly Val
        35                  40                  45

ctc tcg aac atc tca gcg ctg aca gat ctg gga ggc ctt gac ccc gtg 192
Leu Ser Asn Ile Ser Ala Leu Thr Asp Leu Gly Gly Leu Asp Pro Val
    50                  55                  60

tgg ctg ttt gtg gta gtt gga ggc gtc atg tcg gtg ctg ggc ttt gct 240
Trp Leu Phe Val Val Val Gly Gly Val Met Ser Val Leu Gly Phe Ala
65                  70                  75                  80

ggc tgc att ggg gcc ctc cgg gag aac acc ttc ctg ctc aag ttt ttc 288
Gly Cys Ile Gly Ala Leu Arg Glu Asn Thr Phe Leu Leu Lys Phe Phe
                85                  90                  95

tcc gtg ttc ctc ggt ctc atc ttc ttc ctg gag ctg gca aca ggg atc 336
Ser Val Phe Leu Gly Leu Ile Phe Phe Leu Glu Leu Ala Thr Gly Ile
            100                 105                 110

ctg gcc ttt gtc ttc aag gac tgg att cga gac cag ctc aac ctc ttc 384
Leu Ala Phe Val Phe Lys Asp Trp Ile Arg Asp Gln Leu Asn Leu Phe
        115                 120                 125

atc aac aac aac gtc aag gcc tac cgg gac gac att gac ctc cag aac 432
Ile Asn Asn Asn Val Lys Ala Tyr Arg Asp Asp Ile Asp Leu Gln Asn
    130                 135                 140

ctc att gac ttt gct cag gaa tac tgg tct tgc tgc gga gcc cga ggc 480
Leu Ile Asp Phe Ala Gln Glu Tyr Trp Ser Cys Cys Gly Ala Arg Gly
145                 150                 155                 160

ccc aat gac tgg aac ctc aat atc tac ttc aac tgc act gac ttg aac 528
Pro Asn Asp Trp Asn Leu Asn Ile Tyr Phe Asn Cys Thr Asp Leu Asn
                165                 170                 175

ccc agc cgg gag cgc tgc ggg gtg ccc ttc tcc tgc tgc gtc agg gac 576
Pro Ser Arg Glu Arg Cys Gly Val Pro Phe Ser Cys Cys Val Arg Asp
            180                 185                 190

cct gcg gag gat gtc ctc aac acc cag tgt ggc tac gac gtc cgg ctc 624
Pro Ala Glu Asp Val Leu Asn Thr Gln Cys Gly Tyr Asp Val Arg Leu
        195                 200                 205

aaa ctg gtg aga ggg gag ctg gag cag cag ggc ttc atc cac acc aaa 672
Lys Leu Val Arg Gly Glu Leu Glu Gln Gln Gly Phe Ile His Thr Lys
    210                 215                 220

ggc tgc gtg ggc cag ttt gag aag tgg ctg cag gac aac ctg att gtg 720
Gly Cys Val Gly Gln Phe Glu Lys Trp Leu Gln Asp Asn Leu Ile Val
225                 230                 235                 240

gtg gcg gga gtc ttc atg ggc atc gcc ctc ctc cag atc ttt ggc atc 768
Val Ala Gly Val Phe Met Gly Ile Ala Leu Leu Gln Ile Phe Gly Ile
                245                 250                 255

tgc ctg gcc cag aac ctc gtg agt gac atc aag gca gtg aaa gcc aac 816
Cys Leu Ala Gln Asn Leu Val Ser Asp Ile Lys Ala Val Lys Ala Asn
            260                 265                 270

tgg agc aaa tgg aat gat gac ttt gaa aac cac tgg ctt acg ccc acc 864
Trp Ser Lys Trp Asn Asp Asp Phe Glu Asn His Trp Leu Thr Pro Thr
        275                 280                 285

att tcc gag gtc ctg tcc acg gcg ggg cct cag cag aac tct ctg act 912
Ile Ser Glu Val Leu Ser Thr Ala Gly Pro Gln Gln Asn Ser Leu Thr
    290                 295                 300

ggg gcc cct ggc ccg gcc cca ccc agc cga cat gtt ttc ttt ggc ctg 960
Gly Ala Pro Gly Pro Ala Pro Pro Ser Arg His Val Phe Phe Gly Leu
305                 310                 315                 320

ggt ggt tta tac cct gag cca acc ttt aaa aat tgg 996
Gly Gly Leu Tyr Pro Glu Pro Thr Phe Lys Asn Trp
                325                 330

<210> 6
<211> 332
<212> PRT
<213> Homo sapiens

<400> 6

Met Pro Gly Lys His Gln His Phe Gln Glu Pro Glu Val Gly Cys Cys
1               5                   10                  15

Gly Lys Tyr Phe Leu Phe Gly Phe Asn Ile Val Phe Trp Val Leu Gly
            20                  25                  30

Ala Leu Phe Leu Ala Ile Gly Leu Trp Ala Trp Gly Glu Lys Gly Val
        35                  40                  45

Leu Ser Asn Ile Ser Ala Leu Thr Asp Leu Gly Gly Leu Asp Pro Val
    50                  55                  60

Trp Leu Phe Val Val Val Gly Gly Val Met Ser Val Leu Gly Phe Ala
65                  70                  75                  80

Gly Cys Ile Gly Ala Leu Arg Glu Asn Thr Phe Leu Leu Lys Phe Phe
                85                  90                  95

Ser Val Phe Leu Gly Leu Ile Phe Phe Leu Glu Leu Ala Thr Gly Ile
            100                 105                 110

Leu Ala Phe Val Phe Lys Asp Trp Ile Arg Asp Gln Leu Asn Leu Phe
        115                 120                 125

Ile Asn Asn Asn Val Lys Ala Tyr Arg Asp Asp Ile Asp Leu Gln Asn
    130                 135                 140

Leu Ile Asp Phe Ala Gln Glu Tyr Trp Ser Cys Cys Gly Ala Arg Gly
145                 150                 155                 160

Pro Asn Asp Trp Asn Leu Asn Ile Tyr Phe Asn Cys Thr Asp Leu Asn
                165                 170                 175

Pro Ser Arg Glu Arg Cys Gly Val Pro Phe Ser Cys Cys Val Arg Asp
            180                 185                 190

Pro Ala Glu Asp Val Leu Asn Thr Gln Cys Gly Tyr Asp Val Arg Leu
        195                 200                 205

Lys Leu Val Arg Gly Glu Leu Glu Gln Gln Gly Phe Ile His Thr Lys
    210                 215                 220

Gly Cys Val Gly Gln Phe Glu Lys Trp Leu Gln Asp Asn Leu Ile Val
225                 230                 235                 240

Val Ala Gly Val Phe Met Gly Ile Ala Leu Leu Gln Ile Phe Gly Ile
                245                 250                 255

Cys Leu Ala Gln Asn Leu Val Ser Asp Ile Lys Ala Val Lys Ala Asn
            260                 265                 270

Trp Ser Lys Trp Asn Asp Asp Phe Glu Asn His Trp Leu Thr Pro Thr
        275                 280                 285

Ile Ser Glu Val Leu Ser Thr Ala Gly Pro Gln Gln Asn Ser Leu Thr
    290                 295                 300

Gly Ala Pro Gly Pro Ala Pro Pro Ser Arg His Val Phe Phe Gly Leu
305                 310                 315                 320

Gly Gly Leu Tyr Pro Glu Pro Thr Phe Lys Asn Trp
                325                 330

<210>  7
<211>  987
<212>  DNA
<213>  Homo sapiens

<220>
<221>  CDS
<222>  (1)..(987)

<400>  7
atg ccc ggc aag cac cag cat ttc cag gaa cct gag gtc ggc tgc tgc        48
Met Pro Gly Lys His Gln His Phe Gln Glu Pro Glu Val Gly Cys Cys
1               5                   10                  15

ggg aaa tac ttc ctg ttt ggc ttc aac att gtc ttc tgg gtg ctg gga        96
Gly Lys Tyr Phe Leu Phe Gly Phe Asn Ile Val Phe Trp Val Leu Gly
            20                  25                  30

gcc ctg ttc ctg gct atc ggc ctc tgg gcc tgg ggt gag aag ggc gtt       144
Ala Leu Phe Leu Ala Ile Gly Leu Trp Ala Trp Gly Glu Lys Gly Val
        35                  40                  45

ctc tcg aac atc tca gcg ctg aca gat ctg gga ggc ctt gac ccc gtg       192
Leu Ser Asn Ile Ser Ala Leu Thr Asp Leu Gly Gly Leu Asp Pro Val
    50                  55                  60

tgg ctg ttt gtg gta gtt gga ggc gtc atg tcg gtg ctg ggc ttt gct       240
Trp Leu Phe Val Val Val Gly Gly Val Met Ser Val Leu Gly Phe Ala
65                  70                  75                  80

ggc tgc att ggg gcc ctc cgg gag aac acc ttc ctg ctc aag ttt ttc       288
Gly Cys Ile Gly Ala Leu Arg Glu Asn Thr Phe Leu Leu Lys Phe Phe
                85                  90                  95

tcc gtg ttc ctc ggt ctc atc ttc ttc ctg gag ctg gca aca ggg atc       336
Ser Val Phe Leu Gly Leu Ile Phe Phe Leu Glu Leu Ala Thr Gly Ile
            100                 105                 110

ctg gcc ttt gtc ttc aag gac tgg att cga gac cag ctc aac ctc ttc       384
Leu Ala Phe Val Phe Lys Asp Trp Ile Arg Asp Gln Leu Asn Leu Phe
        115                 120                 125

atc aac aac aac gtc aag gcc tac cgg gac gac att gac ctc cag aac       432
Ile Asn Asn Asn Val Lys Ala Tyr Arg Asp Asp Ile Asp Leu Gln Asn
    130                 135                 140

ctc att gac ttt gct cag gaa tac tgg tct tgc tgc gga gcc cga ggc       480
Leu Ile Asp Phe Ala Gln Glu Tyr Trp Ser Cys Cys Gly Ala Arg Gly
145                 150                 155                 160

ccc aat gac tgg aac ctc aat atc tac ttc aac tgc act gac ttg aac       528
Pro Asn Asp Trp Asn Leu Asn Ile Tyr Phe Asn Cys Thr Asp Leu Asn
                165                 170                 175

ccc agc cgg gag cgc tgc ggg gtg ccc ttc tcc tgc tgc gtc agg gac       576
Pro Ser Arg Glu Arg Cys Gly Val Pro Phe Ser Cys Cys Val Arg Asp
            180                 185                 190

cct gcg gag gat gtc ctc aac acc cag tgt ggc tac gac gtc cgg ctc       624

Pro Ala Glu Asp Val Leu Asn Thr Gln Cys Gly Tyr Asp Val Arg Leu
        195                 200                 205

aaa ctg gag ctg gag cag cag ggc ttc atc cac acc aaa ggc tgc gtg        672
Lys Leu Glu Leu Glu Gln Gln Gly Phe Ile His Thr Lys Gly Cys Val
    210                 215                 220

ggc cag ttt gag aag tgg ctg cag gac aac ctg att gtg gtg gcg gga        720
Gly Gln Phe Glu Lys Trp Leu Gln Asp Asn Leu Ile Val Val Ala Gly
225                 230                 235                 240

gtc ttc atg ggc atc gcc ctc ctc cag atc ttt ggc atc tgc ctg gcc        768
Val Phe Met Gly Ile Ala Leu Leu Gln Ile Phe Gly Ile Cys Leu Ala
                245                 250                 255

cag aac ctc gtg agt gac atc aag gca gtg aaa gcc aac tgg agc aaa        816
Gln Asn Leu Val Ser Asp Ile Lys Ala Val Lys Ala Asn Trp Ser Lys
            260                 265                 270

tgg aat gat gac ttt gaa aac cac tgg ctt acg ccc acc att tcc gag        864
Trp Asn Asp Asp Phe Glu Asn His Trp Leu Thr Pro Thr Ile Ser Glu
        275                 280                 285

gtc ctg tcc acg gcg ggg cct cag cag aac tct ctg act ggg gcc cct        912
Val Leu Ser Thr Ala Gly Pro Gln Gln Asn Ser Leu Thr Gly Ala Pro
    290                 295                 300

ggc ccg gcc cca ccc agc cga cat gtt ttc ttt ggc ctg ggt ggt tta        960
Gly Pro Ala Pro Pro Ser Arg His Val Phe Phe Gly Leu Gly Gly Leu
305                 310                 315                 320

tac cct gag cca acc ttt aaa aat tgg                                    987
Tyr Pro Glu Pro Thr Phe Lys Asn Trp
                325

<210>  8
<211>  329
<212>  PRT
<213>  Homo sapiens

<400>  8

Met Pro Gly Lys His Gln His Phe Gln Glu Pro Glu Val Gly Cys Cys
1               5                   10                  15

Gly Lys Tyr Phe Leu Phe Gly Phe Asn Ile Val Phe Trp Val Leu Gly
            20                  25                  30

Ala Leu Phe Leu Ala Ile Gly Leu Trp Ala Trp Gly Glu Lys Gly Val
        35                  40                  45

Leu Ser Asn Ile Ser Ala Leu Thr Asp Leu Gly Gly Leu Asp Pro Val
    50                  55                  60

Trp Leu Phe Val Val Val Gly Gly Val Met Ser Val Leu Gly Phe Ala
65                  70                  75                  80

Gly Cys Ile Gly Ala Leu Arg Glu Asn Thr Phe Leu Leu Lys Phe Phe
                85                  90                  95

Ser Val Phe Leu Gly Leu Ile Phe Phe Leu Glu Leu Ala Thr Gly Ile
            100                 105                 110

Leu Ala Phe Val Phe Lys Asp Trp Ile Arg Asp Gln Leu Asn Leu Phe
        115                 120                 125

Ile Asn Asn Asn Val Lys Ala Tyr Arg Asp Asp Ile Asp Leu Gln Asn

130                 135                 140

Leu Ile Asp Phe Ala Gln Glu Tyr Trp Ser Cys Cys Gly Ala Arg Gly
145                 150                 155                 160

Pro Asn Asp Trp Asn Leu Asn Ile Tyr Phe Asn Cys Thr Asp Leu Asn
                165                 170                 175

Pro Ser Arg Glu Arg Cys Gly Val Pro Phe Ser Cys Cys Val Arg Asp
            180                 185                 190

Pro Ala Glu Asp Val Leu Asn Thr Gln Cys Gly Tyr Asp Val Arg Leu
        195                 200                 205

Lys Leu Glu Leu Glu Gln Gln Gly Phe Ile His Thr Lys Gly Cys Val
    210                 215                 220

Gly Gln Phe Glu Lys Trp Leu Gln Asp Asn Leu Ile Val Val Ala Gly
225                 230                 235                 240

Val Phe Met Gly Ile Ala Leu Leu Gln Ile Phe Gly Ile Cys Leu Ala
                245                 250                 255

Gln Asn Leu Val Ser Asp Ile Lys Ala Val Lys Ala Asn Trp Ser Lys
            260                 265                 270

Trp Asn Asp Asp Phe Glu Asn His Trp Leu Thr Pro Thr Ile Ser Glu
        275                 280                 285

Val Leu Ser Thr Ala Gly Pro Gln Gln Asn Ser Leu Thr Gly Ala Pro
    290                 295                 300

Gly Pro Ala Pro Pro Ser Arg His Val Phe Phe Gly Leu Gly Gly Leu
305                 310                 315                 320

Tyr Pro Glu Pro Thr Phe Lys Asn Trp
                325

<210>  9
<211>  798
<212>  DNA
<213>  Homo sapiens

<220>
<221>  CDS
<222>  (1)..(798)

<400>  9
atg ccc ggc aag cac cag cat ttc cag gaa cct gag gtc ggc tgc tgc      48
Met Pro Gly Lys His Gln His Phe Gln Glu Pro Glu Val Gly Cys Cys
1               5                   10                  15

ggg aaa tac ttc ctg ttt ggc ttc aac att gtc ttc tgg gtg ctg gga      96
Gly Lys Tyr Phe Leu Phe Gly Phe Asn Ile Val Phe Trp Val Leu Gly
            20                  25                  30

gcc ctg ttc ctg gct atc ggc ctc tgg gcc tgg ggt gag aag ggc gtt      144
Ala Leu Phe Leu Ala Ile Gly Leu Trp Ala Trp Gly Glu Lys Gly Val
        35                  40                  45

ctc tcg aac atc tca gcg ctg aca gat ctg gga ggc ctt gac ccc gtg      192
Leu Ser Asn Ile Ser Ala Leu Thr Asp Leu Gly Gly Leu Asp Pro Val

50                  55                  60

tgg ctg ttt gtg gta gtt gga ggc gtc atg tcg gtg ctg ggc ttt gct        240
Trp Leu Phe Val Val Val Gly Gly Val Met Ser Val Leu Gly Phe Ala
65                  70                  75                  80

ggc tgc att ggg gcc ctc cgg gag aac acc ttc ctg ctc aag ttt ttc        288
Gly Cys Ile Gly Ala Leu Arg Glu Asn Thr Phe Leu Leu Lys Phe Phe
                85                  90                  95

tcc gtg ttc ctc ggt ctc atc ttc ttc ctg gag ctg gca aca ggg atc        336
Ser Val Phe Leu Gly Leu Ile Phe Phe Leu Glu Leu Ala Thr Gly Ile
            100                 105                 110

ctg gcc ttt gtc ttc aag gac tgg att cga gac cag ctc aac ctc ttc        384
Leu Ala Phe Val Phe Lys Asp Trp Ile Arg Asp Gln Leu Asn Leu Phe
        115                 120                 125

atc aac aac aac gtc aag gcc tac cgg gac gac att gac ctc cag aac        432
Ile Asn Asn Asn Val Lys Ala Tyr Arg Asp Asp Ile Asp Leu Gln Asn
    130                 135                 140

ctc att gac ttt gct cag gaa tac tgg tct tgc tgc gga gcc cga ggc        480
Leu Ile Asp Phe Ala Gln Glu Tyr Trp Ser Cys Cys Gly Ala Arg Gly
145                 150                 155                 160

ccc aat gac tgg aac ctc aat atc tac ttc aac tgc act gac ttg aac        528
Pro Asn Asp Trp Asn Leu Asn Ile Tyr Phe Asn Cys Thr Asp Leu Asn
                165                 170                 175

ccc agc cgg gag cgc tgc ggg gtg ccc ttc tcc tgc tgc gtc agg gac        576
Pro Ser Arg Glu Arg Cys Gly Val Pro Phe Ser Cys Cys Val Arg Asp
            180                 185                 190

cct gcg gag gat gtc ctc aac acc cag tgt ggc tac gac gtc cgg ctc        624
Pro Ala Glu Asp Val Leu Asn Thr Gln Cys Gly Tyr Asp Val Arg Leu
        195                 200                 205

aaa ctg gtg aga ggg gag ctg gag cag cag ggc ttc atc cac acc aaa        672
Lys Leu Val Arg Gly Glu Leu Glu Gln Gln Gly Phe Ile His Thr Lys
    210                 215                 220

ggc tgc gtg ggc cag ttt gag aag tgg ctg cag gac aac ctg att gtg        720
Gly Cys Val Gly Gln Phe Glu Lys Trp Leu Gln Asp Asn Leu Ile Val
225                 230                 235                 240

gtg gcg gga gtc ttc atg ggc atc gcc ctc ctc cag atc ttt ggc atc        768
Val Ala Gly Val Phe Met Gly Ile Ala Leu Leu Gln Ile Phe Gly Ile
                245                 250                 255

tgc ctg gcc cag aac ctc gag caa atg gaa                                798
Cys Leu Ala Gln Asn Leu Glu Gln Met Glu
            260                 265


<210>  10
<211>  266
<212>  PRT
<213>  Homo sapiens

<400>  10

Met Pro Gly Lys His Gln His Phe Gln Glu Pro Glu Val Gly Cys Cys
1               5                   10                  15


Gly Lys Tyr Phe Leu Phe Gly Phe Asn Ile Val Phe Trp Val Leu Gly
            20                  25                  30


Ala Leu Phe Leu Ala Ile Gly Leu Trp Ala Trp Gly Glu Lys Gly Val
        35                  40                  45


Leu Ser Asn Ile Ser Ala Leu Thr Asp Leu Gly Gly Leu Asp Pro Val
    50                  55                  60

Trp Leu Phe Val Val Val Gly Gly Val Met Ser Val Leu Gly Phe Ala
65                  70                  75                  80

Gly Cys Ile Gly Ala Leu Arg Glu Asn Thr Phe Leu Leu Lys Phe Phe
                85                  90                  95

Ser Val Phe Leu Gly Leu Ile Phe Phe Leu Glu Leu Ala Thr Gly Ile
            100                 105                 110

Leu Ala Phe Val Phe Lys Asp Trp Ile Arg Asp Gln Leu Asn Leu Phe
        115                 120                 125

Ile Asn Asn Asn Val Lys Ala Tyr Arg Asp Asp Ile Asp Leu Gln Asn
    130                 135                 140

Leu Ile Asp Phe Ala Gln Glu Tyr Trp Ser Cys Cys Gly Ala Arg Gly
145                 150                 155                 160

Pro Asn Asp Trp Asn Leu Asn Ile Tyr Phe Asn Cys Thr Asp Leu Asn
                165                 170                 175

Pro Ser Arg Glu Arg Cys Gly Val Pro Phe Ser Cys Cys Val Arg Asp
            180                 185                 190

Pro Ala Glu Asp Val Leu Asn Thr Gln Cys Gly Tyr Asp Val Arg Leu
        195                 200                 205

Lys Leu Val Arg Gly Glu Leu Glu Gln Gln Gly Phe Ile His Thr Lys
    210                 215                 220

Gly Cys Val Gly Gln Phe Glu Lys Trp Leu Gln Asp Asn Leu Ile Val
225                 230                 235                 240

Val Ala Gly Val Phe Met Gly Ile Ala Leu Leu Gln Ile Phe Gly Ile
                245                 250                 255

Cys Leu Ala Gln Asn Leu Glu Gln Met Glu
            260                 265

<210>  11
<211>  958
<212>  DNA
<213>  Homo sapiens

<220>
<221>  CDS
<222>  (1)..(789)

<400>  11
atg ccc ggc aag cac cag cat ttc cag gaa cct gag gtc ggc tgc tgc       48
Met Pro Gly Lys His Gln His Phe Gln Glu Pro Glu Val Gly Cys Cys
1               5                   10                  15

ggg aaa tac ttc ctg ttt ggc ttc aac att gtc ttc tgg gtg ctg gga       96
Gly Lys Tyr Phe Leu Phe Gly Phe Asn Ile Val Phe Trp Val Leu Gly
            20                  25                  30

gcc ctg ttc ctg gct atc ggc ctc tgg gcc tgg ggt gag aag ggc gtt      144
Ala Leu Phe Leu Ala Ile Gly Leu Trp Ala Trp Gly Glu Lys Gly Val
        35                  40                  45

ctc tcg aac atc tca gcg ctg aca gat ctg gga ggc ctt gac ccc gtg     192
Leu Ser Asn Ile Ser Ala Leu Thr Asp Leu Gly Gly Leu Asp Pro Val
    50                  55                  60

tgg ctg ttt gtg gta gtt gga ggc gtc atg tcg gtg ctg ggc ttt gct     240
Trp Leu Phe Val Val Val Gly Gly Val Met Ser Val Leu Gly Phe Ala
65                  70                  75                  80

ggc tgc att ggg gcc ctc cgg gag aac acc ttc ctg ctc aag ttt ttc     288
Gly Cys Ile Gly Ala Leu Arg Glu Asn Thr Phe Leu Leu Lys Phe Phe
                85                  90                  95

tcc gtg ttc ctc ggt ctc atc ttc ttc ctg gag ctg gca aca ggg atc     336
Ser Val Phe Leu Gly Leu Ile Phe Phe Leu Glu Leu Ala Thr Gly Ile
            100                 105                 110

ctg gcc ttt gtc ttc aag gac tgg att cga gac cag ctc aac ctc ttc     384
Leu Ala Phe Val Phe Lys Asp Trp Ile Arg Asp Gln Leu Asn Leu Phe
        115                 120                 125

atc aac aac aac gtc aag gcc tac cgg gac gac att gac ctc cag aac     432
Ile Asn Asn Asn Val Lys Ala Tyr Arg Asp Asp Ile Asp Leu Gln Asn
    130                 135                 140

ctc att gac ttt gct cag gaa tac tgg tct tgc tgc gga gcc cga ggc     480
Leu Ile Asp Phe Ala Gln Glu Tyr Trp Ser Cys Cys Gly Ala Arg Gly
145                 150                 155                 160

ccc aat gac tgg aac ctc aat atc tac ttc aac tgc act gac ttg aac     528
Pro Asn Asp Trp Asn Leu Asn Ile Tyr Phe Asn Cys Thr Asp Leu Asn
                165                 170                 175

ccc agc cgg gag cgc tgc ggg gtg ccc ttc tcc tgc tgc gtc agg gac     576
Pro Ser Arg Glu Arg Cys Gly Val Pro Phe Ser Cys Cys Val Arg Asp
            180                 185                 190

cct gcg gag gat gtc ctc aac acc cag tgt ggc tac gac gtc cgg ctc     624
Pro Ala Glu Asp Val Leu Asn Thr Gln Cys Gly Tyr Asp Val Arg Leu
        195                 200                 205

aaa ctg gag ctg gag cag cag ggc ttc atc cac acc aaa ggc tgc gtg     672
Lys Leu Glu Leu Glu Gln Gln Gly Phe Ile His Thr Lys Gly Cys Val
    210                 215                 220

ggc cag ttt gag aag tgg ctg cag gac aac ctg att gtg gtg gcg gga     720
Gly Gln Phe Glu Lys Trp Leu Gln Asp Asn Leu Ile Val Val Ala Gly
225                 230                 235                 240

gtc ttc atg ggc atc gcc ctc ctc cag atc ttt ggc atc tgc ctg gcc     768
Val Phe Met Gly Ile Ala Leu Leu Gln Ile Phe Gly Ile Cys Leu Ala
                245                 250                 255

cag aac ctc gag caa atg gaa tgatgacttt gaaaaccact ggcttacgcc     819
Gln Asn Leu Glu Gln Met Glu
            260

caccatttcc gaggtcctgt ccacggcggg gcctcagcag aactctctga ctggggcccc     879

tggcccggcc ccacccagcc gacatgtttt ctttggcctg ggtggtttat accctgagcc     939

aacctttaaa aattggtag     958

<210>  12
<211>  263
<212>  PRT
<213>  Homo sapiens

<400>  12

Met Pro Gly Lys His Gln His Phe Gln Glu Pro Glu Val Gly Cys Cys
1               5                   10                  15

Gly Lys Tyr Phe Leu Phe Gly Phe Asn Ile Val Phe Trp Val Leu Gly

20                  25                  30

Ala Leu Phe Leu Ala Ile Gly Leu Trp Ala Trp Gly Glu Lys Gly Val
        35                  40                  45

Leu Ser Asn Ile Ser Ala Leu Thr Asp Leu Gly Gly Leu Asp Pro Val
    50                  55                  60

Trp Leu Phe Val Val Val Gly Gly Val Met Ser Val Leu Gly Phe Ala
65                  70                  75                  80

Gly Cys Ile Gly Ala Leu Arg Glu Asn Thr Phe Leu Leu Lys Phe Phe
                85                  90                  95

Ser Val Phe Leu Gly Leu Ile Phe Phe Leu Glu Leu Ala Thr Gly Ile
            100                 105                 110

Leu Ala Phe Val Phe Lys Asp Trp Ile Arg Asp Gln Leu Asn Leu Phe
        115                 120                 125

Ile Asn Asn Asn Val Lys Ala Tyr Arg Asp Asp Ile Asp Leu Gln Asn
    130                 135                 140

Leu Ile Asp Phe Ala Gln Glu Tyr Trp Ser Cys Cys Gly Ala Arg Gly
145                 150                 155                 160

Pro Asn Asp Trp Asn Leu Asn Ile Tyr Phe Asn Cys Thr Asp Leu Asn
                165                 170                 175

Pro Ser Arg Glu Arg Cys Gly Val Pro Phe Ser Cys Cys Val Arg Asp
            180                 185                 190

Pro Ala Glu Asp Val Leu Asn Thr Gln Cys Gly Tyr Asp Val Arg Leu
        195                 200                 205

Lys Leu Glu Leu Glu Gln Gln Gly Phe Ile His Thr Lys Gly Cys Val
    210                 215                 220

Gly Gln Phe Glu Lys Trp Leu Gln Asp Asn Leu Ile Val Val Ala Gly
225                 230                 235                 240

Val Phe Met Gly Ile Ala Leu Leu Gln Ile Phe Gly Ile Cys Leu Ala
                245                 250                 255

Gln Asn Leu Glu Gln Met Glu
            260

<210>  13
<211>  822
<212>  DNA
<213>  Homo sapiens

<220>
<221>  CDS
<222>  (1)..(822)

<400>  13
atg ccc ggc aag cac cag cat ttc cag gaa cct gag gtc ggc tgc tgc       48
Met Pro Gly Lys His Gln His Phe Gln Glu Pro Glu Val Gly Cys Cys

1                 5                   10                  15

ggg aaa tac ttc ctg ttt ggc ttc aac att gtc ttc tgg gtg ctg gga          96
Gly Lys Tyr Phe Leu Phe Gly Phe Asn Ile Val Phe Trp Val Leu Gly
            20                  25                  30

gcc ctg ttc ctg gct atc ggc ctc tgg gcc tgg ggt gag aag ggc gtt          144
Ala Leu Phe Leu Ala Ile Gly Leu Trp Ala Trp Gly Glu Lys Gly Val
        35                  40                  45

ctc tcg aac atc tca gcg ctg aca gat ctg gga ggc ctt gac ccc gtg          192
Leu Ser Asn Ile Ser Ala Leu Thr Asp Leu Gly Gly Leu Asp Pro Val
    50                  55                  60

tgg ctg ttt gtg gta gtt gga ggc gtc atg tcg gtg ctg ggc ttt gct          240
Trp Leu Phe Val Val Val Gly Gly Val Met Ser Val Leu Gly Phe Ala
65                  70                  75                  80

ggc tgc att ggg gcc ctc cgg gag aac acc ttc ctg ctc aag ttt ttc          288
Gly Cys Ile Gly Ala Leu Arg Glu Asn Thr Phe Leu Leu Lys Phe Phe
                85                  90                  95

tcc gtg ttc ctc ggt ctc atc ttc ttc ctg gag ctg gca aca ggg atc          336
Ser Val Phe Leu Gly Leu Ile Phe Phe Leu Glu Leu Ala Thr Gly Ile
            100                 105                 110

ctg gcc ttt gtc ttc aag gac tgg att cga gac cag ctc aac ctc ttc          384
Leu Ala Phe Val Phe Lys Asp Trp Ile Arg Asp Gln Leu Asn Leu Phe
        115                 120                 125

atc aac aac aac gtc aag gcc tac cgg gac gac att gac ctc cag aac          432
Ile Asn Asn Asn Val Lys Ala Tyr Arg Asp Asp Ile Asp Leu Gln Asn
    130                 135                 140

ctc att gac ttt gct cag gaa tac tgg tct tgc tgc gga gcc cga ggc          480
Leu Ile Asp Phe Ala Gln Glu Tyr Trp Ser Cys Cys Gly Ala Arg Gly
145                 150                 155                 160

ccc aat gac tgg aac ctc aat atc tac ttc aac tgc act gac ttg aac          528
Pro Asn Asp Trp Asn Leu Asn Ile Tyr Phe Asn Cys Thr Asp Leu Asn
                165                 170                 175

ccc agc cgg gag cgc tgc ggg gtg ccc ttc tcc tgc tgc gtc agg gac          576
Pro Ser Arg Glu Arg Cys Gly Val Pro Phe Ser Cys Cys Val Arg Asp
            180                 185                 190

cct gcg gag gat gtc ctc aac acc cag tgt ggc tac gac gtc cgg ctc          624
Pro Ala Glu Asp Val Leu Asn Thr Gln Cys Gly Tyr Asp Val Arg Leu
        195                 200                 205

aaa ctg gtg aga ggg gag ctg gag cag cag ggc ttc atc cac acc aaa          672
Lys Leu Val Arg Gly Glu Leu Glu Gln Gln Gly Phe Ile His Thr Lys
    210                 215                 220

ggc tgc gtg ggc cag ttt gag aag tgg ctg cag gac aac ctg att gtg          720
Gly Cys Val Gly Gln Phe Glu Lys Trp Leu Gln Asp Asn Leu Ile Val
225                 230                 235                 240

gtg gcg gga gtc ttc atg ggc atc gcc ctc ctc cag atc ttt ggc atc          768
Val Ala Gly Val Phe Met Gly Ile Ala Leu Leu Gln Ile Phe Gly Ile
                245                 250                 255

tgc ctg gcc cag aac ctc gtg agt gac atc aag gca gtg aaa gcc aac          816
Cys Leu Ala Gln Asn Leu Val Ser Asp Ile Lys Ala Val Lys Ala Asn
            260                 265                 270

tgg tga                                                                  822
Trp

<210>  14
<211>  273
<212>  PRT
<213>  Homo sapiens

<400> 14

Met Pro Gly Lys His Gln His Phe Gln Glu Pro Glu Val Gly Cys Cys
1               5                   10                  15

Gly Lys Tyr Phe Leu Phe Gly Phe Asn Ile Val Phe Trp Val Leu Gly
            20                  25                  30

Ala Leu Phe Leu Ala Ile Gly Leu Trp Ala Trp Gly Glu Lys Gly Val
        35                  40                  45

Leu Ser Asn Ile Ser Ala Leu Thr Asp Leu Gly Gly Leu Asp Pro Val
    50                  55                  60

Trp Leu Phe Val Val Val Gly Gly Val Met Ser Val Leu Gly Phe Ala
65                  70                  75                  80

Gly Cys Ile Gly Ala Leu Arg Glu Asn Thr Phe Leu Leu Lys Phe Phe
                85                  90                  95

Ser Val Phe Leu Gly Leu Ile Phe Phe Leu Glu Leu Ala Thr Gly Ile
            100                 105                 110

Leu Ala Phe Val Phe Lys Asp Trp Ile Arg Asp Gln Leu Asn Leu Phe
        115                 120                 125

Ile Asn Asn Asn Val Lys Ala Tyr Arg Asp Asp Ile Asp Leu Gln Asn
    130                 135                 140

Leu Ile Asp Phe Ala Gln Glu Tyr Trp Ser Cys Cys Gly Ala Arg Gly
145                 150                 155                 160

Pro Asn Asp Trp Asn Leu Asn Ile Tyr Phe Asn Cys Thr Asp Leu Asn
                165                 170                 175

Pro Ser Arg Glu Arg Cys Gly Val Pro Phe Ser Cys Cys Val Arg Asp
            180                 185                 190

Pro Ala Glu Asp Val Leu Asn Thr Gln Cys Gly Tyr Asp Val Arg Leu
        195                 200                 205

Lys Leu Val Arg Gly Glu Leu Glu Gln Gln Gly Phe Ile His Thr Lys
    210                 215                 220

Gly Cys Val Gly Gln Phe Glu Lys Trp Leu Gln Asp Asn Leu Ile Val
225                 230                 235                 240

Val Ala Gly Val Phe Met Gly Ile Ala Leu Leu Gln Ile Phe Gly Ile
                245                 250                 255

Cys Leu Ala Gln Asn Leu Val Ser Asp Ile Lys Ala Val Lys Ala Asn
            260                 265                 270

Trp

<210> 15
<211> 813

<212> DNA
<213> Homo sapiens

<220>
<221> CDS
<222> (1)..(813)

<400> 15
atg ccc ggc aag cac cag cat ttc cag gaa cct gag gtc ggc tgc tgc 48
Met Pro Gly Lys His Gln His Phe Gln Glu Pro Glu Val Gly Cys Cys
1               5                   10                  15

ggg aaa tac ttc ctg ttt ggc ttc aac att gtc ttc tgg gtg ctg gga 96
Gly Lys Tyr Phe Leu Phe Gly Phe Asn Ile Val Phe Trp Val Leu Gly
            20                  25                  30

gcc ctg ttc ctg gct atc ggc ctc tgg gcc tgg ggt gag aag ggc gtt 144
Ala Leu Phe Leu Ala Ile Gly Leu Trp Ala Trp Gly Glu Lys Gly Val
        35                  40                  45

ctc tcg aac atc tca gcg ctg aca gat ctg gga ggc ctt gac ccc gtg 192
Leu Ser Asn Ile Ser Ala Leu Thr Asp Leu Gly Gly Leu Asp Pro Val
    50                  55                  60

tgg ctg ttt gtg gta gtt gga ggc gtc atg tcg gtg ctg ggc ttt gct 240
Trp Leu Phe Val Val Val Gly Gly Val Met Ser Val Leu Gly Phe Ala
65                  70                  75                  80

ggc tgc att ggg gcc ctc cgg gag aac acc ttc ctg ctc aag ttt ttc 288
Gly Cys Ile Gly Ala Leu Arg Glu Asn Thr Phe Leu Leu Lys Phe Phe
                85                  90                  95

tcc gtg ttc ctc ggt ctc atc ttc ttc ctg gag ctg gca aca ggg atc 336
Ser Val Phe Leu Gly Leu Ile Phe Phe Leu Glu Leu Ala Thr Gly Ile
            100                 105                 110

ctg gcc ttt gtc ttc aag gac tgg att cga gac cag ctc aac ctc ttc 384
Leu Ala Phe Val Phe Lys Asp Trp Ile Arg Asp Gln Leu Asn Leu Phe
        115                 120                 125

atc aac aac aac gtc aag gcc tac cgg gac gac att gac ctc cag aac 432
Ile Asn Asn Asn Val Lys Ala Tyr Arg Asp Asp Ile Asp Leu Gln Asn
    130                 135                 140

ctc att gac ttt gct cag gaa tac tgg tct tgc tgc gga gcc cga ggc 480
Leu Ile Asp Phe Ala Gln Glu Tyr Trp Ser Cys Cys Gly Ala Arg Gly
145                 150                 155                 160

ccc aat gac tgg aac ctc aat atc tac ttc aac tgc act gac ttg aac 528
Pro Asn Asp Trp Asn Leu Asn Ile Tyr Phe Asn Cys Thr Asp Leu Asn
                165                 170                 175

ccc agc cgg gag cgc tgc ggg gtg ccc ttc tcc tgc tgc gtc agg gac 576
Pro Ser Arg Glu Arg Cys Gly Val Pro Phe Ser Cys Cys Val Arg Asp
            180                 185                 190

cct gcg gag gat gtc ctc aac acc cag tgt ggc tac gac gtc cgg ctc 624
Pro Ala Glu Asp Val Leu Asn Thr Gln Cys Gly Tyr Asp Val Arg Leu
        195                 200                 205

aaa ctg gag ctg gag cag cag ggc ttc atc cac acc aaa ggc tgc gtg 672
Lys Leu Glu Leu Glu Gln Gln Gly Phe Ile His Thr Lys Gly Cys Val
    210                 215                 220

ggc cag ttt gag aag tgg ctg cag gac aac ctg att gtg gtg gcg gga 720
Gly Gln Phe Glu Lys Trp Leu Gln Asp Asn Leu Ile Val Val Ala Gly
225                 230                 235                 240

gtc ttc atg ggc atc gcc ctc ctc cag atc ttt ggc atc tgc ctg gcc 768
Val Phe Met Gly Ile Ala Leu Leu Gln Ile Phe Gly Ile Cys Leu Ala
                245                 250                 255

cag aac ctc gtg agt gac atc aag gca gtg aaa gcc aac tgg tga 813
Gln Asn Leu Val Ser Asp Ile Lys Ala Val Lys Ala Asn Trp
            260                 265                 270

<210> 16
<211> 270
<212> PRT
<213> Homo sapiens

<400> 16

Met Pro Gly Lys His Gln His Phe Gln Glu Pro Glu Val Gly Cys Cys
1               5                   10                  15

Gly Lys Tyr Phe Leu Phe Gly Phe Asn Ile Val Phe Trp Val Leu Gly
            20                  25                  30

Ala Leu Phe Leu Ala Ile Gly Leu Trp Ala Trp Gly Glu Lys Gly Val
        35                  40                  45

Leu Ser Asn Ile Ser Ala Leu Thr Asp Leu Gly Gly Leu Asp Pro Val
    50                  55                  60

Trp Leu Phe Val Val Val Gly Gly Val Met Ser Val Leu Gly Phe Ala
65                  70                  75                  80

Gly Cys Ile Gly Ala Leu Arg Glu Asn Thr Phe Leu Leu Lys Phe Phe
                85                  90                  95

Ser Val Phe Leu Gly Leu Ile Phe Phe Leu Glu Leu Ala Thr Gly Ile
            100                 105                 110

Leu Ala Phe Val Phe Lys Asp Trp Ile Arg Asp Gln Leu Asn Leu Phe
        115                 120                 125

Ile Asn Asn Asn Val Lys Ala Tyr Arg Asp Asp Ile Asp Leu Gln Asn
    130                 135                 140

Leu Ile Asp Phe Ala Gln Glu Tyr Trp Ser Cys Cys Gly Ala Arg Gly
145                 150                 155                 160

Pro Asn Asp Trp Asn Leu Asn Ile Tyr Phe Asn Cys Thr Asp Leu Asn
                165                 170                 175

Pro Ser Arg Glu Arg Cys Gly Val Pro Phe Ser Cys Cys Val Arg Asp
            180                 185                 190

Pro Ala Glu Asp Val Leu Asn Thr Gln Cys Gly Tyr Asp Val Arg Leu
        195                 200                 205

Lys Leu Glu Leu Glu Gln Gln Gly Phe Ile His Thr Lys Gly Cys Val
    210                 215                 220

Gly Gln Phe Glu Lys Trp Leu Gln Asp Asn Leu Ile Val Val Ala Gly
225                 230                 235                 240

Val Phe Met Gly Ile Ala Leu Leu Gln Ile Phe Gly Ile Cys Leu Ala
                245                 250                 255

Gln Asn Leu Val Ser Asp Ile Lys Ala Val Lys Ala Asn Trp
            260                 265                 270

<210> 17
<211> 927
<212> DNA
<213> Homo sapiens

<220>
<221> CDS
<222> (1)..(927)

<400> 17
atg ccc ggc aag cac cag cat ttc cag gaa cct gag gtc ggc tgc tgc 48
Met Pro Gly Lys His Gln His Phe Gln Glu Pro Glu Val Gly Cys Cys
1               5                   10                  15

ggg aaa tac ttc ctg ttt ggc ttc aac att gtc ttc tgg gtg ctg gga 96
Gly Lys Tyr Phe Leu Phe Gly Phe Asn Ile Val Phe Trp Val Leu Gly
            20                  25                  30

gcc ctg ttc ctg gct atc ggc ctc tgg gcc tgg ggt gag aag ggc gtt 144
Ala Leu Phe Leu Ala Ile Gly Leu Trp Ala Trp Gly Glu Lys Gly Val
        35                  40                  45

ctc tcg aac atc tca gcg ctg aca gat ctg gga ggc ctt gac ccc gtg 192
Leu Ser Asn Ile Ser Ala Leu Thr Asp Leu Gly Gly Leu Asp Pro Val
    50                  55                  60

tgg ctg ttt gtg gta gtt gga ggc gtc atg tcg gtg ctg ggc ttt gct 240
Trp Leu Phe Val Val Val Gly Gly Val Met Ser Val Leu Gly Phe Ala
65                  70                  75                  80

ggc tgc att ggg gcc ctc cgg gag aac acc ttc ctg ctc aag ttt ttc 288
Gly Cys Ile Gly Ala Leu Arg Glu Asn Thr Phe Leu Leu Lys Phe Phe
                85                  90                  95

tcc gtg ttc ctc ggt ctc atc ttc ttc ctg gag ctg gca aca ggg atc 336
Ser Val Phe Leu Gly Leu Ile Phe Phe Leu Glu Leu Ala Thr Gly Ile
            100                 105                 110

ctg gcc ttt gtc ttc aag gac tgg att cga gac cag ctc aac ctc ttc 384
Leu Ala Phe Val Phe Lys Asp Trp Ile Arg Asp Gln Leu Asn Leu Phe
        115                 120                 125

atc aac aac aac gtc aag gcc tac cgg gac gac att gac ctc cag aac 432
Ile Asn Asn Asn Val Lys Ala Tyr Arg Asp Asp Ile Asp Leu Gln Asn
    130                 135                 140

ctc att gac ttt gct cag gaa tac tgg tct tgc tgc gga gcc cga ggc 480
Leu Ile Asp Phe Ala Gln Glu Tyr Trp Ser Cys Cys Gly Ala Arg Gly
145                 150                 155                 160

ccc aat gac tgg aac ctc aat atc tac ttc aac tgc act gac ttg aac 528
Pro Asn Asp Trp Asn Leu Asn Ile Tyr Phe Asn Cys Thr Asp Leu Asn
                165                 170                 175

ccc agc cgg gag cgc tgc ggg gtg ccc ttc tcc tgc tgc gtc agg gac 576
Pro Ser Arg Glu Arg Cys Gly Val Pro Phe Ser Cys Cys Val Arg Asp
            180                 185                 190

cct gcg gag gat gtc ctc aac acc cag tgt ggc tac gac gtc cgg ctc 624
Pro Ala Glu Asp Val Leu Asn Thr Gln Cys Gly Tyr Asp Val Arg Leu
        195                 200                 205

aaa ctg gag ctg gag cag cag ggc ttc atc cac acc aaa ggc tgc gtg 672
Lys Leu Glu Leu Glu Gln Gln Gly Phe Ile His Thr Lys Gly Cys Val
    210                 215                 220

ggc cag ttt gag aag tgg ctg cag gac aac ctg att gtg gtg gcg gga 720
Gly Gln Phe Glu Lys Trp Leu Gln Asp Asn Leu Ile Val Val Ala Gly
225                 230                 235                 240

gtc ttc atg ggc atc gcc ctc ctc cag agc aaa tgg aat gat gac ttt 768
Val Phe Met Gly Ile Ala Leu Leu Gln Ser Lys Trp Asn Asp Asp Phe
                245                 250                 255

gaa aac cac tgg ctt acg ccc acc att tcc gag gtc ctg tcc acg gcg     816
Glu Asn His Trp Leu Thr Pro Thr Ile Ser Glu Val Leu Ser Thr Ala
            260                 265                 270

ggg cct cag cag aac tct ctg act ggg gcc cct ggc ccg gcc cca ccc     864
Gly Pro Gln Gln Asn Ser Leu Thr Gly Ala Pro Gly Pro Ala Pro Pro
        275                 280                 285

agc cga cat gtt ttc ttt ggc ctg ggt ggt tta tac cct gag cca acc     912
Ser Arg His Val Phe Phe Gly Leu Gly Gly Leu Tyr Pro Glu Pro Thr
    290                 295                 300

ttt aaa aat tgg tag                                                 927
Phe Lys Asn Trp
305

<210> 18
<211> 308
<212> PRT
<213> Homo sapiens

<400> 18

Met Pro Gly Lys His Gln His Phe Gln Glu Pro Glu Val Gly Cys Cys
1               5                   10                  15

Gly Lys Tyr Phe Leu Phe Gly Phe Asn Ile Val Phe Trp Val Leu Gly
            20                  25                  30

Ala Leu Phe Leu Ala Ile Gly Leu Trp Ala Trp Gly Glu Lys Gly Val
        35                  40                  45

Leu Ser Asn Ile Ser Ala Leu Thr Asp Leu Gly Gly Leu Asp Pro Val
    50                  55                  60

Trp Leu Phe Val Val Val Gly Gly Val Met Ser Val Leu Gly Phe Ala
65                  70                  75                  80

Gly Cys Ile Gly Ala Leu Arg Glu Asn Thr Phe Leu Leu Lys Phe Phe
                85                  90                  95

Ser Val Phe Leu Gly Leu Ile Phe Phe Leu Glu Leu Ala Thr Gly Ile
            100                 105                 110

Leu Ala Phe Val Phe Lys Asp Trp Ile Arg Asp Gln Leu Asn Leu Phe
        115                 120                 125

Ile Asn Asn Asn Val Lys Ala Tyr Arg Asp Asp Ile Asp Leu Gln Asn
    130                 135                 140

Leu Ile Asp Phe Ala Gln Glu Tyr Trp Ser Cys Cys Gly Ala Arg Gly
145                 150                 155                 160

Pro Asn Asp Trp Asn Leu Asn Ile Tyr Phe Asn Cys Thr Asp Leu Asn
                165                 170                 175

Pro Ser Arg Glu Arg Cys Gly Val Pro Phe Ser Cys Cys Val Arg Asp
            180                 185                 190

Pro Ala Glu Asp Val Leu Asn Thr Gln Cys Gly Tyr Asp Val Arg Leu
        195                 200                 205

Lys Leu Glu Leu Glu Gln Gln Gly Phe Ile His Thr Lys Gly Cys Val
    210                 215                 220

Gly Gln Phe Glu Lys Trp Leu Gln Asp Asn Leu Ile Val Val Ala Gly
225                 230                 235                 240

Val Phe Met Gly Ile Ala Leu Leu Gln Ser Lys Trp Asn Asp Asp Phe
                245                 250                 255

Glu Asn His Trp Leu Thr Pro Thr Ile Ser Glu Val Leu Ser Thr Ala
            260                 265                 270

Gly Pro Gln Gln Asn Ser Leu Thr Gly Ala Pro Gly Pro Ala Pro Pro
        275                 280                 285

Ser Arg His Val Phe Phe Gly Leu Gly Gly Leu Tyr Pro Glu Pro Thr
    290                 295                 300

Phe Lys Asn Trp
305

<210>  19
<211>  19
<212>  RNA
<213>  Artificial Sequence

<220>
<223>  sense oligonucleotide for siRNA-1

<400>  19
gcaaucaagu ggagacaga                                                    19

<210>  20
<211>  19
<212>  RNA
<213>  Artificial Sequence

<220>
<223>  antisense oligonucleotide for siRNA-1

<400>  20
ucugucucca cuugauugc                                                    19

<210>  21
<211>  19
<212>  RNA
<213>  Artificial Sequence

<220>
<223>  sense oligonucleotide for siRNA-2

<400>  21
agacagaguc ggugggaga                                                    19

<210>  22
<211>  19
<212>  RNA
<213>  Artificial Sequence

<220>
<223>  antisense oligonucleotide for siRNA-2

<400>  22
ucucccaccg acucugucu                                                    19

<210> 23
<211> 19
<212> RNA
<213> Artificial Sequence

<220>
<223> sense oligonucleotide for siRNA-3

<400> 23
cggugggaga cgugacuua 19


<210> 24
<211> 19
<212> RNA
<213> Artificial Sequence

<220>
<223> antisense oligonucleotide for siRNA-3

<400> 24
uaagucacgu cucccaccg 19


<210> 25
<211> 25
<212> DNA
<213> Artificial

<220>
<223> primer

<400> 25
aagcttatga aaccccagtt tgttg 25


<210> 26
<211> 24
<212> DNA
<213> Artificial

<220>
<223> primer

<400> 26
tctagactat ccgctccaca gtcc 24


<210> 27
<211> 25
<212> DNA
<213> Artificial

<220>
<223> primer

<400> 27
gccaccatga aaccccagtt tgttg 25


<210> 28
<211> 25
<212> DNA
<213> Artificial

<220>
<223> primer

<400> 28
actgcgatga aaccccagtt tgttg 25


<210> 29
<211> 24
<212> DNA
<213> Artificial

<220>
<223>  primer

<400>  29
tctagactat ccgctccaca gtcc                                              24


<210>  30
<211>  35
<212>  DNA
<213>  Artificial

<220>
<223>  primer

<400>  30
ccagtcacta tggcgtgctc ctagcgctat atgcg                                  35


<210>  31
<211>  35
<212>  DNA
<213>  Artificial

<220>
<223>  primer

<400>  31
cgcatatagc gctaggagca cgccatagtg actgg                                  35


<210>  32
<211>  50
<212>  DNA
<213>  Artificial

<220>
<223>  His-FNdeBam

<400>  32
tatgcatcat catcatcatc acgctagcca tcatcatcat catcactgag                  50


<210>  33
<211>  52
<212>  DNA
<213>  Artificial

<220>
<223>  His-RNdeBam

<400>  33
gatcctcagt gatgatgatg atgatggcta gcgtgatgat gatgatgatg ca               52


<210>  34
<211>  26
<212>  DNA
<213>  Artificial

<220>
<223>  LY6G-FNDE

<400>  34
catatgaacc gaatgcggtg ctacaa                                            26


<210>  35
<211>  26
<212>  DNA
<213>  Artificial

<220>
<223>  LY6G-RNHE

<400>  35
gctagcgttg cacaggtctc ccaggt                                          26


<210>  36
<211>  66
<212>  DNA
<213>  Artificial

<220>
<223>  HAT-F

<400>  36
ctagcaagga tcatctcatc cacaatgtcc acaaagagga gcacgctcat gcccacaaca      60

agtgag                                                                66


<210>  37
<211>  66
<212>  DNA
<213>  Artificial

<220>
<223>  HAT-R

<400>  37
gatcctcact tgttgtgggc atgagcgtgc tcctctttgt ggacattgtg gatgagatga      60

tccttg


<210>  38
<211>  19
<212>  RNA
<213>  Artificial

<220>
<223>  siRNA1

<400>  38
cccagaaccu cgugaguga                                                  19


<210>  39
<211>  19
<212>  RNA
<213>  Artificial

<220>
<223>  siRNA2

<400>  39
gggccaaucu ggagugaaa                                                  19


<210>  40
<211>  27
<212>  DNA
<213>  Artificial

<220>
<223>  primer

<400>  40
gaattcatgc ccggcaagca ccagcat                                         27


<210>  41
<211>  24
<212>  DNA
<213>  Artificial

<220>
<223>  primer

<400> 41
gaagactccc gccaccacaa tcag 24

<210> 42
<211> 25
<212> DNA
<213> Artificial

<220>
<223> primer

<400> 42
cgtgttcctc ggtctcatct tcttc 25

<210> 43
<211> 27
<212> DNA
<213> Artificial

<220>
<223> primer

<400> 43
tctagaccaa tttttaaagg ttggctc 27

<210> 44
<211> 35
<212> DNA
<213> Artificial

<220>
<223> primer

<400> 44
gctcccctct caccagtttg agccggacgt cgtag 35

<210> 45
<211> 35
<212> DNA
<213> Artificial

<220>
<223> primer

<400> 45
aactggtgag aggggagctg gagcagcagg gcttc 35

<210> 46
<211> 39
<212> DNA
<213> Artificial

<220>
<223> primer

<400> 46
aaagacgatg acgacaagcc cggcaagcac cagcatttc 39

<210> 47
<211> 33
<212> DNA
<213> Artificial

<220>
<223> primer

<400> 47
atgtctagac taccaatttt taaaggttgg ctc 33

<210>  48
<211>  36
<212>  DNA
<213>  Artificial

<220>
<223>  primer

<400>  48
gccgccacca tggactacaa agacgatgac gacaag                                36


<210>  49
<211>  44
<212>  DNA
<213>  Artificial

<220>
<223>  primer

<400>  49
atgtctagat cattccattt gctcgaggtt ctgggccagg caga                       44


<210>  50
<211>  35
<212>  DNA
<213>  Artificial

<220>
<223>  primer

<400>  50
ggcgaattcc cgctcaccat gcccggcaag cacca                                 35


<210>  51
<211>  33
<212>  DNA
<213>  Artificial

<220>
<223>  primer

<400>  51
atgtctagac taccaatttt taaaggttgg ctc                                   33

## Description

### Technical Field

The present invention relates to novel preventive/remedy agents for cancer, agents for promoting the apoptosis in cancer cells, and agents for inhibiting cancer cell growth. Specifically, the present invention relates to preventive/remedy agents for cancer comprising substances that inhibit the expression and/or activities of novel target proteins, agents for promoting the apoptosis in cancer cells, and agents for inhibiting cancer cell growth. The present invention also relates to diagnostic agents for cancer comprising substances that can detect the expression of the target proteins or genes encoding the proteins. Further, the present invention relates to preventive/remedy for cancer using the target proteins or the nucleic acids encoding the proteins, and to a method of screening for the substance that promotes the apoptosis in cancer cells and/or inhibits cell growth.

### Background Art

Rapid advance in molecular biology study has enabled the elucidation of the molecular mechanism of cancer cell growth or malignant alteration. By clarifying the molecular target involved in the mechanism and the function thereof, there has initiated an investigation with a new concept of molecular target-based remedy which leads to a remedy by controlling the function, and developed new molecular target-based medicines such as Herceptin, Gleevec, Iressa, and the like, and thus accomplished certain achievement. However, since the medicines are consistently limited on the effectiveness, it is still an important task to search a novel drug development target molecule for treating cancer.

It is predicted that a cancer could be assessed for its pathological conditions by microarray profiling data for the gene. Actually in leukemia, it is reportedly possible to classify leukemia by gene expression profiles. By clarifying the gene expression profile of each cancerous tissue and accumulating its classification, it is considered possible to predict responsiveness to a particular cancer therapy or discover a novel drug development target protein for a particular cancer. Specifically, where enhanced expression of a certain protein is observed in a certain cancer, it becomes possible to induce an anti-tumor activity in patients newly diagnosed to be antigen positive, by means of (i) reducing its expression level, (ii) suppressing its function, (iii) eliciting immune response of host to the protein, etc. At the same time, patients diagnosed to be antigen negative can immediately switch over to another cancer therapy, assuming to eliminate any concern of imposing a superfluous burden on patients. As such, it is expected that the expression profile analysis would greatly contribute to molecular diagnosis of a cancer and development of molecular target-based drugs.

LY6G6D gene (RefSeq Accession No. NM_021246) is one of the genes assumed to be in the human MHC class III region. The gene is isolated from cDNA of the human monocytic leukemia cell line U937 origin, and encodes a protein comprising 133 amino acids (RefSeq Accession No. NP_067069). Further, a mouse gene (RefSeq Accession No. NM_033478) which is homologous to the LY6G6D gene has been cloned from cDNA of a mouse tissue origin, and encodes a protein comprising 135 amino acids (RefSeq Accession No. NP_258439). The mouse gene has about 77% homology in a base sequence and about 67% homology in an amino acid sequence, to LY6G6D gene. LY6G6D is a molecular which belongs to LY-6 family and expected to be localized on a cell membrane via bonding with GPI.

LY6G6D gene is reported as one of the useful proteins for the diagnosis of cancers (Publication No. WO 2003/024392) and a protein useful for the diagnosis of colon cancer (Publication Nos. WO 2003/083074 and WO 2004/046342). However, in these patent documents, any of the LY6G6D genes are assumed to encode protein comprising 382 amino acids, and the existence and usefulness of those proteins are only analogized from a gene expression analysis, thus a particular case proving the assumption has not yet been clarified.

PTPRO (Protein tyrosine phosphatase receptor type O) gene is the gene which, in 1995, was isolated from a cDNA library of human kidney origin. Thereafter, there are reported six variants (Refseq Accession No. NM_002848, 030667, 030668, 030669, 030670, 030671) as the PTPRO gene, and four isoforms of isoform a comprising 1216 amino acids (Refseq Accession No. NP_109592), isoform b comprising 1118 amino acids (Refseq Accession No. NP_002839), isoform c comprising 405 amino acids (Refseq Accession No. NP_109594, 109596), and isoform d comprising 377 amino acids (Refseq Accession No. NP_109593, 109595) as a protein. Although the PTPRO protein varies in the length of the part which is assumed to be an extracellular domain comprising Fibronectin type III motif via isoform and varies in the presence of repeated 28 amino acids which is assumed to be present in an intracellular domain, the PTPRO protein is expected to be a receptor-type tyrosine phosphatase comprising, commonly, a single cell membrane-spanning site and a single tyrosine phosphatase active site. The function of the member of a receptor-type tyrosine phosphatase family is reported by one research group that as PTPα, one molecule participates in promoting carcinogenesis, and also reported by another research group that the molecule participates in inhibiting carcinogenesis. Thus the function has not uniformly concluded yet.

About PTPRO, there are reports such as suggesting that the PTPRO induces an apoptosis on the occasion of physiological turnover of hemopoietic cell, suggesting that it controls the growth by promoting G0/G1 arrest in growth process of B cell, and that the gene expression in a lung cancer is reduced and it may act as a cancer inhibiting factor (PNAS (2004), 101: 13844-13849). In a prior patent, It is reported that PTPRO is expressed more in bowel cancer and uterine cancer than in a normal tissue, and that it is one of genes useful for a diagnosis and a treatment (Publication WO 2,003/057,160). However, the usefulness thereof is only analogized from a gene expression analysis, thus a particular case proving the assumption has not yet been clarified.

A TM4SF17 gene is a gene partly found from an EST library by applying a high homology to a sequence of the gene interacting with a Skp1 gene by using a yeast two-hybrid screening method as an indicator (Winston et al, Current Biology, 9, 20, 1180-1182). The TM4SF17 gene includes a TM4SF17a gene (Refseq Accession No. NM_012171); a TM4SF17b gene (RefSeq Accession No. NM_130465) and a TM4SF17c gene (RefSeq Accession No. NM_001006616) which are the splicing variants thereof, and encodes proteins each comprising 332 amino acids (Refseq Accession No. NP_036303), 329 amino acids (Refseq Accession No. NP_569732), and 266 amino acids (RefseqAccession No. NP_001006617). Other than these TM4SF17a, TM4SF17b, and TM4SF17c, a splicing variant is also known, and in the present specification, it is referred to as TM4SF17d (swissprot Accession No. Q96FV3) (hereinafter, TM4SF17a to TM4SF17 d genes and their novel variants discovered were may be generically named as a TM4SF17 gene) . In addition, a mouse gene homologous to the TM4SF17 gene (RefSeq Accession No. NM_028841) is cloned from cDNA of a mouse tissue origin, and encodes a protein comprising 270 amino acids (RefSeq Accession No. NP_083117). This mouse gene has about 91% homology in a base sequence and about 96% homology in an amino acid sequence, to the TM4SF17a gene. The TM4SF17 gene has another name of FBX 23, and belongs to a tetraspanin super family which is the four-transmembrane type molecular family and expected to be localized on a cell membrane.

The TM4SF17 gene is reported as one of the useful proteins for the diagnosis of cancers (Publication Nos. WO 2000/56891, WO 2001/77173). However, the usefulness thereof is only analogized from a gene expression analysis, thus a particular case proving the assumption has not yet been clarified.

### Disclosure of the Invention

As described above, a safe novel medicine which leads to a growth inhibition of cancer cells by targeting the molecule specifically expressed in a cancer cell is desired. In a cancer cell, many genes of which the expression significantly varies is known, but there are still many to be elucidated to find among them, which gene and expression product thereof can be the drug development target for treating cancer. Accordingly, the object of the invention is to identify a molecule which can be the novel target of a cancer molecular target-based treatment, to provide a preventive/remedy approach for cancer by controlling the expression or function of the target molecule, and to provide a method of screening for the substance having cancer preventive/remedy activity with the use of the target molecule.

In order to solve the foregoing problems, the present inventors made extensive investigations and as a result, discovered a gene showing a significantly increased expression in cancer tissue. Further, they found that siRNA for the gene inhibits a cancer cell growth, thus proved that the gene can be the target for a cancer preventive/remedy. Further investigation was carried out based on these findings, thereby the inventors completed the invention.

That is, the invention provides
(1) An agent for promoting the apoptosis in cancer cells and/or inhibiting the growth of cancer cells, which comprises an antibody against a protein comprising the same or substantially the same amino acid sequence as the amino acid sequence represented by SEQ ID NO: 2, or against partial peptide thereof,
(2) The agent according to (1) above, which is for a preventive/remedy for cancer,
(3) An agent for promoting the apoptosis in cancer cells and/or inhibiting the growth of cancer cells, which comprises an antisense polynucleotide comprising a base sequence or a part of the base sequence, wherein the base sequence is complimentary or substantially complimentary to a base sequence of polynucleotide encoding a protein which includes the same or substantially the same amino acid sequence as the amino acid sequence represented by SEQ ID NO: 2,
(4) The agent according to (3) above, which is for a preventive/remedy for cancer,
(5) An agent for promoting the apoptosis in cancer cells and/or inhibiting the growth of cancer cells, which comprises a substance that inhibits the expression and/or activity of a protein comprising the same or substantially the same amino acid sequence as the amino acid sequence represented by SEQ ID NO: 2,
(6) The agent according to (5) above, which is for a preventive/remedy for cancer,
(7) A method of promoting the apoptosis in cancer cells and/or inhibiting the growth of cancer cells, the method comprises inhibiting the expression and/or activity of a protein comprising the same or substantially the same amino acid sequence as the amino acid sequence represented by SEQ ID NO: 2,
(8) The method according to (7) above, which is for a preventive/remedy for cancer,
(9) Use of a substance that inhibits the expression and/or activity of a protein comprising the same or substantially the same amino acid sequence as the amino acid sequence represented by SEQ ID NO: 2, to manufacture an agent for promoting the apoptosis in cancer cells and/or inhibiting the growth of cancer cells,
(10) The use according to (9) above, wherein the agent for promoting the apoptosis in cancer cells and/or inhibiting the growth of cancer cells is for a preventive/remedy for cancer,
(11) A method of screening for a substance that promotes the apoptosis in cancer cells and/or inhibits the growth of cancer cells, the method includes using a protein comprising the same or substantially the same amino acid sequence as the amino acid sequence represented by SEQ ID NO: 2, or partial peptide thereof,
(12) The method according to (11) above, wherein the protein comprising the same or substantially the same amino acid sequence as the amino acid sequence represented by SEQ ID NO: 2, or partial peptide thereof, is provided in the form of a cell capable of producing the protein or the partial peptide,
(13) The method according to (12) above, comprises using further any one selected from a group consisting of antibodies against a protein comprising the same or substantially the same amino acid sequence as the amino acid sequence represented by SEQ ID NO: 2, or against partial peptide thereof, and polynucleotide encoding the protein or polynucleotide including a part of the base sequence,
(14) The method according to (11) above, which is for the screening for a preventive/remedy substance for cancer,
(15) An agent for promoting the apoptosis in cancer cells and/or inhibiting the growth of cancer cells, which comprises an antibody against a protein comprising the same or substantially the same amino acid sequence as the amino acid sequence represented by SEQ ID NO: 6, SEQ ID NO: 8, SEQ ID NO: 10, SEQ ID NO: 12, SEQ ID NO: 14, SEQ ID NO: 16, or SEQ ID NO: 18, or against partial peptide thereof,
(16) The agent according to (15) above, which is for a preventive/remedy for cancer,
(17) An agent for promoting the apoptosis in cancer cells and/or inhibiting the growth of cancer cells, which comprises an antisense polynucleotide comprising a base sequence or a part of the base sequence, wherein the base sequence is complimentary or substantially complimentary to a base sequence of polynucleotide encoding a protein which includes the same or substantially the same amino acid sequence as the amino acid sequence represented by SEQ ID NO: 6, SEQ ID NO: 8, SEQ ID NO: 10, SEQ ID NO: 12, SEQ ID NO: 14, SEQ ID NO: 16, or SEQ ID NO: 18,
(18) The agent according to (17) above, which is for a preventive/remedy for cancer,
(19) An agent for promoting the apoptosis in cancer cells and/or inhibiting the growth of cancer cells, which comprises a substance that inhibits the expression and/or activity of a protein comprising the same or substantially the same amino acid sequence as the amino acid sequence represented by SEQ ID NO: 6, SEQ ID NO: 8, SEQ ID NO: 10; SEQ ID NO: 12, SEQ ID NO: 14, SEQ ID NO: 16, or SEQ ID NO: 18,
(20) The agent according to (19) above, which is for a preventive/remedy for cancer,
(21) A method of promoting the apoptosis in cancer cells and/or inhibiting the growth of cancer cells, the method comprises inhibiting the expression and/or activity of a protein comprising the same or substantially the same amino acid sequence as the amino acid sequence represented by SEQ ID NO: 6, SEQ ID NO: 8, SEQ ID NO: 10, SEQ ID NO: 12, SEQ ID NO: 14, SEQ ID NO: 16, or SEQ ID NO: 18,
(22) The method according to (21) above, which is for a preventive/remedy for cancer,
(23) Use of a substance that inhibits the expression and/or activity of a protein comprising the same or substantially the same amino acid sequence as the amino acid sequence represented by SEQ ID NO: 6, SEQ ID NO: 8, SEQ ID NO: 10, SEQ ID NO: 12, SEQ ID NO: 14, SEQ ID NO: 16, or SEQ ID NO: 18, to manufacture an agent for promoting the apoptosis in cancer cells and/or inhibiting the growth of cancer cells,
(24) The use according to (23) above, wherein the agent for promoting the apoptosis in cancer cells and/or inhibiting the growth of cancer cells is for a preventive/remedy for cancer,
(25) A method of screening for a substance that promotes the apoptosis in cancer cells and/or inhibits the growth of cancer cells, the method comprises using a protein comprising the same or substantially the same amino acid sequence as the amino acid sequence represented by SEQ ID NO: 6, SEQ ID NO: 8, SEQ ID NO: 10, SEQ ID NO: 12, SEQ ID NO: 14, SEQ ID NO: 16, or SEQ ID NO: 18, or partial peptide thereof,
(26) A method according to (25) above, wherein the protein comprising the same or substantially the same amino acid sequence as the amino acid sequence represented by SEQ ID NO: 6, SEQ ID NO: 8, SEQ ID NO: 10, SEQ ID NO: 12, SEQ ID NO: 14, SEQ ID NO: 16, or SEQ ID NO: 18, or partial peptide thereof, is provided in the form of a cell capable of producing the protein or the partial peptide,
(27) The method according to (26) above, comprises using further any one selected from a group consisting of antibodies against a protein comprising the same or substantially the same amino acid sequence as the amino acid sequence represented by SEQ ID NO: 6, SEQ ID NO: 8, SEQ ID NO: 10, SEQ ID NO: 12, SEQ ID NO: 14, SEQ ID NO: 16, or SEQ ID NO: 18, or against partial peptide thereof, and polynucleotide encoding the protein or polynucleotide including a part of the base sequence,
(28) The method according to (25) above, which is for the screening for a preventive/remedy substance for cancer,
(29) An agent for promoting the apoptosis in cancer cells and/or inhibiting the growth of cancer cells, which comprises an antibody against a protein comprising the same or substantially the same amino acid sequence as the amino acid sequence represented by SEQ ID NO: 4, or against partial peptide thereof,
(30) The agent according to (29) above, which is for a preventive/remedy for cancer,
(31) An agent for promoting the apoptosis in cancer cells and/or inhibiting the growth of cancer cells, which comprises an antisense polynucleotide comprising a base sequence or a part of the base sequence, wherein the base sequence is complimentary or substantially complimentary to a base sequence of polynucleotide encoding a protein which includes the same or substantially the same amino acid sequence as the amino acid sequence represented by SEQ ID NO: 4,
(32) The agent according to (31) above, which is for a preventive/remedy for cancer,
(33) An agent for promoting the apoptosis in cancer cells and/or inhibiting the growth of cancer cells, which comprises a substance that inhibits the expression and/or activity of a protein comprising the same or substantially the same amino acid sequence as the amino acid sequence represented by SEQ ID NO: 4,
(34) The agent according to (33) above, which is for a preventive/remedy for cancer,
(35) A method of promoting the apoptosis in cancer cells and/or inhibiting the growth of cancer cells, the method comprises inhibiting the expression and/or activity of a protein comprising the same or substantially the same amino acid sequence as the amino acid sequence represented by SEQ ID NO: 4,
(36) The method according to (35) above, which is for a preventive/remedy for cancer,
(37) Use of a substance that inhibits the expression and/or activity of a protein comprising the same or substantially the same amino acid sequence as the amino acid sequence represented by SEQ ID NO: 4, to manufacture an agent for promoting the apoptosis in cancer cells and/or inhibiting the growth of cancer cells,
(38) The use according to (37) above, wherein the agent for promoting the apoptosis in cancer cells and/or inhibiting the growth of cancer cells is for a preventive/remedy for cancer,
(39) A method of screening for a substance that promotes the apoptosis in cancer cells and/or inhibits the growth of cancer cells, the method comprises using a protein comprising the same or substantially the same amino acid sequence as the amino acid sequence represented by SEQ ID NO: 4, or partial peptide thereof,
(40) The method according to (39) above, wherein the protein comprising the same or substantially the same amino acid sequence as the amino acid sequence represented by SEQ ID NO: 4, or partial peptide thereof, is provided in the form of a cell capable of producing the protein or the partial peptide,
(41) The method according to (40) above, comprises using further any one selected from a group consisting of antibodies against a protein comprising the same or substantially the same amino acid sequence as the amino acid sequence represented by SEQ ID NO: 4, or against partial peptide thereof, and polynucleotide encoding the protein or polynucleotide including a part of the base sequence,
(42) The method according to (39) above, which is for the screening for a preventive/remedy substance for cancer, and the like.

The protein comprising the same or substantially the same amino acid sequence as the amino acid sequence represented by SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, or SEQ ID NO: 18, and the polynucleotide encoding the protein are specifically expressed in cancer tissue, thus can be a diagnosis marker for cancer. Further, an antibody against the protein, an antisense polynucleotide to the polynucleotide, and a substance that controls the expression and/or activity of the protein, can be safely used, for example, as preventive/remedy agents for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.) (preferably as preventive/remedy agents for breast cancer, lung cancer, colon cancer, prostate cancer, ovary cancer, pancreatic cancer, etc.), agents for promoting apoptosis in cancer cells, agents for inhibiting cancer cell growth, or the like. Further, the above-mentioned protein, polynucleotide, and antibody and the like are useful for screening for a preventive/remedy substance for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.) (preferably for a preventive/remedy substance for breast cancer, lung cancer, colon cancer, prostate cancer, ovary cancer, pancreatic cancer, etc.), a substance promoting the apoptosis in cancer cells, a substance inhibiting the growth of cancer cells, or the like.

### Brief Description of the Drawings

Fig. 1 shows a gene expression profile of LY6G6D prepared by using various cancer tissue origin mRNAs and neighboring normal tissue origin mRNAs.
FIG. 2 shows a result of administering siRNA for a LY6G6D gene in LY6G6D gene expression cell line SW480. Fig 2A shows that the cell growth of human lung cancer cell line SW480 is inhibited by administering the siRNA; and Fig 2B shows that the level of mRNA expression of the LY6G6D gene is reduced by administering the siRNA.
Fig. 3 shows a result of administering siRNA for a LY6G6D gene in LY6G6D gene expression cell line COLO 320DM. Fig 3A shows that the cell growth of human lung cancer cell line COLO 320DM is inhibited by administering the siRNA; and Fig 3B shows that the level of mRNA expression of the LY6G6D gene is reduced by administering the siRNA.
FIG. 4 shows a result of administering siRNA for a LY6G6D gene in cell line HCT116 which does not express a LY6G6D gene. Fig 4A shows that the cell growth of human lung cancer cell line HCT116 is not inhibited by administering the siRNA; and Fig 4B shows the efficiency of transfecting siRNA by the expression level of mRNA of GAPDH gene.
Fig. 5 shows the effect on the cell growth and signal transduction pathway when LY6G6D is transiently expressed. Fig 5A shows that the cell growth is promoted by expressing a LY6G6D protein. Fig. 5B shows that the cell growth-promoting effect due to the LY6G6D protein expression is inhibited by a PI3K inhibiting agent. Fig. 5C shows that the ability of ATK phosphorylation is increased by expressing the LY6G6D protein.
Fig. 6 shows that in LY6G6D stable expression cell line, a LY6G6D protein is localized on a surface layer of a cell membrane via GPI. Fig. 6A shows the analysis result using FACScan. Fig. 6B shows the western blot analysis result.
FIG. 7 shows that in LY6G6D stable expression cell line, the LY6G6D protein undergoes sugar chain modification.
Fig. 8 shows that the LY6G6D protein is reduced by administering siRNA for LY6G6D gene in LY6G6D gene expression cell line COLO 320DM and SW480.
FIG. 9 shows that the LY6G6D protein is expressed in LY6G6D gene expression human bowel cancer cell line.
Fig. 10 shows that the growth rate of LY6G6D stable expression cell line in plate culturing shows a positive correlation with the expression level of the LY6G6D protein.
FIG. 11 shows that in a cell staining technique using LY6G6D stable expression cell line, the LY6G6D protein is localized on a cell membrane.
Fig. 12 shows that the respiratory activity of mitochondria of LY6G6D stable expression cell line in three-dimensional culturing shows a positive correlation with the expression level of the LY6G6D protein.
FIG. 13 shows an expression profile of the LY6G6D protein in human normal tissue or in human bowel cancer tissue.
Fig. 14 shows a gene expression profile of TM4SF17 prepared by using various cancer tissue origin mRNAs and neighboring normal tissue origin mRNAs.
Fig. 15 shows that the TM4SF17 protein undergoes post-translational modification.
FIG. 16 shows that recombinant TM4SF17 b-3xFLAG, FLAG-b, and FLAG-d proteins undergo sugar chain modification.
FIG. 17 shows that TM4SF17 isoform a and b proteins are localized on a cell membrane.
Fig. 18 shows schematically the comparison between the amino acid sequences of TM4SF17 isoform e, isoform f, and isoform h proteins, cDNA sequences encoding thereof, and other splice variants.

### Best Mode for Carrying Out the Invention

A protein used in the present invention is a protein comprising the same or substantially the same amino acid sequence as the amino acid sequence represented by SEQ ID NO: 2, SEQ ID NO: 4, SEQ ID NO: 6, SEQ ID NO: 8, SEQ ID NO: 10, SEQ 10 NO: 12, SEQ ID NO: 14, SEQ ID NO: 16, or SEQ 10 NO: 18 (hereinafter these proteins are sometimes referred to as the protein of the present invention). The protein of the present invention may be any protein isolated and purified from cells [for example, hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary cells, or interstitial cells; or the corresponding precursor cells, stem cells, cancer cells, etc.] of human and other warm-blooded animals (e.g., guinea pig, rat, mouse, chicken, rabbit, swine, sheep, bovine, simian, etc.); or any tissue where such cells are present [for example, brain or each part of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle (e.g., smooth muscle, skeletal muscle), lung, gastrointestinal tract (e.g., large intestine and small intestine), blood vessel, heart, thymus, spleen, submandibular gland, peripheral blood, prostate, testis, ovary, placenta, uterus, bone, joint, adipose tissue (e.g., white adipose tissue, brown adipose tissue), etc.]. The protein may also be a protein biochemically synthesized by a chemical synthesis or cell-free translation system, or a recombinant protein produced from a transfectant in which the nucleic acid comprising a base sequence encoding the above-mentioned amino acid sequence is transfected.

The amino acid sequence which is substantially the same amino acid sequence as that represented by SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16 or SEQ ID NO: 18, includes amino acid sequences having about 50% or more homology, preferably about 60% or more homology, more preferably about 70% or more homology, even more preferably about 80% or more homology, particularly preferably about 90% or more homology and most preferably about 95% or more homology, to the amino acid sequence shown by SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16 or SEQ ID NO: 18,etc. Herein, the 'Homology' means a ratio (%) of the same amino acid and similar amino acid residue to the total overlapped amino acid residue, in the best alignment when two amino acid sequences are aligned with the use of a mathematical algorithm commonly known in the technical field (preferably, the algorithm considers introduction of gaps on one or both side of the sequence for the best alignment). The term 'similar amino acid' refers to an amino acid similar in its physiochemical properties, and Examples include amino acids classified in a same group such as aromatic amino acid (Phe, Trp, Tyr), aliphatic amino acid (Ala, Leu, Ile, Val), polar amino acid (Gln, Asn), basic amino acid (Lys, Arg, His), acidic amino acid (Glu, Asp), amino acid including a hydroxyl group (Ser, Thr), amino acid having a short side chain(Gly, Ala, Ser, Thr, Met), and the like. A substitution by such similar amino acid is expected to give no change in the phenotype of protein (thus is a conservative amino acid substitution). A specific example of the conservative amino acid substitution is well-known in the technical field, and is disclosed in various documents (for example, refer Bowie et al, Science, 247: 1306-1310 (1990)).

Homology of the amino acid sequences in the present specification can be calculated under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool). Other algorithm for determining homology of the amino acid sequence is exemplified by an algorithm disclosed in Karlin et al, Proc. Natl. Acad. Sci. USA, 90: 5873-5877 (1993) [this algorithm is incorporated in NBLAST and XBLAST program (version 2.0) (Altschul et al, Nucleic Acids Res., 25:3389-3402(1997))]; an algorithm disclosed in Needleman et al, J. Mol. Biol., 48: 444-453 (1970) [This algorithm is incorporated in a GAP program in a GCG software package] ; an algorithm disclosed in Myers and Miller, CABIOS, 4: 11-17 (1988) [This algorithm is incorporated in ALIGN program (version 2.0) which is a part of a CGC sequence alignment software package] ; an algorithm disclosed in Pearson et al. Proc. Natl. Acad. Sci. USA, 85: 2444-2448 (1998) [This algorithm is incorporated in an FASTA program in a GCG software package], etc., and these may be also preferably used.

More preferably, the amino acid sequence which is substantially the same amino acid sequence as that represented by SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16 or SEQ ID NO: 18, includes amino acid sequences having about 50% or more identity, preferably about 60% or more identity, more preferably about 70% or more identity, even more preferably about 80% or more identity, particularly preferably about 90% or more identity and most preferably about 95% or more identity, to the amino acid sequence shown by SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16 or SEQ ID NO: 18.

The protein used in the present invention is a protein comprising substantially the same amino acid sequence as the amino acid sequence represented by SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16 or SEQ ID NO: 18, and having an activity substantially equivalent to the protein comprising the amino acid sequence represented by SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16 or SEQ ID NO: 18.

As the substantially equivalent activity described above, there are, for example, the ligand binding activity, and signal transduction, and the like. The substantially equivalent is used to mean that the nature of the activities is equivalent in terms of quality (e.g., physiologically or pharmacologically). Thus, the activities of the protein of the present invention are preferably equivalent, but differences in quantitative factors such as a level of these activities (e.g., about 0.01 to 100 times, preferably about 0.1 to 10 times, more preferably 0.5 to 2 times), a molecular weight of the protein, and the like may be present and allowable.

The activity such as the ligand binding activity and signal transduction can be assayed by publicly known methods, for example, it can be assayed by the method to be described later , comprises screening for a compound that inhibits the activity of the protein used in the invention, or salts thereof.

Examples of the protein used in the present invention include so-called muteins such as proteins having (i) the amino acid sequence represented by SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16 or SEQ ID NO: 18, of which 1 or 2 or more (e.g., about 1 to about 50, preferably about 1 to about 30, more preferably about 1 to about 10 and much more preferably several (1 to 5, 4, 3 or 2)) amino acids are deleted; (ii) the amino acid sequence represented by SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16 or SEQ ID NO: 18, to which 1 or 2 or more (e.g., about 1 to about 50, preferably about 1 to about 30, more preferably about 1 to about 10 and much more preferably several (1 to 5, 4, 3 or 2)) amino acids are added; (iii) the amino acid sequence represented by SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16 or SEQ ID NO: 18, in which 1 or 2 or more (e.g., about 1 to about 50, preferably about 1 to about 30, more preferably about 1 to about 10 and much more preferably several (1 to 5, 4, 3 or 2)) amino acids are inserted; (iv) the amino acid sequence represented by SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16 or SEQ ID NO: 18, in which 1 or 2 or more (e.g., about 1 to about 50, preferably about 1 to about 30, more preferably about 1 to about 10 and much more preferably several (1 to 5, 4, 3 or 2)) amino acids are substituted by other amino acids; or (v) a combination of these amino acid sequences; and the like.

Where the amino acid sequence is inserted, deleted or substituted as described above, the position of its insertion, deletion, or substitution is not particularly limited.

Preferable examples of the protein used in the present invention include a protein comprising the amino acid sequence represented by SEQ ID NO: 2 (Refseq Accession No. NP_067069.2) or the homolog thereof (for example, mouse homolog registered as RefSeq Accession No. NP_258439.1 in GenBank) in other mammals, a protein comprising the amino acid sequence represented by SEQ ID NO: 4 (Refseq Accession No. NP_109592.1) or the homolog thereof (for example, mouse homolog registered as RefSeq Accession No. NP_035346.2 in GenBank) in other mammals, a protein comprising the amino acid sequence represented by SEQ ID NO: 6 (RefSeq Accession No. NP_036303) or the homolog thereof (for example, mouse homolog registered as RefSeq Accession No. NP_083117 in GenBank) in other mammals, a protein comprising the amino acid sequence represented by SEQ ID NO: 8 (RefSeq Accession No. NP_569732) or the homolog thereof in other mammals, a protein comprising the amino acid sequence represented by SEQ ID NO: 10 (RefSeq Accession No. NP_001006617) or the homolog thereof in other mammals, a protein comprising the amino acid sequence represented by SEQ ID NO: 12 (Swissprot Accession No. Q96FV3) or the homolog thereof in other mammals, a protein comprising the amino acid sequence represented by SEQ ID NO: 14 or the homolog thereof in other mammals, a protein comprising the amino acid sequence represented by SEQ ID NO: 16 or the homolog thereof in other mammals, and a protein comprising the amino acid sequence represented by SEQ ID NO: 18 or the homolog thereof in other mammals, and the like.

In the present specification, the proteins and peptides are represented in accordance with the conventional way of describing peptides, that is, the N-terminus (amino terminus) at the left hand and the C-terminus (carboxyl terminus) at the right hand. In the protein used in the present invention which includes the protein comprising the amino acid sequence represented by SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16, or SEQ ID NO: 18, the C-terminus may be in any form of a carboxyl group (-COOH), a carboxylate (-COO⁻), an amide (-CONH₂) or an ester (-COOR).

Herein, examples of R in the ester include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl such as a phenyl-C₁₋₂ alkyl group, e.g., benzyl, phenethyl, etc.; or α-naphthyl-C₁₋₂ alkyl group such as α-naphthylmethyl, etc.; pivaloyloxymethyl group and the like.

Where the protein used in the present invention contains a carboxyl group (or a carboxylate) at a position other than the C-terminus, the carboxyl group may be amidated or esterified and such an amide or ester is also included within the protein used in the present invention. Examples of the ester in this case may be the C-terminal esters described above, etc.

Furthermore, examples of the protein used in the present invention also include those wherein the amino group at the N-terminal amino acid residues (e.g., methionine residue) is protected with a protecting group (e.g., a C₁₋₆ acyl group such as a C₁₋₆ alkanoyl, e.g., formyl group, acetyl group, etc.); those wherein the N-terminal region is cleaved in vivo and the glutamine residue thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g. , a C₁₋₆ acyl group such as a C₁₋₆ alkanoyl group, e.g., formyl group, acetyl group, etc.), or conjugated proteins such as so-called glycoproteins having sugar chains; etc.

The partial peptide of the protein used in the present invention may be any peptide as long as it is a peptide having a partial amino acid sequence of the protein used in the present invention described above and has the activity substantially equivalent to that of the protein used in the present invention described above. Herein, the 'activity substantially equivalent' means the same as mentioned above. The 'activity substantially equivalent' can also be assayed in a same manner as in the case of protein used in the present invention as above mentioned.

For example, there are used peptides containing, e.g., at least 20 or more, preferably 50 or more, more preferably 70 or more, much more preferably 100 or more and most preferably 150 or more amino acids, in the constituent amino acid sequence of the protein used in the present invention, etc.

The partial peptide used in the present invention may be peptides containing the amino acid sequence, of which 1 or 2 or more (preferably about 1 to about 20, more preferably about 1 to about 10 and much more preferably several (1 to 5, 4, 3 or 2)) amino acids may be deleted; to which 1 or 2 or more (preferably about 1 to about 20, more preferably about 1 to about 10 and much more preferably several (1 to 5, 4, 3 or 2)) amino acids may be added; in which 1 or 2 or more (preferably about 1 to about 20, more preferably about 1 to about 10 and much more preferably several (1 to 5, 4, 3 or 2)) amino acids may be inserted; or in which 1 or 2 or more (preferably about 1 to about 20, more preferably several and much more preferably sevsral (1 to 5, 4, 3 or 2) amino acids may be substituted by other amino acids.

In the partial peptide used in the present invention, the C-terminus may be in any form of a carboxyl group (-COOH), a carboxylate (-COO⁻), an amide (-CONH₂) or an ester (-COOR). Herein, as the 'R' in ester, same ones as in the protein used in the present invention can be exemplified. Where the partial peptide of the present invention contains a carboxyl group (or a carboxylate) at a position other than the C-terminus, the carboxyl group may be amidated or esterified and such an amide or ester is also included within the partial peptide of the present invention. Examples of the ester in this case may be the same as C-terminal esters described above, etc.

Furthermore, the partial peptide used in the present invention also includes those wherein the amino group at the N-terminal amino acid residues (e.g., methionine residue) is protected with a protecting group; those wherein the N-terminal region is cleaved in vivo and the glutamine residue thus formed is pyroglutaminated; those wherein a substituent on the side chain of an amino acid in the molecule is protected with a suitable protecting group, or conjugated peptides such as so-called glycopeptides having sugar chains; etc., as in the protein used in the present invention described above.

The partial peptide used in the present invention may also be used as an antigen for producing antibodies.

The protein, or its partial peptide used in the present invention, may be a free form, or salt form (it is true throughout the present specification unless otherwise specified). As the salts, salts with physiologically acceptable acids (e.g., inorganic acids, organic acids, etc.) or bases (e.g., alkali metal salts, etc.), preferably physiologically acceptable acid addition salts can be used. Examples of such salts include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein used in the present invention may be manufactured by publicly known methods used to purify a protein from human or warm-blooded animal cells or tissues described above. Specifically, mammalian tissues or cells are homogenized in the presence of surfactant, and then crude tissue extract fraction obtained is subject to chromatography techniques such as reverse phase chromatography, ion exchange chromatography, affinity chromatography and the like, thereby the proteins used in the present invention can be prepared.

The protein used in the present invention or its partial peptide can be manufactured by publicly known methods for peptide synthesis.

For the methods for peptide synthesis, for example, either solid phase synthesis or liquid phase synthesis may be used. That is, the partial peptide or amino acids that can constitute the protein or its partial peptide used in the present invention are condensed with the remaining part. Where the product contains protecting groups, these protecting groups are removed to give the desired protein (peptide). Publicly known methods for condensation and elimination of the protecting groups are, for example, described in (i) to (v) below.
(i) M. Bodanszky & M.A. Ondetti: Peptide Synthesis, Interscience Publishers, New York (1966)
(ii) Schroeder & Luebke: The Peptide, Academic Press, New York (1965)
(iii) Nobuo Izumiya, et al.: Peptide Gosei-no-Kiso to Jikken (Basics and experiments of peptide synthesis), published by Maruzen Co. (1975)
(iv) Haruaki Yaj ima & Shunpei Sakakibara: Seikagaku Jikken Koza (Biochemical Experiment) 1, Tanpakushitsu no Kagaku (Chemistry of Proteins) IV, 205 (1977)
(v) Haruaki Yajima ed.: Zoku Iyakuhin no Kaihatsu (A sequel to Development of Pharmaceuticals), Vol. 14, Peptide Synthesis, published by Hirokawa Shoten

The protein (peptide) obtained in the above manner may be purified and isolated by a known purification methods such as solvent extraction, distillation, column chromatography, liquid chromatography and recrystallization.

When the partial peptide obtained by the above methods is in a free form, it can be converted into an appropriate salt by a publicly known method or its modification; conversely when the partial peptide is obtained in a salt form, it can be converted into a free form or other different salt form by a publicly known method or its modification.

To synthesize the protein or its partial peptide used in the present invention, or amides thereof, commercially available resins that are used for protein synthesis may be usually used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids, in which α-amino groups and functional groups on the side chains are appropriately protected, are condensed on the resin in accordance with the sequence of the objective protein according to various condensation methods publicly known in the art. At the end of the reaction, the protein or partial peptide is excised from the resin and at the same time, the protecting groups are removed. Then, intramolecular disulfide bond-forming reaction is performed in a highly diluted solution to obtain the objective protein or partial peptide, or amides thereof.

For condensation of the protected amino acids described above, a variety of activation reagents available for protein synthesis may be used, and carbodiimides are particularly preferable. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) may be added directly to the resin, or the protected amino acids may be previously activated in the form of symmetric acid anhydrides, HOBt esters or HOOBt esters, followed by adding the thus activated protected amino acids to the resin.

Solvents for use to activate the protected amino acids or condense them with the resin may be appropriately chosen from solvents that are known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; or appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to avoid any possible effect on the subsequent reaction.

Protection of the functional groups that should not be involved in the reaction of the starting materials, protecting groups, elimination of the protecting groups, activation of the functional groups involved in the reaction, and the like may be appropriately chosen from publicly known groups and publicly known means.

Examples of the protecting groups used to protect the starting amino groups include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl, e.g., methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower (C₁₋₆) alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, Cl₂-Bzl, 2-nitrobenzyl, Br-Z, t-butyl, etc.

Examples of groups used to protect the imidazole moiety of histidine include Tos, 4-methoxy-2,3,6-trimethyl-benzenesulfonyl, DNP, benzyloxymethyl, Bum, Boc, Trt, Fmoc, etc.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid, trifluoroacetic acid, or a mixture solution of these acids; a treatment with a base such as diisopropylethylamine, triethylamine, piperidine or piperazine; reduction with sodium in liquid ammonia, etc. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2, 4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Examples of the activated carboxyl groups in the starting material include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)]. As example of the activated amino groups in the starting material, the corresponding phosphoric amides are employed.

In another method for obtaining the amides of the desired protein or partial peptide, for example, the α-carboxyl group of the carboxy terminal amino acid is first protected by amidation; the peptide (protein) chain is then extended from the amino group side to a desired length. Subsequently, a protein or partial peptide, in which only the protecting group of the N-terminal α-amino group of the peptide chain has been eliminated, and a protein or partial peptide, in which only the protecting group of the C-terminal carboxyl group has been eliminated, are manufactured. The two proteins or peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To prepare the esterified protein or peptide, for example, the α-carboxyl group of the carboxy terminal amino acid is condensed with a desired alcohol to prepare the amino acid ester, which is followed by procedures similar to the preparation of the amidated protein or peptide above to give the desired esterified protein or peptide.

The partial peptide of the protein used in the present invention can be manufactured by cleaving the protein used in the present invention with an appropriate peptidase.

Further, the protein used in the present invention or partial peptides thereof can also be manufactured by culturing a transformant containing polynucleotides encoding the same and then by separating and purifying the protein or partial peptide from the obtained culture. The polynucleotide encoding the protein used in the present invention or its partial peptide may be DNA or RNA, or DNA/RNA chimera, and preferably is DNA. In addition, the polynucleotide may be a double-strand, or single-strand. The double-strand may include a double-stranded DNA, a double-stranded RNA, and a DNA:RNA hybrid. The single-strand may include a sense strand (that is, coding strand) and an antisense strand (that is, non-coding strand).

The polynucleotide encoding the protein used in the present invention or its partial peptide can be exemplified by genomic DNA, genomic DNA library, cDNA derived from any mammalian (e.g., human, bovine, simian, horse, swine, sheep, goat, canine, feline, guinea pig, rat, mouse, rabbit, hamster, etc.) cells [for example, hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary cells, or interstitial cells; or the corresponding precursor cells, stem cells, cancer cells, etc.]; or any tissues where such cells are present [for example, brain or each part of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e. g. , large intestine and small intestine), blood vessel, heart, thymus, spleen, submandibular gland, peripheral blood, prostate, testis, ovary, placenta, uterus, bone, joint, adipose tissue (e.g., white adipose tissue, brown adipose tissue), skeletal muscle, etc.], synthetic DNA, etc. The genomic DNA and cDNA encoding the protein used in the present invention or its partial peptide can be directly amplified by Polymerase Chain Reaction (hereinafter, abbreviate to "PCR") and Reverse Transcriptase- PCR (hereinafter, abbreviate to "RT-PCR") with the use of each genomic DNA fraction, and total RNA or mRNA fraction prepared from the above-described cells or tissues as a template. Further, the genomic DNA and cDNA encoding the protein used in the present invention or its partial peptide can be respectively cloned from genomic DNA library and cDNA library which are prepared by inserting the fragment of genomic DNA, and total RNA or mRNA prepared from the above-described cells or tissues into an appropriate vector, in accordance with a colony or plaque hybridization assay or PCR method. The vector used for the library may be any of bacteriophage, plasmid, cosmid, phagemid and the like.

Examples of the DNA encoding the protein used in the present invention may be any of DNA comprising a base sequence hybridizable to DNA comprising the base sequence represented by SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15 or SEQ ID NO: 17 under high stringent conditions and encoding a protein which has the activity substantially equivalent to the protein comprising the amino acid sequence represented by SEQ ID NO: 2, SEQ ID NO: 4, SEQ ID NO: 6, SEQ ID NO: 8, SEQ ID NO: 10, SEQ ID NO: 12, SEQ ID NO: 14, SEQ ID NO: 16 or SEQ ID NO: 18.

As the DNA that is hybridizable to the base sequence represented by SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15 or SEQ ID NO: 17 under high stringent conditions, there are employed, for example, DNAs comprising base sequences having about 50% or more homology, preferably about 60% or more homology, more preferably about 70% or more homology, even more preferably about 80% or more homology, particularly preferably about 90% or more homology, and most preferably about 95% or more homology, to the base sequence represented by SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15 or SEQ ID NO: 17; and the like.

Homology of the base sequences in the present specification, for example, can be calculated under the following conditions (an expectation value = 10; gaps are allowed; filtering = ON; match score = 1; mismatch score = -3) using a homology scoring algorithm NCBI BLAST (National Center for Biotechnology Information Basic Local Alignment Search Tool). As the other algorithm for determining homology of the base sequence, same ones as the above-described homology scoring algorithms for the amino acid sequence are preferably exemplified.

The hybridization can be carried out by publicly known methods or by modifications thereof, for example, by the method described in Molecular Cloning, 2nd ed. (J. Sambrook et al., Cold Spring Harbor Lab. Press, 1989). A commercially available library can also be used according to the instructions of the attached manufacturer's protocol. The hybridization can be carried out more preferably under high stringent conditions.

The high stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium salt concentration at about 19 mM at a temperature of about 65°C are most preferred. Those skilled in the art can simply regulate the condition to a desired stringency by appropriately changing a concentration of hybridization solution, temperature of hybridization reaction, probe concentration, length of probe, number of mismatch, time for hybridization reaction, salt concentration of washing solution, temperature for washing, etc.

Preferable examples of the DNA encoding the protein used in the present invention include DNA comprising the base sequence represented by SEQ ID NO: 1 (Refseq Accession No. NM_021246) or the homolog thereof (for example, mouse homolog registered as RefSeq Accession No. NM_033478 in GenBank) in other mammals, DNA comprising the base sequence represented by SEQ ID NO: 3 (Refseq Accession No. NM_030667) or the homolog thereof (for example, mouse homolog registered as RefSeq Accession No. NM_011216 in GenBank) in other mammals, DNA comprising the based sequence represented by SEQ ID NO: 5 (RefSeq Accession No. NM_012171) or the homolog thereof (for example, mouse homolog registered as RefSeq Accession No. NM_028841 in GenBank) in other mammals, DNA comprising the base sequence represented by SEQ ID NO: 7 (RefSeq Accession No. NM_130465) or the homolog thereof in other mammals, DNA comprising the base sequence represented by SEQ ID NO: 9 (RefSeq Accession No. NM_001006616) or the homolog thereof in other mammals, a DNA comprising the base sequence represented by SEQ ID NO: 11 or the homolog thereof in other mammals, DNA comprising the base sequence represented by SEQ ID NO: 13 or the homolog thereof in other mammals, DNA comprising the base sequence represented by SEQ ID NO: 15 or the homolog thereof in other mammals, and DNA comprising the base sequence represented by SEQ ID NO: 17 or the homolog thereof in other mammals, and the like.

The polynucleotide (e.g., DNA) encoding the partial peptide of the protein used in the present invention may be any polynucleotide so long as it contains the base sequence encoding the partial peptide of the protein used in the present invention described above. The polynucleotide may also be any of genomic DNA, genomic DNA library, cDNA derived from the cells and tissues described above, cDNA library derived from the cells and tissues described above, and synthetic DNA.

As the DNA encoding the partial peptide of the protein used in the present invention, there is employed, for example, a DNA containing a partial base sequence of the base sequence represented by SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15 or SEQ ID NO: 17; or DNA containing a base sequence hybridizable to polynucleotide which comprises the base sequence represented by SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15 or SEQ ID NO: 17 under high stringent conditions, and also encoding a peptide which has the activity substantially equivalent to the protein used in the present invention. The DNA hybridizable to the base sequence represented by SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15 or SEQ ID NO: 17 represent the same meaning as described above. Further, same hybridization method and high stringent conditions as described above can be used.

For cloning of DNAs that encode the protein used in the present invention and its partial peptide (hereinafter sometimes merely referred to as the protein of the present invention in the description of cloning of DNAs encoding the same and their expression), the DNA can be either amplified by PCR using synthetic DNA primers containing a part of the base sequence encoding the protein of the present invention, or the DNA inserted into an appropriate vector can be selected by hybridization with those labeled with DNA fragment or synthetic DNA that encodes a part or entire region of the protein of the present invention. The hybridization can be carried out, for example, according to the method described in Molecular Cloning, 2nd ed. (J. Sambrook et al. , Cold Spring Harbor Lab. Press, 1989). Where the hybridization is carried out using commercially available library, the procedures may be conducted in accordance with the protocol described in the attached instructions.

Conversion of the base sequence of DNA can be effected by publicly known methods such as the ODA-LA PCR method, the Gapped duplex method, the Kunkel method, etc., or modification thereof, using PCR, a publicly known kit available as Mutan^{™}-super Express Km (Takara Bio) or Mutan^{™}-K (Takara Bio), etc.

The cloned DNA encoding the protein can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector for the protein of the present invention can be manufactured, for example, by excising the desired DNA fragment from the DNA encoding the protein of the present invention, and then ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc. , animal viruses such as retrovirus, vaccinia virus, baculovirus, etc. as well as pA1-11, pXT1, pRc/CMV, pRc/RSV, pcDNA I/Neo, etc.

The promoter used in the present invention may be any promoter if it matches well with a host to be used for gene expression. For example, when animal cells are used as the host, examples of the promoter are SRα promoter, SV40 promoter, LTR promoter, CMV promoter, HSV-TK promoter, etc.

Among them, it is preferable to use CMV (cytomegalovirus) promoter, SRα promoter, etc. When bacteria of the genus Escherichia is used as a host, preferred examples of the promoter are trp promoter, lac promoter, recA promoter, λP_{L} promoter, lpp promoter, T7 promoter, etc. When bacteria of the genus Bacillus is used as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter are polyhedrin promoter, P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori), etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker using dhfr gene-deficient Chinese hamster cells, the objective gene can also be selected on a thymidine free medium.

If necessary, a signal sequence that matches with a host is added to the N-terminus of the protein of the present invention. The signal sequence that can be used are PhoA signal sequence, OmpA signal sequence, etc. when bacteria of the genus Escherichia is used as the host; α-amylase signal sequence, subtilisin signal sequence, etc. when bacteria of the genus Bacillus is used as the host; MFα signal sequence, SUC2 signal sequence, etc. when yeast is used as the host; and insulin signal sequence, α-interferon signal sequence, antibody molecule signal sequence, etc. when animal cells are used as the host, respectively.

Using the vector containing the DNA encoding the protein of the present invention thus constructed, transformants can be manufactured. Examples of the host, which may be employed, are bacteria belonging to the genus Escherichia, bacteria belonging to the genus Bacillus, yeast, insect cells, insects, animal cells, etc.

Specific examples of the bacteria belonging to the genus Escherichia include Escherichia coli K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cerevisiae AH22, AH22R⁻, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cell (Sf cell), MG1 cell derived from mid-intestine of Trichoplusia ni, High Five^{™} cell derived from egg of Trichoplusia ni, cells derived from Mamestra brassicae, cells derived from Estigmena acrea, etc.; and for the virus BmNPV, Bombyx mori N cell (BmN cell), etc. is used. Examples of the Sf cell which can be used are Sf9 cell (ATCC CRL1711), Sf21 cell (both cells are described in Vaughn, J. L. et al., In Vivo, 13, 213-217 (1977)), etc.

As the insect, for example, a larva of Bombyx mori can be used [Maeda et al., Nature, 315, 592 (1985)].

Examples of animal cells include simian cell COS-1, COS-3, COS-7, Vero, Chinese hamster ovary cell (hereinafter simply referred to as CHO cell), dhfr gene-deficient CHO cell (hereinafter simply referred to as CHO (dhfr⁻) cell), mouse L cell, mouse AtT-20, mouse myeloma cell, mouse ATDC5 cell, rat GH3, human FL cell, human 293 cell, human HeLa cell, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995) (published by Shujunsha), or Virology, 52, 456 (1973).

Thus, the transformants transformed with the expression vectors bearing the DNAs encoding the protein can be obtained.

Where the host is bacteria belonging to the genus Escherichia or the genus Bacillus, the transformant can be appropriately cultured in a liquid medium which contains materials required for growth of the transformant such as carbon sources, nitrogen sources, inorganic materials, and the like. Examples of the carbon sources include glucose, dextrin, soluble starch, sucrose, etc.; examples of the nitrogen sources include inorganic or organic materials such as ammonium salts, nitrate salts, corn steep liquor, peptone, casein, meat extract, soybean cake, potato extract, etc.; and, examples of the inorganic materials are calcium chloride, sodium dihydrogenphosphate, magnesium chloride, etc. In addition, yeast extracts, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15 to 43°C for about 3 to 24 hours. If necessary, the culture may be aerated or agitated.

Where the bacteria belonging to the genus Bacillus are used as the host, the transformant is cultured generally at about 30 to 40°C for about 6 to 24 hours. If necessary, the culture can be aerated or agitated.

Where yeast is used as the host, the transformant is cultivated, for example, in Burkholder's minimal medium [Proc. Natl. Acad. Sci. U.S.A., 77, 4505 (1980)] or in SD medium supplemented with 0.5% Casamino acids [Proc. Natl. Acad. Sci. U.S.A., 81, 5330 (1984)]. Preferably, pH of the medium is adjusted to about 5 to 8. In general, the transformant is cultivated at about 20 to 35°C for about 24 to 72 hours. If necessary, the culture can be aerated or agitated.

Where insect cells or insects are used as the host, the transformant is cultivated in, for example, Grace's Insect Medium (Nature, 195, 788 (1962)) to which an appropriate additive such as inactivated 10% bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

Where animal cells are employed as the host, the transformant is cultured in, for example, MEM medium containing about 5 to 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 to 60 hours and, if necessary, the culture can be aerated or agitated.

As described above, the protein of the present invention can be produced in the transformant, on the cell membrane of the transformant, or outside the transformant.

The protein of the present invention can be separated and purified from the culture described above, for example, by the following procedures.

When the protein of the present invention is extracted from the bacteria or cells of the culture, the bacteria or cell is collected after culturing by a publicly known method and suspended in an appropriate buffer. The bacteria or cell is then disrupted by methods such as ultrasonication, a treatment with lysozyme and/or freeze-thaw cycling, followed by centrifugation, filtration, etc. to produce crude extract of the protein. The buffer used for the procedures may contain a protein denaturant such as urea or guanidine hydrochloride, or a surfactant such as Triton X-100^{™}, etc. When the protein is secreted in the culture broth, the supernatant can be separated, after completion of the cultivation, from the bacteria or cell to collect the supernatant by a publicly known method.

The protein of the present invention contained in the supernatant or the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein of the present invention thus obtained is in a free form, the protein can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein-modifying enzyme so that the protein can be subjected to addition of an appropriate modification or removal of a partial polypeptide. Examples of the protein-modifying enzyme include trypsin, chymotrypsin, arginyl endopeptidase, protein kinase, glycosidase and the like.

The presence of the thus produced protein of the present invention can be determined by an enzyme immunoassay or western blotting using a specific antibody.

The protein used in the present invention or its partial peptide can be synthesized by the in vitro translation with the use of a cell-free protein translation system comprising rabbit reticulocyte lysate, wheat germ lysate, E. coli lysate, etc., and RNA which corresponds to DNA encoding the same as a template. The protein or its partial peptide can also be synthesized by a cell-free transcription/translation system further comprising RNA polymerase with the use of DNA encoding calmodulin or its partial peptide as a template. For the cell-free protein (transcription /) translation system, commercially available ones and a method known per se can be employed. In particular, the Escherichia coli extract solution can be prepared according to a method disclosed in Pratt J.M. et al, "Transcription and Tranlation", Hames B.D. and Higgins S.J. edition, IRL Press, Oxford 179-209 (1984). As the commercially available cell lysate, Escherichia coli derived lysates such as E. coli S30 extract system (manufactured by Promega) and RTS 500 Rapid Tranlation System (manufactured by Roche Ltd.), etc.; rabbit reticulocyte derived lysates such as Rabbit Reticulocyte Lysate System (manufactured by Promega), etc. ; and wheat germ derived lysates such as PROTEIOS^{™}(manufactured by TOYOBO), etc., can be exemplified. Among them, the wheat germ lysate is preferably used. As the method of manufacturing wheat germ lysate, for example, methods disclosed in Johnston F.B. et al, Nature, 179, 160-161 (1957), Erickson A.H. et al, Meth. Enzymol., 96, 38-50 (1996), etc., can be employed.

As a system or device for the protein synthesis, a batch method (Pratt, J.M. et al, (1984) mentions above); continuous cell-free protein synthesis system (Spirin A. S. et al, Science, 242, 1162-1164 (1988)) in which an amino acid, energy source, etc. is continuously supplied in a reaction system; dialysis (Kikawa, et al, 21st Annual meeting of the Molecular Biology Society of Japan, WID6)); a double layer method (PROTEIOS^{™} Wheat germ cell-free protein synthesis core kit instruction manual, manufactured by TOYOBO), and the like can be exemplified. In addition, a method which comprises supplying RNA template, amino acid, energy source, etc., into a synthesis reaction system according to necessity, and eliminating a synthesized or degraded material according to necessity (Japanese Patent Laid-Open No. 2000-333673) can be used.

The antibody against the protein used in the present invention or against its partial peptide (hereinafter, sometimes abbreviated as "antibody of the present invention") can be any of polyclonal or monoclonal antibody, as long as it recognizes the protein of the present invention or its partial peptide. The isotype of the antibody is not particularly limited, but it is preferably IgG, IgM or IgA, particularly preferably IgG.

The antibody of the present invention is not subject to limitation, as long as it has at least a complementality determining region (CDR) for specifically recognizing and binding to the target antigen; in addition to the whole antibody molecule, the antibody may, for example, be a fragment such as Fab, Fab', or F(ab')₂, a genetically engineered conjugate molecule such as scFv, scFv-Fc, minibody, or diabody, or a derivative thereof modified with a molecule having protein stabilizing action, such as polyethylene glycol (PEG), or the like, and the like.

The antibody against the protein used in the present invention or against its partial peptide (hereinafter sometimes merely referred to as the protein of the present invention in the description of antibody) can be manufactured according to a method of producing an antibody or antisera known per se.

Hereinafter, a preparation method of immunogen of an antibody of the present invention and a production method of the antibody will be described.
(1) Preparation of antigen As an antigen used to prepare the antibody of the present invention, any antigen such as the above-mentioned protein of the present invention or its partial peptide, or a (synthetic) peptide having 1 or 2 or more antigenic determinants, which are the same as in the above-mentioned protein of the present invention its partial peptide, etc. may be used (hereinafter these antigens are sometimes merely referred to as the antigen of the present invention).
   The aforesaid protein of the present invention or its partial peptide can be, for example,(a) prepared from warm-blooded animal tissues or cells of human, simian, rat, mouse, chicken, etc., by publicly known methods or with modifications, (b) chemically synthesized by publicly known peptide synthesis methods using a peptide synthesizer, etc., (c) produced by culturing a transformant bearing a DNA encoding protein of the present invention or its partial peptide, or (d) biochemically synthesized from nucleic acid encoding protein of the present invention or its partial peptide as a template by using a cell-free transcription/ translation system.
   (a) Where the protein of the present invention is prepared from the warm-blooded animal tissues or cells, the tissues or cells are homogenized, and then the crude extract (e.g., membrane fraction, soluble fraction) can be also used per se as an antigen. Alternatively, the tissues or cells are extracted with an acid, a surfactant, an alcohol, etc., and the extract solution can be purified and isolated by a combination of salting-out, dialysis, gel filtration, chromatography techniques such as reverse phase chromatography, ion exchange chromatography, affinity chromatography and the like. Thus obtained protein of the present invention can be used as per se as an immunogen, and can also be used as the immunogen in the form of a partial peptide prepared by limited degradation using a peptidase, etc.
   (b) Where the antigen of the present invention is prepared chemically, the synthetic peptides used are, for example, a peptide having the same structure as the protein of the present invention purified from natural material by the above method (a), specifically, a peptide containing 1 or 2 or more amino acid sequences, which are the same amino acid sequences consisting of 3 or more, preferably 6 or more amino acids in an optional region of the amino acid sequence of the protein.
   (c) Where the antigen of the present invention is produced using the DNA-bearing transformants, the DNA can be produced in accordance with publicly known cloning techniques [e.g., the method described in Molecular Cloning 2nd ed., (J. Sambrook et al. , Cold Spring Harbor Lab. Press, 1989), etc.]. The cloning techniques include (1) a method in which DNAs encoding the antigen are isolated from cDNA library by a hybridization method using DNA probes designed on the basis of the gene sequence encoding the protein of the present invention, and (2) a method in which DNAs encoding the antigen are produced from cDNA as a template by PCR using DNA primers designed based on the gene sequence encoding the protein of the present invention, and the DNA are inserted into an expression vector suitable for a host. Incubating the transformants which is obtained from host by being transformed with the expression vector, in appropriate medium, the desired antigen can be obtained.
   (d) When a cell-free transcription/translation system is utilized, a method for synthesizing an mRNA by using an expression vector incorporating a DNA that encodes the antigen (for example, an expression vector wherein the DNA is placed under the control of the T7 or SP6 promoter and the like, and the like) as the template, that was prepared by the same method as (c) above, and a transcription reaction mixture comprising an RNA polymerase matching the promoter and its substrates (NTPs); and thereafter performing a translation reaction with the mRNA as the template using a known cell-free translation system (e. g. , extract from E. *coli,* rabbit reticulocytes, wheat germ etc.), and the like can be mentioned. By adjusting the salt concentration and the like appropriately, the transcription reaction and the translation reaction can also be carried out in the same reaction mixture at one time.

   As the immunogen, a whole protein molecule of the present invention or a peptide having a partial amino acid sequence of the protein molecule can be used. As examples of the partial amino acid sequence, those comprising 3 or more continuous amino acid residues, preferably those comprising 4 or more, more preferably 5 or more, still more preferably 6 or more continuous amino acid residues, can be mentioned. Alternatively, as examples of the amino acid sequence, those comprising 20 or less continuous amino acid residues, preferably those comprising 18 or less, more preferably 15 or less, still more preferably 12 or less continuous amino acid residues, can be mentioned. A portion of these amino acid residues (e.g., 1 to several residues) may be substituted with a substituent group (e.g., Cys, hydroxyl group, etc.). The peptide used as the immunogen has an amino acid sequence comprising one to several such partial amino acid sequences.
   Warm-blooded animal cells itself which express the protein of the present invention can also be used directly as the antigen of the present invention. As the Warm-blooded animal cells, there can be used the naturally occurring cells as described in (a) above, cells transformed by the methods as described in (c) above, etc. Hosts used for the transformation may be any cells as long as they are the cells collected from human, simian, rat, mouse, hamster, chicken etc. and preferably used are HEK293, COS7, CHO-K1, NIH3T3, Balb3T3, FM3A, L929, SP2/0, P3U1, B16, P388, or the like. Naturally occurring warm-blooded animal cells or transformed warm-blooded animal cells, which express the protein of the present invention, can be injected to immune animal as a suspension of the cells in a medium used for tissue culture (e.g., RPMI 1640) or buffer (e.g., Hanks' Balanced Salt Solution). Immunization may be done by any method, as long as it can stimulate antibody production, and preferably used are intravenous injection, intraperitoneal injection, intramuscular injection, subcutaneous injection, etc. The antigen of the present invention permit direct use for immunization in an insolubilized form, as long as it has immunogenicity; when an antigen of low molecular weight (i.e., molecular weight about 3,000 or less) having only one to several antigenic determinants in the molecule thereof (for example, a partial peptide of the protein of the present invention) is used, it can be used for immunization in the form of a complex bound or adsorbed to a suitable carrier because these antigens are normally hapten molecules of low immunogenicity. As the carrier, a naturally occurring or synthetic polymer can be used. As examples of the naturally occurring polymer, serum albumin of a mammal such as bovine, rabbit, or human, thyroglobulin of a mammal such as bovine or rabbit, ovalbumin of chicken etc. , hemoglobin of a mammal such as bovine, rabbit, human, or sheep, keyhole limpet hemocyanin (KLH) and the like can be used. As examples of the synthetic polymer, various latexes of polymers or copolymers of polyamino acids, polystyrenes, polyacryls, polyvinyls, polypropylenes and the like, and the like can be mentioned.
   A mixing ratio of the carrier to the hapten may be in any ratio of any type, as long as the antibody can be efficiently produced to the antigen bound or adsorbed to the carrier. The above described naturally occurring or synthetic high molecular carrier conventionally used to produce an antibody against a hapten may be used in a weight ratio of 0.1 to 100 based on 1 of hapten.
   For coupling of the hapten and the carrier, a variety of condensing agents can be used. Examples of the condensing agents, which are advantageously employed, are diazonium compounds such as bis-diazotized benzidine capable of crosslinking tyrosines, histidines or tryptophans; dialdehyde compounds such as glutaraldehyde, etc. capable of crosslinking amino groups with each other; diisocyanate compounds such as toluene-2,4-diisocyanate, etc.; dimaleimide compounds such as N,N'-o-phenylenedimaleimide, etc. capable of crosslinking thiol groups with each other; maleimide activated ester compounds capable of crosslinking an amino group with a thiol group; carbodiimide compounds capable of crosslinking an amino group with a carboxyl group; etc. In the crosslinking of amino groups with each other, one amino group is reacted with an activated ester reagent having dithiopyridyl group (e.g., N-succinimidyl 3-(2-pyridyldithio)propionate (SPDP), etc.) and then reduced to introduce the thiol group, whereas another amino group is introduced with a maleimide group using a maleimide activated ester reagent, and the two groups may be reacted with each other.
(2) Preparation of monoclonal antibody
   (a) Preparation of monoclonal antibody producing-cell An antigen of the present invention is administered as is, or along with a carrier or a diluent, to a warm-blooded animal at a site enabling antibody production by the methods such as intraperitoneal injection, intravenous injection, subcutaneous injection, intradermal injection and the like. In order to increase antibody productivity upon the administration, Freund's complete adjuvant or Freund's incomplete adjuvant may be administered. Dosing is normally performed about two to 10 times in total with one time every 1 to 6 weeks. As examples of the warm-blooded animal used, simians, rabbits, canines, guinea swine, mice, rats, hamsters, sheep, goats, donkeys and chickens can be mentioned. Although it is preferable to use a mammal of the same species as the recipient in order to avoid the problem of anti-Ig antibody production, mice and rats are generally preferably used for generating a monoclonal antibody.
      Since artificial immunization to humans is ethically difficult, it is preferable, when the antibody of the present invention targets a human, (i) to obtain a human antibody by immunizing a human antibody-producing animal (e.g., mouse) produced according to a method described below, (ii) to produce a chimeric antibody, humanized antibody or fully human antibody according to a method described below, or (iii) to obtain a human antibody using in combination the in vitro immunization method and cell immortalization with virus, human-human (or -mouse) hybridoma production technique, phage display method and the like. Note that the *in vitro* immunization method can also be used preferably as a method for obtaining an antigen against an antigen that is unstable and difficult to prepare in large amounts for the purpose of preparing a non-human animal-derived antibody, because there is the possibility of obtaining an antibody against an antigen for which antibody production is suppressed by ordinary immunization, because it is possible to obtain an antibody with an amount of antigen on the nanogram to microgram order, because immunization completes in several days, and for other reasons.
      As the animal cells used in the *in vitro* immunization method, lymphocytes, preferably B-lymphocytes and the like, isolated from peripheral blood, spleen, lymph node and the like of a human and the above-described warm-blooded animals (preferably mouse or rat) can be mentioned. For example, in the case of mouse or rat cells, the spleen is extirpated from an about 4- to 12-week-old animal, and splenocytes are separated and rinsed with a appropriate medium [e.g., Dulbecco's modified Eagle medium (DMEM), RPMI1640 medium, Ham's F12 medium and the like], after which the splenocytes are suspended in an antigen-containing medium supplemented with fetal calf serum (FCS; about 5 to 20%) and cultured using a CO₂ incubator and the like for about 4 to 10 days. Examples of the antigen concentration include, but are not limited to, 0.05 to 5 µg. It is preferable to prepare a culture supernatant of thymocytes of an animal of the same strain (preferably at about 1 to 2 weeks of age) according to a conventional method, and to add the supernatant to the medium.
      Since it is difficult to obtain a thymocyte culture supernatant in in vitro immunization of human cells, it is preferable to perform immunization by adding, to the medium, several kinds of cytokines such as IL-2, IL-4, IL-5, and IL-6 and the like, and if necessary, an adjuvant substance (e.g., muramyldipeptide and the like) along with the antigen.
      In preparing a monoclonal antibody, it is possible to establish an antibody-producing hybridoma by selecting an individual or cell population showing an elevated antibody titer from among antigen-immunized warm-blooded animals (e.g., mice, rats) or animal cells (e.g., human, mouse, rat), respectively; collecting spleens or lymph nodes at 2 to 5 days after the final immunization or collecting the cells after 4 to 10 days of cultivation after *in vitro* immunization to isolate antibody-producing cells; and fusing the isolated cells with myeloma cells. A measurement of serum antibody titer can be performed by, for example, reacting a labeled antigen and an antiserum, and thereafter determining the activity of the label bound to the antibody.
      Although the myeloma cells are not subject to limitation, as long as they are capable of producing a hybridoma that secretes a large amount of antibody, those that do not produce or secrete the antibody *per se* are preferable, with greater preference given to those of high cell fusion efficiency. To facilitate hybridoma selection, it is preferable to use a cell line that is sensitive to HAT (hypoxanthine, aminopterin, thymidine). As examples of the mouse myeloma cells, NS-1, P3U1, SP2/0, AP-1 and the like can be mentioned; as examples of the rat myeloma cells, R210.RCY3, Y3-Ag 1.2.3 and the like can be mentioned; as examples of the human myeloma cells, SKO-007, GM 1500-6TG-2, LICR-LON-HMy2, UC729-6 and the like can be mentioned.
      Fusion operation can be performed according to a known method, for example, the method of Koehler and Milstein [Nature, 256, 495 (1975)]. As a fusion promoter, polyethylene glycol (PEG), Sendai virus and the like can be mentioned, and PEG and the like are preferably used. Although the molecular weight of PEG is not subject to limitation, PEG1000 to PEG6000, which are of low toxicity and relatively low viscosity, are preferable. As examples of the PEG concentration, about 10 to 80%, preferably about 30 to 50%, can be mentioned. As the solution for diluting PEG, various buffers such as serum-free medium (e.g., RPMI1640), complete medium comprising about 5 to 20% serum, phosphate buffered saline (PBS), and Tris buffer can be used. DMSO (e.g., about 10 to 20%) can also be added as desired. As examples of the pH of the fusion solution, about 4 to 10, preferably about 6 to 8 can be mentioned.
      The ratio by number of antibody-producing cells (splenocytes) and myeloma cells is preferably normally about 1:1 to 20:1, and the cell fusion can be efficiently performed by incubation normally at 20 to 40°C, preferably at 30 to 37°C, normally for 1 to 10 minutes.
      An antibody-producing cell line can also be obtained by infecting antibody-producing cells with a virus capable of transforming lymphocytes to immortalize the cells. As such viruses, for example, Epstein-Barr (EB) virus and the like can be mentioned. Although the majority of persons have immunity because they have ever been infected with this virus in an asymptomatic infection of infectious mononucleosis, virion is also produced when the ordinary EB virus is used; therefore, appropriate purification must be performed. As an EB system free from the possibility of viral contamination, it is also preferable to use a recombinant EB virus that retains the capability of immortalizing B lymphocytes but lacks the capability of replicating virion (for example, deficiency of the switch gene for transition from latent infection state to lytic infection state and the like).
      Since marmoset-derived B95-8 cells secrete EB virus, B lymphocytes can be easily transformed by using a culture supernatant thereof. An antibody-producing B cell line can be obtained by, for example, culturing these cells using a medium supplemented with serum and penicillin/streptomycin (P/S) (e.g., RPMI1640) or a serum-free medium supplemented with a cell growth factor, thereafter separating the culture supernatant by filtration or centrifugation and the like, suspending therein antibody-producing B lymphocytes at a suitable concentration (e.g., about 10⁷ cells/mL), and incubating the suspension normally at 20 to 40°C, preferably at 30 to 37°C, normally for about 0.5 to 2 hours. When human antibody-producing cells are provided as mixed lymphocytes, it is preferable to previously remove T lymphocytes by allowing them to form an E rosette with, for example, sheep erythrocytes and the like, to increase transformation frequency of EB virus, because the majority of persons have T lymphocytes which exhibit cytotoxicity to cells infected with EB virus. It is also possible to select lymphocytes specific for the target antigen by mixing sheep erythrocytes, previously bound with a soluble antigen, with antibody-producing B lymphocytes, and separating the rosette using a density gradient of Percoll and the like. Furthermore, because antigen-specific B. lymphocytes are capped by adding the antigen in large excess so that they no longer present IgG on the surface, mixing with sheep erythrocytes bound with anti-IgG antibody results in the formation of rosette only by antigen-nonspecific B lymphocytes. Therefore, by collecting a layer of cells that don't form rosette from this mixture using a density gradient of Percoll and the like, it is possible to select antigen-specific B lymphocytes.
      Human antibody-secreting cells having acquired the capability of proliferating infinitely by the transformation can be back fused with mouse or human myeloma cells in order to stably sustain the antibody-secreting ability. As the myeloma cells, the same as those described above can be used.
      Hybridoma screening and breeding are normally performed using a medium for animal cells (e.g., RPMI1640) containing 5 to 20% FCS or a serum-free medium supplemented with cell growth factors, with the addition of HAT (hypoxanthine, aminopterin, thymidine). As examples of the concentrations of hypoxanthine, aminopterin and thymidine, about 0.1 mM, about 0.4 µM and about 0.016 mM and the like, respectively, can be mentioned. For selecting a human-mouse hybridoma, ouabain resistance can be used. As human cell lines are more sensitive to ouabain than mouse cell lines, it is possible to eliminate unfused human cells by adding ouabain at about 10⁻⁷ to 10⁻³ M to the medium.
      In selecting a hybridoma, it is preferable to use feeder cells or culture supernatants of certain cells. As the feeder cells, an allogenic cell species having a lifetime limited so that it dies after helping the emergence of hybridoma, cells capable of producing large amounts of a growth factor useful for the emergence of hybridoma with their proliferation potency reduced by irradiation and the like, and the like are used. For example, as the mouse feeder cells, splenocytes, macrophage, blood, thymocytes and the like can be mentioned; as the human feeder cells, peripheral blood mononuclear cells and the like can be mentioned. As examples of the cell culture supernatant, primary culture supernatants of the above-described various cells and culture supernatants of various established cell lines can be mentioned.
      Moreover, a hybridoma can also be selected by reacting a fluorescein-labeled antigen with fusion cells, and thereafter separating the cells that bind to the antigen using a fluorescence-activated cell sorter (FACS). In this case, efforts for cloning can be lessened significantly because a hybridoma that produces an antibody against the target antigen can be directly selected.
      For cloning a hybridoma that produces a monoclonal antibody against the target antigen, various methods can be used.
      It is preferable to remove aminopterin as soon as possible because it inhibits many cell functions. In the case of mice and rats, aminopterin can be removed 2 weeks after fusion and beyond because most myeloma cells die within 10 to 14 days. However, a human hybridoma is normally maintained in a medium supplemented with aminopterin for about 4 to 6 weeks after fusion. It is desirable that hypoxanthine and thymidine be removed more than one week after the removal of aminopterin. That is, in the case of mouse cells, for example, a complete medium (e.g., RPMI1640 supplemented with 10% FCS) supplemented with hypoxanthine and thymidine (HT) is added or exchanged 7 to 10 days after fusion. About 8 to 14 days after fusion, visible clones emerge. Provided that the diameter of clone has reached about 1 mm, the amount of antibody in the culture supernatant can be measured.
      A measurement of the amount of antibody can be performed by, for example, a method comprising adding the hybridoma culture supernatant to a solid phase (e.g., microplate) to which the target antigen or a derivative thereof or its partial peptide (including the partial amino acid sequence used as the epitope) is adsorbed directly or with a carrier, subsequently adding an anti-immunoglobulin (IgG) antibody (an antibody against IgG derived from an animal of the same species as the animal from which the original antibody-producing cells are derived is used) or protein A, which had been labeled with a radioactive substance (e.g., ¹²⁵I, ¹³¹I, ³H, ¹⁴C), enzyme (e.g., β-galactosidase, β-glucosidase, alkaline phosphatase, peroxidase, malate dehydrogenase), fluorescent substance (e.g., fluorescamine, fluorescein isothiocyanate), luminescent substance (e.g., luminol, luminol derivative, luciferin, lucigenin) and the like, and detecting the antibody against the target antigen (epitope) bound to the solid phase, a method comprising adding the hybridoma culture supernatant to a solid phase to which an anti-IgG antibody or protein A is adsorbed, adding the target antigen, or a derivative thereof, or its partial peptide labeled with the same labeling agent as described above, and detecting the antibody against the target antigen (epitope) bound to the solid phase, and the like.
      Although limiting dilution is normally used as the cloning method, cloning using soft agar and cloning using FACS (described above) are also possible. Cloning by limiting dilution can be performed by, for example, the following procedures, which, however, are not to be construed as limiting.
      The amount of antibody is measured as described above, and positive wells are selected. Selected suitable feeder cells are previously added to a 96-well plate. Cells are collected from the antibody-positive wells and suspended in complete medium (e.g., RMPI1640 supplemented with 10% FCS and P/S) to obtain a density of 30 cells/mL; 0.1 mL (3 cells/well) of this suspension is added to the well plate with feeder cells added thereto; the remaining cell suspension is diluted to 10 cells/mL and sown to other wells (1 cell/well) in the same way; the still remaining cell suspension is diluted to 3 cells/mL and sown to other wells (0.3 cells/well). The cells are cultured for about 2 to 3 weeks until a visible clone appears, when the amount of antibody is measured to select positive wells, and the selected cells are recloned in the same way. In the case of human cells, cloning is relatively difficult, so that a plate in which cells are plated at 10 cells/well is also prepared. Although a monoclonal antibody-producing hybridoma can be obtained normally by two times of subcloning, it is desirable to repeat recloning regularly for several more months to confirm the stability thereof.
      Hybridomas can be cultured *in vitro* or *in vivo.*
      As a method of *in vitro* culture, a method comprising gradually scaling up a monoclonal antibody-producing hybridoma obtained as described above, from a well plate, while keeping the cell density at, for example, about 10⁵ to 10⁶ cells/mL, and gradually lowering the FCS concentration, can be mentioned.
      As a method of in vivo culture, for example, a method comprising intraperitoneally injecting a mineral oil to a mouse (a mouse that is histocompatible with the parent strain of the hybridoma) to induce plasmacytoma (MOPC), intraperitoneally injecting about 10⁶ to 10⁷ cells of hybridoma to the mouse 5 to 10 days later, and collecting ascites fluid under anesthesia 2 to 5 weeks later, can be mentioned.
   (b)Purification of the monoclonal antibody Separation and purification of the monoclonal antibody can be performed according to a publicly known method, for example, separation and purification method of immunoglobulin [e.g., salting-out, alcohol precipitation, isoelectric point precipitation, electrophoresis, adsorption-desorption with an ion exchanger (e.g., DEAE, QEAE), ultracentrifugation, gel filtration, specific purification comprising selectively collecting the antibody by means of an antigen-coupled solid phase or an active adsorbent such as protein A or protein G, and dissociating the linkage to obtain the antibody, and the like].
      As described above, a monoclonal antibody can be produced by culturing a hybridoma in or outside the living body of a warm-blooded animal, and harvesting an antibody from the body fluid or culture thereof.
      When using the antibody of the present invention for cancer preventive / remedy, since the antibody is required to have antitumor activity, it is necessary to examine the level of antitumor activity of provided monoclonal antibody. The antitumor activity can be assayed by comparing the cancer cell growth or induction of apoptosis, in the presence and absence of antibody.
      In a preferred mode of embodiment, since the antibody of the present invention is used as a pharmaceutical product having humans as the subject of administration thereof, the antibody of the present invention (preferably a monoclonal antibody) is an antibody whose risk of showing antigenicity when administered to a human has been reduced; to be specific, the antibody is a fully human antibody, a humanized antibody, a mouse-human chimeric antibody and the like, particularly preferably a fully human antibody. A humanized antibody and a chimeric antibody can be prepared by genetic engineering technology according to the method described below. Although a fully human antibody can also be produced from the above-described human-human (or -mouse) hybridoma, it is desirable to produce it using a human antibody-producing animal (e.g., mouse) or the phage display method in order to stably supply the antibody in large amounts at low costs.
      (i) Preparation of chimeric antibody
         As used herein, "a chimeric antibody" means an antibody wherein the sequences of the variable regions of the H chain and L chain (V_{H} and V_{L}) thereof are derived from a mammalian species, and wherein the sequences of the constant regions (C_{H} and C_{L}) are derived from another mammalian species. The sequences of the variable regions are preferably derived from, for example, an animal species permitting easy preparation of a hybridoma, such as mouse, and the sequences of the constant regions are preferably derived from the recipient mammalian species.
         As examples of the method of preparing a chimeric antibody, the method described in US Patent No. 6,331,415 or a partially modified method thereof and the like can be mentioned. To be specific, first, mRNA or total RNA is prepared from a monoclonal antibody-producing hybridoma (for example, mouse-mouse hybridoma) obtained as described above, according to a conventional method, to synthesize cDNA. DNAs that encode V_{H} and V_{L} are amplified and purified by PCR according to a conventional method with the cDNA as the template, using appropriate primers [for example, oligo DNAs comprising the base sequences that encode the N-terminal sequences of V_{H} and V_{L}, respectively, as the sense primers, and oligo DNAs that hybridize to the base sequences that encode the N-terminal sequences of C_{H} and C_{L}, respectively, as the antisense primer (see, for example, Bio/Technology, 9: 88-89, 1991)]. In the same manner, DNAs that encode C_{H} and C_{L} are amplified and purified from an RNA prepared from lymphocytes and the like of other mammal (e.g., human) by RT-PCR. V_{H} and C_{H}, and V_{L} and C_{L}, are ligated together, respectively, using a conventional method, and the chimeric H chain DNA and chimeric L chain DNA obtained are inserted into respective appropriate expression vectors [for example, vectors comprising promoters that have transcription activity in CHO cells, COS cells, mouse myeloma cells and the like (e.g., CMV promoter, SV40 promoter and the like)]. The DNAs that encode the two chains may be inserted into separate vectors, and may be inserted into a single vector in tandem. Host cells are transformed with the chimeric H chain and chimeric L chain expression vector(s) obtained. As the host cells, animal cells, for example, Chinese hamster ovary (CHO) cells, simian-derived COS-7 cells, Vero cells, rat-derived GHS cells and the like, in addition to the above-described mouse myeloma cells, can be mentioned. For the transformation, any method applicable to animal cells can be used, with preference given to electroporation method and the like. It is possible to isolate a chimeric monoclonal antibody by culturing the host cells in a medium suitable thereto for a given period, and thereafter collecting the culture supernatant and purifying it in the same manner as described above. Alternatively, it is also possible to obtain a chimeric monoclonal antibody easily and in large amounts from milk or eggs of transgenic animals which are produced by a conventional method using germ line cells of an animal such as bovine, goat, or chicken as the host cells, for which a transgenic technique has been established and a know-how of mass propagation as a domestic animal (domestic fowl) has been accumulated. Furthermore, it is also possible to obtain a chimeric monoclonal antibody in large amounts from the seeds, leaves and the like of a transgenic plant, produced by using microinjection and electroporation into protoplast, the particle gun method and Ti-vector method for intact cells and the like, with cells of a plant such as corn, rice, wheat, soybean, or tobacco as the host cells, for which a transgenic technique has been established, and which is cultured in large amounts as a major crop.
         When the chimeric monoclonal antibody obtained is digested with papain, Fab is obtained; when the same is digested with pepsin, F(ab')₂ is obtained.
         It is also possible to make scFv by ligating DNAs that encode mouse V_{H} and V_{L} via a suitable linker, for example, a DNA that encodes a peptide consisting of 1 to 40 amino acids, preferably 3 to 30 amino acids, more preferably 5 to 20 amino acids [e.g., [Ser-(Gly)m]n or [(Gly)m-Ser]n (m is an integer from 0 to 10, n is an integer from 1 to 5) and the like]. Furthermore, it is possible to make a minibody monomer by ligating a DNA that encodes C_{H3} via a suitable linker thereto, or make a scFv-Fc by ligating a DNA that encodes C_{H} full length via a suitable linker thereto. The DNA encoding such an antibody molecule modified (coupled) by genetic engineering can be expressed in a microorganism such as E. *coli* or yeast under the control of a suitable promoter, to produce the antibody molecule in large amounts.
         When DNAs encoding mouse V_{H} and V_{L} are inserted into the downstream of one promoter in tandem and introduced into E. coli, a dimer named as Fv is formed by monocistronic gene expression. When an appropriate amino acid in the FRs of V_{H} and V_{L} is substituted with Cys using molecule modeling, a dimer named as dsFv is formed via the intermolecular disulfide bond between the two chains.
      (ii) Humanized antibody As used herein, "a humanized antibody" means an antibody wherein the sequences of all regions present in the variable region, other than the complementality determining region (CDR), [i.e., framework region (FR) in constant region and variable region] are derived from a human, and wherein only the sequence of CDR is derived from another mammalian species. The other mammalian species is preferably an animal species, for example, mouse and the like, with which production of hybridomas can be easily performed.
         As examples of the method of preparing a humanized antibody, the methods described in US Patent Nos. 5,225,539, 5,585,089, 5,693,761 and 5,693,762 or partially modified methods therefrom and the like can be mentioned. To be specific, DNAs that encode V_{H} and V_{L} derived from a non-human mammalian species (e.g., mouse) are isolated in the same manner as with the above-described chimeric antibody, after which sequencing is performed by a conventional method using an automated DNA sequencer (e.g., manufactured by Applied Biosystems and the like), and the base sequences obtained or deduced amino acid sequences therefrom are analyzed using a known antibody sequence database [for example, Kabat database (see Kabat et al., "Sequences of Proteins of Immunological Interest", edited by US Department of Health and Human Services, Public Health Service, NIH, 5th edition, 1991) and the like] to determine the CDR and FR of the two chains. A base sequence wherein the CDR encoding region of a base sequence that encodes the L chain and H chain of a human antibody having an FR sequence similar to the determined FR sequence [e.g., human κ type L chain subgroup I and human H chain subgroup II or III (see Kabat et al., 1991 (*supra*))] is substituted with the determined base sequence that encodes the CDR of another animal species, is designed, and the base sequence is divided into fragments of about 20 to 40 bases, and a sequence complementary to the base sequence is divided into fragments of about 20 to 40 bases so that they alternately overlap with the aforementioned fragments. It is possible to construct DNAs that encode V_{H} and V_{L} having human-derived FR and a CDR derived from another mammalian species by synthesizing individual fragments using a DNA synthesizer, and hybridizing and ligating them in accordance with conventional methods. In order to transfer a CDR derived from another mammalian species into human-derived V_{H} and V_{L} more quickly and more efficiently, it is preferable to use PCR-based site directed mutagenesis. As examples of such a method, the sequential CDR grafting method described in Japanese Patent Unexamined Publication No. HEI-5-227970 and the like can be mentioned. It is possible to obtain cells or transgenic animal/plant that produces a humanized antibody by ligating the thus-obtained DNAs that encode V_{H} and V_{L} to DNAs that encode human-derived C_{H} and C_{L}, respectively, in the same manner as with the above-described chimeric antibody, and introducing the ligated product into suitable host cells.
         A humanized antibody, like a chimeric antibody, can be modified to scFv, scFv-Fc, minibody, dsFv, Fv and the like by using genetic engineering techniques; and they can be produced in a microorganism such as E. coli or yeast by using a suitable promoter.
         The technology for preparing a humanized antibody can also be applied to, for example, preparing a monoclonal antibody that can be preferably administered to another animal species for which no hybridoma production technology has been established. For example, animals widely propagated as domestic animals (domestic fowl) such as bovine, swine, sheep, goat, and chicken, and pet animals such as canines and cats, and the like can be mentioned as the subject animal species.
      (iii) Preparation of fully human antibody using human antibody-producing animal
         Provided that a functional human Ig gene is introduced into a non-human warm-blooded animal having the endogenous immunoglobulin (Ig) gene knocked out (KO) therein, and that this animal is immunized with an antigen, a human antibody is produced in place of the antibody derived from the animal. Therefore, provided that an animal such as mice, for which a technique for producing a hybridoma has been established, is used, it is possible to acquire a fully human monoclonal antibody by the same method as the conventional method used to prepare a mouse monoclonal antibody. First, some of the human monoclonal antibodies, that were generated by using a human antibody-producing mouse (see Immunol. Today, 17: 391-397, 1996) obtained by crossing a mouse transfected with minigenes of the human Ig H chain and L chain using an ordinary transgenic (Tg) technique with a mouse wherein the endogenous mouse Ig gene has been inactivated using an ordinary KO technique, are already in clinical stage, and to date production of anti-human Ig human antibody (HAHA) has not been reported.
         Later, Abgenix Inc. [trade name: XenoMouse (see Nat. Genet., 15: 146-156, 1997; US Patent No. 5,939,598 and the like)] and Medarex Inc. [trade name: Hu-Mab Mouse (see Nat. Biotechnol., 14: 845-851, 1996; US Patent No. 5,545,806 and the like)] established Tg mice transfected with even a larger human Ig gene using a yeast artificial chromosome (YAC) vector, thus enabling the production of human antibodies of richer repertoire. However, because the human Ig gene, for example, in the case of the H chain, exhibits its diversity as the VDJ exon, which is a variable combination of about 80 kinds of V fragments, about 30 kinds of D fragments and 6 kinds of J fragments, encodes the antigen binding site, the full length thereof is as large as about 1.5 Mb (14th chromosome) for the H chain, about 2 Mb (2nd chromosome) for the κL chain, and about 1 Mb (22nd chromosome) for the λL chain. To reproduce the diverse antibody repertoire in another animal species as in human, it is desirable to introduce the full length of each Ig gene. However, a DNA that is insertable into a conventional transfection vector (plasmid, cosmid, BAC, YAC and the like) is normally several kb to several hundred kb in length, and it has been difficult to introduce the full length of Ig genes by the conventional technique for establishing a transgenic animal, which comprises inserting a cloned DNA into a fertilized egg.
         Tomizuka et al. (Nat. Genet., 16: 133-143, 1997) prepared a mouse having the full-length human Ig gene by introducing a natural fragment of a human chromosome harboring the Ig gene (hCF) into a mouse [transchromosomic (TC) mouse]. That is, first, a human-mouse hybrid cell having human chromosomes in which the 14th chromosome comprising the H chain gene and the 2nd chromosome comprising the κL chain gene, both labeled with, for example, a drug-resistance marker and the like, is treated with a spindle formation inhibitor (e.g., colcemid) for about 48 hours to prepare a microcell wherein one to several chromosomes or fragments thereof are enveloped in nuclear membrane, and the chromosomes are introduced into a mouse ES cell by the micronuclear fusion method. A hybrid ES cell retaining the chromosomes having the human Ig gene or fragments thereof is selected using a medium containing a drug, and the cell is microinjected into a mouse embryo in the same manner as with the preparation of an ordinary KO mouse. A germ line chimera is selected among the chimeric mice obtained, with coat color as the index, and the like, to establish a TC mouse strain carrying the human 14th chromosome fragment (TC(hCF14)) and a TC mouse strain carrying the human 2nd chromosome fragment (TC(hCF2)). After establishing mouse strains wherein the endogenous H chain gene and κL chain gene are knocked out, respectively [KO (IgH) and KO (Igκ)] by a conventional method, it is possible to establish a mouse strain having all the four kinds of gene modifications (double TC/KO) by repeating the crossing of these four strains.
         Provided that the same method as that for producing an ordinary mouse monoclonal antibody is applied to a double TC/KO mouse established as described above, it is possible to obtain an antigen-specific human monoclonal antibody-producing hybridoma. However, there is the drawback of a lower efficiency to obtain hybridomas than that with the ordinary mouse, because hCF2 containing the κL chain gene is unstable in the mouse cells.
         On the other hand, because the aforementioned Hu-Mab mouse has a structure wherein the variable region cluster are doubled although it has about 50% of the κL chain gene, it exhibits a κ chain diversity equivalent to that with full length (on the other hand, HuMab mouse exhibits a low H chain diversity and inadequate response to antigen because it carries only about 10% of the H chain gene). And the κ chain is stably retained in the mouse cells because it is inserted in mouse chromosome via a YAC vector (Igκ-YAC). Making use of this advantage, it is possible to get the efficiency for obtaining hybridomas and affinity of antibody to antigen that are equivalent to those with the ordinary mouse, by crossing a TC(hCF14) mouse with a Hu-Mab mouse to establish a mouse that stably retains both hCF14 and Igκ-YAC (trade name: KM mouse).
         Furthermore, it is also possible to establish a human antibody-producing animal in which the λL chain gene is further transfected to reconstruct the diverse human antibody repertoire more completely. Such an animal can also be obtained by producing a TC mouse in which the human 22nd chromosome or a fragment thereof harboring the λL chain gene is introduced in the same manner as described above [TC (hCF22)], and crossing the mouse with the above-described double TC/KO mouse or KM mouse, or can also be obtained by, for example, constructing a human artificial chromosome (HAC) comprising both the H chain locus and the λL chain locus, and introducing it into a mouse cell (Nat. Biotechnol. , 18: 1086-1090, 2000) .
         When an antibody of the present invention is used as a medicine, an antibody of the present invention is desirably a monoclonal antibody, but it may be a polyclonal antibody. When the antibody of the present invention is a polyclonal antibody, it is not necessary to use hybridoma; therefore, provided that a human antibody-producing animal is produced in the same manner as described above using an animal species for which no technique for preparing a hybridoma has been established but a transgenic technique has been established, preferably an ungulate such as bovine, it is also possible to produce a human antibody in larger amounts at low costs (see, for example, Nat. Biotechnol., 20: 889-894, 2002). The human polyclonal antibody thus obtained can be purified by collecting blood, ascites fluid, milk, egg and the like, preferably milk or egg, of the human antibody-producing animal, in combination with the same purification techniques as described above.
      (iv) Preparation of fully human antibody using phage display human antibody library
         Another approach to produce a fully human antibody is a method using phage display. This method sometimes encounters cases in which a mutation due to PCR is introduced into a site other than CDRs; for this reason, a few reports of cases of HAHA production in clinical stage are available. On the other hand, however, the method has advantages such as no risk of cross-species viral infection derived from the host animal and the indefinite specificity of the antibody (antibodies against forbidden clone, sugar chain and the like can also be easily prepared).
         The method of preparing a phage display human antibody library include, but are not limited to, for example, the methods described below.
         Although a phage used is not subject to limitation, filamentous phage (Ff bacteriophage) is normally preferably used. As the method of presenting a foreign protein on the phage surface, a method comprising expressing and presenting the foreign protein as a fusion protein with any of the coat proteins g3p, and g6p to g9p on the coat protein can be mentioned; and a method comprising fusing the foreign protein to the N-terminal side of g3p or g8p is often used. As the phage display vector, 1) one in which the foreign gene is introduced in the form of fusion gene with the coat protein gene of the phage genome, to allow all the coat proteins presented on the phage surface to be presented as a fusion protein with the foreign protein, 2) one in which the gene encoding the fusion protein is inserted separately from the wild-type coat protein gene to allow the fusion protein and the wild-type coat protein to be expressed simultaneously, and 3) one in which an E. *coli* having a phagemid vector harboring the gene that encodes the fusion protein is infected with a helper phage having the wild-type coat protein gene to produce phage particles that express the fusion protein and the wild-type coat protein simultaneously, and the like can be mentioned. However, a phage display vector of the type 2) or 3) is used for the preparation of an antibody library, because in the case of 1), the capability of infection is lost when a large foreign protein is fused.
         As a specific vector, those described by Holt et al. (Curr. Opin. Biotechnol., 11: 445-449, 2000) can be mentioned as examples. For example, pCES1 (see J. Biol. Chem., 274: 18218-18230, 1999) is an Fab-expressing phagemid vector wherein a DNA encoding the κL chain constant region allocated to downstream of the g3p signal peptide, and a DNA encoding CH3, His-tag, c-myc tag, and the amber stop codon (TAG) followed by the g3p coding sequence, allocated to downstream of the g3p signal peptide, are arranged under the control of one lactose promoter. When this is introduced to an E. *coli* having an amber mutation, Fab is presented onto the g3p coat protein, but when it is expressed in the HB2151 strain and the like, which do not have an amber mutation, a soluble Fab antibody is produced. And as the scFv-expressing phagemid vector, for example, pHEN1 (J. Mol. Biol., 222: 581-597, 1991) and the like are used.
         Meanwhile as examples of the helper phage, M13-KO7, VCSM13 and the like can be mentioned.
         And as another phage display vector, a vector that is designed so that a sequence comprising the cysteine-encoding codon is linked to each of the 3' end of the antibody gene and the 5' end of the coat protein gene to express the two genes simultaneously and separately (not in the form of a fusion protein), and to present the antibody onto the coat protein on the phage surface via S-S bonds between the introduced cysteine residues (CysDisplay^{™} technology of Morphosys AG) and the like, can be mentioned.
         As the kind of human antibody library, a naive/non-immunized library, a synthetic library, an immunized library and the like can be mentioned.
         The naive/non-immunized library is a library obtained by acquiring the V_{H} and V_{L} genes retained by a normal human by RT-PCR, and randomly cloning them into the above-described phage display vector. Normally, mRNA derived from lymphocytes of peripheral blood, bone marrow, tonsil and the like of a normal human, and the like are used as the template. A library prepared by selectively amplifying IgM-derived mRNA in which a class switch due to antigen sensitization is not undergoing, to avoid V gene biases such as clinical history, is particularly called a naive library. Representatively, the library of Cambridge Antibody Technology (see J. Mol. Biol., 222: 581-597, 1991; Nat. Biotechnol., 14: 309-314, 1996), the library of Medical Research Council (*see* Annu. Rev. Immunol., 12: 433-455, 1994), the library of Dyax Corp. (see J. Biol. Chem., 1999 *(supra);* Proc. Natl. Acad. Sci. USA, 14: 7969-7974, 2000) and the like can be mentioned.
         A synthetic library is obtained by selecting a functional particular antibody gene in human B cells, and substituting a portion of antigen-binding region in a V gene fragment, for example, CDR3 and the like, with DNAs encoding a random amino acid sequence of appropriate length, to construct a library. It is recognized to be excellent in antibody expression efficiency and stability because the library can be constructed with the combination of the V_{H} and V_{L} genes, which produce functional scFv and Fab, since the beginning. Representatively, the HuCAL library of Morphosys AG (see J. Mol. Biol., 296: 57-86, 2000), the library of Biolnvent (see Nat. Biotechnol. , 18: 852, 2000), the library of Crucell (see Proc. Natl. Acad. Sci. USA, 92: 3938, 1995; J. Immunol. Methods, 272: 219-233, 2003) and the like can be mentioned.
         An immunized library is a library obtained by preparing an mRNA from lymphocytes collected from a human such as a patient with cancer, autoimmune disease, infectious disease and the like or a recipient of vaccination, having an elevated blood antibody titer against the target antigen, or from human lymphocytes and the like which are artificially immunized with the target antigen by the above-described *in vitro* immunization method, in the same manner as with the above-described naive/non-immunized library, and amplifying the V_{H} and V_{L} genes by RT-PCR, to construct a library. It is possible to obtain the desired antibody even from such libraries of relatively small size because the desired antibody gene is contained in the library already at the beginning.
         The wider the diversity of the library is, the better; actually, however, an appropriate library size is about 10⁸ to 10¹¹ clones, taking into consideration of the number of phages handlable in the following panning operation (10¹¹ to 10¹³ phages) and the number of phages necessary to isolate and amplify clones in ordinary panning (100 to 1,000 phages/clone); it is possible to screen for an antibody normally having a Kd value on the order of 10⁻⁹ with a library of about 10⁸ clones.
         The process for selecting an antibody against the target antigen by the phage display method is referred to as panning. To be specific, for example, a phage presenting an antigen-specific antibody is concentrated by repeating a series of operations of bringing an antigen-immobilized carrier and a phage library into contact with each other, washing out the unbound phage, thereafter eluting the bound phage from the carrier, and infecting the phage to E. *coli* to propagate it, about 3 to 5 times. As the carrier for immobilizing the antigen, various carriers used in ordinary antigen-antibody reactions or affinity chromatography, for example, insoluble polysaccharides such as agarose, dextran, and cellulose, synthetic resins such as polystyrene, polyacrylamide, and silicon, or microplates, tubes, membranes, columns, beads and the like comprising glass, metal and the like, and surface plasmon resonance (SPR) sensor chips, and the like can be mentioned. For the antigen immobilization, physical adsorption may be used, and a method using a chemical bond used to insolubilize and immobilize a protein or enzyme and the like is also acceptable. For example, a biotin- (strept) avidin system and the like are preferably used. When the endogenous ligand, that is a target antigen, is a small molecule such as a peptide, it is necessary to pay special attention to prevent masking of the portion used as the epitope by conjugating with the carrier. For washing the unbound phage, a blocking solution such as BSA solution (1 to 2 times), a PBS containing a surfactant such as Tween (3 to 5 times) and the like can be used sequentially. There is also a report mentioning that the use of citrate buffer (pH 5) and the like is preferable for the washing. For elution of the specific phage, an acid (e.g., 0.1 M hydrochloric acid and the like) is normally used; cleavage with a specific protease (for example, a gene sequence that encodes the trypsin cleavage site can be introduced into the linkage site between the antibody gene and the coat protein gene. In this case, E. *coli* infection and propagation are possible even if all the coat protein is expressed in the form of a fusion protein because the wild-type coat protein is presented on the surface of the eluted phage), competitive elution with a soluble antigen, or elution by reduction of S-S bond (for example, in the aforementioned CysDisplay^{™}, the antigen-specific phage can be collected by dissociating the antibody and the coat protein by using a suitable reducing agent after performing panning.) is also possible. When elution has been performed with an acid, the eluate is neutralized with Tris and the like, and the eluted phage is then infected to E. *coli,* which is cultured; after which the phage is collected by a conventional method.
         After the phage presenting the antigen-specific antibody is concentrated by panning, the phage is infected to E. *coli* and the cells are sown onto a plate to perform cloning. The phage is again collected, and the antigen binding activity is confirmed by the above-described antibody titer assay (e.g., ELISA, RIA, FIA and the like) or a measurement utilizing FACS or SPR.
         Isolation and purification of the antibody from the selected phage clone that presents the antigen-specific antibody can be performed by, for example, when using a vector incorporating an amber stop codon at the linker site of the antibody gene and the coat protein gene as the phage display vector, infecting the phage to an E. *coli* that does not have amber mutation (e.g., HB2151 strain) to produce and secrete soluble antibody molecules in periplasm or the medium, lysing the cell wall with lysozyme and the like, collecting the extracellular fraction, and purifying using the same purification technique as described above. Provided that the His-tag or c-myc tag has been introduced in advance, the antibody can easily be purified by using IMAC, an anti-c-myc antibody column and the like. When cleavage with a specific protease is utilized in panning, the antibody molecule is separated from the phage surface by an action of the protease, so that the desired antibody can be purified by performing the same purification operation as above mentioned.
         The technology for producing a fully human antibody using a human antibody-producing animal and a phage display human antibody library can also be applied to the production of a monoclonal antibody derived from another animal species. For example, animals widely propagated as domestic animals (domestic fowl) such as bovine, swine, sheep, goat, and chicken, and pet animals such as canines and cats, and the like can be mentioned as the subject animal species. In non-human animals, the utilization of an immunized library is more effective because there are fewer ethical problems concerning artificial immunization with the target antigen.
(3) Preparation of polyclonal antibody
   The polyclonal antibody of the present invention can be manufactured by publicly known methods or modifications thereof. For example, a warm-blooded animal is immunized with an immunogen (protein or peptide antigen) per se, or with a complex of immunogen and a carrier protein formed in a manner similar to the method described above for the manufacture of monoclonal antibodies. The product containing the antibody against the protein of the present invention is collected from the immunized animal followed by separation and purification of the antibody.

In the complex of immunogen and carrier protein used to immunize a warm-blooded animal, the type of carrier protein and the mixing ratio of carrier protein to hapten may be any type and in any ratio, as long as the antibody is efficiently produced to the hapten immunized by crosslinking to the carrier protein. For example, bovine serum albumin, bovine thyroglobulin or hemocyanin is coupled to hapten in a carrier-to-hapten weight ratio of approximately 0.1 to 20, preferably about 1 to 5.

A variety of condensation agents can be used for the coupling of carrier protein to hapten. Glutaraldehyde, carbodiimide, maleimide activated ester and activated ester reagents containing thiol group or dithiopyridyl group are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 1 to 6 weeks and about 2 to 10 times in total.

The polyclonal antibody can be collected from the blood, ascites, etc., preferably from the blood of warm-blooded animal immunized by the method described above.

The polyclonal antibody titer in antiserum can be assayed, by the same procedure as the assay of antibody titer of the antiserum described above. The separation and purification of the polyclonal antibody can be carried out, following the method for the separation and purification of immunoglobulins performed as in the separation and purification of monoclonal antibodies described hereinabove.

The polynucleotide having the base sequence complementary to the target region of target polynucleotides, that is, the polynucleotides hybridizable to target polynucleotides, can be denoted to be 'antisense' to the target polynucleotides.

The antisense polynucleotide having a complementary or substantially complementary base sequence or a part thereof to a base sequence of the polynucleotide (e.g., DNA (hereinafter these DNAs that encode the protein of the present invention are sometimes collectively referred to as the DNA of the present invention in the description of antisense polynucleotide)) that encodes the protein of the present invention, can be any antisense polynucleotide, as long as it has a complementary or substantially complementary base sequence or a part thereof to a base sequence of the polynucleotide (e.g., DNA) that encodes the protein of the present invention, and is capable of suppressing the expression of the polynucleotide.

The base sequence substantially complementary to the DNA of the present invention may include, for example, a base sequence having about 70% or more homology, preferably about 80% or more homology, more preferably about 90% or more homology and most preferably about 95% or more homology, for the region overlapping to the base sequence complementary to the DNA of the present invention (i.e., complementary strand to the DNA of the present invention), and the like. The term 'homology' has the same meaning as the case of the DNA of the present invention mentioned above. Especially in the entire base sequence of the complementary strand to the DNA of the present invention, preferred are (a) an antisense polynucleotide having about 70% or more homology, preferably about 80% or more homology, more preferably about 90% or more homology and most preferably about 95% or more homology, to the complementary strand of the base sequence which encodes the N-terminal region of the protein of the present invention (e.g., the base sequence around the initiation codon) in the case of antisense polynucleotide directed to translation inhibition and (b) an antisense polynucleotide having about 70% or more homology, preferably about 80% or more homology, more preferably about 90% or more homology and most preferably about 95% or more homology, to the complementary strand of the entire base sequence of the DNA of the present invention having intron, in the case of antisense polynucleotide directed to RNA degradation by RNaseH, respectively.

Specific examples include an antisense polynucleotide containing the entire or part of a base sequence complementary or substantially complementary to the base sequence represented by SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15 or SEQ ID NO: 17, preferably an antisense polynucleotide containing the entire or part of a base sequence complementary to the base sequence represented by SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15 or SEQ ID NO: 17, etc.

The antisense polynucleotide having a complementary or substantially complementary base sequence or a part thereof to a base sequence of the DNA of the present invention (hereinafter it will also be called as the antisense polynucleotide of the invention) can be designed and synthesized on the basis of the base sequence information of cloned or identified protein-encoding DNA of the present invention. Such an antisense polynucleotide can inhibit replication or expression of the genes encoding the protein of the invention (hereinafter will also simply called as the 'gene of the present invention'). Accordingly, an antisense polynucleotide of the present invention is hybridizable to RNA transcribed from a gene of the present invention (mRNA or primary transcript) to inhibit the synthesis (processing) or function (translation to a protein) of said mRNA or is capable of modulating and/or controlling the expression of a gene of the present invention via interaction with RNA associated with the gene of the present invention. Polynucleotides complementary to the selected sequences of RNA associated with the gene of the present invention and polynucleotides specifically hybridizable to RNA associated with the gene of the present invention are useful in modulating/controlling the in vivo and in vitro expression of the gene of the present invention, and are useful for the treatment or diagnosis of diseases, etc.

Target region of an antisense polynucleotide of the present invention is not particularly limited in its length as long as the translation into a protein of the present invention is inhibited as a result of hybridization of an antisense polynucleotide, and it may be a whole sequence or a partial sequence of mRNA encoding the protein which can be exemplified by a short strand of about 10 bases and a long strand of mRNA or whole sequence of early transcription product. In consideration of a simple synthesis and an antigenic problem, oligonucleotide comprising about 10 to 40 bases, particularly about 15 to 30 bases is preferable, but it is not limited thereto. In specific, in the genes of the present invention, the 5' end hairpin loop, 5' end 6-base-pair repeats, 5' end untranslated region, translation initiation codon, protein coding region, ORF translation termination codon, 3' end untranslated region, 3' end palindrome region, 3' end hairpin loop, may be selected as preferred target regions of an antisense polynucleotide, though any other region may be selected as a target in the genes of the present invention. For example, an intron-part of the gene can be exemplified as the target region.

Further, the antisense polynucleotide of the present invention may be a polynucleotide in which the translation into a protein is inhibited by hybridizing with mRNA of a protein of the present invention or with early transcription product, and it may as well as be the polynucleotide capable of forming a triplex by binding with the double-stranded DNA which is the gene of the present invention and inhibiting the transcription of RNA. Moreover, it may be a polynucleotide forming a DNA: RNA hybrid and directing the degradation by RNaseH.

To prevent digestion with a hydrolase such as nuclease, etc., the phosphoric acid residue (phosphate) of each nucleotide that constitutes the antisense polynucleotide may be substituted with chemically modified phosphoric acid residues, e.g., phosphorothioate, methyl phosphonate, phosphorodithionate, etc. Also, the sugar (deoxyribose) in each nucleotide may be replaced by a chemically modified structure such as 2'-O-methylation, etc. The base part (pyrimidine, purine) may also be chemically modified and may be any one which hybridizes to a DNA containing the base sequence represented by SEQ ID NO: 1, SEQ ID NO: 3, SEQ ID NO: 5, SEQ ID NO: 7, SEQ ID NO: 9, SEQ ID NO: 11, SEQ ID NO: 13, SEQ ID NO: 15 or SEQ ID NO: 17.

Examples of the antisense polynucleotides include polynucleotides containing 2-deoxy-D-ribose, polynucleotides containing D-ribose, any other type of polynucleotides which are N-glycosides of a purine or pyrimidine base, or other polymers containing non-nucleotide backbones (e.g., commercially available protein nucleic acids and synthetic sequence-specific nucleic acid polymers) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., α anomeric nucleic acids, etc.), and the like. Herein the terms 'nucleoside', 'nucleotide' and 'nucleic acid' are used to refer to moieties that contain not only the purine and pyrimidine bases, but also other heterocyclic bases, which have been modified. Such modifications may include methylated purines and pyrimidines, acylated purines and pyrimidines or other heterocyclic rings. Modified nucleotides and modified nucleotides also include modifications on its sugar moiety, wherein, for example, one or more hydroxyl groups may optionally be substituted with a halogen atom(s), an aliphatic group (s), etc. , or may be converted into the functional groups such as ethers, amines, or the like.

The antisense polynucleotide of the present invention is RNA, DNA or a modified nucleic acid (RNA, DNA). Specific examples of the modified nucleic acid are, but not limited to, sulfur and thiophosphate derivatives of nucleic acids and those resistant to degradation such as polynucleoside amides or oligonucleoside amides. The antisense polynucleotide of the present invention can be modified, for example, based on the following design, that is, by increasing the intracellular stability of the antisense polynucleotide, enhancing the cell permeability of the antisense polynucleotide, increasing the affinity of the nucleic acid to the targeted sense strand to a higher level, or minimizing the toxicity, if any, of the antisense polynucleotide. Many of such modifications are known in the art, as disclosed in J. Kawakami, et al., Pharm. Tech. Japan, Vol. 8, pp. 247 or pp. 395, 1992; Antisense Research and Applications, CRC Press, 1993; etc.

The antisense polynucleotide of the present invention may contain altered or modified sugars, bases or linkages. The antisense polynucleotide may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

In addition, the antisense polynucleotide of the present invention includes a ribozyme capable of specifically cleaving the internal (intron is included in early transcription products) coding region of mRNA which encodes the protein of the present invention or an early transcription product. 'ribozyme' is RNA having enzymatic activity for cleaving nucleic acid, but since recently it is discovered that the oligoDNA having a base sequence of the enzymatic activity site also has the activity for cleaving nucleic acid, the present specification also includes DNA as long as it has a sequence specific enzymatic activity for cleaving nucleic acid. Riobzyme with mostly high-generality includes self-splicing RNA which can be found in infectious RNA such as viroid, a virusoid, etc. , and hammer-head type or hairpin type are known. The hammer-head type exhibits enzymatic activity at about 40 bases, and it is possible to specifically cleave only the target mRNA by complementary arranging several bases (about 10 bases in total) of both ends adjacent to the part having a hammer-head structure. This type of ribozyme has a further advantage that genomic DNA is never targeted as its substrate is only RNA. When mRNA of protein of the present invention forms itself a double-stranded structure, the target sequence can be formed into a single-strand by using hybrid ribozyme coupled with RNA motif derived from viral nucleic acid which specifically binds to RNA helicase [Proc. Natl. Acad. Sci. USA, 98 (10) : 5572-5577 (2001)]. Also, in a case where ribozyme is used in the form of an expression vector having DNA which encodes the ribozyme, the ribozyme can be hybrid ribozyme further coupled with the sequence of modified tRNA so as to promote transport to cytoplasm of a transcriptional product [Nucleic Acids Res., 29 (13): 2780-2788 (2001)].

In the present specification, the antisense polynucleotide of the present invention is also defined to include so-called siRNA which is double-stranded RNA comprising oligoRNA complementary to a partial sequence of coding region (intron is included in early transcription products) in mRNA of the protein of the present invention or early transcription products and a strand complementary to the oligoRNA. The phenomenon of so-called RNA interference (RNAi) in which mRNA complementary to the RNA introduced is degraded by introducing short-stranded mRNA into a cell, is known to occur in nematode, an insect, plant, etc., but after it is confirmed that the phenomenon also occurs in animal cells [Nature, 411 (6836),:, 494-498 (2001)], it is widely used as an alternative technology of ribozyme. siRNA can be appropriately designed on the basis of base sequence information of target mRNA by using a commercially available software (e.g., RNAi Designer; Invitrogen corp.).

The antisense oligonucleotide of the present invention and ribozyme can be prepared by determining a target sequence of mRNA or early transcription product on the basis of a cDNA sequence or genomic DNA sequence of the gene of the present invention, and synthesizing its complementary sequence with the use of a commercially available DNA/RNA automatic synthesizer (Applied Biosytem,Beckman, etc.). siRNA can be prepared according a process comprising synthesizing a sense strand and an antisense strand respectively with the DNA/RNA automatic synthesizer, denaturing in a suitable annealing-buffer solution at about 90 to 95 °C for about 1 minute, and annealing at about 30 to 70 °C for about 1 to 8 hours. In addition, longer double-stranded polynucleotide can be prepared according to a process comprising synthesizing complementary oligonucleotides in an alternately overlapping manner, annealing the oligonucleotides, and ligating with ligase. Alternatively, siRNA can be designed such that it is synthesized as RNA (shRNA) in which a sense strand and an antisense strand are linked via a linker of suitable length (for example, about 3 to 10 bases), and that it can be subjected for processing by an enzyme dicer or the like in an animal cell to which the RNA is introduced. Further, siRNA may be formed by preparing an expression vector in which DNAs encoding a sense strand and an antisense strand are under control of respective Pol III promoter such as U6 or H1, or an expression vector in which DNA encoding an RNA strand, which comprises the sense strand and the antisense strand linked via a linker, is under control of Pol III promoter, and expressing the vector in an animal cell.

Inhibitory activity of the antisense polynucleotide of the present invention can be examined by transformants to which gene of the present invention is introduced, in vivo or in vitro gene expression system of the present invention, or in vivo or in vitro protein translation system of the present invention.

Hereinafter, the use of protein of the present invention or its partial peptide (hereinafter, sometimes merely abbreviated as 'protein of the present invention'), DNA encoding the protein of the present invention or its partial peptide (hereinafter, sometimes merely abbreviated as 'DNA of the present invention'), antibody of the present invention described above, and antisense polynucleotide of the present invention described above, will be explained.

The protein of the present invention can be used as a disease marker as its expression is increased in cancer tissue. In other words, the protein is useful as a marker for early diagnosis in cancer tissue, judgment of symptomatic disease severity, prediction of disease progression.

In specific, by the inhibition of expression and/or activity of the protein of the present invention, an apoptosis in cancer cells is promoted, growth of cancer cells is inhibited, and mobility of cancer cells is inhibited. Therefore, a pharmaceutical comprising the antisense polynucleotide of the present invention, antibody of the present invention, or substance inhibiting the expression and/or activity of protein described above (e.g., low molecular compound) can be used as an agent for promoting the apoptosis in cancer cells and/or inhibiting the growth of cancer cells and/or as an agent for inhibiting mobility of cancer cells, that is, it can be used as preventive/remedy agent for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.) and/or agent for metastasis suppression (preferably, preventive/remedy agent and/or agent for metastasis suppression for e. g. , breast cancer, lung cancer, colon cancer, prostate cancer, ovary cancer, pancreatic cancer, etc.). In the present specification, term 'cancer cell' is used to include cells directing to turn cancerous in the future.
(1) Pharmaceutical comprising antibody of the present invention
   The antibody (neutralizing antibody) of the present invention is capable of neutralizing activity of the protein of the present invention, thereofore can be used as an agent for promoting apoptosis in cancer cells, agent for inhibiting cancer cell growth, and preventive/remedy agent for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.) (preferably, preventive/remedy agent for breast cancer, lung cancer, colon cancer, prostate cancer, ovary cancer, pancreatic cancer, etc.). The term 'neutralizing' in the present invention is defined to cover antibody-dependent cell-mediated cytotoxicity activity (ADCC activity), complement-dependent cytotoxicity activity (CDC activity), inhibition of growth signal in cancer cells (neutralization activity in the narrow sense), induction of an apoptosis, etc. (not limited thereto).
   The preventive/remedy agents for the above diseases and the promoting agent comprising the antibody of the present invention are low-toxic and can be administered as they are in the form of liquid preparations, or as pharmaceutical compositions of suitable preparations to human or mammals (e. g. , rats, rabbits, sheep, swine, bovine, feline, canine, simian, etc.), orally or parenterally (e.g.; intravascularly, subcutaneously, etc.).
   The antibody of the present invention may be administered in itself, or may be administered as an appropriate pharmaceutical composition. The pharmaceutical composition used for the administration may contain a pharmacologically acceptable carrier, diluent or excipient with the antibody of the present invention and salt thereof. Such a pharmaceutical composition is provided in the form of pharmaceutical preparations suitable for oral or parenteral administration.
   Examples of the composition for parenteral administration are injectable preparations, suppositories, etc. The injectable preparations may include dosage forms such as intravenous, subcutaneous, intracutaneous and intramuscular injections, drip infusions, etc. These injectable preparations may be prepared by methods publicly known. For example, the injectable preparations may be prepared by dissolving, suspending or emulsifying the antibody of the present invention described above or the salts thereof in a sterile aqueous medium or oily medium conventionally used for injections. As the aqueous medium for injections, there are, for example, physiological saline, an isotonic solution containing glucose and other auxiliary agents, etc. , which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule. The suppository used for rectal administration may be prepared by blending the antibody described above or the salts thereof with conventional bases for suppositories.
   For example, the composition for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and may contain a carrier, a diluent or excipient conventionally used in the field of pharmaceutical preparations. Examples of the carrier or excipient for tablets are lactose, starch, sucrose, magnesium stearate, etc.
   Advantageously, the pharmaceutical compositions for parenteral or oral use described above are prepared into pharmaceutical preparations with a unit dose suited to fit a dose of the active ingredients. Such unit dose preparations include, for example, tablets, pills, capsules, injections (ampoules), suppositories, etc. The amount of the antibody contained is generally 5 to 500 mg per dosage unit form; it is preferred that the antibody described above is contained in 5 to 100 mg especially in the form of injection, and in 10 to 250 mg for the other forms.
   The dose of the agent described above containing the antibody of the present invention may vary depending upon subject to be administered, target disease, conditions, route of administration, etc. For example, when used for the purpose of treating/preventing breast cancer in adult, it is advantageous to administer the antibody of the present invention intravenously in a dose of about 0.01 to about 20 mg/kg body weight, preferably about 0.1 to about 10 mg/kg body weight and more preferably about 0.1 to about 5 mg/kg body weight, about 1 to 5 times/day, preferably about 1 to 3 times/day. In other parenteral and oral administration, the agent can be administered in a dose corresponding to the dose given above. When the condition is especially severe, the dose may be increased according to the condition.
   The antibody of the present invention may be administered in itself, or may be administered as an appropriate pharmaceutical composition. The pharmaceutical composition used for the administration may contain a pharmacologically acceptable carrier, diluent, or excipient with the antibody or the salts thereof. Such a composition is provided in the form of pharmaceutical preparations suitable for oral or parenteral administration (e.g., intravascular injection, subcutaneous injection, etc.).
   Each composition described above may further contain other active components unless they cause any adverse interaction with the antibody described above due to blending.
   Furthermore, the antibody of the present invention may be used in combination with other drugs, for example, alkylating agents (e.g., cyclophosphamide, ifosfamide, etc.), metabolic antagonists (e.g., methotrexate, 5-fluorouracil, etc.), antitumor antibiotics (e.g., mitomycin, adriamycin, etc.), plant-derived antitumor agents (e.g., vincristine, vindesine, Taxol, etc.), cisplatin, carboplatin, etoposide, irinotecan, etc. The antibody of the present invention and the substance described above may be administered simultaneously or at staggered times to the patient.
(2) Pharmaceutical comprising antisense polynucleotide
   The antisense polynucleotide of the present invention which is capable of binding complementarily to a transcription product of the gene of the present invention and suppressing the expression of the gene is low toxic and can suppress the in vivo functions or actions of the protein or gene of the present invention to induce apoptosis in cancer cells. Thus, the antisense polynucleotide can also be used as an agent for promoting apoptosis in cancer cells, agent for inhibiting cancer cell growth, and preventive/remedy agent for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.) (preferably, preventive/remedy agents for e.g., breast cancer, lung cancer, colon cancer, prostate cancer, ovary cancer, pancreatic cancer, etc.).
   For the use of the antisense polynucleotide of the present invention as a preventive / remedy agent, an agent for promoting apoptosis, an agent for inhibiting growth as above described and the like, it can be prepared into pharmaceutical preparations by publicly known methods, which are provided for administration.
   In addition, for example, the antisense polynucleotide can be administered alone, or the antisense polynucleotide can be inserted into an appropriate vector such as retrovirus vector, adenovirus vector, adenovirus-associated virus vector, etc., and then administered. The antisense polynucleotide may be administered orally or parenterally to human or a mammal (e.g., rat, rabbit, sheep, swine, bovine, feline, canine, simian etc.) in a conventional manner. The antisense polynucleotide may also be administered as it stands, or may be prepared in pharmaceutical preparations together with a physiologically acceptable carrier such as auxiliary substance to promote its uptake, which are then administered by gene gun or through a catheter such as a hydrogel catheter. Alternatively, the antisense polynucleotide may be prepared into an aerosol, which is topically administered into the trachea as an inhaler.
   Further for the purposes of improving pharmacokinetics in the body, prolonging a half-life, and improving intracellular uptake efficiency, the antisense polynucleotide described above may administered alone, or may be prepared into pharmaceutical preparations (injectable preparations) together with a carrier such as liposome, etc. and the preparations may be administered intravenously, subcutaneously, etc.
   A dose of the antisense polynucleotide may vary depending on target disease, subject to be administered, route for administration, etc. For example, where the antisense polynucleotide of the present invention is administered for the purpose of treating breast cancer, the antisense polynucleotide is generally administered to an adult (60 kg body weight) in a daily dose of about 0.1 to 100 mg.
   In addition, the polynucleotide having a base sequence that encodes the protein of the present invention or a part of the base sequence (also referred to as 'sense polynucleotide of the present invention') and the antisense polynucleotide of the present invention may also be used as a nucleotide probe (or primer) for diagnosis to examine the states of expression of the gene of the present invention in tissues or cells.
(3) Screening for drug candidates for disease
   The protein of the present invention is increasingly expressed in cancer tissues. When expression and/or activity of the protein of the present invention are/is inhibited, apoptosis in cancer cell is induced, whereby cancer cell growth is inhibited. Therefore, the compounds or their salts that inhibit the expression and/or activity of the protein of the present invention are useful as agents for promoting apoptosis in cancer cells, agents for inhibiting cancer cell growth, and preventive/remedy agents for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.) (preferably, preventive/remedy agents for breast cancer, lung cancer, colon cancer, prostate cancer, ovary cancer, pancreatic cancer, etc.).

Accordingly, the protein of the present invention is useful as a reagent for screening for the compounds or their salts that inhibit the expression and/or activity of the protein of the present invention.

That is, the present invention provides a method of screening for the compounds or their salts that inhibit the expression and/ or activity of the protein of the present invention, in which the method is characterized in that the protein of the present invention is used.

The method of screening for the compounds or their salts that inhibits the activities of the protein of the present invention is classified broadly into followings;
(a-1) a method of comparing activity of isolated protein of the present invention in the presence and absence of a test compound, and
(a-2) a method of culturing a cell capable of producing the protein of the present invention in the presence of and absence of a test compound, and comparing activity of the protein of the present invention under two different conditions.
   The protein of the present invention used in the screening method (a-1) above can be isolated and purified by using the above-mentioned method of producing the protein of the present invention or its partial peptide.
   The cell capable of producing the protein of the present invention used in the screening method (a-2) above is not particularly limited, as long as it is a human or other warm-blooded animal cell naturally expressing the protein, or a biological specimen (e.g., blood, tissue, organ, etc.) containing thereof. Examples include human breast cancer-cell line MCF7, T47D, etc. For the cells derived from non-human animal blood, tissue, organ, etc., these may be isolated from an organism and then cultured, or a test compound may be administered to an organism and then those biological specimen may be isolated after a given period of time.
   As the cells capable of producing the protein of the present invention, various transformants prepared by the genetic engineering mentioned above can be exemplified. Preferably, animal cells such as COS7 cells, CHO cells, HEK293 cells, etc. are used as the host.
   Examples of the test compound include proteins, peptides, non-peptide compounds, synthetic compounds, fermentation products, cell extracts, plant extracts, animal tissue extracts, and the like. These compounds may be novel compounds or publicly known compounds.
   The activity of the protein of the present invention in the screening method of (a-1) above is assayed by, for example, bringing (i) the protein of the present invention or (ii) the protein of the present invention a test compound, into contact with the warm-blooded animal cell in which the cell growth signal is activated by the protein of the present invention, and then assaying the cell growth or apoptosis induction in cells. The assay for the activity of the protein of the present invention in the screening method of (a-1) above can be carried out by assaying the cell growth or assaying the apoptosis induction in cells capable of producing the protein of the present invention.
   When an apoptosis (cell death) is used as an indicator, the induction level of apoptosis (cell death) is assessed after completing the following processes of suspending a cell in a suitable medium culture or buffer solution, adding a test compound (or not be added) thereto, stimulating the cells with oxidation stress (e.g., H₂O₂ addition) or the like, and then incubating the obtained solution normally at about 20 to 40 °C and preferably about 30 to 37 °C for about 6 to 72 hours.
   Apoptosis (cell death) can be examined by detecting membrane blebbing, reduction of cell size, chromatin condensation, DNA fragmentation, etc., with the morphological observation of using, for example, optical microscope, phase contrast microscope, fluorescence microscope, etc. For example, cells are incubated in multi-well plates, the abraded cells from the plates or membrane blebbed cells were counted using a microscope, and ratio (%) of a dead cell in a total cell in the visual field, is calculated. The cells were observed at different views and average number of those observed was obtained as a cell death induction ratio. In addition, the cell death induction ratio can be derived by staining the dead cells using a dye such as trypan blue, erythrosine B, Negrosin, eosine Y, fluorescein diacetate, acridine orange, ethidium bromide, etc., and then counting the number of cells using a microscope. The cells may also be counted by staining DNA using fluorescent dye of DAPI, Hoechst 33342 etc., and then counting the cells in which chromatin is condensated using a microscope. Alternatively, the apoptosis induction ratio can be assayed by adding dUTP which is labelled with biotin or fluorescent dye with the use of terminal deoxynucleotidyl transferase (TdT), to 3' end of fragmented DNA (TUNEL method), and counting the number of intensely stained cells by using a light microscope, fluorescence microscope, or the like.
   The apoptosis induction ratio can be assayed by counting the number of cells reduced in size or fragmented by assessing the cell size distribution with the use of a particle-size measuring apparatus (e.g., Coulter multisizer). The cell death induction ratio can also be assayed by identifying the alive/dead cells by detecting the reduction in cell size/fragmentation into apoptotic body by using flow cytometry (FACS).
   Further, apoptosis can be biochemically detected by extracting chromosomal DNA from a cell using a conventional method, and carrying out a gel electrophoresis to measure the level of DNA fragmentation using a densitometer or the like. The cell death induction ratio can be assayed by measuring the absorbance decrease in 570-630nm using a microplate reader as 3-(4,5-dimethyl-thiazol-2-yl)-2,5-diphenyl tetrazolium bromide (MTT) is reduced to formazan by alive cells.
   For example, in the screening method (a-1) above, when the activity of the protein of the present invention in the presence of a test compound is inhibited about 20% or more, preferably about 30% or more, or more preferably about 50% or more as compared to the activity of the protein in the absence of a test compound, the test compound can be selected as a substance which inhibits the activity of the protein of the present invention.
   As stated above, the substance inhibiting the expression of the protein of the present invention promotes apoptosis in cancer cells and/or inhibits growth of cancer cells, thereby is useful for preventive/remedy for cancer.
   In other words, the present invention also provides a method of screening for a substance that promotes apoptosis in cancer cells and/or inhibits growth of cancer cells, and a substance for preventive/remedy for cancer, in which the method is characterized in that the expression of a protein in cells capable of producing the protein of the present invention is compared in the presence of and absence of test compound.
   The expression level of the protein of the present invention can be assayed at a transcriptional level by detecting mRNA thereof with the use of polynucleotide hybridizable to polynucleotide encoding the protein of the present invention under high stringent conditions (that is, polynucleotide containing a base sequence encoding the protein of the present invention above mentioned or a part of the base sequence (also referred to as 'sense polynucleotide of the present invention') or the antisense polynucleotide of the present invention). Alternatively, the expression level can be assayed at a translational level by detecting the protein of the present invention with the use of an antibody of the present invention mentioned above.
   Accordingly, the present invention more specifically provides:
(b) a method of screening for a substance that promotes apoptosis in cancer cells and/or inhibits growth of cancer cells, and a substance for preventive/remedy for cancer, in which the method is characterized in that a cell capable of producing the protein of the present invention is cultured in the presence of and absence of a test compound, and the amount of mRNA encoding the protein of the present invention obtained under two different conditions are measured and compared using sense or antisense polynucleotide of the present invention; and,
(c) a method of screening for a substance that promotes apoptosis in cancer cells and/or inhibits growth of cancer cells, and a substance for preventive/remedy for cancer, in which the method is characterized in that a cell capable of producing the protein of the present invention is cultured in the presence of and absence of a test compound, and the amount of protein of the present invention obtained under two different conditions are measured and compared using an antibody of the present invention.

In the screening method according to (b) and (c) above, same cells used for the screening method (a-2) above can be preferably used as a cell capable of producing the protein of the present invention.

For example, a quantification for mRNA of the protein of the present invention or the protein can be specifically carried out as the following:
(i) normal or disease model non-human warm-blooded animal (for example, mouse, rat, rabbit, sheep, swine, bovine, feline, canine, simian, bird, etc.) is administered with a medicine (for example, TNF-α, interleukin-1, Fas, anticancer agent) or applied with a physicochemical stress (for example, UV, active oxygen, ischaemia), and after a given period of time, blood, particular organs (for example, a brain, a liver, a kidney), or tissues or cells isolated from organs are obtained.
   mRNA of the protein of the present invention contained in the obtained cell can be for example, quantified by a process comprising extracting mRNA from cells etc. according to normally used method and by means of, for example, RT-PCR, or also can be quantified by carrying out a Northern blot analysis known per se. On the other hand, the protein of the present invention can be quantified by a Western blot analysis or by a various immunoassay method described below in detail.
(ii) A transformant to which polynucleotide encoding the protein of the present invention is introduced is prepared according to above-mentioned method, and the protein of the present invention or mRNA encoding the protein included in the transformant can be quantified and analyzed in the same manner as in the (i) above.

A screening for a substance which modifies the expression amount of the protein of the present invention can be carried out as the following:
(i) amount of mRNA which encodes the protein of the present invention or the amount of protein of the present invention included in the cell isolated from an animal is quantified and analyzed, a give time of period after (30 minutes to 3 days after, preferably 1 hour to 2 days after, more preferably 1 to 24 hours after) of administering a test compound to the normal or disease model non-human warm-blooded animal before (30 minutes to 24 hours before, preferably 30 minutes to 12 hours before, more preferably 1 to 6 hours before) or after (30 minutes to 3 days after, preferably 1 hours to 2 days after, more preferably 1 to 24 hours after) a given time of period for applying a medicine or physiochemical stress; or administering them concurrently with the medicine or physiochemical stress to the animal; or
(ii) amount of mRNA of the protein of the present invention contained or the amount of protein of the present invention in a transformant is quantified and analyzed, after adding a test compound to a medium culture when culturing the transformant according to an ordinary method and incubating for a given period of time (1 to 7 days after, preferably 1 to 3 days after, more preferably 2 to 3 days after).

Examples of the test compound include peptides, proteins, non-peptide compounds, synthetic compounds, fermentation products, etc., and these compounds may be novel compounds or publicly known compounds.

Quantification for the protein of the present invention in the screening method (c) above specifically includes, for example:
(i) a method comprising competitively reacting the antibody of the present invention, a sample fluid, and a labeled form of the protein of the present invention, and determining the labeled protein of the present invention that binds to the antibody, thereby to quantify the protein of the present invention in the sample fluid; and
(ii) a method comprising simultaneously or continuously reacting a sample fluid, the immobilized antibody of the present invention on a carrier, and a labeled form of another antibody of the present invention, and measuring the amount (activity) of the label on the immobilizing carrier, thereby to quantify the protein of the present invention in the sample fluid.
   In the quantification method of (ii) above, two species of antibodies are desirably the ones that each recognizes the different part in the protein of the present invention. For example, when one antibody recognizes the N-terminal region of the protein of the present invention, another antibody reacting with the C-terminal region of the protein of the present invention is used.
   Examples of labeling agents, which are employed for the aforesaid measuring methods using labeling agents, are radioisotopes, enzymes, fluorescent substances, luminescent substances, etc. Examples of radioisotopes are [¹²⁵I], [¹³¹I], [³H], [¹⁴C], etc. Preferred examples of the enzymes are those that are stable and have a higher specific activity, which include β-galactosidase, β-glucosidase, alkaline phosphatase, peroxidase, malate dehydrogenase, etc. Examples of the fluorescent substances include fluorescamine, fluorescein isothiocyanate, etc. Examples of the luminescent substances are luminol, a luminol derivative, luciferin, lucigenin, etc. Furthermore, a biotin-(strepto)avidin system may be used as well for binding of an antibody or antigen to a labeling agent.
   As the sample fluid, when the protein of the present invention is localized in a cell, a cell homogenate obtained by suspending cells in an appropriate buffer, and then breaking cells by ultrasonication, freeze-thaw cycling, etc., is used, and when the protein of the present invention is secreted outside the cell, cell culture supernatant is used. If necessary, the quantification can be carried out after separating and purifying the protein of the present invention from a homogenate or a cell-culture supernatant. In addition, an intact cell can be used as a specimen as long as the label detection is possible.
   The quantification method of the protein of the present invention using the antibody of the present invention is not particularly limited. Any quantification method may be used, so long as the amount of an antibody, antigen or antibody-antigen complex corresponding to the amount of antigen in a sample fluid can be detected by chemical or physical means and the amount of the antigen can be calculated from a standard curve prepared from standard solutions containing known amounts of the antigen. For such an assay method, for example, nephrometry, the competitive method, the immunometric method, the sandwich method, etc. are suitably used and in terms of sensitivity and specificity, it is preferred to use, for example, the sandwich method described later.
   In the immobilization of antigens or antibodies, physical adsorption may be used. Alternatively, chemical binding that is conventionally used for immobilization/stabilization of proteins, enzymes, etc. may be used as well. Examples of the carrier include insoluble polysaccharides such as agarose, dextran, cellulose, etc.; synthetic resins such as polystyrene, polyacrylamide, silicone, etc.; or glass; and the like.
   In the sandwich method, the immobilized antibody of the present invention is reacted with a sample fluid (primary reaction), then with a labeled form of another antibody of the present invention (secondary reaction), and the amount or activity of the label on the immobilizing carrier is measured, whereby the amount of the protein of the present invention in the sample fluid can be quantified. The order of the primary and secondary reactions may be reversed, and the reactions may be performed simultaneously or at staggered times. The methods of labeling and immobilization can be performed by the methods described above. In the immunoassay by the sandwich method, the antibody used for immobilized or labeled antibodies is not necessarily one species, but a mixture of two or more species of antibody may be used, for example, to increase the measurement sensitivity.
   The antibody of the present invention can also be used in measuring system other than the sandwich method such as in the competitive method, the immunometric method, nephrometry, etc.
   In the competitive assay, the protein of the present invention in a sample fluid and a labeled form of the protein of the present invention are reacted competitively against an antibody, an unreacted labeled antigen (F) is separated from an antibody-bound labeled antigen (B) (B/F separation), and the labeled amount of B or F is determined, thereby to quantify the protein of the present invention in the sample fluid. The present reaction method includes a liquid phase method in which the B/F separation is carried out by using a soluble antibody as an antibody and using polyethylene glycol or a secondary antibody against against the antibody (primary antibody) etc.; and a solid phase method in which a solid-phased antibody is used as a primary antibody (direct method) or a soluble antibody is used as a primary antibody and a solid-phased antibody is used as a secondary antibody (indirect method).
   In the immunometric assay, the protein of the present invention in a sample fluid and a solid phased protein of the present invention are competitively reacted with a given amount of a labeled form of the antibody followed by separating the solid phase from the liquid phase; or an protein of the present invention in a sample fluid and an excess amount of labeled form of the antibody are reacted, then a solid phase protein of the present invention is added to allow an unreacted labeled form of the antibody to bind to the solid phase, and the solid phase is then separated from the liquid phase. Thereafter, the labeled amount in any of the phases is measured to determine the antigen level in the sample fluid.
   In the nephrometry, the amount of insoluble sediment, which is produced as a result of the antigen-antibody reaction in a gel or in a solution, is measured. Even when the amount of a protein of the present invention in a sample fluid is small and only a small amount of the sediment is obtained, a laser nephrometry utilizing laser scattering and the like can be suitably used.
   For applying each of these immunological methods to the quantification method of the present invention, any setting of particular conditions or procedures is not required. The system for assaying the protein of the present invention may be established by applying the usual technical concern of those skilled in the art, in addition to the usual conditions and operating method for the respective methods. For the details of these general technical means, reference can be made to the following reviews and texts.
   For example, see, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid. , Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing), etc.
   In the above manner, the production amount of protein of the present invention in a cell can be quantified with a good sensitivity by using the antibody of the present invention.
   For example, in the screening method of (b) and (c), when the expression amount (the amount of mRNA or protein) of the protein of the present invention in the presence of a test compound is inhibited by about 20% or more, preferably 30% or more, and more preferably about 50% or more, as compared to the expression amount in the absence of a test product, the test compound can be selected as a substance capable of inhibiting the expression of the protein of the present invention.
   The screening kit of the present invention comprises the protein of the present invention or its partial peptide (hereinafter, merely referred to as 'protein of the present invention'). The protein of the present invention may be isolated and purified by any method described above, or may be provided in the form of a cell (warm-blooded animal cell) capable of producing the protein or partial peptide of the invention as above mentioned.
   The screening kit of the present invention can further comprise the antibody, or the sense or antisense polynucleotide of the present invention mentioned above, thereby measures the expression amount of the protein in cells capable of producing the protein of the present invention.
   The screening kit can optionally comprise reaction buffer, blocking buffer, washing buffer, labeling reagent, labeling detection reagent, and the like, in addition to the above mentioned agents.
   The substance (which may be a free from or a salt form) that inhibits the expression and/or activity of the protein of the present invention, which can be obtained by the screening method or the screening kit of the invention, is useful as a safe and low toxic medicament such as agents for promoting apoptosis in cancer cells, agents for inhibiting cancer cell growth or a preventive/remedy agent for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.) (preferably as preventive/remedy agents for breast cancer, lung cancer, colon cancer, prostate cancer, ovary cancer, pancreatic cancer, etc.).
   Where the compound or its salts obtained by using the screening method or screening kit of the present invention is used as the agents for preventive/remedy described above, the compound and the salts can be converted into pharmaceutical preparations in a conventional manner.
   For example, the composition for oral administration includes solid or liquid preparations, specifically, tablets (including dragees and film-coated tablets), pills, granules, powdery preparations, capsules (including soft capsules), syrup, emulsions, suspensions, etc. Such a composition is manufactured by publicly known methods and contains a carrier, diluent, or excipient conventionally used in the field of pharmaceutical preparations. Examples of the carrier or excipient for tablets are lactose, starch, sucrose, magnesium stearate, etc.
   Examples of the composition for parenteral administration are injectable preparations, suppositories, etc. The injectable preparations may include dosage forms such as intravenous, subcutaneous, intracutaneous, and intramuscular injections, drip infusions, intraarticular injections, etc. These injectable preparations may be prepared by methods publicly known, for example, by dissolving, suspending, or emulsifying the compounds or their salts described above in a sterile aqueous medium or oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, e. g. , sesame oil, soybean oil, etc., which may be used in combination with a solubilizing agent such as benzyl benzoate, benzyl alcohol, etc. The injection thus prepared is usually filled in an appropriate ampoule. The suppository used for rectal administration may be prepared by blending the compound or its salt described above with conventional bases for suppositories.
   Advantageously, the pharmaceutical compositions for oral or parenteral use described above are prepared into pharmaceutical preparations with a unit dose suited to fit a dose of the active ingredients. Such unit dose preparations include, for example, tablets, pills, capsules, injections (ampoules), suppositories, etc. The amount of the aforesaid compound contained is generally 5 to 500 mg per dosage unit form; and it is preferably 5 to 100 mg especially in the form of injection, and 10 to 250 mg for the other forms.
   Each composition described above may further contain other active components unless they cause any adverse interaction with the compound described above due to blending.
   Since the pharmaceutical preparation thus obtained is safe and low toxic, it can be administered orally or parenterally to human or a warm-blooded animal (e.g., mouse, rat, rabbit, sheep, swine, bovine, horse, bird, feline, canine, simian, chimpanzee, etc.).
   The dose of the compound or its salt may vary depending on its effect, target disease, subject to be administered, route of administration, etc. For example, when the substance that inhibits the expression and/or activity of the protein of the present invention is orally administered for the purpose of treating breast cancer, the compound or its salt is administered to the adult (as 60 kg body weight) generally in a daily dose of about 0.1 to 100 mg, preferably about 1.0 to 50 mg, and more preferably about 1.0 to 20 mg. In parenteral administration, a single dose of the inhibition substance may vary depending on a subject to be administered, target disease, etc. When the substance that inhibits the expression and/or the activity of the protein of the present invention is generally administered in the form of an injectable preparation to the adult (as 60 kg body weight) for the purpose of treating breast cancer, it is advantageous to administer the compound or its salt in a single dose of about 0.01 to about 30 mg, preferably about 0.1 to about 20 mg, and more preferably about 0.1 to about 10 mg a day, by injection to cancer lesion. For other animal species, the corresponding dose as converted per 60 kg weight can be administered.
(4) Diagnostic agent for cancer
   The antibody of the present invention can specifically recognize the protein of the present invention, thus can be used for quantifying the protein of the present invention in a test fluid. Thus, in the method of screening for the substance that inhibits expression of the protein of the present invention in which the antibody of the invention mentioned above is used, an expression level of the protein of the present invention in animal can be examined by carrying out an immunoassay using a biological specimen (e.g., blood, plasma, urine, biopsy, etc.) collected from a test warm-blooded animal instead of using a cell capable of producing the protein of the present invention, thereby the method can be used for diagnosis of cancer. As a result of the immunoassay, when increase in protein of the present invention in the specimen is detected, it can be diagnosed that as to suffer from cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.), or have a risk to develop them in the future.
   Similarly, by using the sense or antisense polynucleotide of the invention mentioned above as a probe or primer, an abnormality (gene abnormality) of the DNA or mRNA encoding the protein of the present invention in human or other warm-blooded animal (e.g., rat, mouse, hamster, rabbit, sheep, goat, swine, bovine, horse, feline, canine, simian, chimpanzee, bird, etc.) can be detected. Therefore, the polynucleotide is useful as a gene diagnostic agent for detecting amplification of DNA or overexpression of mRNA, etc., and particularly as a diagnostic agent for cancer. The polynucleotide encoding the protein of the present invention or corresponding antisense polynucleotide is not particularly limited as long as it has a length required as a probe or primer (for example, about 15 bases or more).
   The above-described gene diagnosis in which the sense or antisense polynucleotide of the invention is used can be performed by, for example, the publicly known Northern hybridization, quantitative RT-PCR, PCR-SSCP assay, allele-specific PCR, PCR-SSOP assay, DGGE assay, RNase protection assay, PCR-RFLP assay, etc.
   For example, as a result of Northern hybridization or quantitative RT-PCR for RNA fraction extracted from the cells of a warm-blooded animal, when increase expression of protein of the present invention is detected, it can be diagnosed as to suffer from cancer (e. g. , colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.), or have a risk to develop them in the future.
(5) Pharmaceutical comprising the protein of the present invention
   Since the protein of the present invention is overexpressed in cancer, the protein can be used as a cancer vaccine to activate the immune system in patients with cancer.
   For example, the so-called adoptive immunotherapy, which involves culturing potent antigen presenting cells (e.g., dendritic cells) in the presence of protein of the present invention to allow them to engulf the protein and then putting the cells back into the body of a patient, can preferably be used. The dendritic cells, returned back into the body, can induce and activate cytotoxic T cells specific to a cancer antigen whereby to kill cancer cells.
   The protein of the present invention can also be administered to a mammal (e.g. human, simian, mouse, rat, rabbit, swine) safely, for example, as a vaccine preparation to prevent or treat a cancer (e. g. , colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.).
   The vaccine preparation usually contains the protein of the present invention and a physiologically acceptable carrier. Such carrier includes a liquid carrier such as water, saline (including physiological saline), buffer (e.g., phosphate buffer), alcohol (e.g., ethanol), etc.
   The vaccine preparation can be prepared according to a conventional method of manufacturing a vaccine preparation.
   In general, the protein of the present invention is dissolved or suspended in a physiologically acceptable carrier. Alternatively, the protein of the present invention and the physiologically acceptable carrier may be separately prepared and then mixed at use.
   The vaccine preparation may be further formulated with, for example, an adjuvant (e.g., aluminum hydroxide gel, serum albumin, etc.), a preservative (e.g., thimerosal, etc.), a soothing agent (e.g., glucose, benzyl alcohol, etc.), in addition to the protein of the present invention and the physiologically acceptable carrier. Furthermore, the vaccine preparation may also be formulated with, for example, a cytokine (e.g., an interleukin such as interleukin-2, an interferon such as interferon-y) to enhance the production of the antibody to the protein or receptor of the present invention.
   When used as a vaccine preparation, the protein of the present invention may be used in its active form, or may be denatured to enhance the antigenicity. The protein of the present invention may be denatured usually by heating or treating with a protein-denaturing agent (e.g., formalin, guanidine hydrochloride, and urea).
   The thus-obtained vaccine preparation is low toxic and may usually be administered in an injectable form, e.g., subcutaneously, intracutaneously, intramuscularly, or topically into or near a mass of cancer cells.
   The dose of the protein of the present invention varies depending on a target disease, a subject to be administered, a route for administration, etc. For example, for subcutaneous administration of the protein of the present invention to an adult with cancer (60 kg body weight) in an injectable form, the single dose is normally about 0.1 mg to about 300 mg, preferably about 100 mg to about 300 mg. The administration of the vaccine preparation may be carried out once, or 2 to 4 times in total approximately in about every 2 weeks to 6 months to increase the production of the antibody.
(6) DNA transgenic animal
   The present invention provides a non-human mammal bearing a DNA encoding the exogenous protein of the present invention (hereinafter abbreviated as the exogenous DNA of the present invention) or its variant DNA (sometimes simply referred to as the exogenous variant DNA of the present invention).
   That is, the present invention provides:
   (1) A non-human mammal bearing the exogenous DNA of the present invention or its variant DNA;
   (2) The mammal according to (1), wherein the non-human mammal is a rodent;
   (3) The mammal according to (2), wherein the rodent is mouse or rat; and,
   (4) A recombinant vector containing the exogenous DNA of the present invention or its variant DNA and capable of expressing in a mammal; etc.

   The non-human mammal bearing the exogenous DNA of the present invention or its variant DNA (hereinafter simply referred to as the DNA transgenic animal of the present invention) can be prepared by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method, etc. Also, it is possible to transfect the desired exogenous DNA of the present invention into a somatic cell, a living organ, a tissue cell, or the like by the DNA transfection methods, and utilize the transformant for cell culture, tissue culture, etc. In addition, these cells may be fused with the above-described germinal cell by a publicly known cell fusion method to prepare the DNA transgenic animal of the present invention.
   Examples of the non-human mammal that can be used include bovine, swine, sheep, goat, rabbits, canine, feline, guinea pig, hamsters, mice, rats, etc. Above all, preferred are rodents, especially mice (e.g., C57BL/6 strain, DBA2 strain, etc. for a pure line and for a cross line, B6C3F₁ strain, BDF₁ strain B6D2F₁ strain, BALB/c strain, ICR strain, etc.), rats (Wistar, SD, etc.) or the like, since they are relatively short in ontogeny and life cycle and easy to breed, from a standpoint of creating model animals for human disease.
   "Mammals" in a recombinant vector that can be expressed in the mammals include the aforesaid non-human mammals, human, etc.
   The exogenous DNA of the present invention refers to the DNA of the present invention that is once isolated and extracted from mammals, not the DNA of the present invention inherently possessed by the non-human mammals.
   The variant DNA of the present invention includes those resulting from variation (e.g., mutation, etc.) in the base sequence of the original DNA of the present invention, specifically DNAs resulting from base addition, deletion, substitution with other bases, etc. and also includes abnormal DNA.
   The abnormal DNA is intended to mean DNA that expresses the abnormal protein of the present invention and exemplified by the DNA that expresses a protein for suppressing the function of the normal protein of the present invention.
   The exogenous DNA of the present invention may be any one of those derived from a mammal of the same species as, or a different species from, the target animal. In transfecting the DNA of the present invention into the target animal, it is generally advantageous to use the DNA as a DNA construct in which the DNA is ligated downstream a promoter capable of expressing the DNA in the target animal. For example, in the case of transfecting the human DNA of the present invention, a DNA transgenic mammal that expresses the DNA of the present invention to a high level, can be prepared by microinjecting a DNA construct (e.g., vector, etc.) ligated with the human DNA of the present invention downstream various promoters which are capable of expressing the DNA derived from various mammals (e.g., rabbits, canine, feline, guinea pigs, hamsters, rats, mice, etc.) bearing the DNA of the present invention highly homologous to the human DNA, into a fertilized egg of the target non-human mammal (e.g., mouse fertilized egg).
   As expression vectors for the protein of the present invention, there are Escherichia coli-derived plasmids, Bacillus subtilis-derived plasmids, yeast-derived plasmids, bacteriophages such as λ phage, retroviruses such as Moloney leukemia virus, etc. , and animal viruses such as vaccinia virus, baculovirus, etc. Of these vectors, Escherichia coli-derived plasmids, Bacillus subtilis-derived plasmids, or yeast-derived plasmids, etc. are preferably used.
   Examples of these promoters for regulating the DNA expression described above include (i) promoters of DNA derived from viruses (e.g., simian virus, cytomegalovirus, Moloney leukemia virus, JC virus, mammary tumor virus, poliovirus, etc.), and (ii) promoters derived from various mammals (human, rabbits, canine, feline, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylation enzyme (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), peptide chain elongation factor 1α (EF-1α), β actin, α and β myosin heavy chains, myosin light chains 1 and 2, myelin basic protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human peptide chain elongation factor 1α (EF-1α) promoters, human and chicken β actin promoters, etc., which are capable of high expression in the whole body are preferred.
   Preferably, the vectors described above have a sequence that terminates the transcription of the desired messenger RNA in the DNA transgenic animal (generally termed a terminator); for example, a sequence of each DNA derived from viruses and various mammals, and preferably SV40 terminator of the simian virus and the like are used.
   In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.
   The translational region for the normal protein of the present invention can be obtained as the entire genomic DNA or its portion from DNA of liver, kidney, thyroid cell or fibroblast origin from human or various mammals (e.g., rabbits, canine, feline, guinea pigs, hamsters, rats, mice, etc.) and of various commercially available genomic DNA libraries, or obtained by using cDNA prepared by a publicly known method from RNA of liver, kidney, thyroid cell or fibroblast origin as a starting material. Also, an exogenous abnormal DNA can be produced as the mutant translational region through mutation of the translational region of normal protein obtained from the cells or tissues described above by point mutagenesis.
   The translational region can be prepared by a conventional DNA engineering technique, in which the DNA is ligated downstream the aforesaid promoter and if desired, upstream the translation termination site, as a DNA construct capable of being expressed in the transgenic animal. The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA of the present invention in all of the germinal cells and somatic cells thereof.
   The non-human mammal in which the normal exogenous DNA of the present invention has been transfected can be passaged as the DNA-bearing animal under ordinary rearing environment, by confirming that the exogenous DNA is stably retained by crossing.
   By the transfection of the exogenous DNA of the present invention at the fertilized egg cell stage, the DNA is retained to be excess in all of the germinal and somatic cells of the target mammal. The fact that the exogenous DNA of the present invention is excessively present in the germinal cells of the prepared animal after transfection means that the exogenous DNA of the present invention is excessively present in all of the germinal cells and somatic cells of all offspring of the prepared animal. The offspring of the animal of species that inherits the exogenous DNA of the present invention have excessively the exogenous DNA of the present invention in all of the germinal cells and somatic cells thereof.
   It is possible to obtain homozygous animals having the transfected DNA in both homologous chromosomes and breed male and female of the animal so that all the progeny have this DNA in excess.
   In a non-human mammal bearing the normal DNA of the present invention, the normal DNA of the present invention has expressed at a high level, and may eventually develop hyperfunction in the function of the protein of the present invention by accelerating the function of endogenous normal DNA. Therefore, the animal can be utilized as a pathologic model animal for such a disease. For example, using the normal DNA transgenic animal of the present invention, it is possible to elucidate the mechanism of hyperfunction in the function of the protein of the present invention and the pathological mechanism of the disease associated with the protein of the present invention and to investigate how to treat these diseases.
   Furthermore, since a mammal transfected with the exogenous normal DNA of the present invention exhibits a symptom of the increased protein of the present invention liberated, the animal is usable for screening test of agents for preventing/treating diseases associated with the protein of the present invention, for example, agents for preventing/treating, for example, cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer ,testicular cancer, thyroid cancer, pancreatic cancer, brain tumor, blood tumor, etc.).
   On the other hand, a non-human mammal having the exogenous abnormal DNA of the present invention can be passaged under normal breeding conditions as the DNA-bearing animal by confirming stable retention of the exogenous DNA via crossing. Furthermore, the exogenous DNA of interest can be utilized as a starting material by inserting the DNA into the plasmid described above. The DNA construct with a promoter can be prepared by conventional DNA engineering techniques. The transfection of the abnormal DNA of the present invention at the fertilized egg cell stage is preserved to be present in all of the germinal and somatic cells of the target mammal. The fact that the abnormal DNA of the present invention is present in the germinal cells of the prepared animal after DNA transfection means that all of the offspring of the prepared animal have the abnormal DNA of the present invention in all of the germinal and somatic cells. Such an offspring that inherits the exogenous DNA of the present invention will have the abnormal DNA of the present invention in all of the germinal and somatic cells. A homozygous animal having the introduced DNA on both of homologous chromosomes can be acquired, and by crossing these male and female animals, all the offspring can be bred to retain the DNA.
   In a non-human mammal bearing the abnormal DNA of the present invention, the abnormal DNA of the present invention has expressed to a high level, and may eventually develop the function inactive type inadaptability to the protein of the present invention by inhibiting the functions of endogenous normal DNA. Therefore, the animal can be utilized as a pathologic model animal for such a disease. For example, using the abnormal DNA transgenic animal of the present invention, it is possible to elucidate the pathological mechanism of the function inactive type inadaptability to the protein of the present invention and to investigate how to treat the disease.
   More specifically, the animal expressing the abnormal DNA of the present invention at a high level is expected to serve as a model to elucidate the mechanism of the functional inhibition (dominant negative effect) of a normal protein by the abnormal protein of the present invention in the function inactive type inadaptability of the protein of the present invention.
   Since a mammal bearing the abnormal exogenous DNA of the present invention shows a symptom of the increased protein of the present invention liberated, the animal can be also expected to serve for screening test of agents preventing/treating the function inactive type inadaptability of the protein of the present invention, for example, preventive/remedy agents for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.).
   Other potential applications of two kinds of the DNA transgenic animals of the present invention described above further include, for example:
   (i) Use as a cell source for tissue culture;
   (ii) Elucidation of the relation to a peptide that is specifically expressed or activated by the protein of the present invention, by direct analysis of DNA or RNA in tissues of the DNA transgenic animal of the present invention or by analysis of the peptide tissues expressed by the DNA;
   (iii) Research on the function of cells derived from tissues that are usually cultured only with difficulty, using cells in tissues bearing the DNA cultured by a standard tissue culture technique;
   (iv) Screening for a drug that enhances the functions of cells using the cells described in (iii) above; and,
   (v) Isolation and purification of the variant protein of the present invention and preparation of an antibody thereto.

   Furthermore, clinical conditions of a disease associated wit the protein of the present invention, including the function inactive type inadaptability to the protein of the present invention can be determined by using the DNA transgenic animal of the present invention. Also, pathological findings on each organ in a model of a disease associated with the protein of the present invention can be obtained in more detail, leading to the development of a new method for treatment as well as the research and therapy of any secondary diseases associated with the disease.
   It is also possible to obtain a free DNA-transfected cell by withdrawing each organ from the DNA transgenic animal of the present invention, mincing the organ and degrading with a proteinase such as trypsin, etc., and to culture the cells or establish the line of cultured cells. Furthermore, the DNA transgenic animal of the present invention can serve to identify cells capable of producing the protein of the present invention, and to study the association with apoptosis, differentiation or propagation or the mechanism of signal transduction in these properties to inspect any abnormality thereof. Thus, the DNA transgenic animal can provide an effective research material for the protein of the present invention and for investigation of the function and effect thereof.
   To develop a drug for the treatment of diseases associated with the protein of the present invention, including the function inactive type inadaptability to the protein of the present invention, using the DNA transgenic animal of the present invention, an effective and rapid method for screening can be provided by using the method for inspection and the method for quantification, etc. described above. It is also possible to investigate and develop a method for DNA therapy for the treatment of diseases associated with the protein of the present invention, using the DNA transgenic animal of the present invention or a vector capable of expressing the exogenous DNA of the present invention.
(7) Knockout animal
   The present invention provides a non-human mammal embryonic stem cell bearing the DNA of the present invention inactivated and a non-human mammal deficient in expressing the DNA of the present invention.
   Thus, the present invention provides:
   (1) A non-human mammal embryonic stem cell in which the DNA of the present invention is inactivated;
   (2) The embryonic stem cell according to (1), wherein the DNA is inactivated by introducing a reporter gene (e.g., β-galactosidase gene derived from *Escherichia coli*)*;*
   (3) The embryonic stem cell according to (1), which is resistant to neomycin;
   (4) The embryonic stem cell according to (1), wherein the non-human mammal is a rodent;
   (5) The embryonic stem cell according to (4), wherein the rodent is mouse;
   (6) A non-human mammal deficient in expressing the DNA of the present invention, wherein the DNA is inactivated;
   (7) The non-human mammal according to (6), wherein the DNA is inactivated by inserting a reporter gene (e.g., β-galactosidase gene derived from *Escherichia coli)* therein and the reporter gene is capable of being expressed under control of a promoter for the DNA of the present invention;
   (8) The non-human mammal according to (6), which is a rodent;
   (9) The non-human mammal according to (8), wherein the rodent is mouse; and,
   (10) A method of screening for a compound that promotes or inhibits the promoter activity to the DNA of the present invention, or salt thereof, which comprises administering a test compound to the mammal of (7) and detecting expression of the reporter gene.

   The non-human mammal embryonic stem cell in which the DNA of the present invention is inactivated refers to a non-human mammal embryonic stem cell that suppresses the ability of the non-human mammal to express the DNA by artificially mutating the DNA of the present invention carried by the mammmal, or in which the DNA has no substantial ability to express the protein of the present invention (hereinafter sometimes referred to as the knockout DNA of the present invention) by substantially inactivating the activities of the protein of the present invention encoded by the DNA (hereinafter merely referred to as ES cell).
   As the non-human mammal, the same examples as described above apply.
   Techniques for artificially mutating the DNA of the present invention include deletion of a part or all of the DNA sequence and insertion of or substitution with other DNA, by genetic engineering. By these mutations, the knockout DNA of the present invention may be prepared, for example, by shifting the reading frame of a codon or by disrupting the function of a promoter or exon.
   Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, introducing a DNA strand having a DNA sequence constructed by isolating the DNA of the present invention that the desired non-human mammal possesses, inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the functions of exon, or constructed by inserting a DNA sequence that terminates gene transcription (e.g., polyA additional signal, etc.) in the intron between exons, thus inhibiting the synthesis of complete messenger RNA and eventually destroying the gene (hereinafter simply referred to as a targeting vector), to a chromosome of the target animal by, e.g., homologous recombination. The thus-obtained ES cells are subjected to the southern hybridization analysis with a DNA sequence on or near the DNA of the present invention as a probe, or to PCR analysis with a DNA sequence on the targeting vector and another DNA sequence near the DNA of the present invention which is not used in preparing the targeting vector as primers, to select the knockout ES cell of the present invention.
   The parent ES cells to inactivate the DNA of the present invention by homologous recombination, etc. may be of a strain already established as described above, or may originally be established in accordance with a modification of the known method by Evans and Kaufman. For example, in the case of mouse ES cells, currently it is common practice to use ES cells of the 129 strain. However, since their immunological background is obscure, for example, those established from the C57BL/6 mouse or the BDF₁ mouse (F₁ between C57BL/6 and DBA/2), wherein the low ovum availability of the C57BL/6 mouse has been improved by crossing with DBA/2, may be preferably used, for the purpose of obtaining a pure line of ES cells with the clear immunological genetic background instead of above strain and for other purposes. The BDF₁ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.
   In establishing ES cells, blastocysts at 3.5 days after fertilization are commonly used. Besides, embryos are preferably collected at the 8-cell stage and cultured until the blastocyst stage, and then the embryos can be used to efficiently obtain a large number of early stage embryos.
   Although the ES cells used may be of either sex, male ES cells are generally more convenient for generation of a germ cell line chimera. It is also desirable that sexes are identified as soon as possible to save painstaking culture procedure.
   Methods for sex identification of the ES cell include the method in which a gene in the sex-determining region on the Y-chromosome is amplified by the PCR process and detected, as an example. When this method is used, about one colony of ES cells (about 50 cells) is sufficient for sex-determination analysis, while conventional karyotype analysis, requires about 10⁶ cells; therefore, the first selection of ES cells at the early stage of culture can be based on sex identification, and male cells can be selected early, which saves a significant amount of labor at the early stage of culture.
   Also, second selection can be achieved by, for example, confirmation of the number of chromosomes by the G-banding method. It is usually desirable that the chromosome number of the obtained ES cells be 100% of the normal number. However, when it is difficult to obtain the cells having the normal number of chromosomes due to physical operations, etc. in the cell establishment, it is desirable that the ES cell is again cloned to a normal cell (e.g. , in a mouse cell having the number of chromosomes being 2n = 40) after knockout of the gene of the ES cells.
   Although the embryonic stem cell line thus obtained usually shows a very high growth potential, it must be subcultured with great care, since it tends to lose its ontogenic capability. For example, the embryonic stem cell line is cultured at about 37°C in a carbon dioxide incubator (preferably 5% carbon dioxide and 95% air, or 5% oxygen, 5% carbon dioxide and 90% air) in the presence of LIF (1 to 10000 U/ml) on appropriate feeder cells such as STO fibroblasts, treated with a trypsin/EDTA solution (normally 0.001 to 0.5% trypsin/0.1 to about 5 mM EDTA, preferably about 0.1% trypsin/1 mM EDTA) at the time of passage to obtain separate single cells, which are then plated on freshly prepared feeder cells. This passage is normally conducted every 1 to 3 days; it is desirable that when cells are observed at the passage and found to be morphologically abnormal in culture, the cell culture is abandoned.
   Where ES cells are allowed to reach a high density in mono-layers or to form cell aggregates in suspension under appropriate conditions, it is possible to differentiate the ES cells to various cell types, for example, parietal and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C. Doetschman et al., Journal of Embryology and Experimental Morphology, 87, 27, 1985]. The cells deficient in expression of the DNA of the present invention, which are obtained by differentiating ES cells of the present invention, are useful for cytologically studying the protein of the present invention in vitro.
   The non-human mammal deficient in expression of the DNA of the present invention can be identified from a normal animal by measuring the mRNA level in the subject animal by a publicly known method, and indirectly comparing the degrees of expression.
   As the non-human mammal, the same examples given above apply.
   With respect to the non-human mammal deficient in expression of the DNA of the present invention, the DNA of the present invention can be knockout by transfecting a targeting vector, prepared as described above, to mouse embryonic stem cells or mouse egg cells, and conducting homologous recombination in which a targeting vector DNA sequence, wherein the DNA of the present invention is inactivated by the transfection, is replaced with the DNA of the present invention on a chromosome of a mouse embryonic stem cell or mouse egg cell.
   The knockout cells with the disrupted DNA of the present invention can be identified by the southern hybridization analysis using as a probe a DNA sequence on or near the DNA of the present invention, or by the PCR analysis using as primers a DNA sequence on the targeting vector and another DNA sequence at the proximal region of other than the DNA of the present invention derived from mouse used in the targeting vector. When non-human mammal stem cells are used, a cell line wherein the DNA of the present invention is inactivated by homologous recombination is cloned; the resulting cells are injected to, e.g., a non-human mammalian embryo or blastocyst, at an appropriate stage such as the 8-cell stage. The resulting chimeric embryos are transplanted to the uterus of the pseudopregnant non-human mammal. The resulting animal is a chimeric animal constructed with both cells having the normal locus of the DNA of the present invention and those having an artificially mutated locus of the DNA of the present invention.
   When some germ cells of the chimeric animal have a mutated locus of the DNA of the present invention, an individual, which entire tissue is composed of cells having a mutated locus of the DNA of the present invention can be selected from a group of inidviduals obtained by crossing between such a chimeric inidvidual and a normal inidvidual, e.g., by coat color identification, etc. The individuals thus obtained are normally deficient in heterozygous expression of the protein of the present invention. The individuals deficient in homozygous expression of the protein of the present invention can be obtained from offspring of the intercross between those deficient in heterozygous expression of the protein of the present invention.
   When an egg cell is used, a DNA solution may be injected, e.g., into the nucleus of the egg cell by microinjection thereby to obtain a transgenic non-human mammal having a targeting vector introduced in its chromosome. From such transgenic non-human mammals, those having a mutation at the locus of the DNA of the present invention can be obtained by selection based on homologous recombination.
   As described above, the individuals in which the DNA of the present invention is knockout can be passaged and reared under ordinary rearing conditions, after the DNA of the individuals obtained by their crossing have also proven to have been knockout.
   Furthermore, the germ line may be obtained and retained by conventional methods. That is, by crossing male and female animals each having the inactivated DNA, homozygous animals having the inactivated DNA in both homologous chromosomes can be obtained. The homozygous animals thus obtained may be reared so that one normal animal and plural homozygotes are produced from a mother animal to efficiently obtain such homozygotes. By crossing male and female heterozygotes, homozygotes and heterozygotes having the inactivated DNA are proliferated and passaged.
   The non-human mammal embryonic stem cell, in which the DNA of the present invention is inactivated, is very useful for preparing a non-human mammal deficient in expression of the DNA of the present invention.
   Since the non-human mammal deficient in expression of the DNA of the present invention lacks various biological activities derived from the protein of the present invention, such an animal can be a model of a disease due to inactivated biological activities of the protein of the present invention and thus, is useful to investigate the causes for and therapy for these diseases.
(8a) Method of screening for the compound having a therapeutic/prophylactic effect on diseases caused by deficiency, damages, etc. of the DNA of the present invention
   The non-human mammal deficient in expression of the DNA of the present invention can be employed for screening for the compound having a therapeutic/prophylactic effect on diseases caused by deficiency, damages, etc. of the DNA of the present invention.
   That is, the present invention provides a method of screening for the compound having a therapeutic/prophylactic effect on diseases, e. g. , cancer, caused by deficiency, damages, etc. of the DNA of the present invention, or salt thereof, which comprises administering a test compound to a non-human mammal deficient in expression of the DNA of the present invention and, observing and measuring a change occurred in the animal.
   As the non-human mammal deficient in expression of the DNA of the present invention, which can be employed for the screening method, the same examples as described above apply.
   Examples of the test compound include peptides, proteins, non-peptide compounds, synthetic compounds, fermentation products, cell extracts, plant extracts, animal tissue extracts, blood plasma, etc. These compounds may be novel compounds or publicly known compounds.
   Specifically, the non-human mammal deficient in expression of the DNA of the present invention is treated with a test compound, comparison is made with an intact animal for control, and a change in each organ, tissue, disease conditions, etc. of the animal is used as an indicator to assess the therapeutic/prophylactic effects of the test compound.
   For treating a test animal to be tested with a test compound, for example, oral administration, intravenous injection, etc. are applied, and the treatment can be appropriately selected depending on conditions of the test animal, properties of the test compound, etc. Furthermore, a dose of the test compound to be administered can be appropriately chosen depending on the way of administration, nature of the test compound, etc.
   For screening for the compound having a therapeutic/prophylactic effect on a cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, testicular cancer, thyroid cancer, pancreatic cancer, brain tumor, blood tumor, etc.), a test compound is administered to the non-human mammal deficient in expression of the DNA of the present invention. Differences in incidence of cancer or differences in degree of healing of cancer from the group administered with no test compound are observed in the tissues described above over time.
   In the screening method, when a test compound is administered to a test animal, the test compound can be selected as a compound having therapeutic/prophylactic effects on the above-mentioned diseases, if the symptoms described above in the animal are relieved by about 10% or more, preferably about 30% or more, more preferably about 50% or more.
   Since a compound obtained using the screening method is a compound selected from the above test compounds, and have therapeutic/prophylactic effects on diseases caused by deficiency, damage, etc. of the protein of the present invention, it can be used as a safe and low toxic medicament such as a prophylactic/therapeutic agent for the disease. Further, a compound derived from the compound that is obtained from the above-mentioned screening can also be used as well.
   The compound obtained by the screening method above may form salts, and may be used in the form of salts with physiologically acceptable acids (e.g., inorganic acids, organic acids, etc.) or bases (e.g., alkali metals, etc.), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e. g. , acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.
   A pharmaceutical comprising the compound obtained by the above screening method or salts thereof can be manufactured in a manner similar to the method for preparing the pharmaceutical comprising the protein of the present invention described hereinabove.
   Since the pharmaceutical preparation thus obtained is safe and low toxic, it can be administered to human or a mammal (e.g., rat, mouse, guinea pig, rabbit, sheep, swine, bovine, horse, feline, canine, simian, etc.).
   The dose of the compound or its salt may vary depending upon target disease, subject to be administered, route of administration, etc. For example, when the compound is orally administered, the compound is administered to the adult patient with breast cancer (as 60 kg body weight) generally in a daily dose of about 0.1 to 100 mg, preferably about 1.0 to 50 mg and more preferably about 1.0 to 20 mg. In parenteral administration, a single dose of the compound may vary depending upon subject to be administered, target disease, etc. For example, when the compound is administered to the general adult patient with breast cancer (as 60 kg body weight) in the form of an injectable preparation, it is advantageous to administer the compound in a dose of about 0.01 to about 30 mg, preferably about 0.1 to about 20 mg and more preferably about 0.1 to about 10 mg a day, by intravenous injection. For other animal species, the corresponding dose as converted per 60 kg weight can be administered.
(8b) Method of screening for a compound that promotes or inhibits the activity of a promoter to the DNA of the present invention
   The present invention provides a method of screening for a compound or its salts that promote or inhibit the activity of a promoter to the DNA of the present invention, which comprises administering a test compound to a non-human mammal deficient in expression of the DNA of the present invention and detecting the expression of a reporter gene.

In the screening method described above, an animal in which the DNA of the present invention is inactivated by introducing a reporter gene and the reporter gene is expressed under control of a promoter to the DNA of the present invention is used as the non-human mammal deficient in expression of the DNA of the present invention, among the aforesaid non-human mammals deficient in expression of the DNA of the present invention.

The same examples of the test compound apply to specific compounds described above.

As the reporter gene, the same specific examples apply to this screening method. Preferably, there are used β-galactosidase gene (lacZ), soluble alkaline phosphatase gene, luciferase gene and the like.

Since the reporter gene is present under control of a promoter to the DNA of the present invention in the non-human mammal deficient in expression of the DNA of the present invention wherein the DNA of the present invention is substituted with the reporter gene, the activity of the promoter can be detected by tracing the expression of a substance encoded by the reporter gene.

When a part of the DNA region encoding the protein of the present invention is substituted with, e.g., β-galactosidase gene (lacZ) derived from Escherichia coli, β-galactosidase is expressed in a tissue where the protein of the present invention should originally be expressed, instead of the protein of the present invention. Thus, the state of expression of the protein of the present invention can be readily observed in vivo of an animal by staining with a reagent, e.g., 5-bromo-4-chloro-3-indolyl-β-galactopyranoside (X-gal) which is substrate for β-galactosidase. Specifically, a mouse deficient in the protein of the present invention, or its tissue section is fixed with glutaraldehyde, etc. After washing with phosphate buffered saline (PBS), the sample is reacted with a staining solution containing X-gal at room temperature or about 37 °C for approximately 30 minutes to an hour. After the β-galactosidase reaction is terminated by washing the tissue preparation with 1 mM EDTA/PBS solution, the color development is observed. Alternatively, mRNA encoding lacZ may be detected in a conventional manner.

The compound or salts thereof obtained using the screening method described above are compounds that are selected from the test compounds described above and that promote or inhibit the promoter activity to the DNA of the present invention.

The compound obtained by the screening method above may form salts, and may be used in the form of salts with physiologically acceptable acids (e.g., inorganic acids, etc.) or bases (e.g., alkali metals, etc.) or the like, especially in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The compound or its salts that inhibit the promoter activity to the DNA of the present invention can inhibit the expression of the protein of the present invention to inhibit the functions of the protein. Thus, the compound or its salts are useful as preventive/remedy agents for cancer (e.g., colon cancer, breast cancer, lung cancer, prostate cancer, esophageal cancer, gastric cancer, liver cancer, biliary tract cancer, spleen cancer, renal cancer, bladder cancer, uterine cancer, ovary cancer, testicular cancer, thyroid cancer, pancreatic cancer, brain tumor, blood tumor, etc.).

In addition, compounds derived from the compound obtained by the screening described above may be used as well.

A pharmaceutical comprising the compound obtained by the above screening method or salts thereof can be manufactured in a manner similar to the method for preparing the pharmaceutical comprising the protein of the present invention described above or salt thereof.

Since the pharmaceutical preparation thus obtained is safe and low toxic, it can be administered to human or a mammal (e.g., rat, mouse, guinea pig, rabbit, sheep, swine, bovine, horse, feline, canine, simian, etc.).

A dose of the compound or salts thereof may vary depending on target disease, subject to be administered, route for administration, etc.; when the compound that inhibits the promoter activity to the DNA of the present invention is orally administered, the compound is administered to the adult patient (as 60 kg body weight) with breast cancer normally in a daily dose of about 0.1 to 100 mg, preferably about 1.0 to 50 mg and more preferably about 1.0 to 20 mg. In parenteral administration, a single dose of the compound varies depending on subject to be administered, target disease, etc. but when the compound of inhibiting the promoter activity to the DNA of the present invention is administered to the general adult patient (as 60 kg body weight) with breast cancer in the form of injectable preparation, it is advantageous to administer the compound intravenously by injection to the patient in a daily dose of about 0.01 to about 30 mg, preferably about 0.1 to about 20 mg and more preferably about 0.1 to about 10 mg. For other animal species, the corresponding dose as converted per 60 kg weight can be administered.

As stated above, the non-human mammal deficient in expression of the DNA of the present invention is extremely useful for screening for the compound or its salt that promotes or inhibits the promoter activity to the DNA of the present invention and, can greatly contribute to elucidation of causes for various diseases due to deficiency in expression of the DNA of the present invention or for the development of prophylactic/therapeutic agents for these diseases.

In addition, a so-called transgenic animal (gene transferred animal) can be prepared by using a DNA containing the promoter region of the protein of the present invention, ligating genes encoding various proteins at downstream of the promoter and injecting the resultant into egg cell of an animal. It is thus possible to synthesize the protein therein specifically and study its activity in vivo. When an appropriate reporter gene is ligated to the promoter site described above and a cell line that expresses the gene is established, the resulting system can be utilized as the search system for a low molecular compound having the action of specifically promoting or inhibiting the in vivo productivity of the protein itself of the present invention.

In the specification, where bases, amino acids, etc. are denoted by their codes, they are expressed based on conventional codes in accordance with the IUPAC-IUB Commission on Biochemical Nomenclature or by the common codes in the art, examples of which are shown below. For amino acids that may have the optical isomer, L form is presented unless otherwise indicated.
- DNA: : deoxyribonucleic acid
- cDNA: :complementary deoxyribonucleic acid
- A: : adenine
- T: : thymine
- G: : guanine
- C: : cytosine
- RNA: : ribonucleic acid
- mRNA :: messenger ribonucleic acid
- dATP :: deoxyadenosine triphosphate
- dTTP :: deoxythymidine triphosphate
- dGTP :: deoxyguanosine triphosphate
- dCTP :: deoxycytidine triphosphate
- ATP: : adenosine triphosphate
- EDTA :: ethylenediaminetetraacetic acid
- SDS: : sodium dodecyl sulfate
- Gly: : glycine
- Ala: : alanine
- Val: : valine
- Leu: : leucine
- Ile: : isoleucine
- Ser: : serine
- Thr: : threonine
- Cys: : cysteine
- Met: : methionine
- Glu: : glutamic acid
- Asp: : aspartic acid
- Lys: : lysine
- Arg: : arginine
- His: : histidine
- Phe: : phenylalanine
- Tyr: : tyrosine
- Trp: : tryptophan
- Pro: : proline
- Asn: : asparagine
- Gln: : glutamine
- pGlu: : pyroglutamic acid
- Sec: : selenocysteine

Substituents, protecting groups and reagents frequently used in this specification are presented by the codes described below.
- Me: : methyl group
- Et: : ethyl group
- Bu: : butyl group
- Ph: : phenyl group
- TC: : thiazolidine-4(R)-carboxamido group
- Tos: : p-toluenesulfonyl
- CHO: : formyl
- Bzl: : benzyl
- Cl₂-Bzl: : 2,6-dichlorobenzyl
- Bom: : benzyloxymethyl
- Z: : benzyloxycarbonyl
- Cl-Z: :2-chlorobenzyloxycarbonyl
- Br-Z: :2-bromobenzyloxycarbonyl
- Boc: : t-butoxycarbonyl
- DNP: : dinitrophenyl
- Trt: : trityl
- Bum: : t-butoxymethyl
- Fmoc: :N-9-fluorenyl methoxycarbonyl
- HOBt: :1-hydroxybenztriazole
- HOOBt: :3,4-dihydro-3-hydroxy-4-oxo-1,2,3-benzotriazine
- HONB: :1-hydroxy-5-norbornene-2,3-dicarboxyimide
- DCC: : N,N'-dicyclohexylcarbodiimide

The sequence identification numbers in the sequence listing of the specification indicate the following sequences.
[SEQ ID NO: 1]
   This shows the base sequence of DNA (CDS) encoding LY6G6D.
[SEQ ID NO: 2]
   This shows the amino acid sequence of LY6G6D.
[SEQ ID NO: 3]
   This shows the base sequence of DNA (CDS) encoding PTPRO.
[SEQ ID NO: 4]
   This shows the amino acid sequence of PTPRO.
[SEQ ID NO: 5]
   This shows the base sequence of DNA (CDS) encoding TM4SF17a.
[SEQ ID NO: 6]
   This shows the amino acid sequence of TM4SF17a.
[SEQ ID NO: 7]
   This shows the base sequence of DNA (CDS) encoding TM4SF17b.
[SEQ ID NO: 8]
   This shows the amino acid sequence of TM4SF17b.
[SEQ ID NO: 9]
   This shows the base sequence of DNA (CDS) encoding TM4SF17c.
[SEQ ID NO: 10]
   This shows the amino acid sequence of TM4SF17c.
[SEQ ID NO: 11]
   This shows the base sequence of DNA (CDS) encoding TM4SF17d.
[SEQ ID NO: 12]
   This shows the amino acid sequence of TM4SF17d.
[SEQ ID NO: 13]
   This shows the base sequence of DNA (CDS) encoding TM4SF17e.
[SEQ ID NO: 14]
   This shows the amino acid sequence of TM4SF17e.
[SEQ ID NO: 15]
   This shows the base sequence of DNA (CDS) encoding TM4SF17f.
[SEQ ID NO: 16]
   This shows the amino acid sequence of TM4SF17f.
[SEQ ID NO: 17]
   This shows the base sequence of DNA (CDS) encoding TM4SF17h.
[SEQ ID NO: 18]
   This shows the amino acid sequence of TM4SF17h.
[SEQ ID NO: 19]
   This shows the RNA sequence forming siRNA-1 with SEQ ID NO: 20.
[SEQ ID NO: 20]
   This shows the RNA sequence forming siRNA-1 with SEQ ID NO: 19.
[SEQ ID NO: 21]
   This shows the RNA sequence forming siRNA-2 with SEQ ID NO: 22.
[SEQ ID NO: 22]
   This shows the RNA sequence forming siRNA-2 with SEQ ID NO: 21.
[SEQ ID NO: 23]
   This shows the RNA sequence forming siRNA-3 with SEQ ID NO: 24.
[SEQ ID NO: 24]
   This shows the RNA sequence forming siRNA-3 with SEQ ID NO: 23.
[SEQ ID NO: 25]
   This shows the base sequence of the primer LY-HindATG.
[SEQ ID NO: 26]
   This shows the base sequence of the primer LY-TAGXba.
[SEQ ID NO: 27]
   This shows the base sequence of the primer LY-CoKoATG.
[SEQ ID NO: 28]
   This shows the base sequence of the primer LY-OrKoATG.
[SEQ ID NO: 29]
   This shows the base sequence of the primer LY-TAGXba.
[SEQ ID NO: 30]
   This shows the base sequence of the primer DESNHE-F.
[SEQ ID NO: 31]
   This shows the base sequence of the primer DESNHE-R.
[SEQ ID NO: 32]
   This shows the base sequence of the synthetic DNA His-FNdeBam.
[SEQ ID NO: 33]
   This shows the base sequence of the synthetic DNA His-RNdeBam.
[SEQ ID NO: 34]
   This shows the base sequence of the LY6G-FNDE.
[SEQ ID NO: 35]
   This shows the base sequence of LY6G-RNHE.
[SEQ ID NO: 36]
   This shows the base sequence of the synthetic DNA HAT-F.
[SEQ ID NO: 37]
   This shows the base sequence of the synthetic DNA HAT-R.
[SEQ ID NO: 38]
   This shows the base sequence of the siRNA1.
[SEQ ID NO: 39]
   This shows the base sequence of the siRNA2.
[SEQ ID NO: 40]
   This shows the base sequence of the primer 1.
[SEQ ID NO: 41]
   This shows the base sequence of the primer 2.
[SEQ ID NO: 42]
   This shows the base sequence of the primer 3.
[SEQ ID NO: 43]
   This shows the base sequence of the primer 4.
[SEQ ID NO: 44]
   This shows the base sequence of the primer 5.
[SEQ ID NO: 45]
   This shows the base sequence of the primer 6.
[SEQ ID NO: 46]
   This shows the base sequence of the primer 7.
[SEQ ID NO: 47]
   This shows the base sequence of the primer 8.
[SEQ ID NO: 48]
   This shows the base sequence of the primer 9.
[SEQ ID NO: 49]
   This shows the base sequence of the primer 10.
[SEQ ID NO: 50]
   This shows the base sequence of the primer 11.
[SEQ ID NO: 51]
   This shows the base sequence of the primer 12.

### Examples

Hereinafter, the present invention will be described in more detail with reference to EXAMPLES and REFERENCE EXAMPLES, but the invention is not limited thereto.

### Example 1

### Inhibition of cell growth in human colon cancer cell line by administering siRNA against the LY6G6D gene

Human colon cancer cell line SW480 purchased from American Type Culture Collection (ATCC) was peeled off from the tissue culture dish using trypsin-EDTA (Invitrogen corp.), and carried out a transfection in accordance with the protocol attached to Nucleofector Kit T for Cell line (Amaxa).

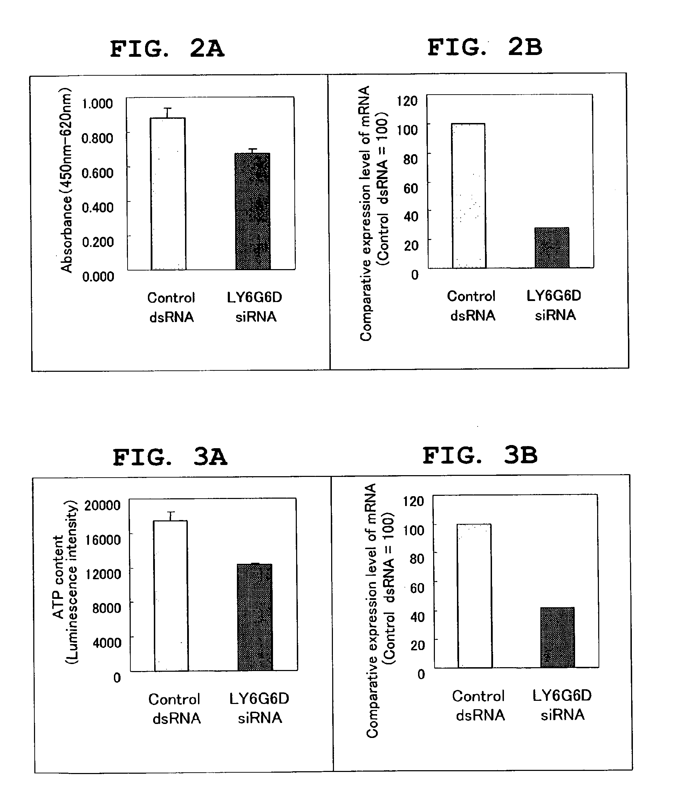

Specifically, siRNA-1 having activity which cleaves mRNA of the LY6G6D gene was produced by hybridizing RNA fragment of SEQ ID NO: 19 with that of SEQ ID NO: 20. In the same method, siRNA-2 was produced by using RNA fragments of SEQ ID NO: 21 and SEQ ID NO: 22, and siRNA-3 was produced by using RNA fragments of SEQ ID NO: 23 and SEQ ID NO: 24. As said RNA fragment, a fragment which 2 bases were added on 3'-terminal with 2'-deoxythymidine thereof was used. Three kinds of siRNAs prepared were mixed by equal quantity, and provided to a transfection (hereinafter abbreviated as siRNA-C). As a control, siRNA (B-Bridge, hereinafter abbreviated as the non-silencing dsRNA), which ensurely exhibits no non-specific RNAi effect against a cell was used. Million cells were suspended in 100µl of T solution (Amaxa), 40 pmol of siRNA against LY6G6D gene, or 40 pmol of non-silencing dsRNA was added, and siRNA transfection was carried out with Nucleofector (Amaxa) T-20 program. After the cells were washed with Leibovitz's L-15 medium [culture medium] (Invitrogen corp.) containing 10% fetal bovine serum (JRH), cells were cultivated at 37°C for 24 hours in the culture medium. After the cultivation, the cells were collected, plated on a 96-well flat-bottomed tissue culture plate with a cell density of 2,000 cells per a well, and during the incubation, cells were further cultivated for 5 days in the culture medium. To the cell culture, 10 µl of Cell Counting Kit-8 reagent (DOJINDO) was added per 1 well, then further cultivated for 3 hours. The mitochondria respiratory activity of the culture after the reaction was determined by measuring the absorbance at 450nm with reference to the absorbance of 620nm wavelength. As a result, the siRNA administered cell against LY6G6D gene showed an luminescence intensity of approximately 23 % lower than the non-silencing dsRNA administered cell, indicating that there is a statistically significant difference (P<0.001) (FIG. 2A).

### Example 2

### Decrease in expression level of mRNA of LY6G6D gene by administering siRNA against LY6G6D gene

Cells collected 24 hours after the transfection in Example 1 were extracted by RNeasy Mini Total RNA Kit (QIAGEN) to obtain the total RNA. A reverse transcription was carried out by using total RNA of about 400 ng as a template and TaqMan Reverse Transcription Reagents (Applied Biosystems) in accordance with the protocol enclosed. For the measurement of expression level of the LY6G6D gene, quantitative PCR reaction was carried out by using cDNA corresponding to 4 ng of the total RNA as a template, and 20µL of reaction liquid which was prepared by adding 10 µL of TaqMan Universal PCR Master Mix (Applied Biosystems), and 1 µL of TaqMan Reagent (Applied Biosystems) containing primer set amplifying base sequence derived from a portion of the second exon and the third exon of the LY6G6D gene. PCR reaction was carried out by reacting at 50°C for 2 minutes, 95°C for 10 minutes and then repeating 40 times the cycle set to include 95°C for 15 seconds and 60°C for 1 minute. On the other hand, the expression level of β-actine gene contained in the template cDNA of same amount was measured by TaqMan β-actine Control Reagent (Applied Biosystems) and used as the internal standard.

In the group administered with siRNA against the LY6G6D gene, the expression level of mRNA of the LY6G6D gene was decreased by 53 % as compared to the group administered with the non-silencing dsRNA which was used as a negative control. These results revealed that the inhibition of cell growth in human colon cancer cell line SW480 was induced by the decrease in expression level of the LY6G6D gene(FIG. 2B).

### Example 3

### Inhibition of cell growth in human lung cancer cell line by administering siRNA of PTPRO gene

Human colon cancer cell line H-29, purchased from American Type Culture Collection (ATCC), was suspended in McCoy's 5A medium (Invitrogen corp.) containing 10% fetal bovine (JRH), and then cultured for 4 days at 37°C under a 5% carbon dioxide gas flow, followed by siRNA transfection.

In specific, 3 kinds of siRNAs (B-Bridge) having activity which cleave mRNA of the PTPRO gene were mixed and provided to transfection. As a control, non-silencing dsRNA used in Example 1 was used. 3µL of solution containing 230 pmol of the siRNA against PTPRO gene, or 230 pmol of the non-silencing dsRNA was mixed with 100µl of Nucleofector Solution R (Amaxa biosystems) in which million cells of HT-29 was suspended, and then transfection was carried out with Nucleofector W-017 program. Full amount of this mixture was added to 4 mL of HT-29 cell culture, and cultured for 24 hours. After the incubation, the cells were collected, and plated on a 96-well flat-bottomed tissue culture plate with a cell density of 5,000 cells/well, and then further cultured 2 days at 37°C in HT-29 cell culture with 5% carbon dioxide gas air flow. To the cell culture, Cell Counting Kit-8 reagent (Wakojunyaku) were added in 10 µl/well, then left stand for additional 2 hours at 37°C with 5% carbon dioxide gas air flow. The number of cells was determined by measuring the absorbance at 450nm. The results revealed that the siRNA administered cell against PTPRO gene showed the absorption of light of approximately 20 % lower than the non-silencing dsRNA administered cell, indicating that there was a statistically significant difference (P<0.001).

### Example 4

### Decrease in expression level of mRNA of the PTPRO gene by administering siRNA of PTPRO gene

Human colon cancer cell line H-29 used in Example 3, was suspended in McCoy's 5A medium (Invitrogen corp.) containing 10% fetal bovine (JRH), and then cultured for 4 days at 37°C with 5% carbon dioxide gas air flow, and then siRNA of transfection was carried out. After the transfection, incubation was carried out another 24 hours, the total RNA was extracted by RNeasy Mini Total RNA Kit (QIAGEN). Using as a template 400 ng of the total RNA, reverse transcription was carried out by TaqMan Reverse Transcription Reagents (Applied Biosystems) in accordance with the protocol attached thereto. For the measurement of expression level of the PTPRO gene, quantitative PCR was carried out to examine the expression level of the PTPRO gene, by using as a template cDNA corresponding to 40 ng of the total RNA, and 20µL of reaction liquid which has prepared by adding 10 µL of TaqMan Universal PCR Master Mix (Applied Biosystems), and 1 µL of TaqMan Reagent (Applied Biosystems) containing primer set amplifying base sequence derived from a portion of the 15th exon and the 16th exon of the PTPRO gene. PCR was carried out by reacting at 50°C for 2 minutes, 95°C for 10 minutes and then repeating 40 times the cycle set to include 95°C for 15 seconds and 60°C for 1 minute. On the other hand, expression level of β-actine gene contained within the same amount of the template cDNA were measured by TaqMan β-actine Control Reagent (Applied Biosystems) to use as the internal standard.

In the group administered with siRNA against the PTPRO gene, the expression level of mRNA of PTPRO was decreased by 70 % as compared to the group administered with the non-silencing dsRNA which was used as a negative control. These results revealed that the inhibition of cell growth in human colon cancer cell line HT-29 was induced by the decrease in expression level of the PTPRO gene.

### Example 5

### Inhibition of cell growth in human breast cancer cell line by administering siRNA of the TM4SF17 gene

Human breast cancer cell line MDA-MB-231, purchased from American Type Culture Collection (ATCC), was suspended in McCoy's L-15 medium (Invitrogen corp.) containing 10% fetal bovine (JRH), and plated on a 96-well flat-bottomed tissue culture plate (BD Falcon) to with a cell density of 2,000 cells/well, and then incubated overnight at 37°C, followed by siRNA transfection.

Specifically, 3 kinds of the siRNAs having activity which cut all mRNA of the TM4SF17a gene, TM4SF17b gene, and TM4SF17c gene, and mixed and provided to transfection. For control, non-silencing dsRNA) used in the example 1 was used. 4 pmol of the siRNA against TM4SF17 gene, or 4 pmol of the non-silencing dsRNA was added to 10µl of Opti-MEM I (Invitrogen corp.), and mixed with 10µl of Opti-MEM I (Invitrogen corp.) in which 0.2µl of Lipofectamine 2000 (Invitrogen corp.) was added, and then left stand for 20 minutes at room temperature. Full amount of the above-mentioned mixture was added to MDA-MB-231 cell culture, and incubated further for 24 hours, and then the medium were replaced. These cells were further cultured for 3 days at 37°C in McCoy's L-15 medium (Invitrogen corp.) containing 10% fetal bovine (JRH). After removing the medium, the plate was allowed to stand for more than 30 minutes and more at -80°C, and then left for another 5 minutes at room temperature. To the plate, aqueous solution containing 0.25% CyQUANT GR reagent (Molecular Probes) and 5% cell lysis buffer(Molecular Probes) were added in 200 µl/well, and left 15 minutes. Next, the DNA content in the cells was determined by measuring the luminescence intensity at the excitation wavelength 485 nm, at the luminescence wavelength 535 nm. The results revealed that the siRNA administered cell against TM4SF17 gene showed the luminescence intensity of approximately 23% lower than the non-silencing dsRNA administered cell, indicating that there was a statistically significant difference (P<0.05).

### Example 6

### Decrease in expression level of mRNA of the TM4SF17 gene by administering siRNA of the TM4SF17 gene

Human breast cancer cell line MDA-MB-231 used in Example 5, was suspended in McCoy's L-15 medium, and plated on a 96-well flat-bottomed tissue culture plate (BD Falcon) with a cell density of 2,000 cells/well. After incubating overnight at 37°C, the transfection of the siRNA was carried out according to Example 5. After the transfection, the incubation was carried out for 24 hours, and then the total RNA was extracted by RNeasy Mini Total RNA Kit (QIAGEN). Using as a template about 100 ng of the total RNA, reverse transcription was carried out by TaqMan Reverse Transcription Reagents (Applied Biosystems) in accordance with the protocol attached thereto. For the measurement of expression level of the TM4SF17 gene, quantitative PCR was carried out to examine the expression level of the TM4SF17 gene, by using as a template cDNA corresponding to 4 ng of the total RNA,and 10µL of reaction liquid which has prepared by adding 5 µL of TaqMan Universal PCR Master Mix (Applied Biosystems), 0.5 µL of TaqMan Reagent (Applied Biosystems) containing primer set amplifying base sequence derived from a portion of the second exon and the third exon of the TM4SF17 gene were added to become. PCR was carried out by reacting at 50°C for 2 minutes, 95°C for 10 minutes and then repeating 40 times the cycle set to include 95°C for 15 seconds and 60°C for 1 minute. On the other hand, expression level of β-actine gene contained within the same amount of the template cDNA were measured by using TaqMan β-actine Control Reagent, reserve primer, probe(each of these Applied Biosystems) to use as the internal standard.

In the group administered with siRNA against the TM4SF17 gene, the expression level of mRNA of TM4SF17 was decreased by 91 % as compared to the group administered with the non-silencing dsRNA which was used as a negative control. These results revealed that the inhibition of cell growth in human breast cancer cell line MDA-MB-231 was induced by the decrease in expression level of the TM4SF17a gene, TM4SF17b gene, or TM4SF17c gene.

### Example 7

### Increase in expression level of the LY6G6D gene in cancer

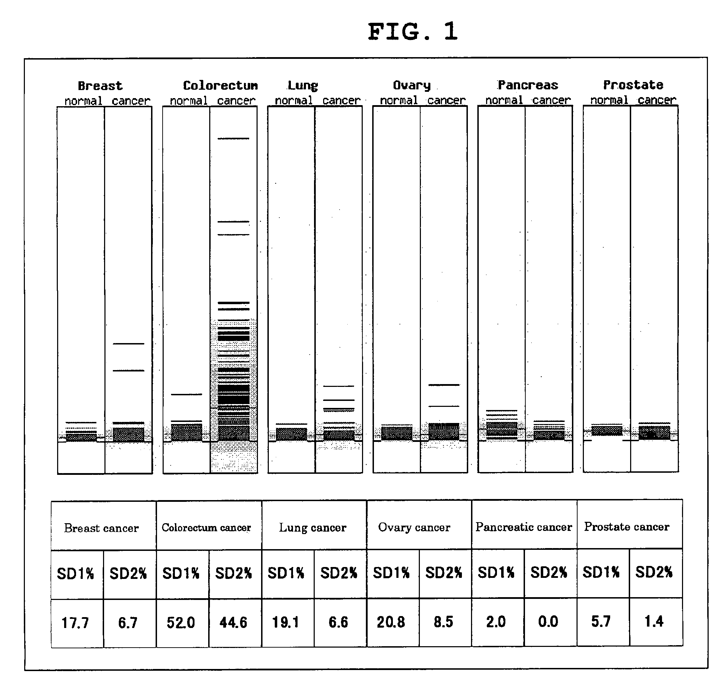

Using the carcinoma tissue-derived mRNA and the around normal tissue-derived mRNA, removed from patient with cancer, hybridization method was carried out to provide the gene expression profile. As the results of the gene expression profile analysis, high expression level of the gene in cancer tissue was found. Particularly in cancer of large intestine and rectum, significant increase of expression level of the gene for around normal tissue was observed (FIG. 1).

### Example 8

### Study of expression level of the LY6G6D gene in human colon culture cell line

The 19 strains used below were purchased from ATCC: human colon cancer cell lines WiDr, COLO 320DM, DLD-1, COLO 205, COLO201, HCT-15, SW 620, SW480, LoVo, SW1116, SW 837, SW948, HCT-8, HCT-116, LS123, LS174T, SNU-C1, Caco-2, and HT-29. Total RNA was prepared from the 19 cell lines described above using RNeasy Mini Total RNA Kit (QIAGEN). Using this total RNA as a template, reverse transcription was carried out by using a random primer in accordance with the protocol attached to TaqMan Reverse Transcription Reagents (Applied Biosystems), to prepare cDNA. Then, by quantitative PCR which was carried out by using this cDNA as a template, the expression level of the LY6G6D gene was examined.

For the measurement of expression level of the LY6G6D gene, quantitative PCR was carried out to examine the expression level of the LY6G6D gene, by using as a template cDNA obtained from 4 ng of the said total RNA, and 10µL of reaction liquid which has prepared by adding 5 µL of TaqMan Universal PCR Master Mix (Applied Biosystems)and 0.5 µL of TaqMan Reagent (Applied Biosystems) containing primer set amplifying base sequence derived from a portion of the second exon and the third exon of the LY6G6D gene. PCR was carried out by reacting at 50°C for 2 minutes, 95°C for 10 minutes and then repeating 40 times the cycle set to include 95°C for 15 seconds and 60°C for 1 minute. On the other hand, expression level of β-actine gene contained within the same amount of the template cDNA were measured by using TaqMan β-actine Control Reagent, to use as the internal standard. In the results, expression level of the LY6G6D gene in human colon cancer cell line, 9 strains such as WiDr, COLO 205, LoVo, SW 837, HCT116, LS123, LS 174T, Caco-2, and HT-29 is below detection limit. On the other hand, expression was seen in 10 strains such as COLO 320DM, DLD-1, COLO201, HCT-15, SW 680, SW480, SW1116, SW948, HCT-8, and SNU-C1. Additionally, high expression that expression level of the LY6G6D gene is approximately 1% of expression level of the β-actine gene was found in COLO 320DM.

### Example 9

### Study of expression level of the LY6G6D gene in human normal tissue and human cancer cell line

Human Marathon-Ready cDNA (Clontech) was used to examine the expression level of the LY6G6D gene in human normal tissue and human culture cell line. As the human normal tissue, Adrenal Gland, Bone Marrow, Brain (whole), Brain (cerebellum), Brain (cerebral cortex), Brain (hippocampus), Brain (hypothalamus), Colon, Heart, Kidney,Leukocyte, Liver, Lung, Lymph Node, Mammary Gland, Ovary, Pancreas, Pituitary Gland, Placenta, Prostate, Skeletal Muscle, Small Intestine, Spleen, Testis, Thymus, Thyroid Gland, Uterus, Fetal Brain, Fetal Kidney, and Fetal Liver were used. As the human cancer cell line, Melanoma (G361), Lung Carcinoma (A549), Burkitt's Lymphoma (Daudi), Lymphoblastic Leukemia (MOLT-4) Promyelocytic Leukemia (HL-60), Prostatic adenocarcinoma (PC-3), and Colorectal adenocarcinoma (SW480) were used. Using 1.5 ng of above mentioned cDNA as the template, expression level of the LY6G6D gene, and β-actine gene as the internal standard were measured by quantitative PCR according to the method described in Example 8. In the results, expression level of LY6G6D gene for expression level of β-actine gene were high level such as 0.58% in human cancer cell line Colorectal adenocarcinoma (SW480). On the other hand, expression level of LY6G6D gene for expression level of β-actine gene were less than 0.1% or below detection limit in most of human normal tissues except that 0.45% is in Testis, 0.15% is in Prostate.

These results show little expression level of LY6G6D gene in human normal tissue were shown.

### Example 10

### Inhibition of cell growth in human colon cancer cell line COLO320 DM by administering siRNA against the LY6G6D gene

Human colon cancer cell line COLO320 DM, purchased from American Type Culture Collection (ATCC), was suspended in RPMI1640 medium (Invitrogen corp.) containing 10% fetal bovine (JRH), and were plated on a 96-well flat-bottomed tissue culture plate (BD Falcon) with a cell density of 1,600 cells/well, and transfection of siRNA was carried out.

Specifically, the siRNA-2 having activity which cleaves mRNA of the LY6G6D gene prepared in Example 1 was prepared to transfection. As a control, non-silencing dsRNA used in Example 1 was used. 10µl of Opti-MEM I (Invitrogen corp.) in which 2 pmol of the siRNA against LY6G6D gene or 2 pmol of the non-silencing dsRNA is added, and 0.12µl of Lipofectamine 2000 (Invitrogen corp.) were mixed together. Then the full amount of this mixture was added to one well of 96-well flat-bottomed tissue culture plate, then allowed to stand for 20 minutes at room temperature. Next, the cells suspended in RPMI1640 medium (Invitrogen corp.) containing 10% fetal bovine (JRH) were plated on a 96-well flat-bottomed tissue culture plate with a cell density of 1,600 cells/well, and then cultured overnight at 37°C with 5% carbon dioxide gas air flow. 1 day after transfection, the medium was replaced, further cultured for 3 days. Then CellTiter-Glo reagent (Promega) were added to the medium in 100 µl/well, and stirred for 30 minutes under light interception, and then the luminescence intensity was measured to determine ATP content in cells. The results revealed that the cell administered with siRNA for LY6G6D showed the luminescence intensity of approximately 29 % lower than the non-silencing dsRNA administered cell, indicating that there was a statistically significant difference (P<0.05) (FIG. 3A).

### Example 11

### Decrease in expression level of mRNA in human colon cancer cell line COLO320 DM by administering siRNA against the LY6G6D gene

The total RNA was extracted from the cells collected one day after transfection in Example 10 by RNeasy Mini Total RNA Kit (QIAGEN). Using the total RNA as a template, reverse transcription was carried out by TaqMan Reverse Transcription Reagents (Applied Biosystems) in accordance with the protocol attached thereto. For the measurement of expression level of the LY6G6D gene, and expression level of β-actine gene used as the internal standard, using as a template cDNA synthesized from total RNA by reverse transcription, in accordance with the method described in Example 8, quantitative PCR was carried out. In the group administered with siRNA against the LY6G6D gene, the expression level of mRNA of LY6G6D was decreased by 77 % as compared to the group administered with the non-silencing dsRNA which was used as a negative control. These results revealed that the inhibition of cell growth in human colon cancer cell line COLO320 DM was induced by the decrease in expression level of the LY6G6D gene.

### Example 12

### Confirmation of the Off-Target effect and the transduction efficiency in human colon cancer cell line HCT116 DM by administering siRNA against the LY6G6D gene

Human colon cancer cell line HCT116 which does not express LY6G6D gene, purchased from American Type Culture Collection (ATCC), was suspended in McCoy's 5A medium (Invitrogen corp.) containing 10% fetal bovine (JRH), and plated on a 96-well flat-bottomed tissue culture plate (BD Falcon) with a cell density of 1,600 cells/well, and at the same time transfection of siRNA was carried out. Alongside, transfection of siRNA against GAPDH gene was carried out, and the transduction efficiency was confirmed by decrease of the GAPDH gene amounts.

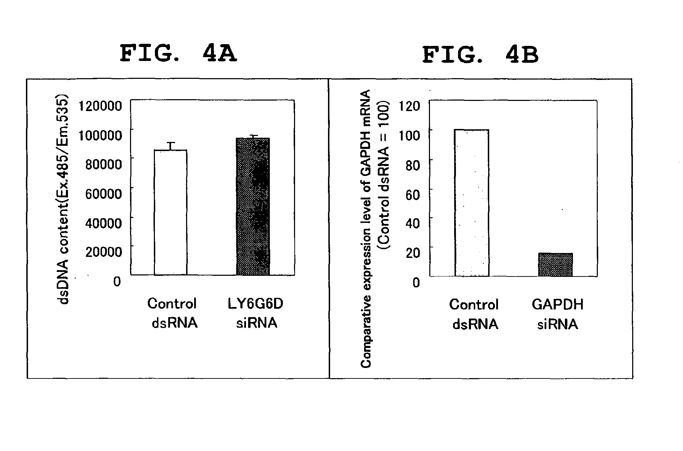

In specific, the siRNA-2 having activity which cleaves mRNA of the LY6G6D gene prepared in Example 1 was prepared to transfection. As a control, non-silencing dsRNA used in Example 1 was used. For the confirmation of the transduction efficiency, siRNA against GAPDH gene was used. 10µl of Opti-MEM I (Invitrogen corp.) added with 2 pmol of the siRNA against LY6G6D gene, siRNA against the non-silencing dsRNA or GAPDH, and 10µl of Opti-MEM I (Invitrogen corp.) added with 0.08µl of Lipofectamine 2000 (Invitrogen corp.) were mixed. Then full amount of this mixture was added to one well of 96-well flat-bottomed tissue culture plate, then were left 20 minutes at room temperature. Next, the cells suspended in McCOy's 5A medium (Invitrogen corp.) containing 10% fetal bovine (JRH) were plated on a 96-well flat-bottomed tissue culture plate with a cell density of 1,600 cells/well, and then cultured overnight at 37°C with 5% carbon dioxide gas air flow. 1 day after transfection, the medium was replaced, and further cultured for 3 days. After incubation, the cells were washed with PBS, and were left for 30 minutes or more at -80°C, then left 5 minutes at room temperature. To the plate, aqueous solution containing 0.25% CyQUANT GR reagent (Molecular Probes) and 5% cell lysis buffer (Molecular Probes) were added in 200 µl/well, and were stirred for 15 minutes. Then the DNA content in the cells were determined by measuring the luminescence intensity at the excitation wavelength 485 nm, at the luminescence wavelength 535 nm. The results revealed that the cell into which was administered siRNA against LY6G6D gene showed the luminescence intensity of approximately 104% compared to the non-silencing dsRNA administered cell, indicating that there was little (FIG. 4A).

On the other hand, the total RNA was extracted from the cells collected 1 day after transfection, and reverse transcription was carried out in accordance with Example 10. For the measurement of expression level of the GAPDH gene, using as a template cDNA synthesized from total RNA, expression level of the GAPDH gene were measured by using Human GAPD (Applied Biosystems), where expression level of β-actine gene used as the internal standard were measured by quantitative PCR in accordance with the same method described in Example 8. In the group administered with siRNA for GAPDH gene, the expression level of the GAPDH gene was decreased by 84 % as compared to the group administered with non-silencing dsRNA, indicating that the siRNA transduction efficiency of the present experiment was excellent (FIG. 4B).

These results revealed that inhibition of cell growth in the cells didn' t express LY6G6D gene by siRNA against LY6G6D, was not induced, indicating that induction of inhibition of cell growth by administering siRNA against LY6G6D, described in Example 11 was not due to the Off-Target effect.

### Example 13

### Construction of animal cell expression vectors for recombinant full-length LY6G6D protein

Using the Marathon-Ready cDNA (CLONTECH) derived from human colon cancer cell line SW480 as a template, using primer of LY-HindATG:5'-AAGCTTATGA AACCCCAGTT TGTTG-3'(SEQ ID NO:25) and LY-TAGXba:5'-TCTAGACTAT CCGCTCCACA GTCC-3'(SEQ ID NO:26), PCR was carried out. The solution 20 µl was comprised of, 0.5 µl of the above mentioned cDNA, 1U LA Taq DNA Polymerase (Takara Bio), 0.5 µM of each of the above mentioned primers, 400µM of dNTPs, and 10µl of 2x GC Buffer I (Takara Bio). PCR was carried out by reacting at 94°C for 1 minute and then repeating 35 times the cycle set to include 94°C for 30 seconds, 60°C for 30 seconds and 72°C for 2 minutes. Additionally, elongation reaction was carried out for 7 minutes at 72°C. The PCR product was purified with PCR Purification Kit (QIAGEN). It was subcloned into the plasmid vector pCR4-TOPO (Invitrogen corp.) to obtain the plasmid LY6G6D/pCR4-TOPO in accordance with prescription of TOPO TA PCR cloning Kit. As a result of the base sequence analysis, the plasmid contains the base sequence of cDNA represented by SEQ ID NO: 1.

The plasmid LY6G6D/pCR4-TOPO obtained above was treated with restriction enzyme HindIII and XbaI. A pcDNA3.1 (+) (Invitrogen corp.) was also treated with restriction enzyme HindIII and XbaI. Then, these were isolated by agarose gel electrocataphoresis. After isolation, DNA fragment containing base sequence encoding LY6G6D and DNA fragment corresponding to pcDNA3.1 (+) were respectively collected and purified with Gel Extraction Kit (QIAGEN). Each of DNA fragment was subjected to the ligation reaction with the use of DNA Ligation Kit ver.2 (Takara Bio) and there after introduced to Escherichia coli TOP10 and selected in LB agar medium containing ampicillin to obtain the plasmide LY6G6D/pcDNA3.1 (+) containing cDNA sequence encoding LY6G6D protein.

Using the plasmide LY6G6D/pcDNA3.1 (+) obtained above as a template, using primer of LY-CoKoATG : 5'-GCCACCATGA AACCCCAGTT TGTTG-3'(SEQ ID NO: 27), LY-OrKoATG:5'-ACTGCGATGA AACCCCAGTT TGTTG-3' (SEQ ID NO: 28)or LY-TAGXba:5'-TCTAGACTAT CCGCTCCACA GTCC-3' (SEQ ID NO: 29), PCR was carried out. The solution 50 µl was comprised of, 1.63ng of the above mentioned cDNA, 1U Platium Pfx DNA Polymerase (Invitrogen corp.), 0.3 µM of each of the above mentioned primers, 300µM of dNTPs, and 1 mM of magnesium sulfate. PCR was carried out by reacting at 95°C for 2 minute and then repeating 25 times the cycle set to include 94°C for 15 seconds, 55°C for 30 seconds and 68°C for 1 minute. In order to add dATP to 3'-terminal of the PCR reaction product, 5U Ex Taq DNA Polymerase (Takara Bio) was added and kept the heat at 72°C for 7 minutes. The obtained PCR reaction product was purified with PCR purification Kit (QIAGEN). The purified PCR product was subcloned into the plasmid vector pCR4-TOPO (Invitrogen corp.) to obtain the plasmid CoLY6G6D/pCR4-TOPO and OrLY6G6D/pCR4-TOPO in accordance with prescription of TOPO TA PCR cloning Kit(Invitrogen corp.).

The plasmide CoLY6G6D/pCR4-TOPO and OrLY6G6D/pCR4-TOPO obtained above was treated with restriction enzyme SPeI and XbaI. A pcDNA3.1 (+) (Invitrogen corp.) was also treated with restriction enzyme SPeI and XbaI. Then, these were isolated by agarose gel electrocataphoresis. After isolation, DNA fragment containing base sequence encoding LY6G6D and DNA fragment corresponding to pcDNA3.1 (+) were respectively collected and purified with Gel Extraction Kit (QIAGEN). Each of DNA fragment was subjected to the ligation reaction with the use of DNA Ligation Kit ver.2 (Takara Bio) and there after introduced to Escherichia coli TOP10 and selected in LB agar medium containing ampicillin to obtain the plasmide CoLY6G6D/pcDNA3.1 (+) and OrLY6G6D/pcDNA3.1 (+) containing cDNA sequence encoding LY6G6D protein.

### Example 14

### Construction of Escherichia coli expression vector for recombinant polyhistidine tagged secretable form LY6G6D protein

In order to prepare LY6G6D-His expression plasmide, firstly, the tagged expression vector which can add 6x His-tag sequence to both the N terminus and the C terminus of the desired gene was prepared. So as to delete Nhe I restriction enzyme cleavage site existing in 229bp of Escherichia coli expression vector pET-3a (MERCK), in accordance with the protocol of QuickChange Site-Directed Mutagenesis Kit(STRATAGENE), using DESNHE-F:5'-CCAGTCACTA TGGCGTGCTC CTAGCGCTAT ATGCG-3' (SEQ ID NO:30) and DESNHE-R:5'-CGCATATAGC GCTAGGAGCA CGCCATAGTG ACTGG-3'(SEQ ID NO:31) as the primer, using pET-3a as the template, PCR was carried out to prepare pET3a-desNhe into which mutation was introduced . Next, to give initiation codon + 6 x His sequence + Nhe I restriction enzyme cleavage site + 6 x His sequence + terminating codon in between restriction enzyme site Nhe I and BamH I of pET3-3a, synthetic DNA His-FNdeBam:5'-TATGCATCATC ATCATCATC ACGCTAGCCA TCATCATCAT CATCACTGAG-3' (SEQ ID NO:32) and His-RNdeBam:5'-GATCCTCAGT GATGATGATG ATGATGGCTA GCGTGATGAT GATGATGATG CA-3' (SEQ ID NO:33) were prepared and diluted with TE to be 10 mM respectively, and equivalent amount were mixed and heated 10 minutes at 95°C, then gradually cooled and annealing was carried out. The pET3a-desNhe obtained above was digested by restriction enzyme Nde I and BamH I (Takara Bio), and electrocataphoresed in agarose gel and extracted from the gel with the use of QIAquick Gel Extraction Kit (QIAGEN). The expression vector treated with restriction enzyme and the annealing solution were mixed and connected with Ligation High (TOYOBO) to prepare Escherichia coli expression vector pET3-HH added with 6 x His-Tag.

Next, for the addition of 6 x His-Tag to the C terminus, PCR was carried out by using the primer capable of adding restriction enzyme cleavage sites of Nde I to 5' terminus and Nhe I to 3' terminus of the LY6G6D gene, respectively and obtained product was inserted to the pET3-HH prepared above to prepare LY6G6D-His expression vector. Using LY6G-FNDE:5'-CATATGAACC GAATGCGGTG CTACAA-3' (SEQ ID NO:34) and LY6G-RNHE : 5'-GCTAGCGTTG CACAGGTCTC CCAGGT-3' (SEQ ID NO:35), and using LY6G6D/pCR4-TOPO as the template obtained in Example 13 with the Pyrobest DNA Polymerase(Takara Bio), PCR was carried out on Gene Amp PCR System 9700(Applied Biosystems). PCR was carried out by repeating 35 times the cycle set to include 98°C for 10 seconds, 60°C for 10 seconds, and 72°C for 30 seconds. The PCR product obtained was isolated by agarose gel electrocataphoresis, the DNA fragment at approximately 200pb was collected with the use of QIAquick Gel Extraction Kit, and then cloned into pCR4blunt-TOPO (Invitrogen corp.) to obtain pCR4/LY6G6D. After confirming the base sequence, the pCR4/LY6G6D and expression vector pET3-HH were digested by restriction enzyme Nde I and Nhe I (Takara Bio), and agarose gel electrocataphoresis was carried out and DNA fragment and expression vector were collected with the use of QIAquick Gel Extraction Kit. Then the DNA fragment and the expression vector which were treated with restriction enzyme Nde I and Nhe I, were connected with Ligation High to prepare LY6G6D expression vector pET3/LY6G6D-His whose C terminus 6 x His-Tag is added to.

The recombinant LY6G6D-His protein was prepared by transforming BL21-Gold (DE3) competent cell (SERATAGENE) with pET3/LY6G6D-His plasmid prepared above. Escherichia coli obtained by transforming was inoculated into 200 mL of LB medium (1% Pepton, 0.5% yeast extract, 0.5% sodium chloride, 0.01% ampicillin) and cultured for 16 hours at 30°C. The obtained culture was implanted to the Jar fermentor containing 3L of main fermentation medium (1.68% sodium hydrogen phosphate, 0.3% sodium dihydrogen phosphate, 0.1% ammonium chloride, 0.05% sodium chloride, 0.024% magnesium sulfate, 0.02 % new pole LB-625, 0.0005% thiamine chloride, 1.5% sorbitol, 1.5% casamino acid, 0.01% ampicillin) and incubation was started at 37°C, ventilation volume 3L/min, stirring rotation 200rpm. At the time when the culture turbidity became approximately 500 Klett unit, cooling incubating temperature to 20°C, then isopropyl-B-D-thiogalactopyranoside (IPTG) was added to the fermentor so as to become 0.5 mM in final concentration, and further incubation was carried out for 4 hours to induce expression of LY6G6D-His. After the incubation, the culture was centrifuged 10 minutes at 5,000rpm, to obtain LY6G6D-His expression Escherichia coli. The LY6G6D-His expression Escherichia coli was suspended in PBS containing 1mM of EDTA, then treated with Microfluidizer (Microfluidics) twice at 100MPa. This spallation solution was ultracentrifuged (10,000xG, 30min, Beckman Coulter), and the supernatant obtained was absorbed through the Ni-NTA Superflow (QIAGEN) column preliminarily equilibrated with PBS, then washed with PBS containing 50mM of Imidazole, and was eluted with PBS containing 250mM of Imidazole. The elute was adjusted to be PH5.0 with 2M of acetic acid, further 10% glycelol was added, then concentrated with Amicon Ultra-15 5K (MILLIPORE). The solution after concentration was gel-filtered with the use of Superdex200 pg column preliminarily equilibrated with PBS containing 10% glycelol to obtain purified LY6G6D-His protein. Concentration of protein was measured by BCA Protein Assay Kit (PIERCE), using BSA as standard.

### Example 15

### Construction of Escherichia coli expression vector for recombinant HAT tagged secretable form LY6G6D protein

The His part of expression vector pET3/LY6G6D-His prepared in Example 14 was carved out, and HAT (Histidine affinity tag) prepared with the synthetic DNA was inserted into the vector to prepare the LY6G6D-HAT expression plasmid. The synthetic DNA HAT-F:5'- CTAGCAAGGA TCATCTCATC CACAATGTCC ACAAAGAGGA GCACGCTCAT GCCCACAACA AGTGAG-3' (SEQ ID NO: 36) and HAT-R:5'- GATCCTCACT TGTTGTGGGC ATGAGCGTGC TCCTCTTTGT GGACATTGTG GATGAGATGA TCCTTG-3'(SEQ ID NO:37) were prepared and diluted with TE to be respectively 10mM, of which the equal amount was mixed and heated 10 minutes at 95°C, then gradually cooled and annealing was carried out.

The pET3/LY6G6D-His prepared above was digested by restriction enzyme Nde I and BamH I, and agarose gel electrocataphoresis was carried out, and then the vector was extracted from gel with the use of QIAquick Gel Extraction Kit. The expression vector treated with the restriction enzyme was mixed with annealing solution and connected with Ligation High to prepare expression vector pET3/LY6G6D-HAT added with HAT.

The recombinant LY6G6D-HAT protein was prepared by transforming BL21-Gold (DE3) competent cell with pET3/LY6G6D-HAT plasmid prepared above. Escherichia coli obtained by transforming was inoculated into 200 mL of LB medium (1% Pepton, 0.5% yeast extract, 0.5% sodium chloride, 0.01% ampicillin) and cultured for 16 hours at 30°C. The culture obtained was implanted to the Jar fermentor containing 3L of main fermentation medium (1.68% sodium hydrogen phosphate, 0.3% sodium dihydrogen phosphate, 0.1% ammonium chloride, 0.05% sodium chloride, 0.024% magnesium sulfate, 0.02% NEWPOLE LB-625, 0.0005% thiamine chloride, 1.5% sorbitol, 1.5% casamino acid, 0.01% ampicillin) and incubation was started at 37°C, ventilation volume 3L/min, stirring rotation 200rpm. At the time when the culture turbidity became approximately 500 Klett unit, cooling incubating temperature to 20°C, then isopropyl-B-D-thiogalactopyranoside (IPTG) was added to the fermentor so as to become 0.5 mM in final concentration, and further incubation was carried out for 4 hours to induce expression of LY6G6D-HAT. After the incubation, the culture solution was centrifuged 10 minutes at 5,000rpm, to obtain LY6G6D-HAT expression Escherichia coli. The LY6G6D-HAT expression Escherichia coli was suspended in PBS containing 1mM of EDTA, then treated with Microfluidizer(Microfluidics) twice at 100MPa. The spallation solution was ultracentrifuged (10,000xG, 30min), and the supernatant obtained was absorbed through the Ni-NTA Superflow column preliminarily equilibrated with PBS, then washed with PBS containing 25mM of Imidazole, and was eluted with PBS containing 250mM of Imidazole. The elute was concentrated with Amicon Ultra-15 5K and then gel-filtered with the use of Superdex200 pg column equilibrated with PBS to obtain purified LY6G6D-HAT protein. Concentration of protein was measured by BCA Protein Assay Kit (PIERCE), using BSA as standard.

### Example 16

### Preparation and purification of anti LY6G6D rabbit polyclonal antibodies

Using the recombinant LY6G6D-His protein prepared in Example 14 as immunogen, the anti LY6G6D rabbit polyclonal antibodies was prepared.

3 domestic rabbits (Oryctolagus cuniculus, female, 3 kg) were immunized subcutaneously on the back or intracutaneously with 0.1 mg/animal of an emulsion which was prepared by mixing a PBS solution of the LY6G6D-HIS protein with Freund's complete adjuvant in an equal volume as the LY6G6D-HIS protein. For the second and subsequent immunizations, a protein emulsion was prepared in a similar manner except that the adjuvant was replaced by Freund's incomplete adjuvant and repeatedly boostered 7 times every 2 weeks.

Prior to the immunization and one week after the fourth and sixth boosters, blood was taken from the ear vein. An increased serum antibody titer was confirmed by ELISA using an immunoplate coated with the LY6G6D-HIS protein. One week after the final immunization, blood was taken from the carotid vein of 3 rabbits under anesthesia to collect antisera in 77.6 ml, 77.0 ml and 71.7 ml from rabbit respectively.

The antisera obtained above were diluted in 20 mM Tris-hydrochloride buffer containing 1M sodium chloride, pH 7. 5 by 2-fold and provided to the protein A-cellulofine column (BIOCHEMICALS). The column was washed with 20 mM Tris-hydrochloride buffer containing 1M sodium chloride, pH 7.5 and eluted with 0.25 M Glycine-hydrochloride containing 1 M sodium chloride, pH 2.5. The eluate was neutralized with 1 M Tris- hydrochloride, pH 9. The elute neutralized was provided to the antigen column prepared by entrenching the recombinant LY6G6D-HAT protein prepared in Example 15 in NHS-Activated Sepharose (Amersham Biosciences). The column was washed with 20 mM Tris-hydrochloride buffer containing 1M sodium chloride, pH 7.5, and then eluted stepwise with 0.1M sodium citrate buffer containing 1M sodium chloride, pH 5, 0.1M sodium citrate buffer containing 1M sodium chloride, pH 4, and 0.1M sodium citrate buffer containing 1M sodium chloride, pH 3. These elute were immediately neutralized with 1 M Tris-hydrochloride, pH 9, then replaced by PBS with the use of Viva spin (Sartorius) equipped with membrane of which the molecular weight cut off is 10,000 to obtain anti LY6G6D rabbit polyclonal antibodies (R1 ,R2 and R3).

### Example 17

### Effects on cell growth by transient expression of LY6G6D

By inducing the plasmid expression vector, LY6G6D protein was transiently expressed to examine the effects on growth rate of cell by LY6G6D protein.

Specifically, 500,000 of HCT116 cells derived from Human colon cancer, purchased from American Type Culture Collection (ATCC), was suspended in 2 mL of McCoy's 5A medium (Invitrogen corp.) containing 10% fetal bovine (JRH), and plated to 6-well tissue culture plate (BD Falcon), then cultured for 1 days at 37°C with 5% carbon dioxide gas air flow. Then 250 µL of Opti-MEM I (Invitrogen corp.) added with 1µg of control vector (pcDNA3.1 (+), Invitrogen corp.) or LY6G6D expression vector (plasmid, pcDNA3.1 (+)/CoLY6G6D, plasmid pcDNA3.1 (+) /OrLY6G6D) prepared in example 13 was mixed with 250 µL of Opti-MEM I (Invitrogen corp.) added with 3L of Lipofectamine 2000 (Invitrogen corp.), and left 20 minutes at room temperature. The liquid mixture was added to culture and cultured for 8 hours, and then the medium was replaced, and further cultured for 1 day.

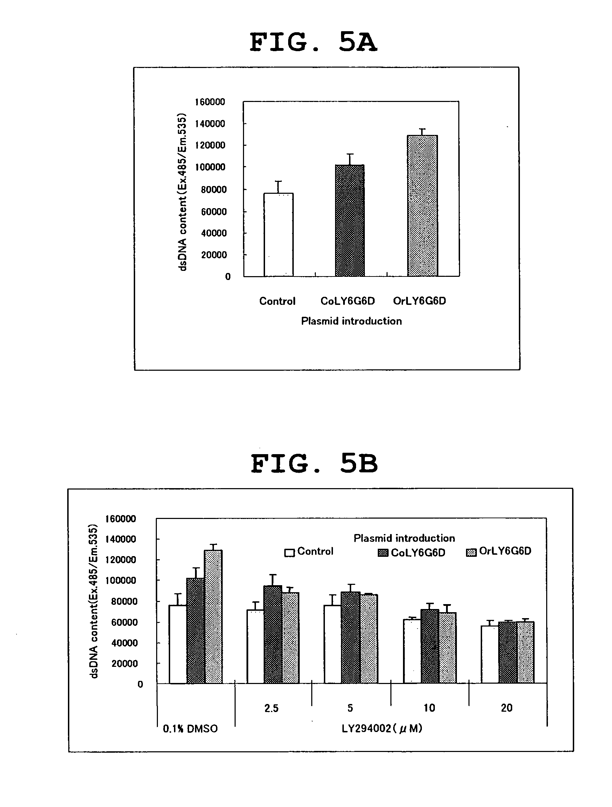

Cells 2 days after transfected with each plasmid was washed with PBS, and distributed to PBS containing 1mM of EDTA and 0.1% BSA, 2,000 of cells was suspended in McCoy' s 5A medium containing 10% fetal bovine (JRH), and plated to 96-well flat-bottomed tissue culture plate (BD Falcon), and DMSO is added as the negative control to the plate to be 0.1% in final concentration. LY294002 of PI3K-Akt pathway inhibitor was added to give 2.5µM, 5µM, 10µM, 20µM, PD98059 of MAPK pathway inhibitor was added to give 1.25µM, 2.5µM, 5µM, 10µM, SB20385 of p38 MAPK pathway inhibitor was added to give 1.25µM, 2.5µ M, 5µM, 10µM, or SP600125of JNK pathway inhibitor was added to give 1.25µM, 2.5µM, 5µM, 10µM. Additionally each intracellular signaling pathway inhibitor was prepared to be 0.1% DMSO. After incubating 3 days at 37°C with 5% carbon dioxide gas air flow, in accordance with the method described in Example 12, the DNA content in the cells were determined. The results revealed that the LY6G6D expression vector (plasmid pcDNA3.1 (+)/CoLY6G6D, plasmid pcDNA3.1 (+)/OrLY6G6D) induced cell showed the luminescence intensity of approximately 1.34-fold and 1.70-fold higher than control vector induced cell in the negative control group, indicating that there was a statistically significant difference (P<0.01, P<0.001)(FIG. 5A). The growth-promoting effect was dose-dependently inhibited by LY294002 of PI3K-Akt pathway inhibitor (FIG. 5B).

### Example 18

### Effects on AKT phosphorylation by transient expression of LY6G6D

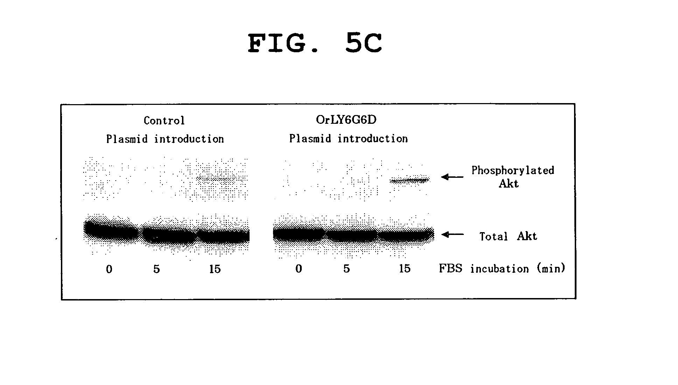

In accordance with the method described in Example 17, plasmid pcDNA3.1 (+) or plasmid pcDNA3.1 (+)/OrLY6G6D was induced, 1 day after transfection, cells were washed with PBS, then further cultured overnight in McCoy's 5A medium not containing fetal bovine. Next, the culture was removed, and then McCoy's 5A medium containing 10% fetal bovine was added, for 5 minutes or 15 minutes with 5% carbon dioxide gas air flow and kept the heat in 37°C. The cell kept the heat in McCoy' s 5A medium containing 10% fetal bovine and the cell before the heat were washed in PBS cooled in ice three times, then 200 µL of altered RIPA buffer cooled in ice [50mM Tris /hydrochloric acid, pH 7.5, 150mM sodium chloride, 1% Triton X-100, 0.05% SDS, 0.25% deoxycholic acid, Complete^{™} tablet (Roche Diagnostics), Phosphatase Inhibitor Cocktail-1 (Sigma), Phosphatase Inhibitor Cocktail-2 (Sigma)] was added, then left for 15 minutes 4°C. This altered RIPA buffer was collected, and centrifuged for 20 minutes at 15,000 rpm to obtain supernatant as cell-free extract. The concentration of the protein of these cell-free extracts were measured with the use of BCA Protein Assay Kit, and the extracts were diluted with altered RIPA buffer in order to assimilate the concentration of the protein, and then one fifth volume of 5-fold SDS-PAGE sample buffer was mixed and heated for 5 minutes at 95°C, and 20 µL of which were provided to the SDS-PAGE in 10% acrylamide gel. The protein electrophoresed and isolated by the method of the art was transferred to Clear Blot membrane-P (ATTO), then left in the blocking solution [50mM Tris / hydrochloric acid, pH 7.5, 150mM sodium chloride, 0.1% Tween 20, 5% Skim milk] for 1 hour at room temperature. Next, rabbit antiphosphorylate Akt antibody (Cell Signaling Technology) or rabbit anti Akt antibody (Cell Signaling Technology) was diluted by 2,000-fold with 50mM Tris / hydrochloric acid, pH 7.5, 150mM sodium chloride, 0.1% Tween 20, 5% BSA solution so that reaction is performed for 2 hours at room temperature, then left in the HRP labeled anti rabbit IgG antibody (GE Healtcare) diluted by 10,000-fold with the blocking solution for 1 hour at room temperature. For the detection, using Immobilon Western HRP substrate (MILLIPORE) in accordance with the protocol attached thereto, phosphorylate Akt or total Akt protein was detected. The results revealed that the plasmid pcDNA3.1 (+)/OrLY6G6D induced cell treated with fetal bovine for 15 minutes showed the amount of phosphorylate Akt of higher than the pcDNA3.1 (+) induced cell treated similarly. On the other hand, there was no difference in the amount of total Akt (FIG. 5C).

These results indicate that the LY6G6D protein has the capability of promoting the cell growth, by activating PI3K-Akt pathway.

### Example 19

### Establishment of stability expression cell line for recombinant full-length LY6G6D protein

In example 17, plasmid pcDNA3.1 (+) /CoLY6G6D or pcDNA3.1 (+)/OrLY6G6D prepared was transfectioned, cultured for 2 days, and the resulting cell was distributed to PBS containing 1mM of EDTA and 0.2% BSA, and suspended in McCoy's 5A medium [G418 selected medium] containing 10% fetal bovine, 800 µg/mL G418 (Progema) and 10mM HEPES, and plated to T-150 tissue culture flask (BD Falcon). The G418 selected medium was replaced every 3 or 4 days, while the incubation was continued for 10 days at 37°C with 5% carbon dioxide gas air flow. G418 resistant cell was plated to 96-well flat-bottomed tissue culture plate with a cell density of 0.8 cells/well, then cell were collected from well, where colony was formed, and plated to 48-well tissue culture plate (BD Falcon). Until the cells were grown at a cell density of 50% or more, the incubation was continued, and then the cell was plated to 6-well tissue culture plate. Until the cells were grown at a cell density of 50% or more, the incubation was continued, and then each clone was suspended in Cell Banker (ZENOAQ), and stored at -80°C. On the other hand, some cells were plated to 12-well tissue culture plate, and cultured to obtain subconfluent, and then each clone was washed with PBS, and then the cells were distributed to the PBS containing 2mM of EDTA and 0.2% BSA, and suspended in BD Pharmingen Stain Buffer(FBS)(BD Biosciences), and R2 antibody obtained in Example 16 was added to be final concentration of 1 µg/mL, and left in ice for 2 hours. Next, the clone was suspended in BD Pharmingen Stain Buffer(FBS) containing 5µg/mL of Alexa488 labeled anti rabbit IgG antibody (Molecular Probes), and left in ice for 1 hour. After additional suspension was carried out with BD Pharmingen Stain Buffer (FBS), the analysis was carried out with the use of FACScan(BD Biosciences), to obtain total 6 of clone such as the high expression clone (C03, C12, and 003), the middle expression clone (C01 and 030), the low expression clone(C07) as stability expression cell line, where LY6G6D protein located in cell surface.

### Example 20

### Demonstration that LY6G6D protein is GPI-anchored membrane protein (PI-PLC treatment)

Whether LY6G6D protein is located in cell surface via GPI (Glycosylphosphatidylinositol) was examined. In specific, 6 clones of LY6G6D stability expression cell line(C01, C03, C07, C12, 003 and 030) obtained in Example 19 were cultured to obtain subconfluent, and the cells washed with PBS were distributed to PBS containing 2mM of EDTA and 0.2% BSA. Million of cells were washed with PBS again, then suspended in 100µ L of OPTI-MEM I including lunits/mL PI-PLC (Phosphatidylinositol-spesific phosphlipase C, Sigma) or 100 µL of OPTI-MEM I to react for 90 minutes at 37 °C.

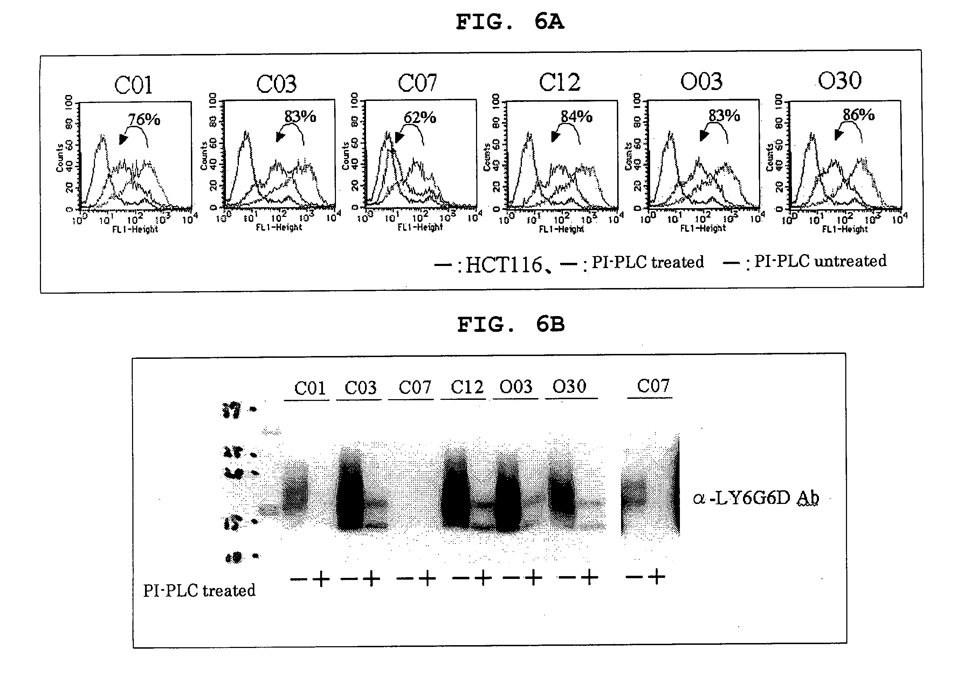

500,000 of cells reacted above were suspended in BD Pharmingen Stain Buffer (FBS) (BD Biosciences), and R2 antibody obtained in Example 16 was added to be final concentration of 3 µg/mL, and left in ice for 1 hour. Next, the clone was suspended in BD Pharmingen Stain Buffer (FBS) containing 5µg/mL of Alexa488 labeled anti rabbit IgG antibody (Molecular Probes), and left in ice for 1 hour. After additional suspension was carried out with BD Pharmingen Stain Buffer (FBS), the analysis was carried out with the use of FACScan(BD Biosciences). As the result, in all of LY6G6D stability expression cell line, staining intensity is declining by reacting with PI-PLC (FIG. 6A) .

On the other hand, 500,000 of cells reacted above were washed with PBS, then suspended in 100µL of SDS-PAGE sample buffer [62. 5 mM Tris / hydrochloric acid, pH 6.8, 20% glycerol, 1% SDS, 0.02% Bromophenol blue, and 2% 2-mercaptoethanol], and heated for 5 minutes at 95°C, and 20 µL of which were provided to the SDS-PAGE in 15% acrylamide gel. The protein electrophoresed and isolated by the method of the art was transferred to Clear Blot membrane-P (ATTO), then left in the blocking solution [50mM Tris / hydrochloric acid, pH 7.5, 150mM sodium chloride, 0.1% Tween 20, 5% Skim milk] for 1 hour at room temperature. Next, R2 antibody obtained in Example 16 was diluted with 50mM Tris / hydrochloric acid, pH 7.5, 150mM sodium chloride, 0.1% Tween 20, 5% BSA solution to be final concentration of 3 µg/mL, and kept the heat at 4°C for 2 hours, then left in the HRP labeled anti rabbit IgG antibody (GE Healtcare) diluted by 100, 000-fold with the blocking solution for 1 hour at room temperature. For the detection, using Immobilon Western HRP substrate (MILLIPORE) in accordance with the protocol attached thereto, LY6G6D protein was detected. As the result, the specific band derived from LY6G6D protein of molecular weight 15kD to 20kD seen in all of LY6G6D stability expression cell line is declining by reacting with PI-PLC (FIG. 6B).

These results indicate that the LY6G6D protein is located in the cell surface via GPI.

### Example 21

### Study of sugar chain modification of LY6G6D protein

In order to characterize the LY6G6D protein biochemically, sugar chain modification was examined. Specifically, LY6G6D stability expression cell line C03 Clone was distributed to PBS containing 2mM of EDTA and 0.2% BSA, 1,000,000 cells were washed with PBS, and then suspended in the hypotonic solution of 10 mM Tris·HCl buffer, pH7.5 containing Complete^{™} tablet (Roche Diagnostics), left on ice for 20 minutes to break the cells. The broken cell was washed hypotonic solution 3 times, suspended in 50mM phosphate buffer, pH7.0 containing 1% Triton X-100, 0.1% SDS, and 0.4% 2-mercaptoethanol, and heated for 5 minutes at 95°C, then reacted at 37°C for 4 hours, in accordance with the prescription of ENZYMATIC DEGLYCOSYLATION KIT (PROZYME), under the conditions of 1) without enzyme, 2) with N-Glycanase (PNGase F), 3)with N-Glycanase(PNGase F) and Sialidase A, or 4) with N-Glycanase(PNGase F),Sialidase A, and O-Glycanase. Then one fifth volume of 5-fold SDS-PAGE sample buffer [312.5 mM Tris. HC1 buffer, pH 6.8, 40% glycerol, 5% SDS, 0.1% Bromophenol blue, and 10% 2-mercaptoethanol] was mixed and heated for 5 minutes at 95°C, and 2 µL of which were provided to the SDS-PAGE in 15% acrylamide gel. The detection was carried out in accordance with the same method described in Example 20. As the result, the specific band derived from LY6G6D protein of molecular weight 15kD to 20kD was focused toward 15kD.

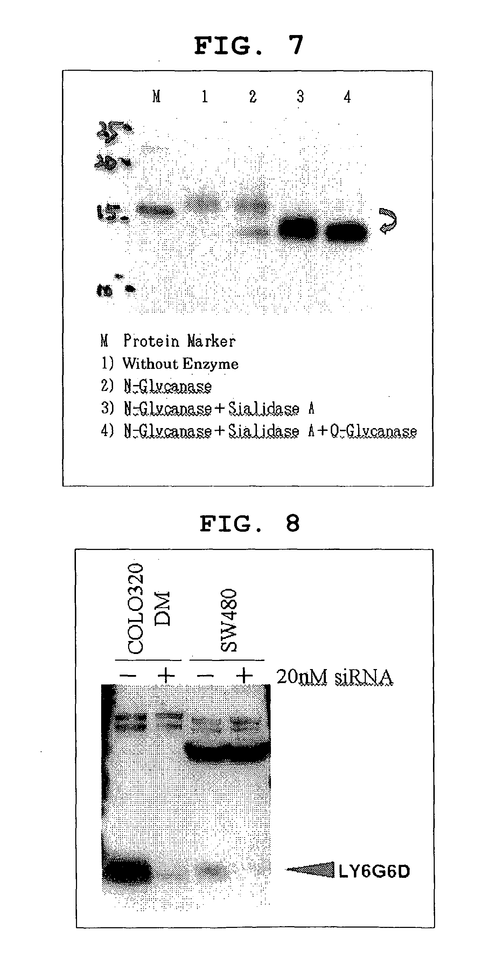

These results indicate that the LY6G6D protein is modified by N-linked sugar chain containing sialic acid and O-linked sugar chain (FIG. 7).

### Example 22

### Decrease in expression level of protein in human colon cancer cell line COLO320 and SW480 by administering siRNA against the LY6G6D gene

Human colon cancer cell line COLO 320DM or SW480, purchased from American Type Culture Collection (ATCC), was suspended in RPMI1640 medium (Invitrogen corp.) containing 10% fetal bovine (JRH) or DMEM medium (Invitrogen corp.)[culture medium], and plated on a 10cmø tissue culture dish (BD Falcon) with a cell density of 750,000 cells/well, and at the same time siRNA was transfectioned.Specifically, the siRNA-2 prepared in Example 1 having activity which cleaves mRNA of the LY6G6D gene was provided to transfection. As a control, non-silencing dsRNA used in Example 1 was used. 1.25mL of Opti-MEM I (Invitrogen corp.) added with 400 pmol of the siRNA against LY6G6D gene or the non-silencing dsRNA, was mixed with 1.25mL of Opti-MEM I (Invitrogen corp.) added with 15µl of Lipofectamine 2000 (Invitrogen corp.). Then full amount of the mixture was added to 10cmø tissue culture dish to left at room temperature for 20 minutes. Then, to the 10cmø tissue culture dish the cells suspended in each of culture medium were plated with a cell density of 750,000 cells/well, and then cultured overnight at 37°C with 5% carbon dioxide gas air flow. 1 day after transfection, the medium was replaced, and the cells further cultured 1day were distributed to PBS containing 1Mm EDTA and 0.1% BSA. Then the cells were broken in the method described in Example 21, then suspended in the aqueous solution containing 0.5% SDS and 1% 2-mercaptoethanol, then heated at 95°C for 10 minutes, thereafter further added to give 1% Triton X-100 and 50mM phosphate buffer in accordance with the protocol of ENZYMATIC DEGLYCOSYLATION KIT (PROZYME). Further, N-Glycanase (PNGase F), Sialidase A and O-Glycanase were added to react at 37°C for 4 hours. Then one fifth volume of 5-fold SDS-PAGE sample buffer was mixed and heated for 5 minutes at 95°C, and 25 µL of react production were provided to the SDS-PAGE in 15% acrylamide gel. Using R2 antibody obtained in example 16 as the concentration of 5µg/mL, the detection was carried out in accordance with the same method described in Example 20. As the result, the specific band derived from LY6G6D protein of molecular weight 15kD was decreased by administering siRNA against the LY6G6D gene(FIG. 8).

These results indicate that down regulation of the LY6G6D protein induce inhabitation of the cell growth.

### Example 23

### Analysis expression level of the LY6G6D protein in colon cancer cell line

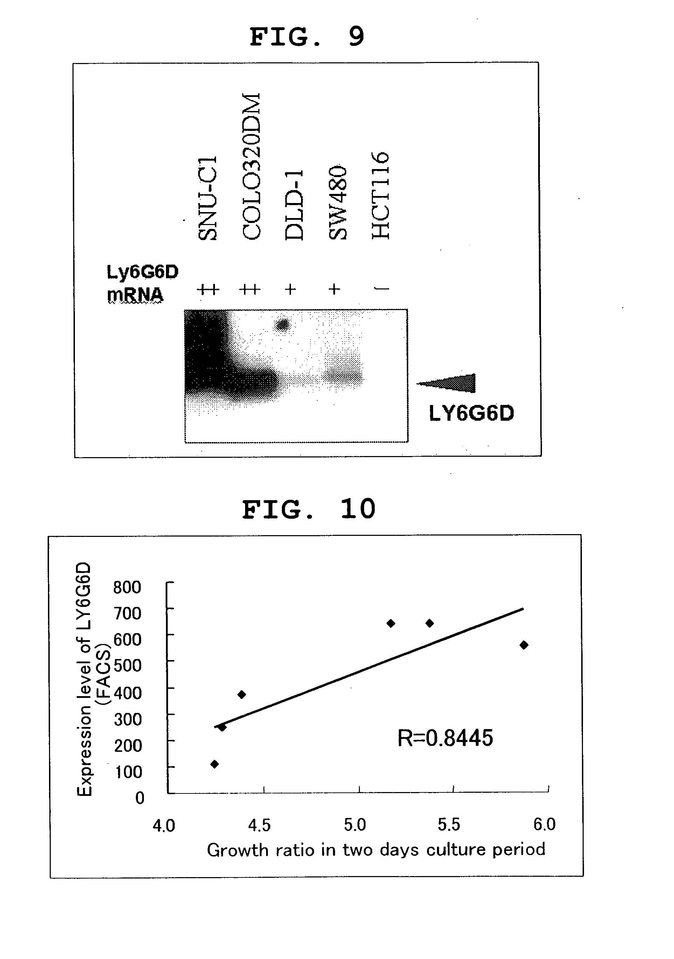

Expression of LY6G6D protein in LY6G6D gene expression cell line was examined. Specifically, Human colon cancer cell line SNU-C1, COLO 320DM, DLD-1, SW480 or HCT116, purchased from American Type Culture Collection (ATCC), were cultured in RPMI1640 medium (Invitrogen corp.) containing 10% fetal bovine (JRH) or DMEM medium (Invitrogen corp.), to obtain subconfluent, then distributed to PBS containing 0.5Mm EDTA and 0.2% BSA. 2 million of the cells were washed with PBS, and broken in accordance with the method described in example 21, then suspended in 50mM phosphate buffer, pH7.0 containing 0.5% SDS and 1% 2-mercaptoethanol, then heated at 95°C for 10 minutes, thereafter further added to give 1% Triton X-100 in accordance with the protocol of ENZYMATIC DEGLYCOSYLATION KIT (PROZYME). Further, N-Glycanase (PNGase F), Sialidase A, and O-Glycanase were added to react at 37°C for 4 hours. Then one fifth volume of 5-fold SDS-PAGE sample buffer was mixed and heated for 5 minutes at 95°C, and 20 µL of react production were provided to the SDS-PAGE in 15% acrylamide gel. Using R2 antibody obtained in example 16 as the concentration of 3µg/mL, the detection was carried out in accordance with the same method described in Example 20. As the result, the specific band derived from LY6G6D protein of molecular weight 15kD was not detected in the LY6G6D gene expression cell line SNU-C1, COLO 320DM, DLD-1 and SW480. On the other hand, the band was not detected in the LY6G6D gene non-expressing cell line HCT116 (FIG. 9).

### Example 24

### Cell growth rate of LY6G6D stable expression cell line (plate culture)

Whether LY6G6D protein influences on a cell growth rate was examined. 6 clones (C01, C03, C07, C12, 003, and 030) of the LY6G6D stable expression cell line obtained in Example 19 were cultured to become subconfluent, were peeled off from a tissue culture dish with trypsin-EDTA (Invitrogen corp.), were suspended in an McCoy's 5A medium (Invitrogen corp.) containing 10% fetal bovine serum (JRH), 800 µg/mL of G418 (Promega), and 10 mM of HEPES, were plated into 96-well flat-bottomed tissue culture plate (BD Falcon) with 3,000 cells/well, and were cultured at 37°C with 5% carbon dioxide gas air flow. The intracellular DNA content of the cells which had been cultured for 1 day and 3 days were determined in accordance with the method described in Example 12 and the ratio of the intracellular DNA content of the third day to that of the first day was calculated as the growth rate. As a result, a positive correlation (R=0.8445) existed between the expression level of LY6G6D protein (FACScan analysis result of PI-PLC non-processed cells in Example 20) and the cell growth (Fig. 10).

### Example 25

### Study of localization of LY6G6D protein (cell staining)

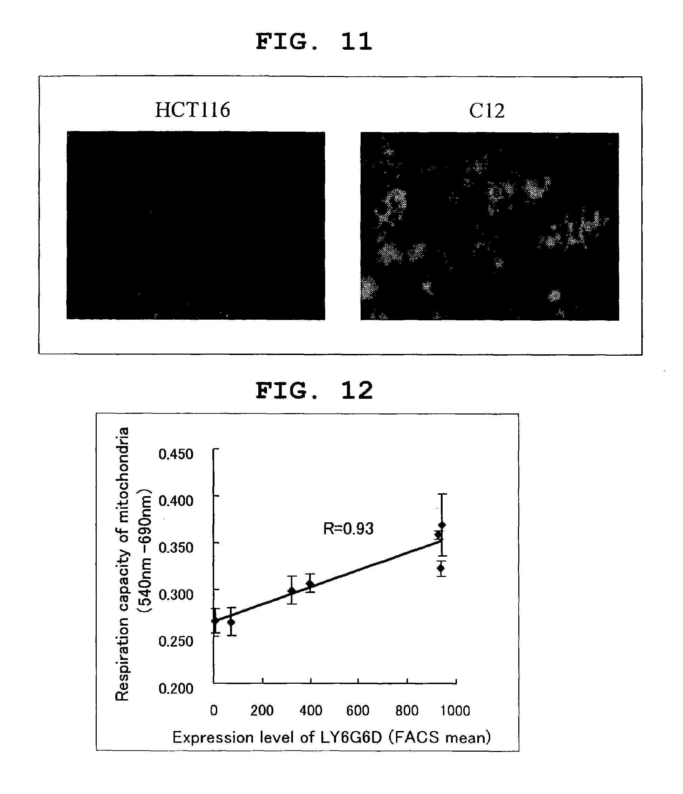

The LY6G6D stable expression cell line clone C12 obtained in Example 19 which was conditioned for two months in the McCoy's 5A culture medium containing 10% fetal bovine serum (JRH) and human colon cancer cell line HCT116 were peeled off from the tissue culture dish with trypsin-EDTA (Invitrogen corp.), were suspended in the culture medium, were plated into poly D-lysine coated 8-well chamber slide (BD Falcon), and were cultured at 37°C with 5% carbon dioxide gas air flow for 2 days. Thereafter, the cells were washed with PBS twice, were fixed at the room temperature for 20 minutes by the use of 10% neutral buffer formalin, and were permeabilized at the room temperature for 5 minutes with PBC containing 0.1% Triton X-100. The resultant cells were washed with the PBS two times and were blocked at the room temperature for 1 hour with PBS containing 1% BSA. A blocking solution containing 1 µg/mL of the R2 antibodies obtained in Example 16 was added, and the resultant cells were left at 4°C for 2 hours and then were washed with the PBS three times. Next, a blocking solution containing 10 µg/mL of Alexa488-labeled anti-rabbit IgG antibody (Molecular Probes) was added thereto, and the resultant cells were left at the room temperature for 1 hour and then were washed with the PBS four times. Nucleuses were stained with an encapsulating agent containing DAPI. The stained portion of LY6G6D protein was observed with a fluorescent microscope. As a result, in, the cells were more adjacent to each other, the LY6G6D protein was stained deeper in outline of the cells (Fig. 11).

### Example 26

### Cell growth rate of LY6G6D stable expression cell line (three-dimensional culture)

The cell growth potential in three-dimensional culture and after conditioned the expression level of LY6G6D protein were compared between 6 clones (C01, C03, C07, C12, 003, and 030) of the LY6G6D stable expression cell line obtained in Example 19 which was conditioned for two months in McCoy's 5A culture medium containing 10% fetal bovine serum (JRH) and human colon cancer cell line HCT116 purchased from American Type Culture Collection (ATCC). Specifically, the cells cultured to become subconfluent were peeled off from a tissue culture dish with trypsin-EDTA (Invitrogen corp.), were suspended in the said culture medium, and plated into 96-well spheoid culture plate (SUMILON) with a cell density of 15,000 cells/well, and were cultured at 37°C with 5% carbon dioxide gas air flow for 4 days. Thereafter, 10µl of PBS containing 5 mg/mL of MTT was added, and the cells were further cultured for 4 hours, and then the culture was removed, isopropanol containing 0.04N hydrochloric acid was added, and the mixture was stirred at the room temperature for 1 hour with light shielded. By measuring an absorption degree of light at 540nm using 690nm as a control, respiration capacity of mitochondria was measured. On the other hand, the cells cultured to become subconfluent were distributed to the PBS containing 0.5 mM of EDTA and 0.2% BSA, 20 µg/mL of the R2 antibody obtained in Example 16 was added and the cells were left at 4°C for 1 hour, and 5 µg/mL of Alexa488-labeled anti-rabbit IgG antibody (Molecular Probes) was added and cells were left at 4°C for 30 minutes, and then the cells were assayed using FACScan (BD Biosciences). As a result, a positive correlation (R=0.935) existed between the expression level of LY6G6D protein and the respiration capacity of mitochondria (Fig. 12).

### Example 27

### Analysis of expression level of LY6G6D protein in human colon cancer tissue and human normal tissue

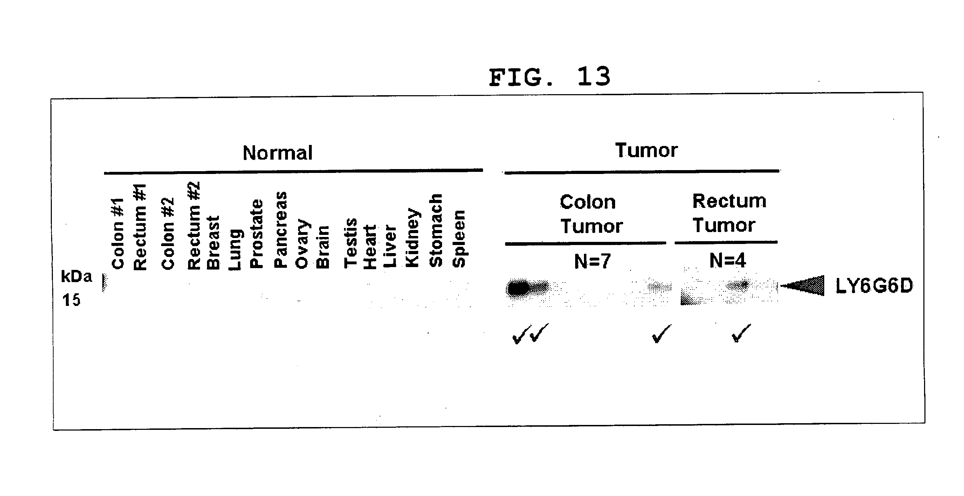

The expression level of LY6G6D protein in the human colon cancer tissue and the human normal tissue was assayed using Total Protein-Human Tumor (Biochain) and Total Protein-Human Normal (Biochain). 7 samples of Colon cancer and 4 samples of Rectum cancer were used as samples of the human colon cancer tissue. Each 2 samples of Colon and Rectum and each one sample of Breast, Lung, Prostate, Pancreas, Ovary, Brain, Testis, Heart, Liver, Kidney, Stomach, and Spleen were used as samples of the human normal tissue. The samples were diluted with an SDS-PAGE sample buffer [62.5 mM Tris·HCl buffer, pH 6.8, 10% glycerol, 1% SDS, 0.02% bromophenol blue, and 2% 2-mercaptoethanol] so that the protein concentration of the tissue extract was 3.3 mg/mL and were heated at 95°C for 10 minutes for deformation. Thereafter, 45 µg of protein was added to become 50 mM phosphate buffer containing 1% Triton X-100 in accordance with the guideline of Enzymatic Deglycosilation Kit (PROZYME INC.). Then N-glycanase (PNGase F), Sialidase A, and O-glycanase were added, and then they were allowed to react at 37°C for 3 hours. One fifth volume of 5-fold SDS-PAGE sample buffer [312.5 mM Tris·HCl buffer, pH 6.8, 40% glycerol, 5% SDS, 0.1% bromophenol blue and 10% 2-mercaptoethanol] was mixed, the mixture was heated at 95°C for 5 minutes, and then 10 µL thereof was provided to SDS-PAGE in 15% acrylamide gel. The protein electrophoresed and isolated was transfered to a clear blot P membrane (ATTO) by a conventional method, and then was left in a blocking solution [50 mM Tris·HCl buffer, pH 7.5, 150 mM sodium chloride, 0.1% tween 20, and 5% BSA] at the room temperature for 1 hour. Next, the R2 antibody obtained in Example 16 and diluted to become 3 µg/mL was maintained in the blocking solution at the room temperature for 2 hours. Subsequently, the HRP-labeled anti-rabbit IgG antibody (Cell Signaling Technology) diluted to 2,000 folds with 50 mM Tris·HCl buffer, pH 7.5, 150 mM sodium chloride, 0.1% tween 20, and 5% skimmed milk was maintained at the room temperature for 1 hour. The LY6G6D protein was detected in accordance with the protocol attached thereto by the use of Immobilon Western HRP substrate (MILLIPORE). As a result, a specific molecular weight 15 kD band derived from the LY6G6D protein was detected from 4 samples of the 11 samples of the human colon cancer tissue. On the other hand, the specific molecular weight 15 kD band derived from the LY6G6D protein was not detected from any sample of the human normal tissue (Fig. 13).

### Example 28

### Increased expression of TM4SF17 gene in cancer

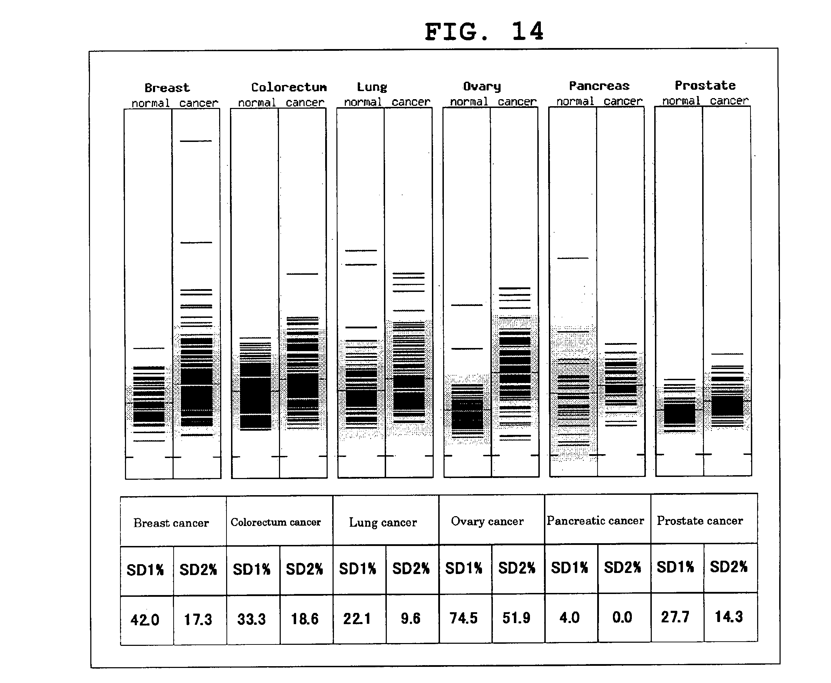

By carrying out the hybridization method using mRNA derived from cancer tissue and mRNA derived from peripheral normal tissue, which are extracted from a cancer patient, a gene expression profile was prepared. As a result of analysis of the gene expression profile, it was found that the aforementioned gene is expressed highly in the cancer tissue. Particularly, in breast cancer, lung cancer, and ovary cancer, remarkable increased expression was observed in comparison with the peripheral normal tissue (Fig. 14).

### Example 29

### Inhibition of cell growth in human breast cancer cell line by administering siRNA against TM4SF17 gene

Human breast cancer cell lines MDA-MB-231 purchased from American Type Culture Collection (ATCC) were suspended in a Leibovitz's L-15 medium (Invitrogen corp.) containing 10% fetal bovine serum (JRH), were plated on a 96-well flat-bottomed tissue culture plate (BD Falcon) with a cell density of 2,000 cells/well, and were cultured at 37°C overnight. Thereafter, a siRNA was transfected.

In specific, three kinds of siRNA having activities of cleaving all mRNAs of TM4SF17a gene, TM4SF17b gene, and TM4SF17c gene were mixed and provided to the transfection. As a control, dsRNA secured not to exhibit a non-specific RNAi effect to a cell (hereinafter, abbreviated as non-silencing dsRNA) was used. 4 pmol siRNA against TM4SF17 gene or 4 pmol non-silencing dsRNA was added to 10 µL Opti-MEM I (Invitrogen corp.), was mixed with 10 µL Opti-MEM I (Invitrogen corp.) to which 0.2 µL of Lipofectamine 2000 (Invitrogen corp.) was added, and then was left at the room temperature for 20 minutes. The mixed solution was all added to a cell culture of MDA-MB-231 and was further cultured for 24 hours, and then the medium was exchanged. The cells were further cultured in the Leibovitz' s L-15 medium (Invitrogen corp.) containing 10% fetal bovine serum (JRH) at 37°C for 3 days. After the medium was removed, the plate was left at rest at -80°C for 30 minutes or more and then was left at the room temperature for 5 minutes. An aqueous solution containing 0.25% CyQUANT GR reagent (Molecular Probes) and 5% cell lysis buffer (Molecular Probes) was added thereto by 200 µL/well and then the mixture was left for 15 minutes. Thereafter, by measuring fluorescence intensity thereof at an excitation wavelength of 485 nm and at an emission wavelength of 535 nm, the intracellular DNA content was assayed. As a result, the cell administered with siRNA against TM4SF17 gene had the fluorescence intensity lowered by about 23% than that of the cell administered with the non-silencing dsRNA, which indicated a statistically significant difference (P<0.05) (Table 1).

### Example 30

### Decrease in expression level of mRNA of TM4SF17 gene by administering siRNA agaisnt TM4SF17 gene

The human breast cancer cell lines MDA-MB-231 used in Example 29 were suspended in the Leibovitz's L-15 medium and were plated on a 96-well flat-bottomed tissue culture plate (BD Falcon) with a cell density of 2,000 cells/well. The cell lines were cultured at 37°C overnight and then a siRNA was transfected in accordance with the method used in Example 29. After the transfection, the cell lines were continuously cultured for 24 hours and then total RNAs were extracted therefrom by the use of RNeasy Mini Total RNA Kit (QIAGEN). Using as a template about 100 ng of the total RNA, reverse transcription was carried out using TaqMan Reverse Transcription Reagents (Applied Biosystems Inc.) in accordance with the protocol attached thereto. Using as a template cDNA corresponding to 4 ng when converted into the total RNA, the expression level of the TM4SF17 gene was assayed using a quantitative PCR method by adding 5 µL of TaqMan Universal PCR master Mix (Applied Biosystems Inc.) and 0.5 µL of TaqMan Reagents (Applied Biosystems Inc.) containing a primer set amplifying base sequences derived from a part of a first exon and a part of a second exon of the TM4SF17 gene to prepare a 10 µL reaction solution. In the PCR reaction, after 2 minutes at 50°C and 10 minutes at 95°C, a cycle of 15 seconds at 95°C and 1 minute at 60°C was repeated 40 times. On the other hand, the expression level of a β-actin gene contained in the same amount of template cDNA was assayed using TaqMan β-actin forward primer, reverse primer, and probe (all of which were made by Applied Biosystems Inc.), which was used as an internal standard.

In the group administered with siRNA against the TM4SF17 gene, the expression level of mRNA of the TM4SF17 genes was decreased by 91% as compared to the group administered with the non-silencing dsRNA which was used as a negative control. These results revealed that the inhibition of cell growth of the human breast cancer cell lines MDA-MB-231 was induced by the decrease in expression level of the TM4SF17a gene, the TM4SF17b gene, or the TM4SF17c gene (Table 1).

### Example 31

### Inhibition of cell growth in human breast cancer cell line MDA-MB-436 by administering siRNA against TM4SF17 gene

Human breast cancer cell lines MDA-MB-436 purchased from American Type Culture Collection (ATCC) were suspended in a Leibovitz's L-15 medium (Invitrogen corp.) containing 10% fetal bovine serum (JRH), were plated on a 96-well flat-bottomed tissue culture plate (BD Falcon) with a cell density of 3,000 cells/well, and were cultured at 37°C overnight. Thereafter, a siRNA was transfected.

In specific, two kinds of siRNA1 and siRNA 2 having activities of cleaving all mRNAs of TM4SF17a gene, TM4SF17b gene and TM4SF17c gene were provided to the transfection. These siRNAs were used by annealing a single-stranded oligo RNA in which an overhang of dTdT is attached to the 3' -terminal in the shown base sequence with a single-stranded oligo RNA in which an overhang of dTdT was attached to the 3'-terminal of the base sequence complementary thereto. As a control, a dsRNA secured not to exhibit a non-specific RNAi effect to a cell (hereinafter, abbreviated as non-silencing dsRNA) was used. 1 pmol of siRNA against TM4SF17 gene or 1 pmol non-silencing dsRNA was added to 10 µL of Opti-MEM I (Invitrogen corp.), was mixed with 10 µL Opti-MEM I (Invitrogen corp.) to which 0.1 µL of Lipofectamine 2000 (Invitrogen corp.) was added, and then was left at the room temperature for 20 minutes. The mixed solution was all added to a cell culture of MDA-MB-436 and was further cultured for 24 hours, and then the medium was exchanged. The cells were further cultured in the Leibovitz's L-15 medium (Invitrogen corp.) containing 10% fetal bovine serum (JRH) at 37°C for 4 days. After the medium was removed, the plate was left at rest at -80°C for 30 minutes or more and then was left at the room temperature for 5 minutes. An aqueous solution containing 0.25% CyQUANT GR reagent (Molecular Probes) and 5% cell lysis buffer (Molecular Probes) was added thereto by 175 µL/well and then the mixture was left for 15 minutes. Thereafter, by measuring fluorescence intensity thereof at an excitation wavelength of 485 nm and at an emission wavelength of 535 nm, the intracellular DNA content was assayed. As a result, the cell administered with two kinds of siRNAs against the TM4SF17 gene had the fluorescence intensity lowered by about 28% (P=0.0114) in the siRNA1 and by about 21% (P=0.1070) in the siRNA2 as compared to the cell administered with the non-silencing dsRNA (Table 1).
siRNA1: cccagaaccucgugaguga(SEQ ID No: 38)
siRNA2: gggccaaucuggagugaaa(SEQ ID No: 39)

### Example 32

### Inhibition of cell growth in human breast cancer cell lines MDA-MB-4355 and MDA-MB-175VII by administering siRNA against TM4SF17 gene

Human breast cancer cell lines MDA-MB-435S and MDA-MB-175VII purchased from American Type Culture Collection (ATCC) were suspended in a Leibovitz's L-15 medium (Invitrogen corp.) containing 10% fetal bovine serum (JRH), were plated on a 96-well flat-bottomed tissue culture plate (BD Falcon) with a cell density of 2,000 cells/well, and were cultured at 37°C overnight. Thereafter, a siRNA was transfected.

Specifically, two kinds of siRNA1 and siRNA 2 having activities of cleaving all mRNAs of TM4SF17a gene, TM4SF17b gene and TM4SF17c gene were provided to the transfection. These siRNAs were used by annealing a single-stranded oligo RNA in which an overhang of dTdT is attached to the 3'-terminal in the shown base sequence with a single-stranded oligo RNA in which an overhang of dTdT was attached to the 3'-terminal of the base sequence complementary thereto. As a control, n dsRNA secured not to exhibit a non-specific RNAi effect to a cell (hereinafter, abbreviated as non-silencing dsRNA) was used. 1 pmol of siRNA agaisnt TM4SF17 gene or 1 pmol non-silencing dsRNA was added to 10 µL of Opti-MEM I (Invitrogen corp.), was mixed with 10 µL Opti-MEM I (Invitrogen corp.) to which 0.1 µL of Lipofectamine 2000 (Invitrogen corp.) was added, and then was left at the room temperature for 20 minutes. The mixed solution was all added to cell cultures of MDA-MB-435S and MDA-MB-175VII and was further cultured for 24 hours, and then the medium was exchanged. The cells were further cultured in the Leibovitz's L-15 medium (Invitrogen corp.) containing 10% fetal bovine serum (JRH) at 37°C for 3 days. After the medium was removed, the plate was left at rest at -80°C for 30 minutes or more and then was left at the room temperature for 5 minutes. An aqueous solution containing 0.25% CyQUANT GR reagent (Molecular Probes) and 5% cell lysis buffer (Molecular Probes) was added thereto by 150 µL/well and then the mixture was left for 15 minutes. Thereafter, by measuring fluorescence intensity thereof at an excitation wavelength of 485 nm and at an emission wavelength of 535 nm, the intracellular DNA content was assayed. As a result, the cell administered with two kinds of siRNAs against the TM4SF17 gene had the fluorescence intensity lowered by about 64% (P=0. 0002) in the siRNA1 and about 57% (P<0.0001) in the siRNA2 in MDA-MB-435S and by about 41% (P=0.0072) in the siRNA1 and about 40% (P=0.0086) in the siRNA2 in MDA-MB-175VII, compared with the cell administered with the non-silencing dsRNA (Table 1).

### Example 33

### Inhibition of cell growth in human breast cancer cell lines HCC1937 and T-47D by administering siRNA against TM4SF17 gene

Human breast cancer cell lines HCC1937 and T-47D purchased from American Type Culture Collection (ATCC) were suspended in a RPMI1640 medium (Invitrogen corp.) containing 10% fetal bovine serum (JRH) and 1 mM sodium pyruvate (Invitrogen corp.), were plated on a 96-well flat-bottomed tissue culture plate (BD Falcon) with a cell density of 3,000 cells/well, and were cultured at 37°C overnight. Thereafter, a siRNA was transfected.

Specifically, two kinds of siRNA1 and siRNA 2 having activities of cleaving all mRNAs of TM4SF17a gene, TM4SF17b gene and TM4SF17c gene were provided to the transfection. These siRNAs were used by annealing a single-stranded oligo RNA in which an overhang of dTdT is attached to the 3'-terminal in the shown base sequence with a single-stranded oligo RNA in which an overhang of dTdT was attached to the 3'-terminal of the base sequence complementary thereto. As a control, n dsRNA secured not to exhibit a non-specific RNAi effect to a cell (hereinafter, abbreviated as non-silencing dsRNA) was used. 1 pmol of siRNA against TM4SF17 gene or 1 pmol non-silencing dsRNA was added to 10 µL of Opti-MEM I (Invitrogen corp.), was mixed with 10 µL Opti-MEM I (Invitrogen corp.) to which 0.1 µL of Lipofectamine 2000 (Invitrogen corp.) was added, and then was left at the room temperature for 20 minutes. The mixed solution was all added to cell cultures of HCC1937 and T-47D and was further cultured for 24 hours, and then the medium was exchanged. The cells were further cultured in the RPMI1640 medium (Invitrogen corp.) containing 10% fetal bovine serum (JRH) and 1 mM sodium pyruvate (Invitrogen corp.) at 37°C for 3 days. After the medium was removed, the plate was left at rest at -80°C for 30 minutes or more and then was left at the room temperature for 5 minutes. An aqueous solution containing 0.25% CyQUANT GR reagent (Molecular Probes) and 5% cell lysis buffer (Molecular Probes) was added thereto by 175 µL/well for HCC1937 and by 150 µL/well for T-47D and then the mixture was left for 15 minutes. Thereafter, by measuring fluorescence intensity thereof at an excitation wavelength of 485 nm and at an emission wavelength of 535 nm, the intracellular DNA content was assayed. As a result, the cell administered two kinds of siRNAs against the TM4SF17 gene had the fluorescence intensity lowered by about 36% (P=0.0195) in the siRNA1 and about 25% (P=0.0124) in the siRNA2 in HCC1937 and by about 24% (P=0.0061) in the siRNA1 and about 28% (P=0.0743) in the siRNA2 in T-47D, compared with the cell administered with the non-silencing dsRNA (Table 1).

### Example 34

### Decrease in expression level of mRNA of breast cancer cell line MDA-MB-436 by administering siRNA against TM4SF17

The human breast cancer cell lines MDA-MB-436 used in Example 31 were suspended in the Leibovitz's L-15 medium (Invitrogen corp.) containing 10% fetal bovine serum (JRH) and were plated on a 96-well flat-bottomed tissue culture plate (BD Falcon) with a cell density of 3,000 cells/well. The cell lines were cultured at 37°C overnight and then a siRNA was transfected in accordance with the method used in Example 31. After the transfection, the cell lines were continuously cultured for 24 hours and then total RNAs were extracted therefrom by the use of RNeasy Mini Total RNA Kit (QIAGEN). Using as a template about 150 ng of the total RNA, reverse transcription was carried out using TaqMan Reverse Transcription Reagents (Applied Biosystems Inc.) in accordance with the protocol attached thereto. Using as a template cDNA corresponding to 10 ng when converted into the total RNA, the expression level of the TM4SF17 gene was assayed using a quantitative PCR method by adding 5 µL of TaqMan Universal PCR master Mix (Applied Biosystems Inc.) and 0.5 µL of TaqMan Reagents (Applied Biosystems Inc.) containing a primer set amplifying base sequences derived from a part of a first exon and a part of a second exon of the TM4SF17 gene to prepare a 10 µL reaction solution. In the PCR reaction, after 2 minutes at 50°C and 10 minutes at 95°C, a cycle of 15 seconds at 95°C and 1 minute at 60°C was repeated 40 times. On the other hand, the expression level of a β-actin gene contained in the same amount of template cDNA was assayed using TaqMan β-actin forward primer, reverse primer, and probe (all of which were made by Applied Biosystems Inc.), which was used as an internal standard.

In the group administered with siRNA against the TM4SF17 gene, the expression level of mRNA of the TM4SF17 genes was decreased by 102% in siRNA1 and about 91% in siRNA2, compared with the group administered with the non-silencing dsRNA which was used as a negative control. These results revealed that the inhibition of cell growth of the human breast cancer cell lines MDA-MB-436 was induced by the decrease in expression level of the TM4SF17a gene, the TM4SF17b gene or the TM4SF17c gene (Table 1).

### Example 35

### Decrease in expression level of mRNA of breast cancer cell lines MDA-MB-435S and MDA-MB-175VII by administering siRNA against TM4SF17

The human breast cancer cell lines MDA-MB-435S and MDA-MB-175VII used in Example 32 were suspended in the Leibovitz's L-15 medium (Invitrogen corp.) containing 10% fetal bovine serum (JRH) and were plated on a 96-well flat-bottomed tissue culture plate (BD Falcon) with a cell density of 2,000 cells/well. The cell lines were cultured at 37°C overnight and then a siRNA was transfected in accordance with the method used in Example 32. After the transfection, the cell lines were continuously cultured for 24 hours and then total RNAs were extracted therefrom by the use of RNeasy Mini Total RNA Kit (QIAGEN). Using as a template about 150 ng of the total RNA, reverse transcription was carried out using TaqMan Reverse Transcription Reagents (Applied Biosystems Inc.) in accordance with the protocol attached thereto. Using as a template about 200 ng of cDNA in MDA-MB-435S and about 120 ng of total RNA in MDA-MB-175VII, the expression level of the TM4SF17 gene was assayed using a quantitative PCR method by adding 5 µL of TaqMan Universal PCR master Mix (Applied Biosystems Inc.) and 0.5 µL of TaqMan Reagents (Applied Biosystems Inc.) containing a primer set amplifying base sequences derived from a part of a first exon and a part of a second exon of the TM4SF17 gene to prepare a 10 µL reaction solution. In the PCR reaction, after 2 minutes at 50°C and 10 minutes at 95°C, a cycle of 15 seconds at 95°C and 1 minute at 60°C was repeated 40 times. On the other hand, the expression level of a β-actin gene contained in the same amount of template cDNA was assayed using TaqMan β-actin forward primer, reverse primer, and probe (all of which were made by Applied Biosystems Inc.), which was used as an internal standard.

In the group administered with the siRNA against the TM4SF17 gene, the expression level of mRNA of the TM4SF17 genes was decreased by about 93% in siRNA1 and about 89% in siRNA2 in MDA-MB-435S and by about 94% in siRNA1 and about 64% in siRNA2 in MDA-MB-175VII, compared with the group administered with the non-silencing dsRNA which was used as a negative control. These results revealed that the inhibition of cell growth of the human breast cancer cell lines MDA-MB-435S and MDA-MB-175VII was induced by the decrease in expression level of the TM4SF17a gene, the TM4SF17b gene, or the TM4SF17c gene (Table 1).

### Example 36

### Decrease in expression level of mRNA of breast cancer cell lines HCC1937 and T-47D by administering siRNA against TM4SF17

The human breast cancer cell lines HCC1937 and T-47D used in Example 33 were suspended in the RPMI1640 medium (Invitrogen corp.) containing 10% fetal bovine serum (JRH) and 1 mM sodium pyruvate (Invitrogen corp.) and were plated on a 96-well flat-bottomed tissue culture plate (BD Falcon) with a cell density of 3,000 cells/well. The cell lines were cultured at 37°C overnight and then a siRNA was transfected in accordance with the method used in Example 33. After the transfection, the cell lines were continuously cultured for 24 hours and then total RNAs were extracted therefrom by the use of RNeasy Mini Total RNA Kit (QIAGEN). Using as a template about 400 ng of total RNA for HCC1937 and about 85 ng of total RNA in T-47D, reverse transcription was carried out using TaqMan Reverse Transcription Reagents (Applied Biosystems Inc.) in accordance with the protocol attached thereto. Using as a template cDNA corresponding to 4 ng when converted into the total RNA, the expression level of the TM4SF17 gene was assayed using a quantitative PCR method by adding 5 µL of TaqMan Universal PCR master Mix (Applied Biosystems Inc.) and 0.5 µL of TaqMan Reagents (Applied Biosystems Inc.) containing a primer set amplifying base sequences derived from a part of a first exon and a part of a second exon of the TM4SF17 gene to prepare a 10 µL reaction solution. In the PCR reaction, after 2 minutes at 50°C and 10 minutes at 95°C, a cycle of 15 seconds at 95°C and 1 minute at 60°C was repeated 40 times. On the other hand, the expression level of a β-actin gene contained in the same amount of template cDNA was assayed using TaqMan β-actin forward primer, reverse primer, and probe (all of which were made by Applied Biosystems Inc.), which was used as an internal standard.

In the group administered with the siRNA against the TM4SF17 gene, the expression level of mRNA of the TM4SF17 genes was decreased by about 112% in siRNA1 and about 85% in siRNA2 in HCC1937 and by about 76% in siRNA1 and about 74% in siRNA2 in T-47D, compared with the group administered with the non-silencing dsRNA which was used as a negative control. These results revealed that the inhibition of cell growth of the human breast cancer cell lines HCC1937 and T-47D was induced by the decrease in expression level of the TM4SF17a gene, the TM4SF17b gene or the TM4SF17c gene (Table 1).

**(Table 1)**

| Cell line | siRNA | Expression level of mRNA (% to NS) | Cell growth (% to NS) |
|---|---|---|---|
| MDA-MB-231 | mixture | 9 | 77 |
| HCC1937 | siRNA1 | -12 | 64 |
| | siRNA2 | 15 | 75 |
| MDA-MB-435S | siRNA1 | 7 | 36 |
| | siRNA2 | 11 | 43 |
| MDA-MB-175-VII | siRNA1 | 6 | 59 |
| | siRNA2 | 36 | 60 |
| T-47D | siRNA1 | 24 | 72 |
| | siRNA2 | 26 | 76 |
| MDA-MB-436 | siRNA1 | -2 | 72 |
| | siRNA2 | 9 | 79 |

### Example 37

### Construction of animal cell expression vector for recombinant C terminal 3xFLAG tagged TM4SF17 isoform b protein

Using cDNA of human lung cancer cell line A549 origin as a template, PCR was carried out with respect to a fragment 1 from ATG to 726bp using primer 1 (SEQ ID No: 40) and primer 2 (SEQ ID NO: 41), and fragment 2 from 291bp to 987bp just before the termination codon of isoform b using primer 3 (SEQ ID No: 42) and primer 4 (SEQ ID No: 43), respectively. In addition to a sequence coinciding with TM4SF17 isoform b, the primer 1 was added with recognition sequence 6bp of restriction enzyme EcoRI, and the primer 4 was added with recognition sequence 6bp of restriction enzyme XbaI. The composition of reaction liquid in the reaction was 20 µL of liquid volume by using about 50 ng of the cDNA as a template, and adding 0.2 µL of TAKARA LA taq (TaKaRa Bio), each 500 nM of the primer 1 (SEQ ID No: 40) and the primer 2 (SEQ ID NO: 41) or the primer 3 (SEQ ID No: 42) and the primer 4 (SEQ ID No: 43), 400 µM of dNTPs and 10 µL of 2xGC Buffer (TaKaRa Bio). PCR was carried out by reacting at 94°C for 1 minute and then repeating 40 times the cycle set to include 94°C for 30 seconds, 60°C for 30 seconds and 72°C for 2 minutes. The PCR product obtained was separated by agarose gel electrocataphoresis, and the DNA fragment corresponding to the length expected in the fragment 1 and fragment 2 was confirmed. Among the PCR product, 15 µL of reaction liquid was purified by using MinElute PCR purification kit (QIAGEN) to obtain 8 µL of product. 4 µL of the purified product was combined to pCR-TOPO vector by TOPO TA Cloning Kit for Sequencing (Invitrogen corp.) and introduced into Escherichia coli One Shot TOP10 (Invitrogen corp.), and then selected in LB agar culture medium including ampicillin. As a result of analyzing each clone sequence, among cDNA sequence (SEQ ID NO: 7) encoding TM4SF17 isoform b protein (SEQ ID NO:8), each pCR4-TOPO vector including clone having fragment from ATG to 726bp, and fragment 2 from 291bp to 987bp just before termination codon of isoform b was obtained.

About 1 µg of pCR4-TOPO vector including fragment 1 was treated with restriction enzymes EcoRI and BamHI and separated by agarose gel electrocataphoresis. The fragment 1 was purified by MinElute Gel Extraction Kit (QIAGEN). Similarly, About 1 µg of pCR4-TOPO vector including fragment 2 was treated with restriction enzymes BamHI and XbaI and separated by agarose gel electrocataphoresis. The fragment 2 was purified by MinElute Gel Extraction Kit (QIAGEN). Simultaneously, about 1 µg of p3XFLAG-CMV-14 (SIGMA) was treated with restriction enzymes EcoRI and XbaI and purified by QIAquick PCR Purification Kit (QIAGEN) . The vector and fragments 1 and 2 were subjected to ligation reaction with the use of DNA Ligation Kit Ver.2 (TaKaRa Bio), and thereafter introduced into Escherichia coli One Shot TOP10 (Invitrogen corp.) and selected in LB agar culture medium including ampicillin. As a result of analyzing each clone sequence, animal cell expression vector p3xFLAG-CMV-14-TM4SF17b (hereinafter, it is sometimes referred to as b-3F or b-3xFLAG) having cDNA sequence (SEQ ID NO: 7) encoding TM4SF17 isoform b protein (SEQ ID NO: 8) was obtained.
Primer 1 GAATTCATGCCCGGCAAGCACCAGCAT (SEQ ID NO: 40)
Primer 2 GAAGACTCCCGCCACCACAATCAG (SEQ ID NO: 41)
Primer 3 CGTGTTCCTCGGTCTCATCTTCTTC (SEQ ID NO: 42)
Primer 4 TCTAGACCAATTTTTAAAGGTTGGCTC (SEQ ID NO: 43)

### Example 38

### Construction of animal cell expression vector for recombinant C terminal 3xFLAG tagged TM4SF17 isoform a protein

Using TM4SF17 isoform b vector prepared in Example 37 as a template, TM4SF17 isoform a was subjected to PCR with respect to a fragment 3 using primer 1 (SEQ ID No: 40) and primer 5 (SEQ ID NO: 44) and fragment 4 using primer 6 (SEQ ID No: 45) and primer 4 (SEQ ID No: 43), respectively. The composition of reaction liquid in the reaction was 20 µL of liquid volume by using about 400 ng of the vector as a template, and adding 0.2 µL of TAKARA LA taq (TaKaRa Bio), each 500 nM of the primer 1 (SEQ ID No: 40) and the primer 5 (SEQ ID NO: 44) or the primer 6 (SEQ ID No: 45) and the primer 4 (SEQ ID No: 43), 400 µM of dNTPs and 10 µL of 2xGC Buffer (TaKaRa Bio). PCR was carried out by reacting at 94°C for 1 minute and then repeating 30 times the cycle set to include 94°C for 30 seconds, 60°C for 30 seconds and 72°C for 2 minutes. The PCR product obtained was separated by agarose gel electrocataphoresis, and the DNA fragment corresponding to the length expected in the fragment 3 and fragment 4 was confirmed. Among the PCR product, 17 µL of reaction liquid was purified by using MinElute PCR purification kit (Qiagen) to obtain 8 µL of product, respectively. By using this, PCR reaction for amplifying total length of TM4SF17 isoform a was carried out. The composition of reaction liquid in the reaction was 20 µL of liquid volume by using a mixture of 1 µL of the purified product of fragment 3 and 0.3 µL of the purified product of fragment 4 as a template, and adding 0.2 µL of TAKARA LA taq (TaKaRa Bio), each 500 nM of the primer 1 (SEQ ID No: 40) and the primer 4 (SEQ ID NO: 43), 400 µM of dNTPs and 10 µL of 2xGC Buffer (TaKaRa Bio). PCR was carried out by reacting at 94°C for 1 minute and then repeating 25 times the cycle set to include 94°C for 30 seconds, 60°C for 30 seconds and 72°C for 2 minutes. The PCR product obtained was separated by agarose gel electrocataphoresis, and the DNA fragment corresponding to the expected length was confirmed. It was combined to pCR-TOPO vector by TOPO TA Cloning Kit for Sequencing (Invitrogen corp.) and introduced into Escherichia coli One Shot TOP10 (Invitrogen corp.), and then selected in LB agar culture medium including ampicillin. As a result of analyzing each clone sequence, pCR4-TOPO vector including cDNA sequence (SEQ ID NO: 5) encoding TM4SF17 isoform a protein (SEQ ID NO: 6) was obtained.

About 2 µg of pCR4-TOPO vector including TM4SF17 isoform a sequence was treated with restriction enzymes EcoRI and XbaI and separated by agarose gel electrocataphoresis, and then was purified by MinElute Gel Extraction Kit (QIAGEN). Simultaneously, about 0.3 µg of p3XFLAG-CMV-14 (SIGMA) was treated with restriction enzymes EcoRI and XbaI and purified by QIAquick PCR Purification Kit (QIAGEN). The vector and the fragment were subjected to ligation reaction with the use of DNA Ligation Kit Ver.2 (TaKaRa Bio), and thereafter introduced into Escherichia coli ECOS DH5α (Nippon Gene) and selected in LB agar culture medium including ampicillin. As a result of analyzing each clone sequence, animal cell expression vector p3xFLAG-CMV-14-TM4SF17a (hereinafter, it is sometimes referred to as a-3F or a-3xFLAG) having cDNA sequence (SEQ ID NO: 5) encoding TM4SF17 isoform a protein (SEQ ID NO: 6) was obtained.
Primer 5 GCTCCCCTCTCACCAGTTTGAGCCGGACGTCGTAG (SEQ ID NO: 44)
Primer 6 AACTGGTGAGAGGGGAGCTGGAGCAGCAGGGCTTC (SEQ ID NO: 45)

### Example 39

### Construction of animal cell expression vector for recombinant N terminal FLAG tagged TM4SF17 isoform b protein

About 2.5 µg of TM4SF17 isoform b in p3XFLAG-CMV-14 was cut by restriction enzyme EcoRI and XbaI, and the fragment was purified by MinElute Gel Extraction kit (Qiagen) to be used as a template. PCR reaction using primer 7 (SEQ ID NO: 46) and primer 8 (SEQ ID NO: 47) was carried out by using Pfu Ultra. The composition of PCR reaction liquid was 20 µL of liquid volume by using about 120 ng of the purified fragment as a template, and adding 0.4 µL of Pfu Ultra High-Fidelity DNA Polymerase (Stratagene), each 500 nM of the primer 7 (SEQ ID No: 46) and the primer 8 (SEQ ID NO: 47), 400 µM of dNTPs and 2 µL of 10x Pfu Ultra High-Fidelity DNA Polymerase Reaction Buffer(Stratagene). PCR was carried out by reacting at 95°C for 2 minute and then repeating 30 times the cycle set to include 95°C for 30 seconds, 60°C for 30 seconds and 72°C for 2 minutes. The PCR product obtained was separated by agarose gel electrocataphoresis and confirmed that the fragment having the expected length was amplified, and thereafter it was diluted by 50 times with distilled water to be used as a template for subsequent PCR reaction using primer 9 (SEQ ID NO: 48) and primer 8 (SEQ ID NO: 47). The composition of PCR reaction liquid was 20 µL of liquid volume by using about 1 µL of the diluted PCR product as a template, and adding 0.4 µL of Pfu Ultra High-Fidelity DNA Polymerase (Stratagene), each 500 nM of the primer 9 (SEQ ID No: 48) and the primer 8 (SEQ ID NO: 47), 400 µM of dNTPs and 2 µL of 10x Pfu Ultra High-Fidelity DNA Polymerase Reaction Buffer (Stratagene). PCR was carried out by reacting at 95°C for 2 minute and then repeating 30 times the cycle set to include 95°C for 30 seconds, 60°C for 30 seconds and 72°C for 2 minutes. The product was separated by agarose gel electrocataphoresis, and purified by MinElute Gel Extraction kit (Qiagen). The fragment was combined to pCR-Blunt II-TOPO vector by TOPO TA Cloning Kit for Sequencing (Invitrogen corp.) and introduced into Escherichia coli One Shot TOP10 (Invitrogen corp.), and then selected in LB agar culture medium including kanamycin. As a result of analyzing each clone sequence, pCR-Blunt II-TOPO vector including cDNA sequence (SEQ ID NO: 7) encoding TM4SF17 isoform b protein (SEQ ID NO: 8) which has 1xFLAG at N terminal and termination codon at C terminal was obtained.

About 2.5 µg of pCR-Blunt II-TOPO vector was treated with restriction enzymes EcoRI and XbaI and separated by agarose gel electrocataphoresis, and then the fragment was purified by MinElute Gel Extraction Kit (QIAGEN). Simultaneously, about 1 µg of pcDNA3.1(+) (Invitrogen corp.) was treated with restriction enzymes EcoRI and XbaI and purified by QIAquick PCR Purification Kit (QIAGEN). The vector and the fragment were subjected to ligation with the use of DNA Ligation Kit Ver.2 (TaKaRa Bio), and thereafter introduced into Escherichia coli ECOS DH5α (Nippon Gene) and selected in LB agar culture medium including ampicillin. As a result of analyzing each clone sequence, animal cell expression vector pcDNA3.1(+)-FLAG-TM4SF17b (hereinafter, it is sometimes referred to as F-b or FLAG-b) having cDNA sequence (SEQ ID NO: 7) encoding TM4SF17 isoform b protein (SEQ ID NO: 8) which has 1xFLAG at N terminal and termination codon at C terminal was obtained.
Primer 7 AAAGACGATGACGACAAGCCCGGCAAGCACCAGCATTTC (SEQ ID NO: 46)
Primer 8 ATGTCTAGACTACCAATTTTTAAAGGTTGGCTC (SEQ ID NO: 47)
Primer 9 GCCGCCACCATGGACTACAAAGACGATGACGACAAG (SEQ ID NO: 48)

### Example 40

### Construction of animal cell expression vector for recombinant N terminal FLAG tagged TM4SF17 isoform d protein

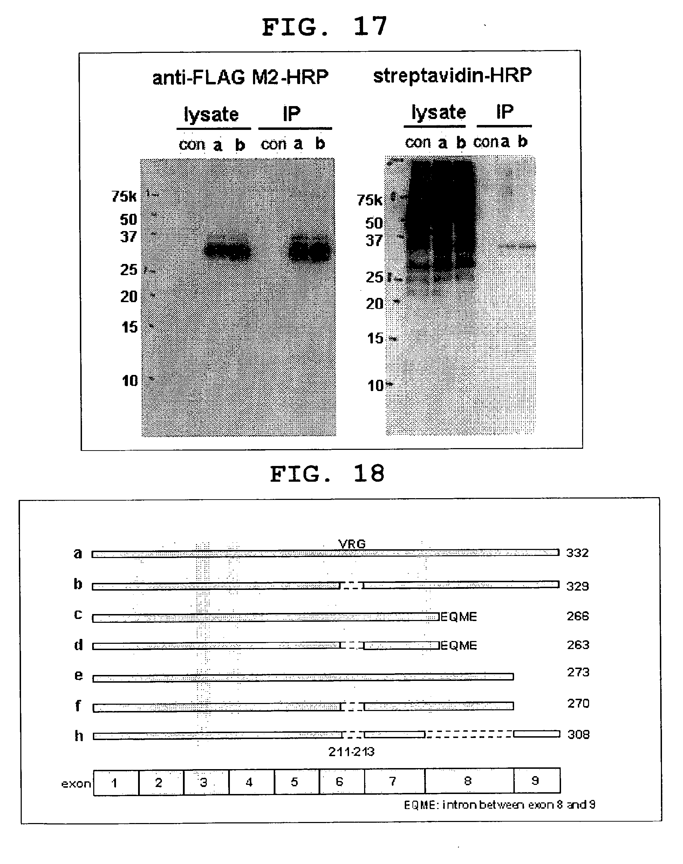

Isoform d is isoform of sequence to which 3 amino acids of 211-213 of isoform c are concerned (Fig. 18). The pcDNA3.1(+) having cDNA sequence encoding N terminal FLAG tagged TM4SF17 isoform b protein was used as a template, and PCR reaction using primer 9 (SEQ ID NO: 48) and primer 10 (SEQ ID NO: 49) was carried out by using Pfu Ultra. The composition of PCR reaction liquid was 20 µL of liquid volume by using about 300 ng of the purified fragment as a template, and adding 0.4 µL of Pfu Ultra High-Fidelity DNA Polymerase (Stratagene), each 500 nM of the primer 9 (SEQ ID No: 48) and the primer 10 (SEQ ID NO: 49), 400 µM of dNTPs and 2 µL of 10x Pfu Ultra High-Fidelity DNA Polymerase Reaction Buffer (Stratagene). PCR was carried out by reacting at 95°C for 2 minute and then repeating 30 times the cycle set to include 95°C for 30 seconds, 60°C for 30 seconds and 72°C for 3 minutes. The product was purified by using QIAquick PCR Purification Kit (QIAGEN), and the fragment was treated with restriction enzymes EcoRI and XbaI and purified by using QIAquick PCR Purification Kit (QIAGEN). Simultaneously, about 1 µg of pcDNA3.1(+) (Invitrogen corp.) was treated with restriction enzymes EcoRI and XbaI and purified by QIAquick PCR Purification Kit (QIAGEN). The vector and the fragment were subjected to ligation with the use of DNA Ligation Kit Ver. 2 (TaKaRa Bio), and thereafter introduced into Escherichia coli ECOS DH5α (Nippon Gene) and selected in LB agar culture medium including ampicillin. As a result of analyzing each clone sequence, animal cell expression vector pcDNA3.1(+)-FLAG-TM4SF17d (hereinafter, it is sometimes referred to as F-d or FLAG-d) having cDNA sequence (SEQ ID NO: 11) encoding TM4SF17 isoform d protein (SEQ ID NO: 12) which has 1xFLAG at N terminal and termination codon at C terminal was obtained.
Primer 10 ATGTCTAGATCATTCCATTTGCTCGAGGTTCTGGGCCAGGCAGA (SEQ ID NO: 49).

### Example 41

### Enforced expression of recombinant TM4SF17 proteins; a-3 xFLAG, b-3 xFLAG, FLAG-b and FLAG-d in human embryonic kidney cell HEK 293

Enforced expression of recombination protein in human embryonic kidney cell HEK 293 was carried out transiently using animal cell expression vectors p3xFLAG-CMV-14-TM4SF17a (a-3xFLAG), p3xFLAG-CMV-14-TM4SF17b (b-3xFLAG), pcDNA3.1(+)-FLAG-TM4SF17b (FLAG-b) or pcDNA3.1(+)-FLAG-TM4SF17d (FLAG-d) which were designed according to Example 38, 37, 39 and 40. In specific, human embryonic kidney cell HEK 293 purchased from the American Type Culture Collection (ATCC) was suspended in MEM medium (Invitrogen corp.) containing 10% fetal bovine serum (JRH Biosciences), 0.1 mM of MEM non-essential amino acid solution, and 1mM of sodium pyruvate, and then 30,000 cells/well were plated in 6-well culture dish (BD FALCON). Plates were incubated overnight at 37°C. The cell was transfected with an animal cell expression vector.

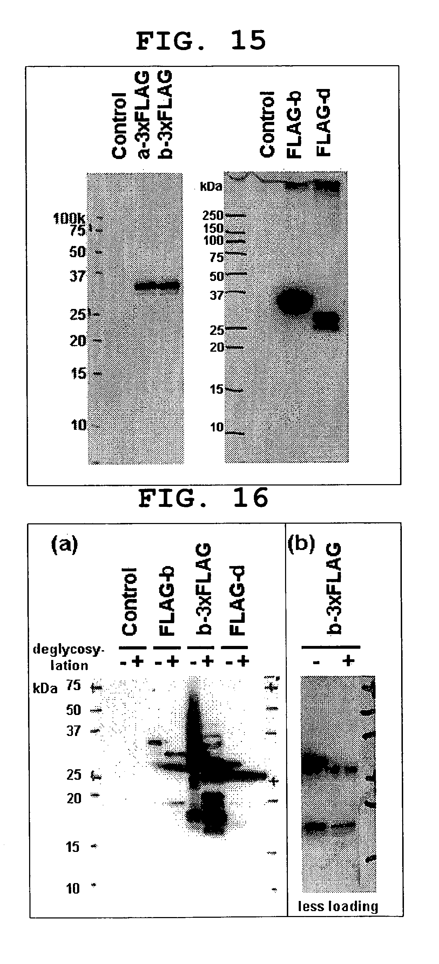

In specific, 1µg of p3xFLAG-CMV-14-TM4SF17a, p3xFLAG-CMV-14-TM4SF17b, pcDNA3.1(+)-FLAG-TM4SF17b or pcDNA3.1(+)-FLAG-TM4SF17d was added to a mixture comprising 3 µL of FuGENE6 (Roche Ltd.) and 97 µL of OPTI-MEM (Invitrogen corp.), and allowed to stand at room temperature for 15 minutes. Whole mixture solution was added to a HEK 293 cell culture solution of each well previously placed with a medium 4 hours before, and then incubated at 37°C for 24 hours, 48 hours or 72 hours. As a control, the reaction without transfecting a vector was carried out in the same manner. After cultivation, medium was removed, and cells were collected using PBS(-) (Invitrogen corp.) which includes 0.5% of EDTA-Na and 0.2% of BSA, washed with PBS (-), and mixed with Laemmli Sample Buffer comprising 5% of 2-mercaptoehanol to give 5,000,000 cells per 1mL. The mixture was heated at 95°C for 5 minutes to prepare a cell lysate. The prepared samples were assayed by Western Blot and detected with anti-FLAG M2-Peroxidase conjugate antibody (SIGMA). As a result, the expression of a-3xFLAG, b-3xFLAG, FLAG-b and FLAG-d protein was detected. The protein of those was detected at a location of slightly lower in molecular weight than expected. In addition, there was a case where many protein bands were observed at a location of much lower in molecular weight. As above, it was shown that the recombinant TM4SF17 protein is expressed in HEK 293 cell with the use of any vectors, and suggested that the recombinant TM4SF17 protein is possibly modified after translation such as protein degradation (Fig. 15 and Table 2).

**(Table 2)**

| Plasmid | Code | amino acid | Estimated molecular weight |
|---|---|---|---|
| Isoform a-3xFLAG (p3XFLAG-CMV-14) | a-3F a-3xFLAG | 360 | 40382.21 |
| Isoform b-3xFLAG (p3XFLAG-CMV-14) | b-3F b-3xFLAG | 357 | 40069.84 |
| 1xFLAG-Isoform b (pcDNA3.1(+)) | F-b FLAG-b | 337 | 37737.45 |
| 1xFLAG-Isoform d (pcDNA3.1(+)) | F-d FLAG-d | 271 | 30564.44 |

### Example 42

### Glycosylation of enforced expression TM4SF17 protein FLAG-b, b-3xFLAG and FLAG-d in human embryonic kidney cell HEK 293

Glycosylation of recombinant TM4SF17 protein was investigated using a cell lysate prepared according to Example 41 which comprises b-3xFLAG, FLAG-b and FLAG-d protein. A control, b-3 xFLAG, FLAG-b and FLAG-d were forcibly expressed, and mixed with Laemmli Sample Buffer comprising 5% of 2-mercaptoehanol to give 5,000,000 cells per 1mL. The mixture was heated at 95°C for 5 minutes to prepare a 20 µL of cell lysate. For deglycosylation, Enzymatic Deglycosylation Kit (PROzyme Inc.) was used. Thereto, 5 µL of 5X incubation buffer (PROzyme Inc.) was added and then heated at 95°C for 10 minutes. To the solution cooled to room temperature, 2.5 µL of 10% Triton-X solution was added, mixed, and thereafter 0.5 µL of N-Glycanase, Sialidase A and O-Glycanase were respectively added. The mixture solution was kept at 37°C for 5 hours. Thereto, 5X Laemmli Sample Buffer was added, heated at 95°C for 10 minutes, and assayed by Western Blot. When the recombinant TM4SF17 protein was detected with anti-FLAG M2-Peroxidase conjugate antibody (SIGMA), maximum molecular weight in each sample around the time of deglycosylation was moved to a lower side of the molecular weight. In this way, it was found that recombinant TM4SF17 protein; b-3 xFLAG, FLAG-b and FLAG-d, were glycosylated (Fig. 16).

### Example 43

### Localization of enforced expression TM4SF17 isoform a and b protein to cell membrane in human embryonic kidney cell HEK 293

A recombinant protein was transiently force expressed in human embryonic kidney cell HEK 293 using animal cell expression vectors p3xFLAG-CMV-14-TM4SF17a and p3xFLAG-CMV-14-TM4SF17b prepared according to Examples 38 and 37, respectively. Specifically, human embryonic kidney cell HEK 293 purchased from the American Type Culture Collection (ATCC) was suspended in a MEM medium (Invitrogen corp.) containing 10% fetal bovine serum (JRH), 0.1 mM of MEM non-essential amino acid solution and 1 mM of sodium pyruvate, and then 670,000 cells/dish were plated in 6cm Organ Culture Dish (BD FALCON). The culture was incubated overnight at 37 °C, and then transfected with an animal cell expression vector.

In specific, 2µg of p3xFLAG-CMV-14-TM4SF17a or p3xFLAG-CMV-14-TM4SF17b was added to a mixture comprising 6 µL of FuGENE6 (Roche Ltd.) and 194 µL of OPTI-MEM (Invitrogen corp.), and allowed to stand at room temperature for 15 minutes. Whole mixture solution was added to a HEK 293 cell culture solution previously placed with a medium 4 hours before, and then incubated at 37°C for 48 hours. As a control, the reaction without transfecting a vector was carried out in the same manner. After medium was removed, cells were collected by pipetting in PBS (-) (Invitrogen corp.), washed with PBS (-), and suspended in 500 µL of PBS (-) . For biotin labeling, Cellular Labelling and Immunoprecipitation Kit (Roche Ltd.) was used. To 500 µL of cell suspension solution, biotin-7-NHS (Roche Ltd.) was added to give a final concentration of 50 µg/mL, and then allowed to stand at room temperature for 15 minutes. Thereafter, 25 µL of stop solution (Roche Ltd.) was added thereto, and kept at 4°C for 15 minutes. After washing the cells with PBS (-), the cells were dissolved in 500 µL of RIPA buffer (1% Triton-X100, 0.25% deoxycholic acid, 150mM sodium chloride, 50mM Tris-HCl (pH 7.5)), and centrifuged supernatant was obtained as a biotin labeled lysate. 100 µL thereof was mixed with 100 µL of Laemmli sample buffer (Bio-Rad) containing 4% 2-Mercaptoethanol (WAKO), and then heated at 95°C for 5 minutes to obtain a SDS-PAGE sample. The remaining 400 µL was mixed with 100 µL slurry of anti-FLAG M2-agarose (Sigma) equilibrated in RIPA buffer to which a M2-anti-FLAG antibody (Sigma) is conjugated, and mixed overnight at 4°C. Beads were washed with RIPA buffer, 100 µL of 2X Laemmli Sample Buffer (Bio-Rad) containing 2% 2-Mercaptoethanol (WAKO) was added, and heated at 95°C for 5 minutes. After centrifugation, supernatant was obtained as a sample for immunoprecipitation. The biotin labled lysate and immunoprecipitation sample were assayed by Western blot, and detected with anti-FLAG M2-Peroxidase conjugate antibody (SIGMA) and Streptavidin-horseradish peroxidase conjugate (Amersham). AS a result, the expression of protein TM4SF17 isoform a and b was detected in the anti-FLAG M2 antibody. Further, since a streptavidin band was detected at the same location of molecular weight from the immunoprecipitation sample, it was shown that TM4SF17 isoform a and b protein is localized on a cell membrane (Fig. 17).

### Example 44

### Idendification of TM4SF17 isoform e and isoform f

TM4SF17 isoform b was cloned according to Example 37, and at the same time, novel isoform e and f were identified. PCR reaction using primer 1 (SEQ ID NO: 40) and primer 2 (SEQ ID NO: 41) and PCR reaction using primer 3 (SEQ ID NO: 42) and primer 4 (SEQ ID NO: 43) were carried out by using cDNA derived from human lung cancer cell line A549 as a DNA template. For the components of reaction solution for the reaction, about 50 ng of the cDNA as a template, 0.2 µL of TAKARA LA taq polymerase (TaKaRa Bio), 500nM of primer 1 (SEQ ID NO: 40) and primer 2 (SEQ ID NO: 41), or primer 3 (SEQ ID NO: 42) and primer 4 (SEQ ID NO: 43), respectively, 400 µM of dNTPs, and 10 µL of 2X GC Buffer (TaKaRa Bio) were added to prepare a solution of 20 µL in total volume. PCR was carried out by reacting at 94°C for 1 minute and then repeating 40 times the cycle set to include 94°C for 30 seconds, 60°C for 30 seconds and 72°C for 2 minutes. Thus-obtained PCR products were separated with agarose gel electrophoresis, and DNA fragments that correspond to the length expected for each PCR reaction were confirmed, but fragments longer than as expected were also observed. 15 µL of the reaction liquid from the PCR product was purified using MinElute PCR purification kit (Qiagen), and 8 µL of new product was obtained. From this product, 4 µL was inserted into pCR4-TOPO vector using TOPO TA Cloning Kit for Sequencing (Invitrogen corp.), introduced to E.Coli One Shot TOP10 (Invitrogen corp.), and selected in the Ampicillin-containing LB agar medium. As a result of analyzing the sequences of each clone, from the PCR reaction using primer 1 (SEQ ID NO: 40) and primer 2 (SEQ ID NO: 41), a clone having a 726bp-length fragment 1 from ATG of cDNA sequence (SEQ ID NO: 7) which encodes protein TM4SF17 isoform b (SEQ ID NO:8); and a clone having a 735bp-length fragment 5 from ATG of cDNA sequence (SEQ ID NO: 5) which encodes protein TM4SF17 isoform a (SEQ ID NO:6) were obtained. From the PCR reaction using primer 3 (SEQ ID NO: 42) and primer 4 (SEQ ID NO: 43), in addition to a clone having a fragment 2 starting from 291bp to just before the termination codon, 987bp of cDNA sequence (SEQ ID NO: 7) which encodes the protein TM4SF17 isoform b (SEQ ID NO: 8), a clone having a novel fragment 6 which is the partial sequence of TM4SF17 isoform, not known till now, was also observed. This is caused by shortening a peptide than that of the TM4SF17 isoform a protein (SEQ ID NO: 6) by a termination codon presenting at the head of the intron sequence, since in the sequence of TM4SF17 isoform a protein (SEQ ID NO: 6), a sequence corresponding to intron followed by exon 8 is transcribed into mRNA. TM4SF17 isoform e was termed considering that there is an isoform comprising mRNA in which fragment 5 and fragment 6 are continuously connected. 273 amino acid sequence (SEQ ID NO: 14) of TM4SF17 isoform e protein and cDNA sequence (SEQ ID NO: 13) coding thereof were indicated. Also, TM4SF17 isoform f was termed considering that there is an isoform comprising mRNA in which fragment 1 and fragment 6 are continuously connected. 270 amino acid sequence (SEQ ID NO: 16) of TM4SF17 isoform f protein and cDNA sequence (SEQ ID NO: 15) coding thereof were indicated. In addition, a view showing a frame format of comparing other splicing variant was shown in Fig. 18.

### Example 45

### Idendification of TM4SF17 isoform h

Human Marathon Ready cDNA (Clonetech) derived from human colon cancer cell line SW480 as a template, PCR was carried out using primer 11 (SEQ ID NO: 50), primer 12 (SEQ ID NO: 51). For the components of reaction solution for the reaction, about 50 ng of the cDNA as a template, 0.4 µL of Pfu Ultra High-Fidelith DNA polymerase (Stratagene), 500nM of primer 11 (SEQ ID No: 50) and primer 12 (SEQ ID NO: 51), 400 µM of dNTPs, and 2 µL of 10X Pfu Ultra High-Fidelith DNA Polymerase Reaction Buffer (Stratagene) were added to prepare a solution with 20 µL in volume. PCR was carried out by reacting at 95°C for 2 minute and then repeating 30 times the cycle set to include 95°C for 30 seconds, 60°C for 30 seconds and 72°C for 3 minutes. Thus-obtained PCR products were separated with agarose gel electrophoresis, and the fragment was purified using MinElute PCR purification kit (Qiagen). The fragment was treated with restriction enzyme such as EcoR I and Xba I, and then purified using MinElute PCR purification kit (Qiagen). The purified product was inserted into a pCR-Blunt II-TOPO vector using TOPO TA Cloning Kit for Sequencing (Invitrogen corp.), and then introduced into E.Coli One Shot TOP10 (Invitrogen corp.). Transformed E. Coli cell was selected in a Kanamycin-containing LB agar medium. As a result of analyzing the sequence of each clone, a clone having a novel sequence of TM4SF17 isoform, not known till now, was discovered. It is the sequence in which a part corresponding to exon 8 is deleted from the sequence of TM4SF17 isoform b protein (SEQ ID NO: 8). This was termed as TM4SF17 isoform h. 308 amino acid sequence (SEQ ID NO: 18) of TM4SF17 isoform h protein and cDNA sequence (SEQ ID NO: 17) coding thereof were indicated. In addition, a view showing a frame format of comparing other splicing variant was shown in Fig. 18.
Primer 11: GGCGAATTCCCGCTCACCATGCCCGGCAAGCACCA (SEQ ID NO: 50)
Primer 12: ATGTCTAGACTACCAATTTTTAAAGGTTGGCTC (SEQ ID NO: 51)

### INDUSTRIAL APPLICABILITY

The proteins used in the present invention or a polynucleotide encoding the proteins are specifically expressed in cancer tissue, thus can be a diagnosis marker for cancer. Also, by suppressing the activity and/or expression of the protein, promoting effect on apoptosis in cancer cells and inhibiting effect on the growth of cancer cells are obtained, thus an antibodies against the proteins, antisense polynucleotides against the polynucleotides,compounds that inhibit activities of the proteins or salt thereof, and a compounds that inhibit expression of genes of the proteins or salt thereof, can be safely used as preventive/remedy agents for cancer, agents for promoting apoptosis in cancer cells, and agents for inhibiting the growth of cancer cells. Further, the proteins, the polynucleotides, the antibodies, and the like, are useful in screening for a preventive/remedy substance for cancer, a substance that promotes apoptosis in cancer cells, and/or a substance that inhibits the growth of cancer cells.

This application is based on a patent application No. 2005-230797 filed in Japan (filing date: August 9, 2005), the contents of which are incorporated in full herein by this reference.

## Claims

1. An agent for promoting the apoptosis in cancer cells and/or inhibiting the growth of cancer cells, which comprises an antibody against a protein comprising the same or substantially the same amino acid sequence as the amino acid sequence represented by SEQ ID NO: 2, or against partial peptide thereof.

2. The agent according to Claim 1, which is for preventive/remedy for cancer.

3. An agent for promoting the apoptosis in cancer cells and/or inhibiting the growth of cancer cells, which comprises an antisense polynucleotide comprising a base sequence or a part of the base sequence, wherein the base sequence is complimentary or substantially complimentary to a base sequence of polynucleotide encoding a protein which includes the same or substantially the same amino acid sequence as the amino acid sequence represented by SEQ ID NO: 2.

4. The agent according to Claim 3, which is for preventive/remedy for cancer.

5. An agent for promoting the apoptosis in cancer cells and/or inhibiting the growth of cancer cells, which comprises a substance that inhibits the expression and/or activity of a protein comprising the same or substantially the same amino acid sequence as the amino acid sequence represented by SEQ ID NO: 2.

6. The agent according to Claim 5, which is for preventive/remedy for cancer.

7. A method of promoting the apoptosis in cancer cells and/or inhibiting the growth of cancer cells, the method comprises inhibiting the expression and/or activity of a protein comprising the same or substantially the same amino acid sequence as the amino acid sequence represented by SEQ ID NO: 2.

8. The method according to Claim 7, which is for preventive/remedy for cancer.

9. Use of a substance that inhibits the expression and/or activity of a protein comprising the same or substantially the same amino acid sequence as the amino acid sequence represented by SEQ ID NO: 2, to manufacture an agent for promoting the apoptosis in cancer cells and/or inhibiting the growth of cancer cells.

10. The use according to Claim 9, wherein the agent for promoting the apoptosis in cancer cells and/or inhibiting the growth of cancer cells is for preventive/remedy for cancer.

11. A method of screening for a substance that promotes the apoptosis in cancer cells and/or inhibits the growth of cancer cells, the method includes using a protein comprising the same or substantially the same amino acid sequence as the amino acid sequence represented by SEQ ID NO: 2, or partial peptide thereof.

12. The method according to Claim 11, wherein the protein comprising the same or substantially the same amino acid sequence as the amino acid sequence represented by SEQ ID NO: 2, or partial peptide thereof, is provided in the form of a cell capable of producing the protein or the partial peptide.

13. The method according to Claim 12, comprises using further any one selected from a group consisting of antibodies against a protein comprising the same or substantially the same amino acid sequence as the amino acid sequence represented by SEQ ID NO: 2, or against partial peptide thereof, and polynucleotide encoding the protein or polynucleotide including a part of the base sequence.

14. The method according to Claim 11, which is for the screening for a preventive/remedy substance for cancer.

15. An agent for promoting the apoptosis in cancer cells and/or inhibiting the growth of cancer cells, which comprises an antibody against a protein comprising the same or substantially the same amino acid sequence as the amino acid sequence represented by SEQ ID NO: 6, SEQ ID NO: 8, SEQ ID NO: 10, SEQ ID NO: 12, SEQ ID NO: 14, SEQ ID NO: 16, or SEQ ID NO: 18, or against partial peptide thereof.

16. The agent according to Claim 15, which is for preventive/remedy for cancer.

17. An agent for promoting the apoptosis in cancer cells and/or inhibiting the growth of cancer cells, which comprises an antisense polynucleotide comprising a base sequence or a part of the base sequence, wherein the base sequence is complimentary or substantially complimentary to a base sequence of polynucleotide encoding a protein which includes the same or substantially the same amino acid sequence as the amino acid sequence represented by SEQ ID NO: 6, SEQ ID NO: 8, SEQ ID NO: 10, SEQ ID NO: 12, SEQ ID NO: 14, SEQ ID NO: 16, or SEQ ID NO: 18.

18. The agent according to Claim 17, which is for preventive/remedy for cancer.

19. An agent for promoting the apoptosis in cancer cells and/or inhibiting the growth of cancer cells, which comprises a substance that inhibits the expression and/or activity of a protein comprising the same or substantially the same amino acid sequence as the amino acid sequence represented by SEQ ID NO: 6, SEQ ID NO: 8, SEQ ID NO: 10, SEQ ID NO: 12, SEQ ID NO: 14, SEQ ID NO: 16, or SEQ ID NO: 18.

20. The agent according to Claim 19, which is for preventive/remedy for cancer.

21. A method of promoting the apoptosis in cancer cells and/or inhibiting the growth of cancer cells, the method comprises inhibiting the expression and/or activity of a protein comprising the same or substantially the same amino acid sequence as the amino acid sequence represented by SEQ ID NO: 6, SEQ ID NO: 8, SEQ ID NO: 10, SEQ ID NO: 12, SEQ ID NO: 14, SEQ ID NO: 16, or SEQ ID NO: 18.

22. The method according to Claim 21, which is for preventive/remedy for cancer.

23. Use of a substance that inhibits the expression and/or activity of a protein comprising the same or substantially the same amino acid sequence as the amino acid sequence represented by SEQ ID NO: 6, SEQ ID NO: 8, SEQ ID NO: 10, SEQ ID NO: 12, SEQ ID NO: 14, SEQ ID NO: 16, or SEQ ID NO: 18, to manufacture an agent for promoting the apoptosis in cancer cells and/or inhibiting the growth of cancer cells.

24. The use according to Claim 23, wherein the agent for promoting the apoptosis in cancer cells and/or inhibiting the growth of cancer cells is for preventive/remedy for cancer.

25. A method of screening for a substance that promotes the apoptosis in cancer cells and/or inhibits the growth of cancer cells, the method comprises using a protein comprising the same or substantially the same amino acid sequence as the amino acid sequence represented by SEQ ID NO: 6, SEQ ID NO: 8, SEQ ID NO: 10, SEQ ID NO: 12, SEQ ID NO: 14, SEQ ID NO: 16, or SEQ ID NO: 18, or partial peptide thereof.

26. A method according to Claim 25, wherein the protein comprising the same or substantially the same amino acid sequence as the amino acid sequence represented by SEQ ID NO: 6, SEQ ID NO: 8, SEQ ID NO: 10, SEQ ID NO: 12, SEQ ID NO: 14, SEQ ID NO: 16, or SEQ ID NO: 18, or partial peptide thereof, is provided in the form of a cell capable of producing the protein or the partial peptide.

27. The method according to Claim 26, comprises using further any one selected from a group consisting of antibodies against a protein comprising the same or substantially the same amino acid sequence as the amino acid sequence represented by SEQ ID NO: 6, SEQ ID NO: 8, SEQ ID NO: 10, SEQ ID NO: 12, SEQ ID NO: 14, SEQ ID NO: 16, or SEQ ID NO: 18, or against partial peptide thereof, and polynucleotide encoding the protein or polynucleotide including a part of the base sequence.

28. The method according to Claim 25, which is for the screening for a preventive/remedy substance for cancer.

29. An agent for promoting the apoptosis in cancer cells and/or inhibiting the growth of cancer cells, which comprises an antibody against a protein comprising the same or substantially the same amino acid sequence as the amino acid sequence represented by SEQ ID NO: 4, or against partial peptide thereof.

30. The agent according to Claim 29, which is for preventive/remedy for cancer.

31. An agent for promoting the apoptosis in cancer cells and/or inhibiting the growth of cancer cells, which comprises an antisense polynucleotide comprising a base sequence or a part of the base sequence, wherein the base sequence is complimentary or substantially complimentary to a base sequence of polynucleotide encoding a protein which includes the same or substantially the same amino acid sequence as the amino acid sequence represented by SEQ ID NO: 4.

32. The agent according to Claim 31, which is for preventive/remedy for cancer.

33. An agent for promoting the apoptosis in cancer cells and/or inhibiting the growth of cancer cells, which comprises a substance that inhibits the expression and/or activity of a protein comprising the same or substantially the same amino acid sequence as the amino acid sequence represented by SEQ ID NO: 4.

34. The agent according to Claim 33, which is for preventive/remedy for cancer.

35. A method of promoting the apoptosis in cancer cells and/or inhibiting the growth of cancer cells, the method comprises inhibiting the expression and/or activity of a protein comprising the same or substantially the same amino acid sequence as the amino acid sequence represented by SEQ ID NO: 4.

36. The method according to Claim 35, which is for preventive/remedy for cancer.

37. Use of a substance that inhibits the expression and/or activity of a protein comprising the same or substantially the same amino acid sequence as the amino acid sequence represented by SEQ ID NO: 4, to manufacture an agent for promoting the apoptosis in cancer cells and/or inhibiting the growth of cancer cells.

38. The use according to Claim 37, wherein the agent for promoting the apoptosis in cancer cells and/or inhibiting the growth of cancer cells is for a preventive/remedy for cancer.

39. A method of screening for a substance that promotes the apoptosis in cancer cells and/or inhibits the growth of cancer cells, the method comprises using a protein comprising the same or substantially the same amino acid sequence as the amino acid sequence represented by SEQ ID NO: 4, or partial peptide thereof.

40. The method according to Claim 39, wherein the protein comprising the same or substantially the same amino acid sequence as the amino acid sequence represented by SEQ ID NO: 4, or partial peptide thereof, is provided in the form of a cell capable of producing the protein or the partial peptide.

41. The method according to Claim 40, comprises using further any one selected from a group consisting of antibodies against a protein comprising the same or substantially the same amino acid sequence as the amino acid sequence represented by SEQ ID NO: 4, or against partial peptide thereof, and polynucleotide encoding the protein or polynucleotide including a part of the base sequence.

42. The method according to Claim 39, which is for the screening for a preventive/remedy substance for cancer.
